(19) Europäisches Patentamt
European Patent Office
Office européen des brevets

(11) EP 1 227 090 A1

(12) EUROPEAN PATENT APPLICATION
published in accordance with Art. 158(3) EPC

(43) Date of publication:
31.07.2002 Bulletin 2002/31

(21) Application number: 00964676.1

(22) Date of filing: 05.10.2000

(51) Int Cl.[7]: C07D 401/14, C07D 405/14, C07D 409/14, A61K 31/4709, A61K 31/496, A61K 31/497, A61P 43/00, A61P 1/00, A61P 3/10, A61P 13/12, A61P 35/00

(86) International application number:
PCT/JP00/06937

(87) International publication number:
WO 01/25228 (12.04.2001 Gazette 2001/15)

(84) Designated Contracting States:
AT BE CH CY DE DK ES FI FR GB GR IE IT LI LU MC NL PT SE
Designated Extension States:
AL LT LV MK RO SI

(30) Priority: 07.10.1999 JP 28693999
11.07.2000 JP 2000215837

(71) Applicant: Tadeka Chemical Industries, Ltd.
Osaka-shi Osaka 541-0045 (JP)

(72) Inventors:
- KATO, Kaneyoshi
Kawanishi-shi Hyogo 666-0152 (JP)
- TERAUCHI, Jun
Ikeda-shi Osaka 563-0024 (JP)
- SUZUKI, Nobuhiro
Hyogo 663-8031 (JP)
- TAKEKAWA, Shiro
Hyogo 662-0976 (JP)

(74) Representative: Lewin, John Harvey
Takeda Patent Office,
11-12 Charles II Street
London SW1Y 4QU (GB)

(54) **AMINE DERIVATIVES**

(57) A compound of the formula:

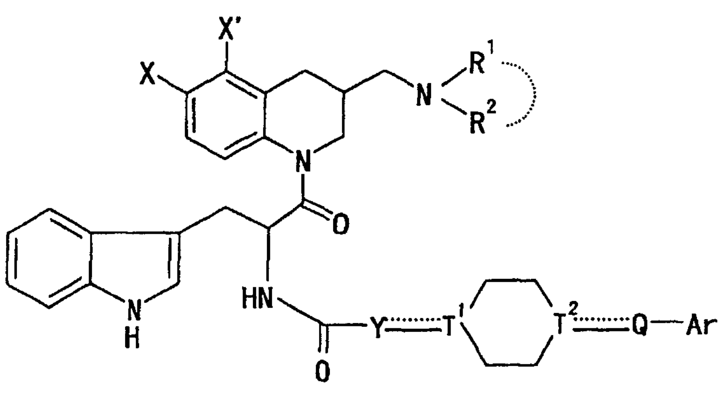

(I)

wherein

X and X' are the same or different, and each represents a hydrogen atom, a fluorine atom, a chlorine atom or an amino optionally having substituents, and at least one of X and X' represents a fluorine atom, a chlorine atom or an amino optionally having substituents;

EP 1 227 090 A1

$R^1$ and $R^2$ represent a hydrogen atom or $C_{1-6}$ alkyl optionally having substituents, or $R^1$ and $R^2$, together with the adjacent nitrogen atom, form a nitrogen-containing heterocyclic ring optionally having substituents;
Y and Q are the same or different, and each represents a bond or a spacer having a main chain of 1 to 6 atoms;
... represents a single bond or a double bond;
$T^1$ and $T^2$ are the same or different, and each represents $C(R^9)$ ($R^9$ represents a hydrogen atom, a hydroxy or $C_{1-6}$ alkyl) or N, when each of the adjacent ... is a single bond, and C when the adjacent ... is a double bond; and
Ar represents an aromatic group optionally having substituents, a $C_{3-9}$ cycloalkyl group optionally having substituents, a 3 to 9-membered saturated heterocyclic group optionally having substituents, a hydrogen atom or a halogen atom; provided that 6-chloro-3-(R,S)-(N,N-dimethylamino)methyl-1-[3-(indol-3-yl)-2-[(R)-(4-phenylpiperazin-1-yl)carbonylamino]propanoyl]-1,2,3,4-tetrahydroquinoline; 6-chloro-3-(R,S)-(N,N-dimethylamino)methyl-1-[3-(indol-3-yl)-2-[(R)-4-(2-oxo-2,3-dihydro-1H-benzimidazol-1-yl)piperidinocarbonylamino]propanoyl]-1,2,3,4-tetrahydroquinoline and 1-benzoyl-N-[(R)-2-[6-chloro-3-[(N,N-dimethylamino)methyl]-1,2,3,4-tetrahydroquinolin-1-yl]-1-[3-(indol-3-yl)propanoyl]-4-piperidinecarboxamide are excluded; a salt thereof or a prodrug thereof has an excellent somatostatin receptor binding inhibition action and is useful for preventing and/or treating diseases associated with somatostatin.

## Description

### TECHNICAL FIELD

The present invention relates to novel amine derivatives. In further detail, the present invention relates to a compound which has a somatostatin receptor binding inhibition activity, and is useful for preventing and/or treating diseases associated with somatostatin.

### BACKGROUND ART

Somatostatin was found to be a growth hormone inhibiting factor (somatotropin release inhibiting factor; SRIF) in 1973.

Somatostatin receptors were found to comprise five subtypes that have been named as SSTR1, SSTR2, SSTR3, SSTR4 and SSTR5 respectively (e.g., Endocrinology, vol.136, pp.3695-3697, 1995; Trends in Pharmacological Sciences, pp.87-94, Vol.18, 1997; Life Science, Vol.57, pp.1249-1265, 1995).

Somatostatin is known to inhibit production and/or secretion of various hormones, growth factors, and physiologically active substances. As the hormones inhibited by somatostatin, mentioned are growth hormone (GH), thyroid-stimulating hormones (TSH), prolactin, insulin, and glucagon. Therefore, somatostatin has various functions in endocrine systems, exocrine systems and nerve systems, and drugs targeting somatostatin are being developed (e.g., Endocrinology, vol.136, p.3695-3697, 1995; Trends in Pharmacological Sciences, pp.87-94, vol.18, 1997).

Diseases caused by somatostatin include life-style related diseases such as diabetes; central nervous system diseases, immune system diseases, and hormone-dependent tumors. Trials to develop somatostatin itself or somatostatin analogues as a drug have been conducted. For instance, octreotide known as a somatostatin receptor agonist has been marketed as a drug for treating hormone-dependent tumors.

As a compound having a somatostatin receptor binding activity, especially a selective SSTR1 antagonist activity, there is known a compound represented by the formula:

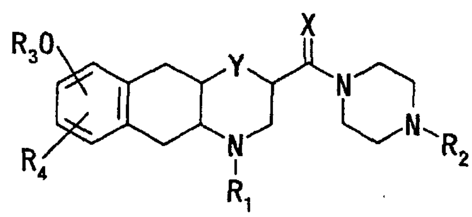

wherein X represents O or H, H; Y represents $-CH_2-$, -O-, -NH- or -S-; $R_1$ represents H or $C_{1-4}$ alkyl; $R_2$ represents H, benzyl, etc.; $R_3$ represents H, $C_{1-4}$ alkyl, etc.; and $R_4$ represents hydrogen atom or halogen (WO97/03054).

As a compound which has a selective SSTR4 binding activity and is expected to have a glaucoma treating activity, there is known a compound represented by the formula:

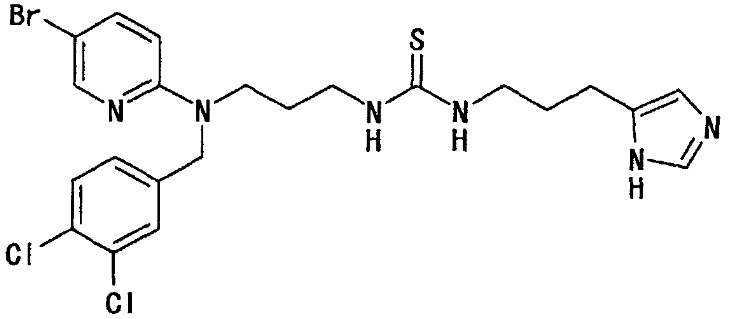

(J. Am. Chem. Soc., vol.120, pp.1368-1373, 1998; WO97/43278).

As a compound having a somatostatin receptor binding activity, especially a selective SSTR2 agonist activity, a compound represented by the formula:

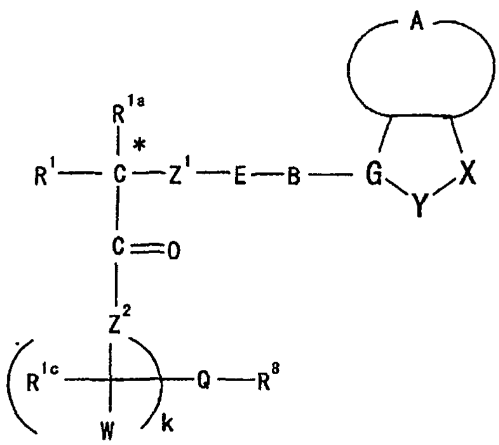

wherein $R^1$ represents $C_{1-10}$alkyl, etc.; $R^{1a}$ represents H, etc.; $Z^1$ represents -O-, etc.; E represents $-SO_2-$, etc.; B represents

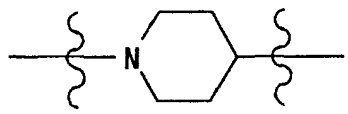

etc.;

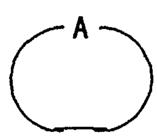

represents 5- or 6-membered aromatic or non-aromatic ring; G represents N, CH or C; Y represents -C(O)-, etc.; X represents $-N(R^{11})-$ ($R^{11}$ represents H, etc.), etc.;

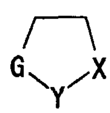

represents 5- to 10-membered condensed aryl, etc.; $Z^2$ represents -O-, etc.; $R^{1c}$ represents H, etc.; W represents H, etc.; k represents 0 or 1; Q represents $-(CH_2)_x-V-(CH_2)_y-$ (x and y each represent 0, 1, 2, 3, 4, 5 or 6; V represents 6- to 12-membered aromatic monocyclic or bi-cyclic ring), etc.; and $R^8$ represents H, etc. (WO98/44921); and a compound represented by the formula:

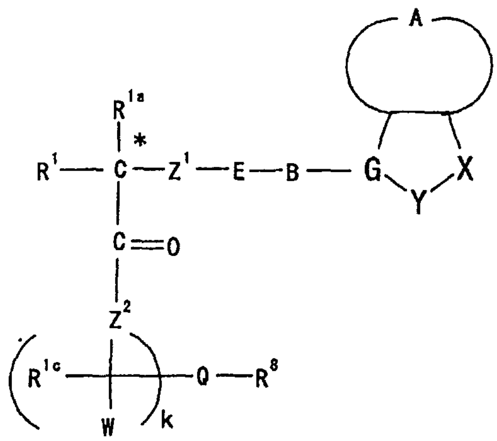

wherein $R^1$ represents $C_{1-10}$ alkyl, etc.; $R^{1a}$ represents H, etc.; $Z^1$ represents -O-, etc., E represents $-SO_2-$, etc.; B represents

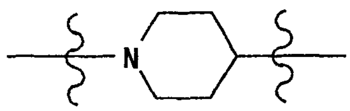

etc.,

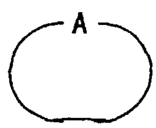

represents 5- or 6-membered aromatic or non-aromatic ring; G represents N, CH or C; Y represents -C(O)-, etc.; X represents $-N(R^{11})-$ ($R^{11}$ represents H, etc.), etc.;

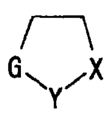

represents 5- to 10-membered condensed aryl, etc.; $Z^2$ represents -O-, etc.; $R^{1c}$ represents H, etc.; W represents H, etc.; k represents 0 or 1; Q represents $-(CH_2)_x-V-(CH_2)_y-$ (x and y each represent 0, 1, 2, 3, 4, 5 or 6; V represents $C_{3-10}$ saturated or partially saturated aromatic monocyclic or bi-cyclic ring containing 1 to 4 nitrogen atoms and 0 to 2 oxygen atoms or sulfur atoms), etc.; and $R^8$ represents H, etc.; (WO98/45285).

On the other hand, the following compounds are known as amine derivatives.

1) J. Med. Chem., vol.34, pp.2624-2633, 1991 describes, as a compound having a weak analgesic activity, a compound represented by the following formula:

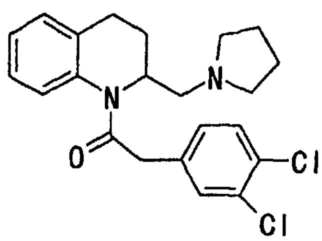

2) JP-A-8-176087 describes 3-(N,N-dimethylaminomethyl)-1,2,3,4-tetrahydroquinoline as a synthetic intermediate for a compound represented by the formula:

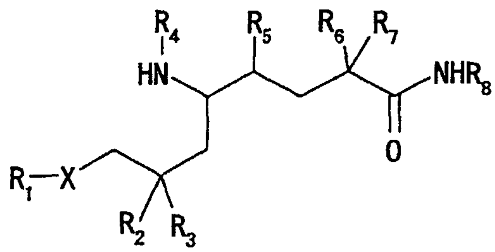

wherein $R_1$ represents arylamino such as

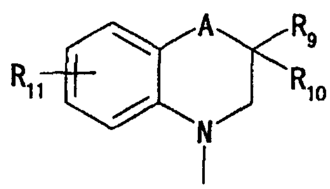

(A represents a direct bond, methylene, ethylene, imino, oxy or thio; $R_9$ represents $C_{1-4}$ alkoxycarbonylamino-$C_{1-4}$ alkyl, etc.; $R_{10}$ represents hydrogen or $C_{1-4}$ alkyl; $R_{11}$ represents hydrogen or halogen; etc.); X represents carbonyl, etc.; $R_2$ and $R_3$ represent hydrogen, etc.; $R_5$ represents hydroxyl, etc.; $R_6$ represents hydrogen, etc.; $R_7$ represents hydrogen, etc.; $R_8$ represents aliphatic group, etc., which is described to be useful in the treatment of hypertension.

3) WO97/12860 describes, as a compound having an acyl-coenzyme A: cholesterol acyltransferase inhibiting activity and a lipid peroxidation inhibiting activity, a heterocyclic derivative represented by the formula:

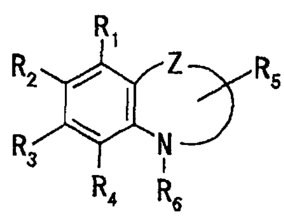

wherein at least one of $R_1$, $R_2$ and $R_5$ represents alkyl or alkenyl which is substituted by hydroxy, an acidic group, alkoxycarbonyl or $-NR_9R_{10}$ ($R_9$ and $R_{10}$ each represent hydrogen atom or lower alkyl), etc., and the remaining two groups independently represent hydrogen atom, lower alkyl or lower alkoxy; either $R_2$ or $R_5$ represents a group represented by the formula: $-NHCOR_7$ wherein $R_7$ represents alkyl, etc., and the remaining group represents hydrogen atom, lower alkyl or lower alkoxy; $R_6$ represents alkyl, alkenyl, alkoxyalkyl, alkylthioalkyl, cycloalkyl, cycloalkylalkyl or arylalkyl; Z represents nitrogen atom substituted by $R_6$, or a linker group forming 5-membered ring or 6-membered ring together with a carbon atom of benzene ring attached to the nitrogen atom and a carbon atom adjacent to the

carbon atom, or a pharmaceutically acceptable salt thereof.

Conventional somatostatin and its analogues are all peptides. They are problematic in their oral absorbability, pharmacokinetics, etc. and are therefore unsatisfactory as medicines. It is desired to develop a compound which is different from conventional known compounds in its chemical structure, and which has a selective or nonselective affinity to somatostatin receptor subtypes, or an excellent somatostatin receptor binding inhibitory activity, etc., and which has satisfactory properties as a medicine.

## DISCLOSURE OF INVENTION

The present inventors have studied various compounds having a somatostatin receptor binding inhibitory activity, and, as a result, have found, for the first time, that a compound of the formula:

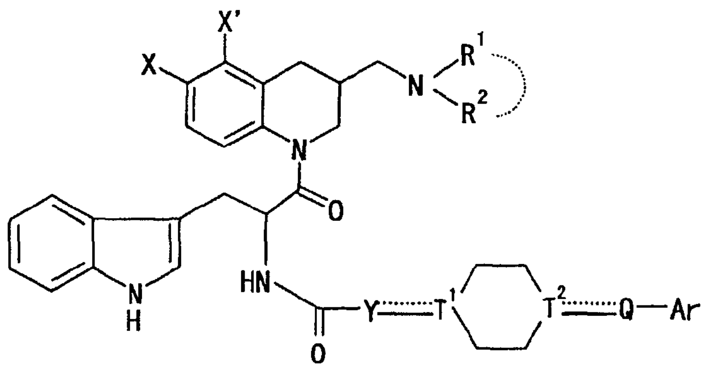

(I)

wherein X and X' are the same or different, and each represents a hydrogen atom, a fluorine atom, a chlorine atom or an amino optionally having substituents, and at least one of X and X' represents a fluorine atom, a chlorine atom or an amino optionally having substituents;

$R^1$ and $R^2$ represent a hydrogen atom or $C_{1-6}$ alkyl optionally having substituents, or $R^1$ and $R^2$, together with the adjacent nitrogen atom, form a nitrogen-containing heterocyclic ring optionally having substituents;
Y and Q are the same or different, and each represents a bond or a spacer having a main chain of 1 to 6 atoms;
... represents a single bond or a double bond;
$T^1$ and $T^2$ are the same or different, and each represents $C(R^9)$ ($R^9$ represents a hydrogen atom, a hydroxy or $C_{1-6}$ alkyl) or N, when each of the adjacent ... is a single bond, and C when the adjacent ... is a double bond; and Ar represents an aromatic group optionally having substituents, a $C_{3-9}$ cycloalkyl group optionally having substituents, a 3 to 9-membered saturated heterocyclic group optionally having substituents, a hydrogen atom or a halogen atom; provided that 6-chloro-3-(R,S)-(N,N-dimethylamino)methyl-1-[3-(indol-3-yl)-2-[(R)-(4-phenylpiperazin-1-yl)carbonylamino]propanoyl]-1,2,3,4-tetrahydroquinoline; 6-chloro-3-(R,S)-(N,N-dimethylamino)methyl-1-[3-(indol-3-yl)-2-[(R)-4-(2-oxo-2,3-dihydro-1H-benzimidazol-1-yl)piperidinocarbonylamino]propanoyl]-1,2,3,4-tetrahydroquinoline and 1-benzoyl-N-[(R)-2-[6-chloro-3-[(N,N-dimethylamino)methyl]-1,2,3,4-tetrahydroquinolin-1-yl]-1-[3-(indol-3-yl)propanoyl]-4-piperidinecarboxamide are excluded; or a salt thereof [hereinafter sometimes referred to as compound (I)] has, based on its characteristic structure, an unexpectedly excellent somatostatin receptor binding inhibitory activity, and that these compounds have low toxicity, etc and are therefore satisfactory as medicines. Based on these findings, the inventors have completed the present invention.

Specifically, the present invention relates to:

[1] a compound of the formula:

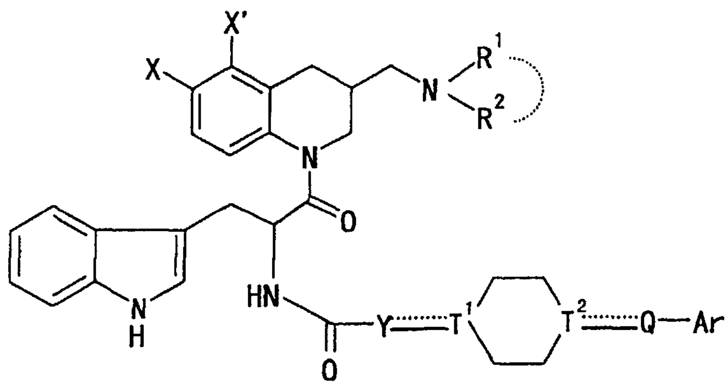

(I)

wherein X and X' are the same or different, and each represents a hydrogen atom, a fluorine atom, a chlorine atom or an amino optionally having substituents, and at least one of X and X' represents a fluorine atom, a chlorine atom or an amino optionally having substituents;

$R^1$ and $R^2$ represent a hydrogen atom or $C_{1-6}$ alkyl optionally having substituents, or $R^1$ and $R^2$, together with the adjacent nitrogen atom, form a nitrogen-containing heterocyclic ring optionally having substituents;
Y and Q are the same or different, and each represents a bond or a spacer having a main chain of 1 to 6 atoms;
... represents a single bond or a double bond;
$T^1$ and $T^2$ are the same or different, and each represents $C(R^9)$ ($R^9$ represents a hydrogen atom, a hydroxy or $C_{1-6}$ alkyl) or N, when each of the adjacent ... is a single bond, and C when the adjacent ... is a double bond; and
Ar represents an aromatic group optionally having substituents, a $C_{3-9}$ cycloalkyl group optionally having substituents, a 3 to 9-membered saturated heterocyclic group optionally having substituents, a hydrogen atom or a halogen atom; provided that 6-chloro-3-(R,S)-(N,N-dimethylamino)methyl-1-[3-(indol-3-yl)-2-[(R)-(4-phenylpiperazin-1-yl)carbonylamino]propanoyl]-1,2,3,4-tetrahydroquinoline; 6-chloro-3-(R,S)-(N,N-dimethylamino)methyl-1-[3-(indol-3-yl)-2-[(R)-4-(2-oxo-2,3-dihydro-lH-benzimidazol-1-yl)piperidinocarbonylamino]propanoyl]-1,2,3,4-tetrahydroquinoline and 1-benzoyl-N-[(R)-2-[6-chloro-3-[(N,N-dimethylamino)methyl]-1,2,3,4-tetrahydroquinolin-1-yl]-1-[3-(indol-3-yl)propanoyl]-4-piperidinecarboxamide are excluded; or a salt thereof;

[2] a compound of the formula:

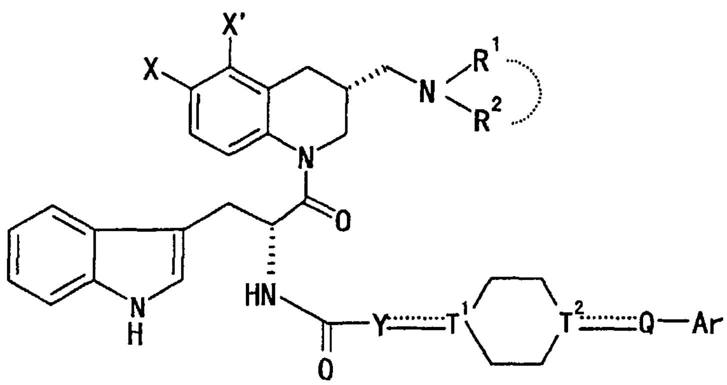

(I')

wherein X and X' are the same or different, and each represents a hydrogen atom, a fluorine atom, a chlorine atom or an amino optionally having substituents, and at least one of X and X' represents a fluorine atom, a chlorine atom or an amino optionally having substituents;

$R^1$ and $R^2$ represent a hydrogen atom or $C_{1-6}$ alkyl optionally having substituents, or $R^1$ and $R^2$, together with the adjacent nitrogen atom, form a nitrogen-containing heterocyclic ring optionally having substituents;
Y and Q are the same or different, and each represents a bond or a spacer having a main chain of 1 to 6 atoms;
... represents a single bond or a double bond;

$T^1$ and $T^2$ are the same or different, and each represents $C(R^9)$ ($R^9$ represents a hydrogen atom, a hydroxy or $C_{1-6}$ alkyl) or N, when each of the adjacent ... is a single bond, and C when the adjacent ... is a double bond; and

Ar represents an aromatic group optionally having substituents, a $C_{3-9}$ cycloalkyl group optionally having substituents, a 3 to 9-membered saturated heterocyclic group optionally having substituents, a hydrogen atom or a halogen atom; or a salt thereof;

[3] the compound according to item [1], wherein compound (I) is represented by the formula:

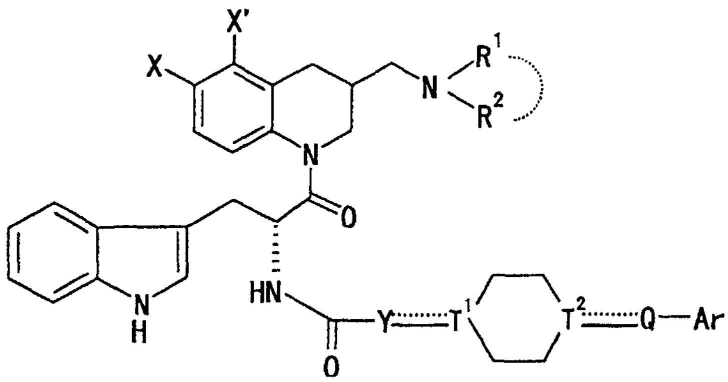

(I'')

wherein each symbol has the same meaning as in item [1];

[4] the compound according to any of items [1] - [3], wherein X and X' are the same or different, and each represents a hydrogen atom, a fluorine atom or a chlorine atom, and at least one of X and X' represents a fluorine atom or a chlorine atom;

... represents a single bond;

$T^1$ and $T^2$ are the same or different, and each represents CH or N; and

Ar is an aromatic group optionally having substituents;

[5] the compound according to any of items [1] - [3], wherein X is a fluorine atom or a chlorine atom and X' is a hydrogen atom;

[6] the compound according to any of items [1] - [3], wherein X is a chlorine atom and X' is a hydrogen atom;

[7] the compound according to any of items [1] - [3], wherein $R^1$ and $R^2$ are each $C_{1-6}$ alkyl, or $R^1$ and $R^2$ form a 5- or 6-membered cyclic amino group together with the adjacent nitrogen atom;

[8] the compound according to any of items [1] - [3], wherein $R^1$ and $R^2$ are each $C_{1-6}$ alkyl;

[9] the compound according to item [1], wherein the spacer having a main chain of 1 to 6 atoms represented by Y and Q is a divalent group comprising of 1 to 3 groups selected from -O-, -S-, -CO-, -SO-, $-SO_2-$, $-NR^8-$ ($R^8$ is a hydrogen atom, an optionally halogenated $C_{1-6}$ alkyl, an optionally halogenated $C_{1-6}$ alkyl-carbonyl, an optionally halogenated $C_{1-6}$ alkylsulfonyl) and an optionally halogenated divalent $C_{1-6}$ non-cyclic hydrocarbon group;

[10] the compound according to any of items [1] - [3], wherein Y is a bond, $C_{1-2}$ alkylene or $-CH_2O-$;

[11] the compound according to any of items [1] - [3], wherein Y is a bond or $C_{1-2}$ alkylene;

[12] the compound according to any of items [1] - [3], wherein Q is =CH-, $-CH_2-$, -O-, -S-, -CO-, $-SO_2-$, $-CO-CH_2-$, $-CH_2-NH-CO-$ or $-CH_2-O-CH_2-$;

[13] the compound according to any of items [1] - [3], wherein Q is -CO-;

[14] the compound according to any of items [1] - [3], wherein ... represents a single bond, $T^1$ is CH and $T^2$ is N;

[15] the compound according to any of items [1] - [3], wherein ... represents a single bond, $T^1$ is N and $T^2$ is CH;

[16] the compound according to any of items [1] - [3], wherein ... represents a single bond, $T^1$ is N and $T^2$ is N;

[17] the compound according to any of items [1] - [3], wherein Ar is a monocyclic aromatic group optionally having substituents;

[18] the compound according to any of items [1] - [3], wherein Ar is a fused aromatic group optionally having substituents;

[19] the compound according to item [17], wherein Ar is phenyl which may have 1 or 2 substituents selected from a halogen atom, a cyano, an optionally halogenated $C_{1-6}$ alkyl and an optionally halogenated $C_{1-6}$ alkoxy;

[20] the compound according to item [18], wherein Ar is indol-2-yl which may have 1 or 2 substituents selected from a halogen atom, an optionally halogenated $C_{1-6}$ alkyl and an optionally halogenated $C_{1-6}$ alkoxy;

[21] the compound according to item [18], wherein Ar is inden-2-yl, isoquinolyl or 2-oxo-2,3-dihydro-1H-benzimidazol-1-yl;
[22] the compound according to item [2], which is of the formula:

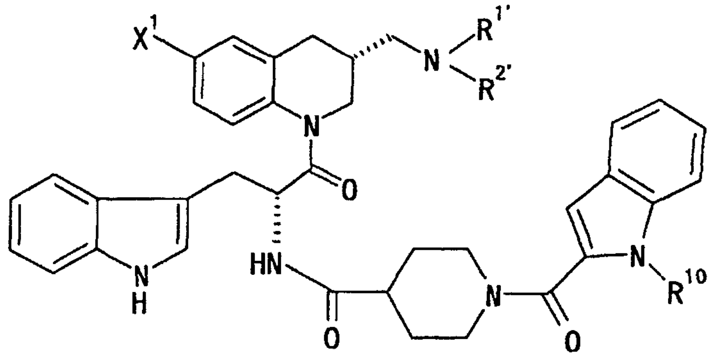

or

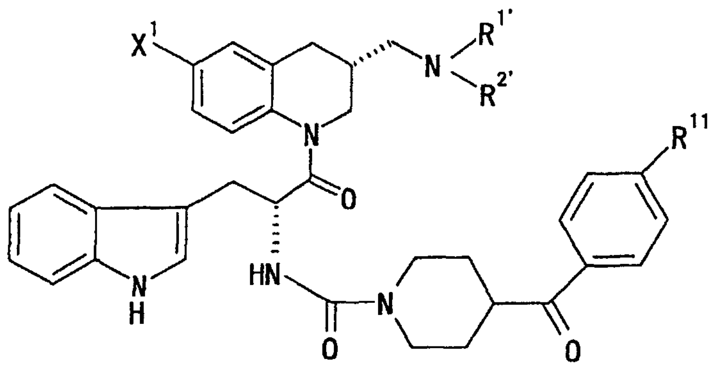

wherein $X^1$ represents a hydrogen atom, a fluorine atom, a chlorine atom or an amino optionally having substituents;

$R^{1'}$ and $R^{2'}$ each represent a hydrogen atom or a $C_{1-6}$ alkyl;
$R^{10}$ represents a $C_{1-6}$ alkyl; and
$R^{11}$ represents a halogen atom;

[23] the compound according to item [22], wherein $X^1$ represents a chlorine atom, $R^{1'}$ and $R^{2'}$ each represent a $C_{1-3}$ alkyl, $R^{10}$ represents a $C_{1-3}$ alkyl, and $R^{11}$ represents a halogen atom;
[24] N-[(1R)-2-[(3R)-6-chloro-3-[(dimethylamino)-methyl]-1,2,3,4-tetrahydro-1-quinolinyl]-1-(1-indol-3-ylmethyl)-2-oxoethyl]-1-(1-methylindol-2-ylcarbonyl)-4-piperidinecarboxamide (Example 51),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-1-(3-isoquinolylcarbonyl)-4-piperidinecarboxamide (Example 118),
N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-(4-fluorobenzoyl)-1-piperidinecarboxamide (Example 129),
4-(4-chlorobenzoyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-1-piperidinecarboxamide (Example 130),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-(4-chlorophenoxy)-1-piperidinecarboxamide (Example 142),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-phenoxy-l-piperidinecarboxamide (Example 145),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-[(4-fluorophenyl)sulfonyl]-1-piperidinecarboxamide (Example 148),
N-[(1R)-2-[(3R)-6-chloro-3-[(dimethylamino)methyl]-1,2,3,4-tetrahydro-1-quinolinyl]-1-(1H-indol-3-ylmethyl)-

2-oxoethyl]-4-[(4-chlorophenyl)sulfonyl]-1-piperidinecarboxamide (Example 150),
3-(1-benzoyl-4-piperidinyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide (Example 31),
2-[(1-benzoyl-4-piperidinyl)oxy]-N-[(1R)-2-[(3R)-6-chloro-3-[dimethylamino]methyl]-1,2,3,4-tetrahydro-1-quinolinyl]-1-(1-indol-3-ylmethyl)-2-oxoethyl]acetamide (Example 33), or a salt thereof;
[25] a prodrug of the compound according to any of items [1] - [3];
[26] a method for producing a compound of item [1] or a salt thereof, which comprises reacting a compound of the formula:

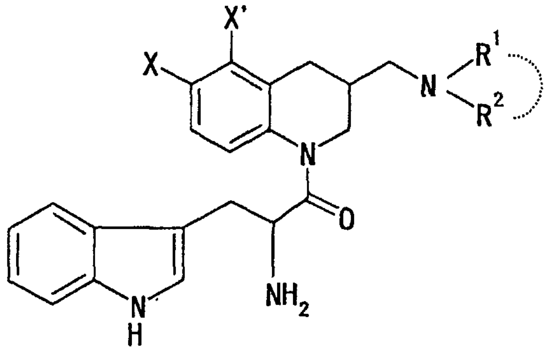

(IV)

wherein each symbol has the same meaning as in item [1], or a salt thereof, and a compound of the formula:

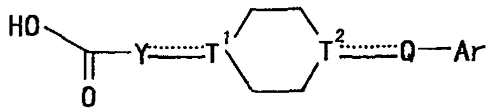

wherein each symbol has the same meaning as in item [1], or a salt thereof;
[27] a method for producing a compound of the formula:

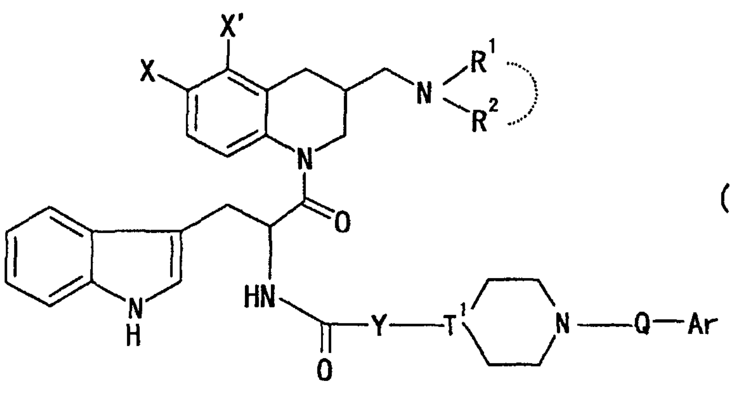

(Ia)

wherein each symbol has the same meaning as in item [1], or a salt thereof, which comprises reacting a compound of the formula:

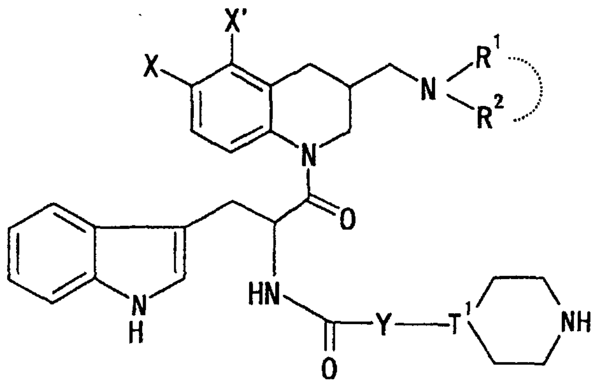

(VII)

wherein each symbol has the same meaning as above, or a salt thereof, and a compound of the formula:

$$L^1\text{-Q-Ar}$$

wherein $L^1$ is a leaving group, and other symbols have the same meanings as in item [1], or a salt thereof;
[28] a pharmaceutical composition which comprises the compound according to any of items [1] - [3], a salt thereof or a prodrug thereof;
[29] the composition according to item [28], which is a somatostatin receptor binding inhibitor;
[30] the composition according to item [29], which is a somatostatin subtype 2 receptor binding inhibitor;
[31] the composition according to item [28], which is a somatostatin receptor agonist;
[32] the composition according to item [31], which is a somatostatin subtype 2 receptor agonist;
[33] the composition according to item [28], which is a prophylactic or therapeutic agent for diabetes or diabetic nephropathy;
[34] the composition according to item [28], which is a prophylactic or therapeutic agent for tumors such as acromegaly, TSH-producing tumors, nonsecretory (afunctional) hypophysial tumors, ectopic ACTH (adrenocorticotrophic hormone)-producing tumors, medullar thyroid carcinoma, VIP-producing tumors, glucagon-producing tumors, gastrin-producing tumors, insulinoma and carotinoid;
[35] the composition according to item [28], which is a prophylactic or therapeutic agent for diarrhea due to neuroendocrine tumors, or diarrhea due to AIDS;
[36] a method for inhibiting somatostatin receptor binding, which comprises administering to a mammal an effective amount of the compound according to any of items [1] - [3], a salt thereof or a prodrug thereof;
[37] use of the compound according to any of items [1] - [3], a salt thereof or a prodrug thereof for manufacturing a somatostatin receptor binding inhibitor; and
[38] a compound of the formula:

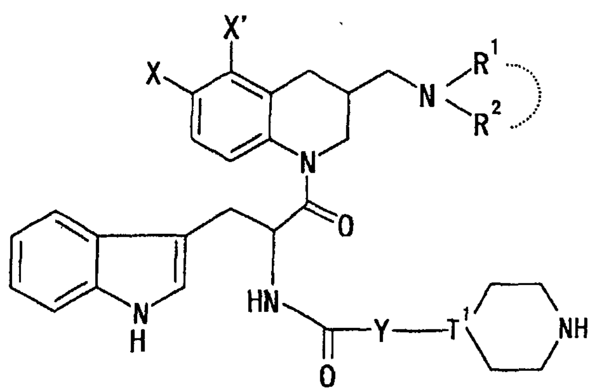

(VII)

wherein each symbol has the same meaning as in item [1], or a salt thereof.

BEST MODE FOR CARRYING OUT THE INVENTION

In the above formula, X and X' are the same or different, they represent a hydrogen atom, a fluorine atom, a chlorine atom or an amino optionally having substituents and at least one of X and X' represents a fluorine atom, a chlorine atom or an amino optionally having substituents.

Preferably, X and X' are the same or different, they represent a hydrogen atom, a fluorine atom or a chlorine atom, and at least one of X and X' represents a fluorine atom or a chlorine atom. More preferably, X represents a fluorine atom or a chlorine atom and X' represents a hydrogen atom.

The substituent in the "amino optionally having substituents" represented by X and X' includes an optionally halogenated $C_{1-6}$ alkyl, formyl, an optionally halogenated $C_{1-6}$ alkyl-carbonyl, $C_{1-6}$ alkoxy-carbonyl, and an optionally halogenated $C_{1-6}$ alkylsulfonyl.

The "optionally halogenated $C_{1-6}$ alkyl" is exemplified by those mentioned as the "substituent" in the later-described "nitrogen-containing heterocyclic ring optionally having substituents" formed by $R^1$ and $R^2$ together with the adjacent nitrogen atom.

The "optionally halogenated $C_{1-6}$ alkyl-carbonyl", "optionally halogenated $C_{1-6}$ alkoxy-carbonyl" and "optionally halogenated $C_{1-6}$ alkylsulfonyl" are exemplified by those mentioned as the later described "substituent" in "$C_{1-6}$ alkyl optionally having substituents" represented by $R^1$ and $R^2$.

In the above formula, the "$C_{1-6}$ alkyl" in the "$C_{1-6}$ alkyl optionally having substituents" includes, for example, methyl, ethyl, propyl, isopropyl, butyl, isobutyl, sec-butyl, tert-butyl, pentyl, hexyl, etc. Among those, methyl, ethyl, propyl are preferred.

The "substituent" in said "$C_{1-6}$ alkyl optionally having substituents" includes, for example, halogen atoms (e.g., fluorine, chlorine, bromine, iodine, etc.), $C_{1-3}$ alkylenedioxy (e.g., methylenedioxy, ethylenedioxy, etc.), nitro, cyano, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino (e.g., methylamino, ethylamino, propylamino, isopropylamino, butylamino, etc.), di-$C_{1-6}$ alkylamino (e.g., dimethylamino, diethylamino, dipropylamino, dibutylamino, ethylmethylamino, etc.), formyl, carboxy, carbamoyl, thiocarbamoyl, optionally halogenated $C_{1-6}$ alkyl-carbonyl, $C_{1-6}$ alkoxy-carbonyl (e.g., methoxycarbonyl, ethoxycarbonyl, propoxycarbonyl, tert-butoxycarbonyl, etc.), mono-$C_{1-6}$ alkyl-carbamoyl (e.g., methylcarbamoyl, ethylcarbamoyl, etc.), di-$C_{1-6}$ alkyl-carbamoyl (e.g., dimethylcarbamoyl, diethylcarbamoyl, ethylmethylcarbamoyl, etc.), optionally halogenated $C_{1-6}$ alkylsulfonyl, formylamino, optionally halogenated $C_{1-6}$ alkyl-carboxamide, $C_{1-6}$ alkoxy-carboxamide (e.g., methoxycarboxamide, ethoxycarboxamide, propoxycarboxamide, butoxycarboxamide, etc.), $C_{1-6}$ alkylsulfonylamino (e.g., methylsulfonylamino, ethylsulfonylamino, etc.), $C_{1-6}$ alkyl-carbonyloxy (e.g., acetoxy, propanoyloxy, etc.), $C_{1-6}$ alkoxy-carbonyloxy (e.g., methoxycarbonyloxy, ethoxycarbonyloxy, propoxycarbonyloxy, butoxycarbonyloxy, etc.), mono-$C_{1-6}$ alkyl-carbamoyloxy (e.g., methylcarbamoyloxy, ethylcarbamoyloxy, etc.), di-$C_{1-6}$ alkyl-carbamoyloxy (e.g., dimethylcarbamoyloxy, diethylcarbamoyloxy, etc.), aromatic group optionally having substituents, etc. The number of the substituents is, for example, 1 to 5, preferably, 1 to 3. When the number of the substituents is 2 or more, these substituents may be the same or different.

The above-mentioned "optionally halogenated $C_{3-6}$ cycloalkyl" includes, for example, a $C_{3-6}$ cycloalkyl (e.g., cyclopropyl, cyclobutyl, cyclopentyl, cyclohexyl) which may have 1 to 5, preferably, 1 to 3 halogen atoms (e.g., fluorine, chlorine, bromine, iodine, etc.). Concrete examples are cyclopropyl, cyclobutyl, cyclopentyl, cyclohexyl, 4,4-dichlorocyclohexyl, 2,2,3,3-tetrafluorocyclopentyl, 4-chlorocyclohexyl, etc.

The above-mentioned "optionally halogenated $C_{1-6}$ alkoxy" includes, for example, $C_{1-6}$ alkoxy (e.g., methoxy, ethoxy, propoxy, butoxy, pentyloxy, etc.) which may have 1 to 5, preferably, 1 to 3 halogen atoms (e.g., fluorine, chlorine, bromine, iodine, etc.). Concrete examples are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, etc.

The above-mentioned "optionally halogenated $C_{1-6}$ alkylthio" includes, for example, $C_{1-6}$ alkylthio (e.g., methylthio, ethylthio, propylthio, isopropylthio, butylthio, sec-butylthio, tert-butylthio, etc.) which may have 1 to 5, preferably, 1 to 3 halogen atoms (e.g., fluorine, chlorine, bromine, iodine, etc.). Concrete examples are methylthio, difluoromethylthio, trifluoromethylthio, ethylthio, propylthio, isopropylthio, butylthio, 4,4,4-trifluorobutylthio, pentylthio, hexylthio, etc.

The above-mentioned "optionally halogenated $C_{1-6}$ alkyl-carbonyl" includes, for example, $C_{1-6}$ alkyl-carbonyl (e.g., acetyl, propanoyl, butanoyl, pentanoyl, hexanoyl, etc.) which may have 1 to 5, preferably, 1 to 3 halogen atoms (e.g., fluorine, chlorine, bromine, iodine, etc.). Concrete examples are acetyl, monochloroacetyl, trifluoroacetyl, trichloroacetyl, propanoyl, butanoyl, pentanoyl, hexanoyl, etc.

The above-mentioned "optionally halogenated $C_{1-6}$ alkylsulfonyl" includes, for example, $C_{1-6}$ alkylsulfonyl (e.g., methylsulfonyl, ethylsulfonyl, propylsulfonyl, isopropylsulfonyl, butylsulfonyl, sec-butylsulfonyl, tert-butylsulfonyl, etc.) which may have 1 to 5, preferably, 1 to 3 halogen atoms (e.g., fluorine, chlorine, bromine, iodine, etc.). Concrete examples are methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl, hexylsulfonyl, etc.

The above-mentioned "optionally halogenated $C_{1-6}$ alkyl-carboxamide" includes, for example, $C_{1-6}$ alkylcar-

boxamide (e.g., acetamide, propanamide, butanamide, etc.) which may have 1 to 5, preferably, 1 to 3 halogen atoms (e.g., fluorine, chlorine, bromine, iodine, etc.). Concrete examples are acetamide, trifluoroacetoamide, propanamide, and butanamide.

The above-mentioned "aromatic group optionally having substituents" is exemplified by one mentioned as the later-described Ar.

The "nitrogen-containing heterocyclic ring" for the "nitrogen-containing heterocyclic ring optionally having substituents" as formed by $R^1$ and $R^2$ together with the adjacent nitrogen atom includes, for example, 3- to 8-membered nitrogen-containing heterocyclic rings containing, in addition to carbon atoms, at least one nitrogen atom and optionally 1 to 3 heteroatoms selected from the group consisting of nitrogen, sulfur and oxygen atoms. Concretely mentioned are aziridine, azetidine, morpholine, thiomorpholine, piperidine, piperazine, pyrrolidine, hexamethyleneimine, heptamethyleneimine, hexahydropyrimidine, 1,4-diazepane, and unsaturated cyclic amines thereof (e.g., 1,2,5,6-tetrahydropyridine, etc.), etc. Among these, preferred are morpholine, piperidine, piperazine, pyrrolidine, etc. More preferred is 5- or 6-membered cyclic amino group (e.g., pyrrolidine, piperidine).

The "substituent" in said "nitrogen-containing heterocyclic ring optionally having substituents" includes, for example, oxo, optionally halogenated $C_{1-6}$ alkyl, optionally halogenated $C_{1-6}$ alkyl-carbonyl, optionally halogenated $C_{1-6}$ alkylsulfonyl, $C_{6-14}$ aryl optionally having substituents, $C_{7-19}$ aralkyl optionally having substituents, $C_{6-14}$ aryl-carbonyl optionally having substituents, 5- to 10-membered aromatic heterocyclic group optionally having substituents, etc. The number of the substituents is, for example, 1 to 5, preferably, 1 to 3. When the number of the substituents is 2 or more, these substituents may be the same or different.

The above "optionally halogenated $C_{1-6}$ alkyl" includes, for example, $C_{1-6}$ alkyl (e.g., methyl, ethyl, propyl, isopropyl, butyl, isobutyl, sec-butyl, tert-butyl, pentyl, hexyl, etc.) which may have 1 to 5, preferably, 1 to 3 halogen atoms (e.g., fluorine, chlorine, bromine, iodine, etc.). Concrete examples are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc.

The "optionally halogenated $C_{1-6}$ alkyl-carbonyl", "optionally halogenated $C_{1-6}$ alkylsulfonyl" are exemplified by those mentioned as the "substituent" in the above "$C_{1-6}$ alkyl optionally having substituents".

The "$C_{6-14}$ aryl" in the "$C_{6-14}$ aryl optionally having substituents" includes, for example, phenyl, 1-naphthyl, 2-naphthyl, 2-indenyl, and 2-anthryl. Among those, phenyl is preferred.

The "$C_{7-19}$ aralkyl" in the "$C_{7-19}$ aralkyl optionally having substituents" includes, for example, benzyl, phenethyl, diphenylmethyl, triphenylmethyl, 1-naphthylmethyl, 2-naphthylmethyl, 2,2-diphenylethyl, 3-phenylpropyl, 4-phenylbutyl, 5-phenylpentyl, etc. Among those, benzyl is preferred.

The "$C_{6-14}$ aryl-carbonyl" in the "$C_{6-14}$ aryl-carbonyl optionally having substituents" includes, for example, benzoyl, 1-naphthoyl, 2-naphthoyl, etc.

The "5- to 10-membered aromatic heterocyclic group" in the "5- to 10-membered aromatic heterocyclic group optionally having substituents" includes, for example, 5- to 10-membered (monocyclic or bicyclic) aromatic heterocyclic group containing, in addition to carbon atoms, preferably 1 to 4 of 1 or 2 kinds of heteroatoms selected from the group consisting of nitrogen, sulfur and oxygen atoms. Concretely, for example, 2- or 3-thienyl; 2-, 3- or 4-pyridyl; 2- or 3-furyl; 2-, 4- or 5-thiazolyl; 2-, 4- or 5-oxazolyl; 1-, 3- or 4-pyrazolyl; 2-pyrazinyl; 2-, 4- or 5-pyrimidinyl; 1-, 2-or 3-pyrrolyl; 1-, 2- or 4-imidazolyl; 3- or 4-pyridazinyl; 3-isothiazolyl; 3-isooxazolyl; 1,2,4-oxadiazol-5-yl; 1,2,4-oxadiazol-3-yl; 2-, 3-, 4-, 5- or 8-quinolyl; 1-, 3-, 4-, 5-, 6-, 7- or 8-isoquinolyl; 1-, 2-, 3-, 4-, 5-, 6- or 7-indolyl; 1-, 2-, 4- or 5-isoindolyl; 1-, 5- or 6-phthalazinyl; 2-, 3- or 5-quinoxalinyl; 2-, 3-, 4-, 5- or 6-benzofuranyl; 2-, 4-, 5- or 6-benzothiazolyl; 1-, 2-, 4-, 5- or 6-benzimidazolyl, etc.

The "substituents" of the above "$C_{6-14}$ aryl optionally having substituents", "$C_{7-19}$ aralkyl optionally having substituents", "$C_{6-14}$ aryl-carbonyl optionally having substituents" and "5- to 10-membered aromatic heterocyclic group optionally having substituents" includes, for example, halogen atoms (e.g., fluorine, chlorine, bromine, iodine, etc.), $C_{1-3}$ alkylenedioxy (e.g., methylenedioxy, ethylenedioxy, etc.), nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino (e.g., methylamino, ethylamino, propylamino, isopropylamino, butylamino, etc.), di-$C_{1-6}$ alkylamino (e.g., dimethylamino, diethylamino, dipropylamino, dibutylamino, ethylmethylamino, etc.), formyl, carboxy, carbamoyl, thiocarbamoyl, optionally halogenated $C_{1-6}$ alkyl-carbonyl, $C_{1-6}$ alkoxy-carbonyl (e.g., methoxycarbonyl, ethoxycarbonyl, propoxycarbonyl, tert-butoxycarbonyl, etc.), mono-$C_{1-6}$ alkylcarbamoyl (e.g., methylcarbamoyl, ethylcarbamoyl, etc.), di-$C_{1-6}$ alkyl-carbamoyl (e.g., dimethylcarbamoyl, diethylcarbamoyl, ethylmethylcarbamoyl, etc.), optionally halogenated $C_{1-6}$ alkylsulfonyl, formylamino, optionally halogenated $C_{1-6}$ alkylcarboxamide, $C_{1-6}$ alkoxy-carboxamide (e.g., methoxycarboxamide, ethoxycarboxamide, propoxycarboxamide, butoxycarboxamide, etc.), $C_{1-6}$ alkylsulfonylamino (e.g., methylsulfonylamino, ethylsulfonylamino, etc.), $C_{1-6}$ alkyl-carbonyloxy (e.g., acetoxy, propanoyloxy, etc.), $C_{1-6}$ alkoxy-carbonyloxy (e.g., methoxycarbonyloxy, ethoxycarbonyloxy, propoxycarbonyloxy, butoxycarbonyloxy, etc.), mono-$C_{1-6}$ alkyl-carbamoyloxy (e.g., methylcarbamoyloxy, ethylcarbamoyloxy, etc.), di-$C_{1-6}$ alkyl-carbamoyloxy

(e.g., dimethylcarbamoyloxy, diethylcarbamoyloxy, etc.), etc. The number of the substituents is, for example, 1 to 5, preferably, 1 to 3. When the number of the substituents is 2 or more, these substituents may be the same or different.

The "optionally halogenated $C_{1-6}$ alkyl" is exemplified by those mentioned as the "substituent" in the above "nitrogen-containing heterocyclic ring optionally having substituents".

The "optionally halogenated $C_{3-6}$ cycloalkyl", "optionally halogenated $C_{1-6}$ alkoxy", "optionally halogenated $C_{1-6}$ alkylthio", "optionally halogenated $C_{1-6}$ alkyl-carbonyl", "optionally halogenated $C_{1-6}$ alkylsulfonyl" and "optionally halogenated $C_{1-6}$ alkyl-carboxamide" are exemplified by those mentioned as the "substituent" in the above "$C_{1-6}$ alkyl optionally having substituents".

For $R^1$ and $R^2$, preferred are $C_{1-6}$ alkyl, more preferred are methyl, ethyl and propyl; the most preferred is methyl.

In the above formula, the "spacer having a main chain of 1 to 6 atoms" represented by Y and Q means a spacer in which 1 to 6 atoms of a main chain are combined in a straight-chain form. The "number of atoms of a main chain" is counted so as the number of atoms of the main chain is minimum.

The "spacer having a main chain of 1 to 6 atoms" includes, for example, divalent group comprising 1 to 3 groups selected from -O-, -S-, -CO-, -SO-, $-SO_2-$, $-NR^8-$($R^8$ is hydrogen atom, optionally halogenated $C_{1-6}$ alkyl, optionally halogenated $C_{1-6}$ alkyl-carbonyl, optionally halogenated $C_{1-6}$ alkylsulfonyl) and optionally halogenated divalent $C_{1-6}$ non-cyclic hydrocarbon group.

The "optionally halogenated $C_{1-6}$ alkyl" is exemplified by those mentioned as the "substituent" in the above "nitrogen-containing heterocyclic ring optionally having substituents".

The "optionally halogenated $C_{1-6}$ alkyl-carbonyl" and "optionally halogenated $C_{1-6}$ alkylsulfonyl" are each exemplified by those mentioned as the "substituent" in the above-mentioned "$C_{1-6}$ alkyl optionally having substituents".

The "divalent $C_{1-6}$ non-cyclic hydrocarbon group" in the "optionally halogenated divalent $C_{1-6}$ non-cyclic hydrocarbon group" includes, for example,

(1) $C_{1-6}$ alkylene (e.g., $-CH_2-$, $-CF_2-$, $-(CH_2)_2-$, $-(CH_2)_3-$, $-(CH_2)_4-$, $-(CH_2)_5-$, $-(CH_2)_6-$, $-CH(CH_3)-$, $-C(CH_3)_2-$, $-OH(CF_3)-$, $-CH(CH_3)CH_2-$, $-C(CH_3)_2CH_2-$, $-(CH(CH_3))_2-$, $-(CF_2)_2-$, $-(CH_2)_2C(CH_3)_2-$, $-(CH_2)_3C(CH_3)_2-$, etc.);
(2) $C_{2-6}$ alkenylene (e.g., -CH=CH-, $-CH_2-CH=CH-$, $-CH_2-CF=CH-$, $-C(CH_3)_2-CH=CH-$, $-CH_2-CH=CH-CH_2-$, $-CH_2-CH_2-CH=CH-$, -CH=CH-CH=CH-, $-CH=CH-CH_2-CH_2-CH_2-$, etc.) ;
(3) $C_{2-6}$ alkynylene (e.g., -C≡C-, $-CH_2-C≡C-$, $-CH_2-C≡C-CH_2-CH_2-$, etc.);

each of which may have 1 to 5, preferably 1 to 3 halogen atoms (e.g., fluorine, chlorine, bromine, iodine, etc.), etc.

The preferable examples of said "spacer having a main chain of 1 to 6 atoms" are

(1) $C_{1-6}$ alkylene which may have 1 to 3 halogen atoms (e.g., $-CH_2-$, $-CF_2-$, $-(CH_2)_2-$, $-(CH_2)_3-$, $-(CH_2)_4-$, $-(CH_2)_5-$, $-(CH_2)_6-$, $-CHCH_3-$, $-C(CH_3)_2-$, $-CH(CF_3)-$, $-CH(CH_3)CH_2-$, $-C(CH_3)_2CH_2-$, $-(CH(CH_3))_2-$, $-(CF_2)_2-$, $-(CH_2)_2C(CH_3)_2-$, $-(CH_2)_3C(CH_3)_2-$, etc.) ;
(2) $C_{2-6}$ alkenylene which may have 1 to 3 halogen atoms (e.g., -CH=CH-, $-CH_2-CH=CH-$, $-CH_2-CF=CH-$, $-C(CH_3)_2-CH=CH-$, $-CH_2-CH=CH-CH_2-$, $-CH_2-CH_2-CH=CH-$, -CH=CH-CH=CH-, $-CH=CH-CH_2-CH_2-CH_2-$, etc.);
(3) $C_{2-6}$ alkynylene which may 1 to 3 halogen atoms, (e.g., -C≡C-, $-CH_2-C≡C-$, $-CH_2-C≡C-CH_2-CH_2-$, etc.);
(4) $-(CH_2)_{w1}O(CH_2)_{w2}-$, $-(CH_2)_{w1}S(CH_2)_{w2}-$, $-(CH_2)_{w1}CO(CH_2)_{w2}-$, $-(CH_2)_{w1}SO(CH_2)_{w2}-$, $-(CH_2)_{w1}SO_2(CH_2)_{w2}-$, $-(CH_2)_{w1}NR^8(CH_2)_{w2}-$ ;
(5) $-(CH_2)_{w3}CONR^8(CH_2)_{w4}-$, $-(CH_2)_{w3}NR^8CO(CH_2)_{w4}-$, $-(CH_2)_{w3}SO_2NR^8(CH_2)_{w4}-$, $-(CH_2)_{w3}NR^8SO_2(CH_2)_{w4}-$, $-(CH_2)_{w3}COO(CH_2)_{w4}-$;
(6) $-(CH_2)_{w5}NR^8CONR^{8b}(CH_2)_{w6}-$;

($R^8$ has the same meanings as above; $R^{8b}$ has the same meanings as $R^8$; w1 and w2 represent an integer of 0 to 5 and w1 + w2 represents 0 to 5; w3 and w4 represent an integer of 0 to 4 and w3 + w4 represents 0 to 4; w5 and w6 represent an integer of 0 to 3 and w5 + w6 represents 0 to 3)

The "spacer having a main chain of 1 to 6 atoms" represented by Y is, preferably, $C_{1-2}$ alkylene (e.g., $-CH_2-$, $-(CH_2)_2-$, etc.), $-(CH_2)_{w1}O(CH_2)_{w2}-$ (the symbols have the same meanings as above), etc. More preferred is $C_{1-2}$ alkylene (e.g., $-CH_2-$, $-(CH_2)_2-$, etc.), etc.

The "spacer having a main chain of 1 to 6 atoms" represented Q is, preferably, $C_{1-2}$ alkylene (e.g., $-CH_2-$, $-(CH_2)_2-$, etc.), $-(CH_2)_{w1}CO(CH_2)_{w2}-$, $-(CH_2)_{w3}COO(CH_2)_{w4}-$, $-(CH_2)_{w3}NR^8CO(CH_2)_{w4}-$, $-(CH_2)_{w1}SO_2(CH_2)_{w2}-$ (the symbols have the same meanings as above); more preferably, $-(CH_2)_{w1}CO(CH_2)_{w2}-$, $-(CH_2)_{w3}COO(CH_2)_{w4}-$ (the symbols have the same meanings as above), etc.

Y represents, preferably, a bond, $C_{1-2}$ alkylene (e.g., $-CH_2-$, $-(CH_2)_2-$, etc.), $-CH_2O-$, etc. More preferred is a bond

or $C_{1-2}$ alkylene (e.g., $-CH_2-$, $-(CH_2)_2-$, etc.), etc.
Q represents, preferably, a bond, $C_{1-2}$ alkylene (e.g., $-CH_2-$, $-(CH_2)_2-$, =CH-, etc.), $-(CH_2)_{w1}CO(CH_2)_{w2}-$, $-(CH_2)_{w3}COO(CH_2)_{w4}-$, $-(CH_2)_{w3}NR^8CO(CH_2)_{w4}-$, $-(CH_2)_{w1}SO_2\ (CH_2)_{w2}-$, $-\ (CH_2)_{w1}O(CH_2)_{w2}-$ (the symbols have the same meanings as above); more preferred is a bond, $-\ (CH_2)_{w1}CO(CH_2)_{w2}-$, $-(CH_2)_{w3}COO(CH_2)_{w4}-$, $-(CH_2)_{w1}O(CH_2)_{w2}-$ (the symbols have the same meanings as above), etc. Among those, =CH-, $-CH_2-$, -O-, -S-, -CO-, $-SO_2-$, $-CO-CH_2-$, $-CH_2-NH-CO-$, $-CH_2-O-CH_2-$are preferred and -CO- is particularly preferred.

In the above formula, ... represents a single bond or a double bond, preferably, a single bond.

In the above formula, when each of adjacent ...is a single bond, $T^1$ and $T^2$ are the same or different, they represent $C(R^9)$ ($R^9$ represents a hydrogen atom, a hydroxy or $C_{1-6}$ alkyl) or N and when each of the adjacent ... is a double bond, $T^1$ and $T^2$ represent C.

The "$C_{1-6}$ alkyl" represented by $R^9$ is exemplified by those mentioned in the "$C_{1-6}$ alkyl optionally having substituents" represented by the above-mentioned $R^1$ and $R^2$.

When each of adjacent ... is a single bond, $T^1$ and $T^2$ are the same or different, and they represent CH or N. Among those, preferred is the case in which $T^1$ is =CH- and $T^2$ is =N-.

Also, preferred are the case in which $T^1$ is N and $T^2$ is CH, and the case in which both $T^1$ and $T^2$ represent N.

In the above formula, the "aromatic group" in the "aromatic group optionally having substituents" includes, for example, monocyclic aromatic group, fused aromatic group, aromatic ring assembly group, etc.

Said monocyclic aromatic group includes, for example, a monovalent group which is derived by removing an optional hydrogen atom from monocyclic aromatic group. The "monocyclic aromatic ring" includes, for example, benzene and a 5- or 6-membered aromatic heterocyclic ring.

The "5- or 6-membered aromatic heterocyclic ring" includes, for example, 5- or 6-membered aromatic heterocyclic rings containing, in addition to carbon atoms, one or more (e.g., 1 to 3) heteroatoms selected from the group consisting of nitrogen, sulfur and oxygen atoms, etc. Concretely mentioned are thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, furazane, etc.

The concrete examples of "monocyclic aromatic group" are phenyl, 2- or 3-thienyl, 2- or 3-furyl, 2-, 3- or 4-pyridyl, 2-, 4- or 5-thiazolyl, 2-, 4- or 5-oxazolyl, 3-or 4-pyrazolyl, 2-pyrazinyl, 2-, 4- or 5-pyrimidinyl, 1-, 2- or 3-pyrrolyl, 1-, 2- or 4-imidazolyl, 3- or 4-pyridazinyl, 3-isothiazolyl, 3-isooxazolyl, 1,2,4-oxadiazol-5-yl, 1,2,4-oxadiazol-3-yl, etc.

The "fused aromatic group" includes, for example, a monovalent group derived by removing an optional hydrogen atom from a fused polycyclic (preferably bi- to tetra-cyclic, preferably bi- or tri-cyclic) aromatic ring. The "fused polycyclic aromatic ring" includes, for example, a fused polycyclic aromatic hydrocarbon, a fused polycyclic aromatic heterocyclic ring, etc.

Said "fused polycyclic aromatic hydrocarbon" includes, for example, a $C_{9-14}$ fused polycyclic (bi- or tri-cyclic) aromatic hydrocarbon (e.g., naphthalene, indene, fluorene, anthracene, etc.), etc.

Said "fused polycyclic aromatic heterocyclic ring" includes, for example, 9- to 14-membered, preferably 9-or 10-membered fused polycyclic aromatic heterocyclic rings containing, in addition to carbon atoms, one or more (e.g., 1 to 4) heteroatoms selected from the group consisting of nitrogen, sulfur and oxygen atoms, etc. The concrete examples of "fused polycyclic aromatic heterocyclic ring" are benzofuran, benzothiophene, benzimidazole, benzoxazole, benzothiazole, benzisothiazole, naphtho[2,3-b]thiophene, isoquinoline, quinoline, indole, quinoxaline, phenanthridine, phenothiazine, phenoxazine, phthalazine, naphthyridine, quinazoline, cinnoline, carbazole, β-carboline, acridine, phenadine, phthalimido, etc.

Specific examples of the "fused aromatic group" includes, for example, 1-naphthyl; 2-naphthyl; inden-1-yl; inden-2-yl; 2-, 3-, 4-, 5- or 8-quinolyl; 1-, 3-, 4-, 5-, 6-, 7- or 8-isoquinolyl; 1-, 2-, 3-, 4-, 5-, 6- or 7-indolyl; 1-, 2-, 4- or 5-isoindolyl; 1-, 5- or 6-phthalazinyl; 2-, 3- or 5-quinoxalinyl; 2-, 3-, 4-, 5-or 6-benzothienyl; 2-, 3-, 4-, 5- or 6-benzofuranyl; 2-, 4-, 5- or 6-benzothiazolyl; 1-, 2-, 4-, 5- or 6-benzimidazolyl, 2-oxo-2,3-dihydro-1H-benzimidazol-1-yl; etc.

The "aromatic ring assembly group" includes, for example, a group derived by removing an optional hydrogen atom from aromatic ring assemblies in which two or more, preferably two or three aromatic rings are directly connected with each other by single bond(s) and the number of such direct ring junctions is one less than the number of the aromatic rings involved.

The above-mentioned aromatic ring assemblies include, for example, aromatic ring assemblies formed by two or three (preferably two) groups selected from a $C_{6-14}$ monocyclic or fused polycyclic aromatic hydrocarbon (e.g., benzene ring, naphthalene ring, etc.) and 5- to 10-membered (preferably 5- or 6-membered) aromatic heterocyclic rings.

Preferred examples of the aromatic ring assemblies include one composed of two or three aromatic rings selected from the group consisting of benzene, naphthalene, pyridine, pyrimidine, thiophene, furan, thiazole, isothiazole, oxazole, 1,2,4-oxadiazole, 1,3,4-oxadiazole, 1,2,4-thiadiazole, 1,3,4-thiadiazole, quinoline, isoquinoline, indole, benzothiophene, benzoxazole, benzothiazole, and benzofuran.

As specific examples of the "aromatic ring assembly group", mentioned are 2-, 3- or 4-biphenylyl; 3-(1-naphthyl)-1,2,4-oxadiazol-5-yl; 3-(2-naphthyl)-1,2,4-oxadiazol-5-yl; 3-(2-benzofuranyl)-1,2,4-oxadiazol-5-yl; 3-phenyl-1,2,4-oxadiazol-5-yl; 3-(2-benzoxazolyl)-1,2,4-oxadiazol-5-yl; 3-(3-indolyl)-1,2,4-oxadiazol-5-yl; 3-(2-indolyl)-1,2,4-oxadiazol-5-yl; 4-phenylthiazol-2-yl; 4-(2-benzofuranyl)thiazol-2-yl; 4-phenyl-1,3-oxazol-5-yl; 5-phenyl-isothiazol-4-yl; 5-phenyloxazol-2-yl; 4-(2-thienyl)phenyl; 4-(3-thienyl)phenyl; 3-(3-pyridyl)phenyl; 4-(3-pyridyl)phenyl; 6-phenyl-3-pyridyl; 5-phenyl-1,3,4-oxadiazol-2-yl; 4-(2-naphthyl)phenyl; 4-(2-benzofuranyl)phenyl; 4,4'-terphenyl; etc.

Among the "aromatic group" described in the above, preferred is "monocyclic aromatic group" and "fused aromatic group".

Said "monocyclic aromatic group" is, preferably, phenyl, 2- or 3-thienyl, 2-, 3- or 4-pyridyl.

Said "fused aromatic group" is, preferably, fused polycyclic aromatic heterocyclic group and more preferably, 2-benzothienyl, 2-benzofuranyl, indol-2-yl, indol-3-yl.

The "substituent" in the "aromatic group optionally having substituents" represented by Ar includes, for example, oxo, halogen atoms (e.g., fluorine, chlorine, bromine, iodine, etc.), $C_{1-3}$ alkylenedioxy (e.g., methylenedioxy, ethylenedioxy, etc.), nitro, cyano, optionally halogenated $C_{1-6}$ alkyl, $C_{6-14}$ aryloxy-$C_{1-6}$ alkyl (e.g., phenoxymethyl, etc.), $C_{1-6}$ alkyl-$C_{6-14}$ aryl-$C_{2-6}$ alkenyl (e.g., methylphenylethenyl, etc.), optionally halogenated $C_{3-6}$ cycloalkyl, $C_{7-19}$ aralkyl optionally having substituents, optionally halogenated $C_{1-6}$ alkoxy, optionally halogenated $C_{1-6}$ alkylthio, hydroxy, $C_{6-14}$ aryloxy optionally having substituents, $C_{7-19}$ aralkyloxy optionally having substituents, amino, mono-$C_{1-6}$ alkylamino (e.g., methylamino, ethylamino, propylamino, isopropylamino, butylamino, etc.), di-$C_{1-6}$ alkylamino (e.g., dimethylamino, diethylamino, dipropylamino, dibutylamino, ethylmethylamino, etc.), 5- to 7-membered saturated cyclic amino optionally having substituents, acyl, acylamino, acyloxy, etc.

The "aromatic group" represented by Ar may have 1 to 5, preferably 1 to 3 of the above substituents at substitutable positions on the aromatic group. When the number of the substituents is two or more, those substituents may be the same or different.

The "optionally halogenated $C_{1-6}$ alkyl" and "$C_{7-19}$ aralkyl optionally having substituents" are exemplified by those mentioned as the "substituent" in the above "nitrogen-containing heterocyclic ring optionally having substituents".

The "optionally halogenated $C_{3-6}$ cycloalkyl", "optionally halogenated $C_{1-6}$ alkoxy" and "optionally halogenated $C_{1-6}$ alkylthio" are exemplified by those mentioned as the "substituent" in the above "$C_{1-6}$ alkyl optionally having substituents".

The "$C_{6-14}$ aryloxy" in the "$C_{6-14}$ aryloxy optionally having substituents" mentioned above includes, for example, phenyloxy, 1-naphthyloxy, 2-naphthyloxy, etc.

The "$C_{7-19}$ aralkyloxy" in the "$C_{7-19}$ aralkyloxy optionally having substituents" mentioned above includes, for example, benzyloxy, phenethyloxy, diphenylmethyloxy, triphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy, etc.

The "substituents" in the "$C_{6-14}$ aryloxy optionally having substituents" and "$C_{7-19}$ aralkyloxy optionally having substituents" are exemplified by those mentioned as the "substituent" in the above "$C_{6-14}$ aryl optionally having substituents". The number of the substituents is, for example, 1 to 5, preferably, 1 to 3. When the number of the substituents is 2 or more, these substituents may be the same or different.

The "5- to 7-membered saturated cyclic amino" for the above "5- to 7-membered saturated cyclic amino optionally having substituents" includes, for example, morpholino, thiomorpholino, piperazin-1-yl, piperidino, pyrrolidin-1-yl, etc. The "5- to 7-membered saturated cyclic amino" may be condensed with benzene ring.

The "substituent" in said "5- to 7-membered saturated cyclic amino" is exemplified by those mentioned as the "substituent" in the above "nitrogen-containing heterocyclic ring optionally having substituents". The number of the substituents is, for example, 1 to 5, preferably, 1 to 3. When the number of the substituents is 2 or more, these substituents may be the same or different.

The "acyl" mentioned above includes, for example, an acyl represented by the following formulas: $-CO-R^3$, $-CO-OR^3$, $-CO-NR^3R^4$, $-CS-NR^3R^4$, $-SO_2-R^{3a}$, $-SO-R^{3a}$ and $-SO_2-NR^3R^4$,

wherein $R^3$ is (i) hydrogen atom, (ii) hydrocarbon group optionally having substituents, or (iii) heterocyclic group optionally having substituents;

$R^{3a}$ is (i) hydrocarbon group optionally having substituents, or (ii) heterocyclic group optionally having substituents;
$R^4$ represents hydrogen atom or $C_{1-6}$ alkyl;
$R^3$ and $R^4$, taken together with the adjacent nitrogen atom, may form a nitrogen-containing heterocyclic ring optionally having substituents.

The "hydrocarbon group" represented by $R^3$ and $R^{3a}$ in the "hydrocarbon group optionally having substituents" include, for example, chain or cyclic hydrocarbon group such as alkyl, alkenyl, alkynyl, cycloalkyl, aryl, aralkyl, etc. Among them, the following $C_{1-19}$ chain or cyclic hydrocarbon groups are preferable:

a) $C_{1-6}$ alkyl (e.g., methyl, ethyl, propyl, isopropyl, butyl, isobutyl, sec-butyl, tert-butyl, pentyl, hexyl, etc.),

b) $C_{2-6}$ alkenyl (e.g., vinyl, allyl, isopropenyl, 2-butenyl, etc.),

c) $C_{2-6}$ alkynyl (e.g., ethynyl, propargyl, 2-butynyl, etc.),

d) $C_{3-6}$ cycloalkyl (e.g., cyclopropyl, cyclobutyl, cyclopentyl, cyclohexyl, etc.), and $C_{3-6}$ cycloalkyl being optionally condensed with one benzene ring,

e) $C_{6-14}$ aryl (e.g., phenyl, 1-naphthyl, 2-naphthyl, 2-indenyl, 2-anthryl, etc.), preferably phenyl,

f) $C_{7-19}$ aralkyl (e.g., benzyl, phenethyl, diphenylmethyl, triphenylmethyl, 1-naphthylmethyl, 2-naphthylmethyl, 2,2-diphenylethyl, 3-phenylpropyl, 4-phenylbutyl, 5-phenylpentyl, etc.), preferably benzyl.

The "substituent" in the "hydrocarbon group optionally having substituents" includes, for example, halogen atoms, (e.g., fluorine, chlorine, bromine, iodine, etc.), $C_{1-3}$ alkylenedioxy (e.g., methylenedioxy, ethylenedioxy, etc.), nitro, cyano, optionally halogenated $C_{1-6}$ alkoxy, optionally halogenated $C_{1-6}$ alkylthio, hydroxy, amino, mono-$C_{1-6}$ alkylamino (e.g., methylamino, ethylamino, propylamino, isopropylamino, butylamino, etc.), di-$C_{1-6}$ alkylamino (e.g., dimethylamino, diethylamino, dipropylamino, dibutylamino, ethylmethylamino, etc.), 5- to 7-membered saturated cyclic amino optionally having substituents, formyl, carboxy, carbamoyl, thiocarbamoyl, optionally halogenated $C_{1-6}$ alkyl-carbonyl, $C_{1-6}$ alkoxy-carbonyl (e.g., methoxycarbonyl, ethoxycarbonyl, propoxycarbonyl, tert-butoxycarbonyl, etc.), $C_{6-14}$ aryl-carbonyl (e.g., benzoyl, 1-naphthoyl, 2-naphthoyl, etc.), 5- or 6-membered heterocyclic carbonyl (e.g., nicotinoyl, isonicotinoyl, 2-thenoyl, 3-thenoyl, 2-furoyl, 3-furoyl, morpholinocarbonyl, piperidinocarbonyl, pyrrolidin-1-ylcarbonyl, etc.), $C_{6-14}$ aryloxy-carbonyl (e.g., phenyloxycarbonyl, 1-naphthyloxycarbonyl, 2-naphthyloxycarbonyl, etc.), $C_{7-19}$ aralkyloxy-carbonyl (e.g., benzyloxycarbonyl, phenethyloxycarbonyl, diphenylmethyloxycarbonyl, triphenylmethyloxycarbonyl, 1-naphthylmethyloxycarbonyl, 2-naphthylmethyloxycarbonyl, 2,2-diphenylethyloxycarbonyl, 3-phenylpropyloxycarbonyl, 4-phenylbutyloxycarbonyl, 5-phenylpentyloxycarbonyl, etc.), mono-$C_{1-6}$ alkyl-carbamoyl (e.g., methylcarbamoyl, ethylcarbamoyl, etc.), di-$C_{1-6}$ alkyl-carbamoyl (e.g., dimethylcarbamoyl, diethylcarbamoyl, ethylmethylcarbamoyl, etc.), $C_{6-14}$ aryl-carbamoyl (e.g., phenylcarbamoyl, etc.), 5- or 6-membered heterocyclic carbamoyl (e.g., morpholinocarbamonyl, piperidinocarbamoyl, 2-pyridylcarbamoyl, 3-pyridylcarbamoyl, 4-pyridylcarbamoyl, 2-thienylcarbamoyl, 3-thienylcarbamoyl, etc.), optionally halogenated $C_{1-6}$ alkylsulfonyl, $C_{6-14}$ arylsulfonyl (e.g., phenylsulfonyl, 1-naphthylsulfonyl, 2-naphthylsulfonyl, etc.), formylamino, optionally halogenated $C_{1-6}$ alkyl-carboxamide, $C_{6-14}$ aryl-carboxamide (e.g., phenylcarboxamide, naphthylcarboxamide, etc.), $C_{1-6}$ alkoxy-carboxamide (e.g., methoxycarboxamide, ethoxycarboxamide, propoxycarboxamide, butoxycarboxamide, etc.), $C_{1-6}$ alkylsulfonylamino (e.g., methylsulfonylaminol, ethylsulfonylamino, etc.), $C_{1-6}$ alkyl-carbonyloxy (e.g., acetoxy, propanoyloxy, etc.), $C_{6-14}$ aryl-carbonyloxy (e.g., benzoyloxy, 1-naphthoyloxy, 2-naphthoyloxy, etc.), $C_{1-6}$ alkoxy-carbonyloxy (e.g., methoxycarbonyloxy, ethoxycarbonyloxy, propoxycarbonyloxy, butoxycarbonyloxy, etc.), mono-$C_{1-6}$ alkyl-carbamoyloxy (e.g., methylcarbamoyloxy, ethylcarbamoyloxy, etc.), di-$C_{1-6}$ alkyl-carbamoyloxy (e.g., dimethylcarbamoyloxy, diethylcarbamoyloxy, etc.), $C_{6-14}$ aryl-carbamoyloxy (e.g., phenylcarbamoyloxy, naphthylcarbamoyloxy, etc.), 5- or 6-membered heterocyclic carbonyloxy (e.g., nicotinoyloxy, etc.), $C_{6-14}$ aryloxy (e.g., phenoxy, naphthoxy, etc.), etc. The number of the substituents is, for example, 1 to 5, preferably, 1 to 3. When the number of the substituents is 2 or more, these substituents may be the same or different.

The "optionally halogenated $C_{1-6}$ alkoxy", "optionally halogenated $C_{1-6}$ alkylthio", "optionally halogenated $C_{1-6}$ alkyl-carbonyl", "optionally halogenated $C_{1-6}$ alkylsulfonyl" and "optionally halogenated $C_{1-6}$ alkyl-carboxamide" are exemplified by those mentioned as the "substituent" in the above "$C_{1-6}$ alkyl optionally having substituents".

The "5- to 7-membered saturated cyclic amino optionally having substituents" is exemplified by those mentioned as the "substituent" in the above "aromatic group optionally having substituents".

The "heterocyclic group" in the "heterocyclic group optionally having substituents" represented by $R^3$ or $R^{3a}$ includes, for example, a monovalent group derived by removing an optional hydrogen atom from 5- to 14-membered (monocyclic, di- or tri-cyclic) heterocyclic rings containing, in addition to carbon atoms, 1 to 4 of 1 or 2 kinds of heteroatoms selected from the group consisting of nitrogen, sulfur and oxygen atoms, etc., preferably (i) aromatic heterocyclic rings, (ii) 5- to 10-membered non-aromatic heterocyclic rings, and (iii) 7- to 10-membered bridged heterocyclic rings.

The "aromatic heterocyclic ring" includes, for example, 5- to 14-membered, preferably 5- to 10-membered aromatic heterocyclic rings containing, in addition to carbon atoms, one or more (e.g., 1 to 4) heteroatoms selected from the group consisting of nitrogen, sulfur and oxygen atoms, etc. Concretely mentioned is an aromatic heterocyclic ring such as thiophene, furan, pyrrole, imidazole, pyrazole, thiazole, isothiazole, oxazole, isooxazole, pyridine, pyrazine, pyrimidine, pyridazine, 1,2,4-oxadiazole, 1,3,4-oxadiazole, 1,2,4-thiadiazole, 1,3,4-thiadiazole, furazan, benzothiophene, benzofuran, benzimidazole, benzoxazole, benzothiazole, benzisothiazole, naphtho[2,3-b]thiophene, phenoxathine, indole, isoindole, 1H-indazole, purine, 4H-quinolidine, isoquinoline, quinoline, phthalazine, naphthyridine, quinoxaline, quinazoline, cinnoline, carbazole, β-carboline, phenanthridine, acridine, phenazinephenothiazine, phenoxazine, phthalimide, etc.; and a ring as formed through condensation of the above ring, preferably monocyclic ring, with one or more, preferably one or two aromatic rings (e.g., benzene ring, etc.), etc.

The above-mentioned "5- to 10-membered non-aromatic heterocyclic rings" includes, for example, 2- or 3-pyrroline, pyrrolidine, 2- or 3-imidazoline, 2-oxazoline, oxazolidine, 2- or 3-pyrazoline, pyrazolidine, 2-thiazoline, piperidine, piperazine, hexamethyleneimine, morpholine, thiomorpholine, etc.

The above-mentioned "7- to 10-membered bridged heterocyclic ring" includes, for example, quinuclidine, 7-azabicyclo[2.2.1]heptane, etc.

Said "heterocyclic group" is preferably 5- to 10-membered (monocyclic or dicyclic) heterocyclic groups containing, in addition to carbon atoms, preferably 1 to 4 of 1 or 2 kinds of heteroatoms selected from the group consisting of nitrogen, sulfur and oxygen atoms, etc. Concretely mentioned are aromatic heterocyclic groups such as 2- or 3-thienyl; 2-, 3- or 4-pyridyl; 2- or 3-furyl; 2-, 4- or 5-thiazolyl; 2-, 4- or 5-oxazolyl; 1-3- or 4-pyrazolyl; 2-pyrazinyl, 2-, 4- or 5-pyrimidinyl; 1-, 2- or 3-pyrrolyl; 1-, 2- or 4-imidazolyl; 3- or 4-pyridazinyl; 3-isothiazolyl; 3-isooxazolyl; 1,2,4-oxadiazol-5-yl; 1,2,4-oxadiazol-3-yl; 2-, 3-, 4-, 5- or 8-quinolyl; 1-, 3-, 4-, 5-, 6-, 7-, or 8-isoquinolyl; 1-, 2-, 3-, 4-, 5-, 6- or 7-indolyl; 1-, 2-, 4- or 5-isoindolyl; 1-, 5- or 6-phthalazinyl; 2-, 3- or 5-quinoxalinyl; 2-, 3-, 4-, 5- or 6-benzofuranyl; 2-, 3-, 4-, 5- or 6-benzothienyl; 2-, 4-, 5- or 6-benzothiazolyl; 1-, 2-, 4-, 5- or 6-benzimidazolyl; etc; non-aromatic heterocyclic group such as 1-, 2- or 3-pyrrolidinyl; 1-, 2- 4- or 5-imidazolidinyl; 2- or 4-imidazolinyl; 2-, 3- or 4-pyrazolydinyl; piperidino; 2-, 3- or 4-piperidyl; 1- or 2-piperazinyl; morpholino; thiomorpholino; etc.

The "substituent" in the "heterocyclic group optionally having substituents" is exemplified by those mentioned as the "substituent" in the above "$C_{6-14}$ aryl optionally having substituents". The number of the substituents is, for example, 1 to 5, preferably, 1 to 3. When the number of the substituents is 2 or more, these substituents may be the same or different.

The "$C_{1-6}$ alkyl" represented by $R^4$ include, for example, methyl, ethyl, propyl, isopropyl, butyl, isobutyl, sec-butyl, tert-butyl, pentyl, hexyl, etc.

For the "nitrogen-containing heterocyclic ring optionally having substituents" formed by $R^3$ and $R^4$ together with the adjacent nitrogen atom, those similar to the nitrogen-containing heterocyclic ring optionally having substituents formed by $R^1$ and $R^2$ as mentioned above can be used.

Said "acyl" is, preferably, formyl, carboxy, carbamoyl, optionally halogenated $C_{1-6}$ alkyl-carbonyl (e.g., acetyl, etc.), $C_{1-6}$ alkoxy-carbonyl (e.g., methoxycarbonyl, ethoxycarbonyl, propoxycarbonyl, tert-butoxycarbonyl, etc.), $C_{6-14}$ aryl-carbonyl optionally having substituents (e.g., benzoyl, 1-naphthoyl, 2-naphthoyl, etc.), $C_{6-14}$ aryloxy-carbonyl optionally having substituents (e.g., phenoxycarbonyl, etc.), $C_{7-19}$ aralkyloxy-carbonyl optionally having substituents (e.g., benzyloxycarbonyl, phenethyloxycarbonyl, etc.), 5- or 6-membered heterocyclic carbonyl optionally having substituents (e.g., nicotinoyl, isonicotinoyl, 2-thenoyl, 3-thenoyl, 2-furoyl, 3-furoyl, morpholinocarbonyl, piperidinocarbonyl, pyrrolidin-1-ylcarbonyl, etc.), mono-$C_{1-6}$ alkyl-carbamoyl (e.g., methylcarbamoyl, ethylcarbamoyl, etc.), di-$C_{1-6}$ alkyl-carbamoyl (e.g, dimethylcarbamoyl, diethylcarbamoyl, ethylmethylcarbamoyl, etc.), $C_{6-14}$ aryl-carbamoyl optionally having substituents (e.g., phenylcarbamoyl, 1-naphthylcarbamoyl, 2-naphthylcarbamoyl, etc.), 5- or 6-membered heterocyclic carbamoyl optionally having substituents (e.g., 2-pyridylcarbamoyl, 3-pyridylcarbamoyl, 4-pyridylcarbamoyl, 2-thienylcarbamoyl, 3-thienylcarbamoyl, etc.), optionally halogenated $C_{1-6}$ alkylsulfonyl (e.g., methylsulfonyl, etc.), $C_{6-14}$ arylsulfonyl optionally having substituents, sulfamoyl, etc. and more preferably, optionally halogenated $C_{1-6}$ alkyl-carbonyl, $C_{1-6}$ alkoxy-carbonyl, $C_{6-14}$ aryl-carbonyl optionally having substituents, $C_{6-14}$ arylsulfonyl optionally having substituents (e.g., benzenesulfonyl, 1-naphthalenesulfonyl, 2-naphthalenesulfonyl, etc.), etc.

Furthermore, the "substituent" in the "$C_{6-14}$ aryl-carbonyl optionally having substituents", "$C_{6-14}$ aryloxy-carbonyl optionally having substituents", "$C_{7-19}$ aralkyloxy-carbonyl optionally having substituents", "5-or 6-membered heterocyclic carbonyl optionally having substituents", "$C_{6-14}$ aryl-carbamoyl optionally having substituents", "5- or 6-membered heterocyclic carbamoyl optionally having substituents" and "$C_{6-14}$ arylsulfonyl optionally having substituents" is exemplified by those mentioned as the "substituent" in the above "$C_{6-14}$ aryl optionally having substituents". The number of the substituents is, for example, 1 to 5, preferably, 1 to 3. When the number of the substituents is 2 or more, these substituents may be the same or different.

The above-mentioned "acylamino" includes, for example, amino which is substituted by 1 or 2 of the above-mentioned "acyl" and preferably, acylamino represented by the formula: $-NR^5-COR^6$, $-NR^5-COOR^{6a}$, $-NR^5-SO_2R^{6a}$ or $-NR^5-CONR^{6a}R^{6b}$,

wherein, $R^5$ represents hydrogen atoms or $C_{1-6}$ alkyl; $R^6$ has the same meanings as the above $R^3$; $R^{6a}$ has the same meanings as the above $R^{3a}$; $R^{6b}$ has the same meanings as the above $R^4$; etc.

For the "$C_{1-6}$ alkyl" represented by $R^5$, those similar to the "$C_{1-6}$ alkyl" represented by $R^4$ as mentioned above can be used.

Said "acylamino" is, preferably, formylamino, optionally halogenated $C_{1-6}$ alkyl-carboxamide (e.g., acetylamino), $C_{6-14}$ aryl-carboxamide optionally having substituents (e.g., phenylcarboxamide, naphthylcarboxamide, etc.), optionally halogenated $C_{1-6}$ alkoxy-carboxamide (e.g., methoxycarboxamide, ethoxycarboxamide, propoxycarboxamide, butoxycarboxamide, etc.), optionally halogenated $C_{1-6}$ alkylsulfonylamino (e.g., methylsulfonylamino, ethylsulfonylamino, etc.), etc.

Furthermore, the "substituent" in the "$C_{6-14}$ aryl-carboxamide optionally having substituents" is exemplified by those mentioned as the "substituent" in the above "$C_{6-14}$ aryl optionally having substituents". The number of the

substituents is, for example, 1 to 5, preferably, 1 to 3. When the number of the substituents is 2 or more, these substituents may be the same or different.

The above-mentioned "acyloxy" includes, for example, oxy which is substituted by one of the above-mentioned "acyl", and preferably, acyloxy represented by the formula: -O-COR$^7$, -O-COOR$^7$, -O-CONHR$^7$,

wherein, $R^7$ has the same meanings as the above-mentioned $R^3$; etc.

Said "acyloxy" is preferably, $C_{1-6}$ alkyl-carbonyloxy (e.g., acetoxy, propanoyloxy, isobutanoyloxy, pivaloyloxy, etc.), $C_{6-14}$ aryl-carbonyloxy optionally having substituents (e.g., benzoyloxy, 1-naphthoyloxy, 2-naphthoyloxy, etc.), optionally halogenated $C_{1-6}$ alkoxy-carbonyloxy (e.g., methoxycarbonyloxy, trifluoromethoxycarbonyloxy, ethoxycarbonyloxy, propoxycarbonyloxy, butoxycarbonyloxy, etc.), mono-$C_{1-6}$ alkyl-carbamoyloxy (e.g, methylcarbamoyloxy, ethylcarbamoyloxy, etc.), di-$C_{1-6}$ alkyl-carbamoyloxy (e.g.,dimethylcarbamoyloxy, diethylcarbamoyloxy, etc.), $C_{6-14}$ aryl-carbamoyloxy optionally having substituents (e.g., phenylcarbamoyloxy, naphthylcarbamoyloxy, etc.), nicotinoyloxy, etc.

Furthermore, the "substituent" in the "$C_{6-14}$ aryl-carbonyloxy optionally having substituents" and "$C_{6-14}$ aryl-carbamoyloxy optionally having substituents" is exemplified by those mentioned as the "substituent" in the above "$C_{6-14}$ aryl optionally having substituents". The number of the substituents is, for example, 1 to 5, preferably, 1 to 3. When the number of the substituents is 2 or more, these substituents may be the same or different.

The "$C_{3-9}$ cycloalkyl group" in the "$C_{3-9}$ cycloalkyl group optionally having substituents" represented by Ar includes, for example, cyclopropyl, cyclobutyl, cyclopentyl, cyclohexyl, cycloheptyl, cyclooctyl, cyclononyl, etc.

The "substituent" in said "$C_{3-9}$ cycloalkyl group optionally having substituents" includes, for example, oxo, optionally halogenated $C_{1-6}$ alkyl, halogen atom, $C_{1-3}$ alkylenedioxy, nitro, cyano, optionally halogenated $C_{3-6}$ cycloalkyl, optionally halogenated $C_{1-6}$ alkoxy, optionally halogenated $C_{1-6}$ alkylthio, hydroxy, amino, mono- $C_{1-6}$ alkylamino, di-$C_{1-6}$ alkylamino, formyl, carboxy, carbamoyl, thiocarbamoyl, optionally halogenated $C_{1-6}$ alkyl-carbonyl, $C_{1-6}$ alkoxy-carbonyl, mono-$C_{1-6}$ alkyl-carbamoyl, di-$C_{1-6}$ alkyl-carbamoyl, optionally halogenated $C_{1-6}$ alkylsulfonyl, formylamino, optionally halogenated $C_{1-6}$ alkyl-carboxamide, $C_{1-6}$ alkoxy-carboxamide, $C_{1-6}$ alkylsulfonylamino, $C_{1-6}$ alkyl-carbonyloxy, $C_{1-6}$ alkoxy-carbonyloxy, mono-$C_{1-6}$ alkyl-carbamoyloxy, di-$C_{1-6}$ alkyl-carbamoyloxy, etc.

Said "optionally halogenated $C_{1-6}$ alkyl" is exemplified by those mentioned as the "substituent" in the "nitrogen-containing heterocyclic ring optionally having substituents" formed by $R^1$ and $R^2$ together with the adjacent nitrogen atom.

The above-mentioned "halogen atom", "$C_{1-3}$ alkylenedioxy", "optionally halogenated $C_{3-6}$ cycloalkyl", "optionally halogenated $C_{1-6}$ alkoxy", "optionally halogenated $C_{1-6}$ alkylthio", "mono-$C_{1-6}$ alkylamino", "di-$C_{1-6}$ alkylamino", "optionally halogenated $C_{1-6}$ alkyl-carbonyl", "$C_{1-6}$ alkoxy-carbonyl", "mono-$C_{1-6}$ alkyl-carbamoyl", "di-$C_{1-6}$ alkyl-carbamoyl", "optionally halogenated $C_{1-6}$ alkylsulfonyl", "optionally halogenated $C_{1-6}$ alkyl-carboxamide", "$C_{1-6}$ alkoxy-carboxamide", "$C_{1-6}$ alkylsulfonylamino", "$C_{1-6}$ alkyl-carbonyloxy", "$C_{1-6}$ alkoxy-carbonyloxy", "mono-$C_{1-6}$ alkyl-carbamoyloxy" and "di-$C_{1-6}$ alkyl-carbamoyloxy" are exemplified by those mentioned as the "substituent" in the "$C_{1-6}$ alkyl optionally having substituents" represented by $R^1$ and $R^2$.

The number of the substituents is, for example, 1 to 5, preferably, 1 to 3. When the number of the substituents is 2 or more, these substituents may be the same or different.

The preferable examples of the "$C_{3-9}$ cycloalkyl group optionally having substituents" include, for example, cyclopentyl, cyclohexyl, 4,4-dimethylcyclohexyl, 4-oxocyclohexyl, etc.

The "3- to 9-membered saturated heterocyclic group" in the "3- to 9-membered saturated heterocyclic group optionally having substituents" represented by Ar includes, for example, 3- to 9-membered saturated nitrogen-containing heterocyclic groups containing, in addition to carbon atoms, one or more (e.g., 1 to 3) heteroatoms selected from the group consisting of oxygen, nitrogen and sulfur atoms, etc. Concretely mentioned are tetrahydrofuranyl, tetrahydropyranyl, pyrrolidinyl, piperidinyl, morpholinyl, thiomorpholinyl, tetrahydrothienyl, tetrahydrothiopyranyl, etc.

For the "substituent" in said "3- to 9-membered saturated heterocyclic group optionally having substituents", those similar to the substituent in the "$C_{3-9}$ cycloalkyl group optionally having substituents" as mentioned above can be used.

The number of the substituents is, for example, 1 to 5, preferably, 1 to 3. When the number of the substituents is 2 or more, these substituents may be the same or different.

The preferable examples of the "3- to 9-membered saturated heterocyclic group optionally having substituents" include, 2- or 3-tetrahydrofuranyl; 2-, 3-or 4-tetrahydropyranyl; 1-, 2- or 3-pyrrolidinyl; 1-, 2-, 3- or 4-piperidinyl; etc.

The "halogen atom" represented by Ar includes, for example, fluorine, chlorine, bromine, iodine, etc.

Ar represents, preferably, "aromatic group optionally having substituents", more preferably, "monocyclic aromatic group optionally having substituents" or "fused aromatic group optionally having substituents".

The "monocyclic aromatic group" in the "monocyclic aromatic group optionally having substituents" includes, preferably, phenyl, 2- or 3-thienyl, or 2-, 3- or 4-pyridyl.

Moreover, "fused aromatic group" in the "fused aromatic group optionally having substituents" includes, preferably, fused polycyclic aromatic heterocyclic group, more preferably, 2-benzothienyl, 2-benzofuranyl, indol-2-yl, and

indol-3-yl.

The "substituent" in the "monocyclic aromatic group optionally having substituents" and "fused aromatic group optionally having substituents" includes, preferably, 1 or 2 substituents selected from halogen atoms, optionally halogenated $C_{1-6}$ alkyl and optionally halogenated $C_{1-6}$ alkoxy.

The most preferably, Ar represents phenyl, indol-2-yl or indol-3-yl, each of which may have 1 or 2 substituents selected from halogen atoms, optionally halogenated $C_{1-6}$ alkyl and optionally halogenated $C_{1-6}$ alkoxy.

Above all, preferred are phenyl and indol-2-yl, each of which may have 1 or 2 substituents selected from halogen atoms, optionally halogenated $C_{1-6}$ alkyl and optionally halogenated $C_{1-6}$ alkoxy.

The preferable examples of compound (I), (I') or (I'') of the present invention include the following compounds:

1) a compound:
wherein either X or X' represents fluorine atoms and the other represents hydrogen atoms;

$R^1$ and $R^2$ each represent $C_{1-6}$ alkyl (preferably, methyl);
Y represents a bond or $C_{1-2}$ alkylene;
Q represents a bond,$-(CH_2)_{w1}CO(CH_2)_{w2}-$ or $-(CH_2)_{w3}COO(CH_2)_{w4}-$
(wherein the symbols have the same meanings as above);
$\underline{...}$ represents a single bond;
$T^1$ represents CH, $T^2$ represents N; and
Ar represents monocyclic aromatic group (preferably, phenyl, 2- or 3-thienyl, 2-, 3- or 4-pyridyl) or fused aromatic group (preferably, 2-benzothienyl, 2-benzofuranyl, indol-2-yl, indol-3-yl), each of which may have 1 or 2 substituents selected from halogen atoms, optionally halogenated $C_{1-6}$ alkyl and optionally halogenated $C_{1-6}$ alkoxy,

2) a compound:
wherein X represents chlorine atom, X' represents hydrogen atoms;

$R^1$ and $R^2$ each represent $C_{1-6}$ alkyl (preferably, methyl) ;
Y represents $C_{1-2}$ alkylene;
Q represents a bond,$-(CH_2)_{w1}CO(CH_2)_{w2}-$ or $-(CH_2)_{w3}COO(CH_2)_{w4}-$ (wherein the symbols have the same meanings as above);
$\underline{...}$ represents a single bond;
$T^1$ represents CH, $T^2$ represents N; and
Ar represents monocyclic aromatic group (preferably, phenyl, 2- or 3-thienyl, 2-, 3- or 4-pyridyl) or fused aromatic group (preferably, 2-benzothienyl, 2-benzofuranyl, indol-2-yl, indol-3-yl), each of which may have 1 or 2 substituents selected from halogen atoms, optionally halogenated $C_{1-6}$ alkyl and optionally halogenated $C_{1-6}$ alkoxy,

3) a compound:
wherein X represents hydrogen atom, X' represents chlorine atom;

$R^1$ and $R_2$ each represents $C_{1-6}$ alkyl (preferably, methyl) ;
Y represents a bond or $C_{1-2}$ alkylene;
Q represents a bond,$-(CH_2)_{w1}CO(CH_2)_{w2}-$ or $-(CH_2)_{w3}COO(CH_2)_{w4}-$ (wherein the symbols have the same meanings as above);
$\underline{...}$ represents a single bond;
$T^1$ represents CH, $T^2$ represents N; and
Ar represents monocyclic aromatic group (preferably, phenyl, 2- or 3-thienyl, 2-,3- or 4-pyridyl) or fused aromatic group (preferably, 2-benzothienyl, 2-benzofuranyl, indol-2-yl, indol-3-yl), each of which may have 1 or 2 substituents selected from halogen atoms, optionally halogenated $C_{1-6}$ alkyl and optionally halogenated $C_{1-6}$ alkoxy,

4) a compound:
wherein X represents chlorine atom, X' represents hydrogen atom;

$R^1$ and $R^2$ each represents $C_{1-6}$ alkyl (preferably, methyl);
Y represents a bond;
Q represents a bond,$-(CH_2)_{w1}CO(CH_2)_{w2}-$ or $-(CH_2)_{w3}COO(CH_2)_{w4}-$ (wherein the symbols have the same

meanings as above);
... represents a single bond;
$T^1$ represents CH, $T^2$ represents N; and
Ar represents monocyclic aromatic group (preferably, phenyl, 2- or 3-thienyl, 2-,3- or 4-pyridyl) or fused aromatic group (preferably, 2-benzothienyl, 2-benzofuranyl, indol-2-yl, indol-3-yl), each of which may have 1 or 2 substituents selected from halogen atoms, optionally halogenated $C_{1-6}$ alkyl and optionally halogenated $C_{1-6}$ alkoxy,

5) a compound:
wherein X represents chlorine atom, X' represents hydrogen atom;

$R^1$ and $R^2$ each represents $C_{1-6}$ alkyl (preferably, methyl);
Y represents a bond;
Q represents a bond,$-(CH_2)_{w1}CO(CH_2)_{w2}-$ or $-(CH_2)_{w3}COO(CH_2)_{w4}-$
(wherein the symbols have the same meanings as above) ;
... represents a single bond;
$T^1$ represents CH, $T^2$ represents N; and
Ar represents monocyclic aromatic group (preferably, phenyl, 2- or 3-thienyl, 2-,3- or 4-pyridyl) or fused aromatic group (preferably, 2-benzothienyl, 2-benzofuranyl, indol-2-yl, indol-3-yl), each of which may have 1 or 2 substituents selected from halogen atoms, optionally halogenated $C_{1-6}$ alkyl and optionally halogenated $C_{1-6}$ alkoxy,

6) a compound of the formula:

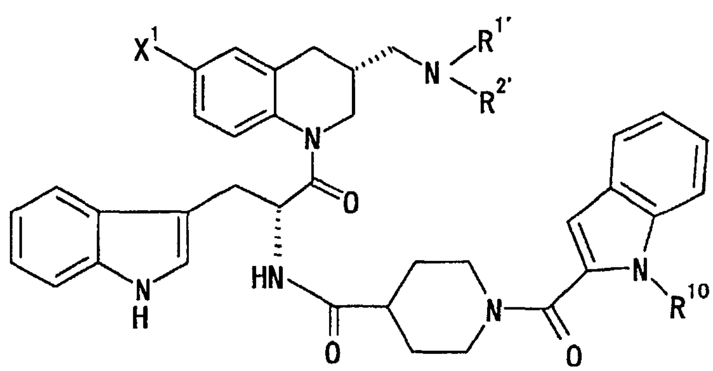

or

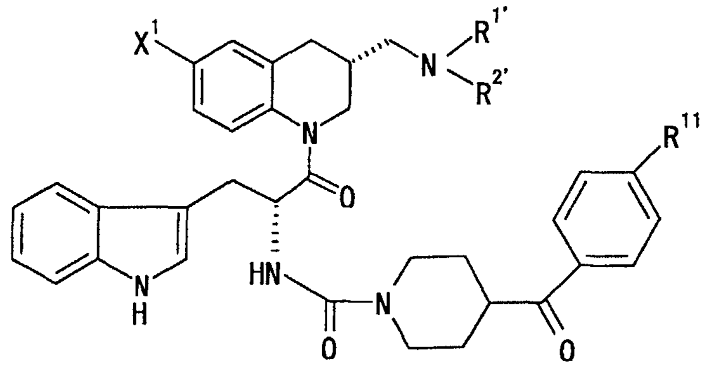

wherein, $X^1$ represents hydrogen atom, fluorine atom, chlorine atom or amino optionally having substituents;

$R^{1'}$ and $R^{2'}$ represent hydrogen atom or $C_{1-6}$ alkyl;
$R^{10}$ represents $C_{1-6}$ alkyl; and

$R^{11}$ represents halogen atoms.

The amino optionally having substituents represented by X' has the same meanings as the above-mentioned amino optionally having substituents represented by X. The $C_{1-6}$ alkyl represented by $R^{1'}$, $R^{2'}$ and $R^{10}$ has the same meaning as the above-mentioned $C_{1-6}$ alkyl represented by $R^1$. The halogen atoms represented by $R^{11}$ include fluorine atom, chlorine atom, etc. Above all, preferably, $X^1$ represents chlorine atom, $R^{1'}$ and $R^{2'}$ represent $C_{1-3}$ alkyl (e.g., methyl, ethyl, etc. and especially, methyl), $R^{10}$ represents $C_{1-3}$ alkyl (e.g., methyl, ethyl, etc. and especially, methyl) and $R^{11}$ represents halogen atom (especially, chlorine atoms),

7) N-[(1R)-2-[(3R)-6-chloro-3-[(dimethylamino)-methyl]-1,2,3,4-tetrahydro-1-quinolinyl]-1-(1-indol-3-yl-methyl)-2-oxoethyl]-1-(1-methylindol-2-ylcarbonyl)-4-piperidinecarboxamide (Example 51),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-1-(3-isoquinolylcarbonyl)-4-piperidinecarboxamide (Example 118),
N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-(4-fluorobenzoyl)-1-piperidinecarboxamide (Example 129),
4-(4-chlorobenzoyl)-N-[(1R)-2-[(3 R)-6-chloro-3-[(dimethylamino)methyl]-1,2,3,4-tetrahydro-1-quinolinyl]-1-(1-indol-3-ylmethyl)-2-oxoethyl]-1-piperidinecarboxamide (Example 130),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-(4-chlorophenoxy)-1-piperidinecarboxamide (Example 142),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-phenoxy-1-piperidinecarboxamide (Example 145),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-[(4-fluorophenyl)sulfonyl]-1-piperidinecarboxamide (Example 148),
N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-[(4-chlorophenyl)sulfonyl]-1-piperidinecarboxamide (Example 150),
3-(1-benzoyl-4-piperidinyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-propanamide (Example 31),
2-[(1-benzoyl-4-piperidinyl)oxy]-N-[(1R)-2-[(3R)-6-chloro-3-[dimethylamino]methyl]-1,2,3,4-tetrahydro-1-quinolinyl]-1-(1-indol-3-ylmethyl)-2-oxoethyl]acetamide (Example 33), or a salt thereof.

As the salts of compound (I), (I') or (I''), for example, inorganic salts, ammonium salts, salts with organic bases, salts with inorganic acids, salts with organic acids and salts with basic or acidic amino acids can be mentioned. Preferable examples of inorganic salts include alkali metal salts such as sodium salt and potassium salt, etc; alkaline earth metal salts such as calcium salts, magnesium salts and barium salts, etc; aluminum salts, etc. Preferred salts with organic bases are exemplified by salts with trimethylamine, triethylamine, pyridine, picoline, ethanolamine, diethanolamine, triethanolamine, dicyclohexylamine, N,N'-dibenzylethylenediamine, etc. Preferred salts with inorganic acids are exemplified by salts with hydrochloric acid, hydrobromic acid, nitric acid, sulfuric acid, phosphoric acid, etc. Preferred salts with organic acids are exemplified by salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc. Preferred salts with basic amino acids are exemplified by salts with arginine, lysine, ornithine, etc. Preferred salts with acidic amino acids are exemplified by salts with aspartic acid, glutamic acid, etc.

Among these, pharmaceutically acceptable salts are preferable. Preferable examples include, when compound (I), (I') or (I'') has an acidic functional group, inorganic salts such as alkali metal salts (e.g., sodium salt, potassium salt, etc.), alkaline earth metal salts (e.g., calcium salt, magnesium salt, barium salt, etc.) and ammonium salts, etc; and when compound of the present invention has a basic functional group, inorganic salts such as hydrochloride, sulfate, phosphate and hydrobromide, or, organic salts such as acetate, maleate, fumarate, succinate, methanesulfonate, p-toluenesulfonate, citrate and tartarate.

The prodrugs of the compound (I), (I'), (I'') or salts thereof (hereinafter abbreviated as the compound of the present invention) means a compound which is converted into the compound of the present invention through a reaction due to an enzyme, a gastric acid, etc. under the physiological condition in the living body, that is, a compound which is enzymatically converted into the compound of the present invention with oxidation, reduction, hydrolysis, etc.; a compound which is converted into the compound of the present invention with gastric acid, etc.; etc. Examples of the prodrug of the compound of the present invention include a compound wherein an amino group of the compound of the present invention is substituted with acyl, alkyl, phosphoric acid, etc. (e.g., a compound wherein an amino group of the compound of the present invention is substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc.); a compound wherein a hydroxy group of the compound of the present invention is substituted with acyl, alkyl, phosphoric acid, boric acid, etc. (e.g., a compound wherein a hydroxy group the compound of the present invention is substituted

with acetyl, palmitoyl, propanoyl, pivaloyl, succinyl, fumaryl, alanyl, dimethylaminomethylcarbonyl, etc.); a compound wherein a carboxyl group of the compound of the present invention is modified with ester, amide, etc. (e.g., a compound wherein a carboxyl group of the compound of the present invention is modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); etc. These compounds can be produced by *per se* known methods from the compound of the present invention.

The prodrug of the compound of the present invention may be a compound which is converted into the compound of the present invention under the physiological conditions as described in "Pharmaceutical Research and Development", Vol. 7 (Drug Design), pages 163-198 published in 1990 by Hirokawa Publishing Co. (Tokyo, Japan).

Process for producing the compound (I) is mentioned below.

The compound (I) can be produced by per *se* known means, for example, by the methods exemplified by the following schemes or a similar method thereto, etc.

Compounds described in the following schemes may be in the form of salts. These salts are exemplified by those similar to the salts of the compound (I).

"Room temperature" is normally meant to indicate a temperature falling between 0°C and 30°C in the present specification.

The following reaction such as alkylation, hydrolysis, amination, esterification, amidation, etherification, oxidation, reduction, urea reaction, etc. may be conducted according to *per se* known methods, for example, those described in Organic Functional Group Preparations, 2nd Ed., Academic Press Inc., 1989 and in Comprehensive Organic Transformations, VCH Publishers Inc., 1989. or a similar method thereto.

[scheme 1]

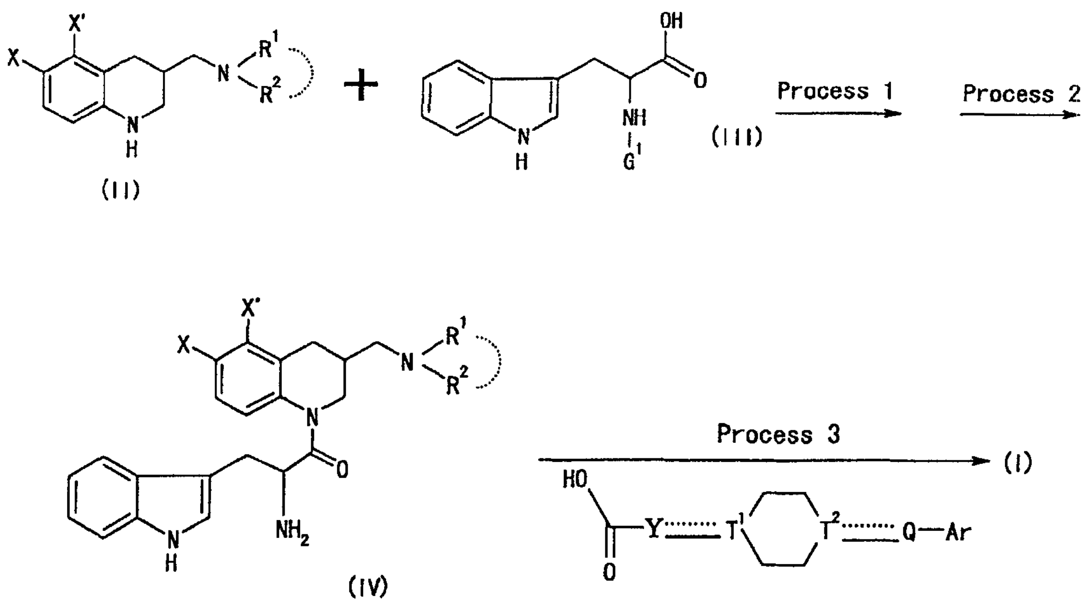

(II) (III) (IV) (I)

wherein, $G^1$ represents the protective group of amino group (e.g., 9-fluorenylmethoxycarbonyl, etc.) and the other symbols have the same meanings as above.

The protective group of amino group represented by $G^1$ includes the same protective group as those for amino group which will be later described. Among those, preferred is 9-fluorenylmethoxycarbonyl, etc.

Process 1: amidation

Said "amidation" includes, for example, the below mentioned method such as i) the method using a dehydrating/condensing agent, ii) the method in which carboxy is converted into the reactive derivative and then, condensed.

i) The method using a dehydrating/condensing agent

Compound (II), about 1 to about 5 equivalents of Compound (III), and about 1 to about 2 equivalents of a dehydrating/consensing agent are reacted in an inert solvent under room temperature for about 10 to 24 hours.
Said "dehydrating/condensing agent" includes, for example, dicyclohexylcarbodiimide (DCC), 1-ethyl-3-(3-dimethylaminopropyl)carbodiimide hydrochloride (WSC), etc. Among those, WSC is preferred.
The "inert solvent" includes, for example, nitriles, amides, halogenated hydrocarbons, ethers, etc., which may be used as a mixture of two or more species. Among those, preferred is acetonitrile, DMF, dichloromethane, THF, etc.
In the present reaction, about one equivalent to about 1.5 equivalents of 1-hydroxybenzotriazole (HOBt) and/or about one equivalent to 5 equivalents of a base may be added if necessary.
Said base includes, for example;

1) strong bases such as alkali metal or alkaline earth metal hydrides (e.g., lithium hydride, sodium hydride, potassium hydride, calcium hydride, etc.), alkali metal or alkaline earth metal amides (e.g., lithium amide, sodium amide, lithium diisopropylamide, lithium dicyclohexylamide, lithium hexamethyldisilazide, sodium hexamethyldisilazide, potassium hexamethyldisilazide, etc.), alkali metal or alkaline earth metal lower-alkoxides (e.g., sodium methoxide, sodium ethoxide, potassium tert-butoxide, etc.), etc;
2) inorganic bases such as alkali metal or alkaline earth metal hydroxides (e.g., sodium hydroxide, potassium hydroxide, lithium hydroxide, barium hydroxide, etc.), alkali metal or alkaline earth metal carbonates (e.g., sodium carbonate, potassium carbonate, cesium carbonate, etc.), alkali metal or alkaline earth metal hydrogen carbonates (e.g., sodium hydrogen carbonate, potassium hydrogen carbonate, etc.), etc.; and
3) organic bases such as amines exemplified by triethylamine, diisopropylethylamine, N-methylmorpholine, dimethylaminopyridine, DBU (1,8-diazabicyclo[5.4.0]-undec-7-ene), DBN (1,5-diazabicyclo[4.3.0]non-5-ene), etc., basic heterocyclic compounds exemplified by pyridine, imidazole, 2,6-lutidine, etc. Among these, preferred are triethylamine and 4-dimethylaminopyridine, etc.

ii) The method using the reactive derivative of carboxy

The reactive derivative of Compound (III) and about 1 to about 5 equivalents (preferably about 1 to about 3 equivalents) of Compound (II) are reacted in an inert solvent.
The reactive derivatives in the "reactive derivative of Compound (III)" include acid halide (e.g., acid chloride, acid bromide, etc.), mixed acid anhydride (e.g., anhydride with $C_{1-6}$ alkyl carboxylic acid, $C_{6-10}$ aryl carboxylic acid or $C_{1-6}$ alkyl carbonic acid, etc.), active ester (e.g., ester with phenol optionally having substituents, 1-hydroxybenzotriazole or N-hydroxysuccinimide, etc.). The "substituent" in said "phenol optionally having substituents" includes, 1 to 5 of halogen atoms, nitro, optionally halogenated $C_{1-6}$ alkyl or optionally halogenated $C_{1-6}$ alkoxy. The concrete examples of "phenol optionally having substituents" are phenol, pentachlorophenol, pentafluorophenol, p-nitrophenol, etc. The reactive derivative is preferably acid halide.
The "inert solvent" includes, for example, ethers, halogenated hydrocarbons, aromatic solvents, nitriles, amides, ketones, sulfoxides, water, esters, etc., which may be used as a mixture of two or more species. Among these, preferred are tetrahydrofuran (THF), acetonitrile, dichloromethane, chloroform, ethyl acetate, etc.
The reaction temperature may be between about -20°C and about 50°C, preferably at room temperature.
The reaction time falls between about 5 minutes and about 40 hours, preferably about 1 to about 5 hours.
In the present reaction, about 1 to about 10 equivalents, preferably about 1 to about 3 equivalents of a base is used if necessary.
As said "base", those exemplified in the above-mentioned "the method using a dehydrating/condensing agent" are used. Among them, preferred is sodium hydride, potassium carbonate, sodium carbonate, sodium hydroxide, potassium hydroxide, sodium hydrogen carbonate, potassium hydrogen carbonate, triethylamine, pyridine, etc.
In the present reaction, about 0.1 to about 1 equivalent, preferably about 0.1 to about 0.5 equivalents of phase-transfer catalyst is used if necessary.
Said "phase-transfer catalyst" includes, for example, quaternary ammonium salt such as tetrabutylammonium hydrogensulfate, benzyltriethylammonium chloride, etc. Among those, preferred is tetrabutylammonium hydrogensulfate.

Process 2: deprotection

The present reaction is carried out by *per se* known method according to the kind of $G^1$ which is a protective group of amino group.

Process 3: amidation

Compound (I) can be produced according to amidation in the same manner as in Process 1 by reacting Compound (IV) and Compound (V).

Compound of the general formula (I) where $T^2$ is =N-and ... is a single bond can be also produced according to the method represented by Scheme 2 and Scheme 3.

[Scheme 2]

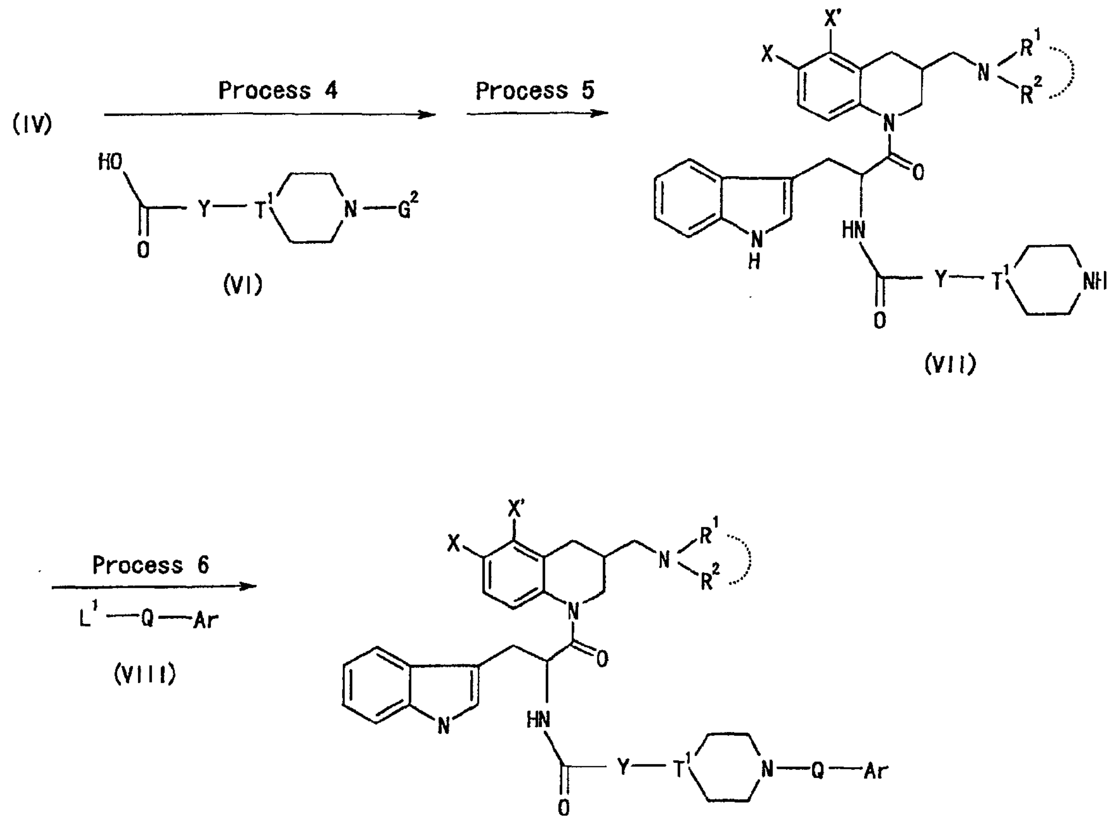


(Ia)

wherein $G^2$ represents a protective group of an amino group (e.g., acetyl, trifluoroacetyl or benzyloxycarbonyl, etc.), $L^1$ represents a leaving group, the other symbols have the same meanings as above.

The "leaving group" represented by $L^1$ includes, for example, (1) halogen atoms (e.g., chlorine, bromine, iodine, etc.), (2) optionally halogenated $C_{1-6}$ alkyl sulfonyloxy (e.g., methane sulfonyloxy, ethane sulfonyloxy, trifluoromethane sulfonyloxy, etc.), (3) $C_{6-10}$ aryl sulfonyloxy optionally having substituents, (4) hydroxy, etc.

The substituent in said "$C_{6-10}$ aryl sulfonyloxy optionally having substituents" includes, for example, 1 to 3 of halogen atoms, optionally halogenated $C_{1-6}$ alkyl, optionally halogenated $C_{1-6}$ alkoxy, etc. The concrete examples of "$C_{6-10}$ aryl sulfonyloxy optionally having substituents" are benzenesulfonyloxy, p-toluene sulfonyloxy, 1-naphthalene sulfonyloxy, 2-naphthalene sulfonyloxy, etc.

Process 4: amidation

According to the same amidation as in the above-mentioned Process 1, Compound (IV) and Compound (VI) are reacted.

Process 5: Deprotection

Compound (VII) can be produced by deprotecting the amide compound obtained in the above-mentioned Process 4. The present reaction can be carried out by *per se* known methods according to the kind of $G^2$ which is a protective group of amino group.
For example, when $G^2$ is trifluoroacetyl, the amide compound obtained in the above-mentioned process 4 is reacted with 1 to 20 equivalents (preferably 1 to 5 equivalents) of base in an inert solvent.
As said "base", those exemplified in the above-mentioned Process 1 are used. The base is, preferably potassium carbonate, sodium carbonate, sodium hydroxide, potassium hydroxide, etc., more preferably, potassium carbonate.
The "inert solvent" includes, for example, alcohols, ethers, halogenated hydrocarbons, aromatic solvents, nitriles, amides, ketones, sulfoxides, water, etc., which may be used as a mixture of two or more species. Among those, preferred are alcohols (e.g., methanol, ethanol, etc.), water or the mixture of these.
For example, when $G^2$ is benzyloxycarbonyl, catalytic reduction is conducted on the amide compound obtained in the above-mentioned Process 4.
Said catalytic reduction is carried out under the presence of a catalyst in an inert solvent under 1 to 100 torr (preferably 1 to 5 torr) of the hydrogen pressure.
The "catalyst" includes, for example, palladium catalysts (e.g., palladium-carbon, palladium-metal, etc.), platinum catalyst (e.g., platinum oxide, etc.), nickel catalysts (e.g, raney nickel, etc.), etc. Among those, preferred is palladium-carbon.
The amount of the catalyst used is generally about 0.01 to about 1 equivalent, preferably about 0.01 to about 0.5 equivalent of the amide compound.
The "inert solvent" includes, for example, alcohols, ethers, halogenated hydrocarbons, aromatic solvents, nitriles, amides, ketones, sulfoxides, water, etc., which may be used as a mixture of two or more species. Among these, preferred is alcohols (e.g., methanol, ethanol, etc.), etc.
The reaction temperature may be between room temperature and 100°C, preferably at room temperature.
The reaction time falls between 0.1 hour and 24 hours, preferably 0.1 to 5 hours.
Further, Compound (VII) is a novel synthetic intermediate for producing Compound (I).
Process 6: Induction of the group represented by the formula -Q-Ar (wherein the symbols have the same meanings as above).
Compound (VII) and Compound (VIII) are reacted to obtain Compound (Ia). The present reaction can be carried out by *per se* known methods according to the kind of $G^2$ which is a protective group of amino group.
When a functional group adjacent to the leaving group $L^1$ is CO, SO or $SO_2$ at Q in Compound (VIII), the present reaction is conducted in the same manner as the amidation in the above-mentioned Process 1.
Moreover, when a functional group adjacent to the leaving group $L^1$ is non-carbonyl carbon atom at Q in Compound (VIII), the present reaction is conducted by alkylation.
Said alkylation is conducted by, for example, reacting Compound (VII) and about 1 to about 5 equivalents (preferably about 1 to about 2 equivalents) of compound (VIII) under the presence of base in an inert solvent.
As said "base", those exemplified in the above-mentioned Process 1 are used. Among those, preferred is potassium carbonate, sodium hydride, potassium hydroxide, etc.
The "inert solvent" includes, for example, alcohols, ethers, halogenated hydrocarbons, aromatic solvents, nitriles, amides, ketones, sulfoxides, water, etc., which may be used as a mixture of two or more species. Among these, preferred are acetonitrile, N,N-dimethyl formamide (DMF), acetone, ethanol, pyridine, water, etc.
The reaction temperature may be between about -20°C and about 100°C, preferably between room temperature and 80°C.
The reaction time falls between 0.5 hour and 1 day.
The aldehyde compound represented by the formula: OHC-Q'-Ar (Q' represents a spacer having a main chain of 1 to 5 atoms) can be used in the above-mentioned Process 6 instead of Compound (VIII).
The "spacer having a main chain of 1 to 5 atoms" represented by Q' includes, one obtained by removing a "spacer having a main chain of 1 atom" from the above-mentioned "spacer having a main chain of 1 to 6 atoms" exemplified as Q.
The "spacer having a main chain of 1 to 5 atoms" represented by Q' includes, for example, preferably

$$-(CH_2)_{w7}CO(CH_2)_{w8}-,\ -(CH_2)_{w9}COO(CH_2)_{w10}-$$

(w7 and w8 represent an integer of 0 to 4, and w7 + w8 represents 0 to 4; w9 and w10 represent an integer of 0 to 3, and w9 + w10 represents 0 to 3), etc.

When an aldehyde compound is used in Process 6, Compound (Ia) is produced by subjecting the aldehyde compound and Compound (VII) to a reductive alkylation. The present reaction may be conducted according to *per se* known methods.

For said reductive alkylation, for example, Compound (VII) and about 1 to about 5 equivalents (preferably 1 to 2 equivalents) of the aldehyde compound are reacted under the presence of metal hydride in an inert solvent.

The "metal hydride" includes, for example, aluminum hydride, lithium aluminum hydride, sodium borohydride, lithium borohydride, sodium cyanoborohydride, lithium cyanoborohydride, sodium triacetoxyborohydride, borane complexes (e.g., borane-THF complex, catechol-borane, etc.), dibutyl aluminum hydride, and the mixtures of these metal hydrides and Lewis acids (e.g., aluminum chloride, titanium tetrachloride, cobalt chloride, etc.) or phosphorus oxychloride, etc. Preferred metal hydrides are sodium cyanoborohydride and sodium triacetoxyborohydride.

The reaction temperature varies, depending on the metal hydride used, but normally falls between about -70°C and about 100°C, preferably between room temperature and 80°C.

The reaction time falls between about 0.1 hour and about 48 hours.

The inert solvent includes, for example, alcohols (preferably ethanol), ethers (preferably THF), nitriles (preferably acetonitrile), acetic acid, etc., which may be used as a mixture of two or more species.

[scheme 3]

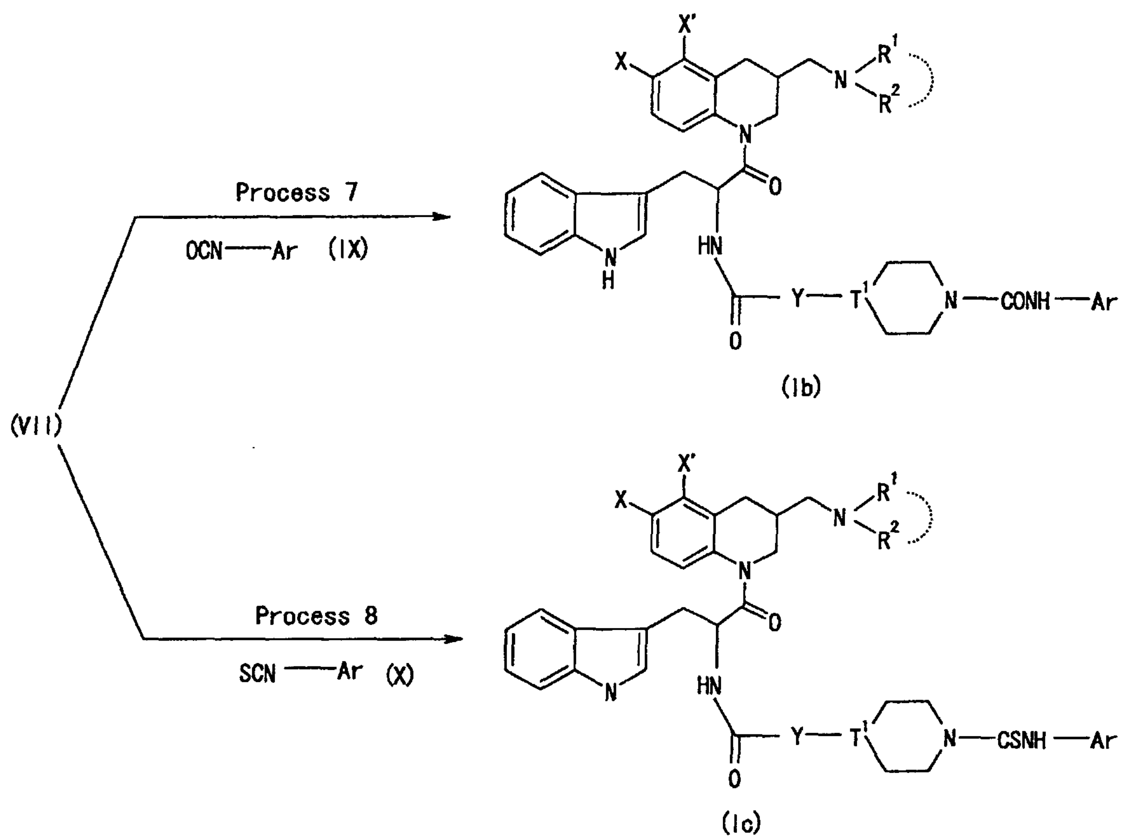


wherein the symbols have the same meanings as above

Process 7: urea reaction

The compound where $T^2$ is =N-, ... is a single bond and Q is -CONH- in the general formula (I), that is, Compound (Ib), can be produced by subjecting the Compound (VII) to urea reaction.

For said urea reaction, for example, Compound (VII) and 1 to 2 equivalents of Compound (IX) (e.g., phenylisocyanate, etc.) are reacted in an inert solvent.

The "inert solvent" includes, for example, ethers, halogenated hydrocarbons, aromatic solvents, nitriles, amides, ketones, sulfoxides, water, etc., which may be used as a mixture of two or more species. Among those, preferred are acetonitrile, N,N-dimethylformamide (DMF), acetone, pyridine, water, etc.

The reaction temperature is between about -20°C and about 100°C, preferably between room temperature and 80°C.

The reaction time falls between 0.5 hour and 1 day.

The present reaction can be conducted under the presence of a base if necessary. Said "base" includes those exemplified in the above-mentioned Process 1. Among those, preferred are sodium hydride, potassium carbonate, carbonic acid, sodium hydroxide, potassium hydroxide, sodium hydrogen carbonate, potassium hydrogen carbonate, triethylamine, pyridine, etc. The amount of the base used is, for example, catalyst amount to 2 equivalents of Compound (VII).

Process 8: thiourea reaction

The compound where $T^2$ is =N-, ... is a single bond and Q is -CSNH- in the general formula (I), that is, Compound (Ic), can be produced by subjecting Compound (VII) to thiourea reaction.

For said thiourea reaction, for example, Compound (VII) and 1 to 2 equivalents of Compound (X) (e.g., phenylisothiocyanate, etc.) are reacted in an inert solvent. The present reaction is conducted in the same manner as in the above-mentioned urea reaction.

The compound where Y is a bond and $T^1$ is =N- in the general formula (I), that is, Compound (Id), can be also produced according to Scheme 4.

[Scheme 4]

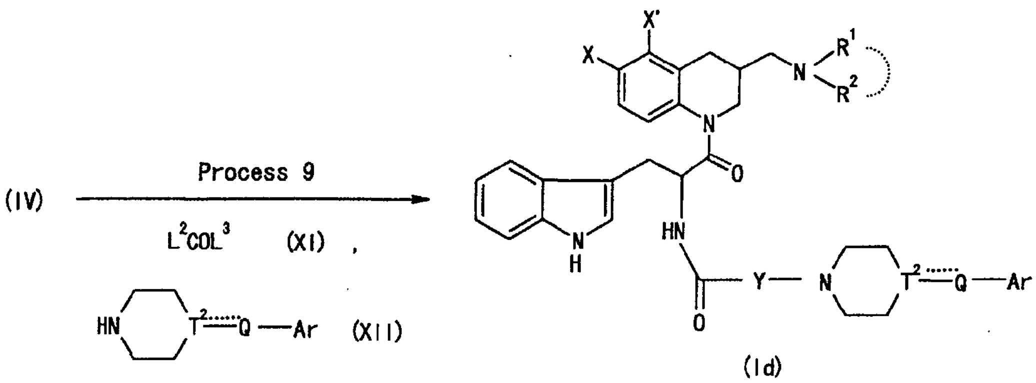


(Id)

wherein $L^2$ and $L^3$ represent a leaving group; the other symbols have the same meanings as above.

The leaving group represented by $L^2$ and $L^3$ includes those exemplified as the above-mentioned $L^1$. Among those, preferred are chlorine or succinimideoxy and especially succinimideoxy is preferred.

Process 9: urea reaction

Compound (IV) and 1 to 2 equivalents of Compound (XI) are reacted in an inert solvent at room temperature for about 0.5 to 5 hours, and then 1 to 2 equivalents of Compound (XII) is reacted in an inert solvent at room temperature for about 0.5 to 24 hours.

The "inert solvent" includes, for example, nitriles, ethers, halogenated hydrocarbons, etc., which may be used as a mixture of two or more species. Among those, acetonitrile, THF, dichloromethane are preferred.

In the present reaction, about 1 to about 5 equivalents of a base (e.g., N-ethyldiisopropylamine, etc.) may be added if necessary.

In the thus obtained compound (I), intermolecular functional groups can be converted into the desired functional groups by combination of *per se* known chemical reactions. Examples of the chemical reactions include oxidation, reduction, alkylation, hydrolysis, amination, esterification, aryl-coupling reaction, deprotection, etc.

The above-mentioned "alcohols" includes, for example, methanol, ethanol, isopropanol, tert-butanol, etc.

The above-mentioned "ethers" includes, for example, diethyl ether, tetrahydrofuran (THF), 1,4-dioxane, 1,2-dimethoxyethane, etc.

The above-mentioned "halogenated hydrocarbons" includes, for example, dichloromethane, chloroform, 1,2-dichloroethane, carbon tetrachloride, etc.

The above-mentioned "aromatic solvents" includes, for example, benzene, toluene, xylene, pyridine, etc.

The above-mentioned "amides" includes, for example, N,N-dimethylformamide (DMF), N,N-dimethylacetamide, N-methylpyrrolidone, etc.

The above-mentioned "ketones" includes, for example, acetone, methylethylketone, etc.

The above-mentioned "sulfoxides" includes for example, dimethylsulfoxide (DMSO), etc.

The above-mentioned "nitriles" includes, for example, acetonitrile, propionitrile, etc.

The above-mentioned "esters" includes, for example, ethyl acetate, etc.

In the above-mentioned reactions where the starting compounds are substituted by any of amino, carboxy, hydroxy or carbonyl, those groups may be protected by ordinary protective groups which are generally used in peptide chemistry, etc. The protective groups may be removed after the reaction, if necessary, to give the desired compounds.

The protective group of amino includes, for example, formyl, $C_{1-6}$ alkyl-carbonyl (e.g., acetyl, propionyl, etc.), $C_{1-6}$ alkoxy-carbonyl (e.g., methoxycarbonyl, ethoxycarbonyl, tert-butoxycarbonyl, etc.), benzoyl, $C_{7-10}$ aralkyl-carbonyl (e.g., benzylcarbonyl, etc.), $C_{7-14}$ aralkyloxy-carbonyl (e.g., benzyloxycarbonyl, 9-fluorenylmethoxycarbonyl, etc.), trithyl, phthaloyl, N,N-dimethylaminomethylene, silyl (e.g., trimethylsilyl, triethylsilyl, dimethylphenylsilyl, tert-butyldimethylsilyl, tert-butyldiethylsilyl, etc.), $C_{2-6}$ alkenyl (e.g., 1-allyl, etc.), etc. These groups may be substituted by 1 to 3 of halogen atoms (e.g., fluorine, chlorine, bromine, iodine, etc.), $C_{1-6}$ alkoxy (e.g., methoxy, ethoxy, propoxy, etc.) or nitro, etc.

The protective group of carboxy includes, for example, $C_{1-6}$ alkyl (e.g., methyl, ethyl, propyl, isopropyl, butyl, tert-butyl, etc.), $C_{7-11}$ aralkyl (e.g., benzyl, etc.), phenyl, trithyl, silyl (e.g., trimethylsilyl, triethylsilyl, dimethylphenylsilyl, tert-butyldimethylsilyl, tert-butyldiethylsilyl, etc.), $C_{2-6}$ alkenyl (e.g., 1-allyl, etc.), etc. These groups may be substituted by 1 to 3 of halogen atoms (e.g., fluorine, chlorine, bromine, iodine, etc.), $C_{1-6}$ alkoxy (e.g., methoxy, ethoxy, propoxy, etc.) or nitro, etc.

The protective group of hydroxy includes, for example, $C_{1-6}$ alkyl (e.g., methyl, ethyl, propyl, isopropyl, butyl, tert-butyl, etc.), phenyl, trithyl, $C_{7-10}$ aralkyl (e.g., benzyl, etc.), formyl, $C_{1-6}$ alkyl-carbonyl (e.g., acetyl, propionyl, etc.), benzoyl, $C_{7-10}$ aralkyl-carbonyl (e.g., benzylcarbonyl, etc.), 2-tetrahydropyranyl, 2-tetrahydrofuranyl, silyl (e.g., trimethylsilyl, triethylsilyl, dimethylphenylsilyl, tert-butyldimethylsilyl, tert-butyldiethylsilyl, etc.), $C_{2-6}$ alkenyl (e.g., 1-allyl, etc.), etc. These groups may be substituted by 1 to 3 of halogen atoms (e.g., fluorine, chlorine, bromine, iodine, etc.), $C_{1-6}$ alkyl (e.g., methyl, ethyl, propyl, etc.), $C_{1-6}$ alkoxy (e.g., methoxy, ethoxy, propoxy, etc.), or nitro, etc.

The protective group of carbonyl includes, for example, cyclic acetal (e.g., 1,3-dioxane, etc.), non-cyclic acetal (e.g., di-$C_{1-6}$ alkylacetal, etc.), etc.

Those protective groups may be removed by per se known methods, for example, the methods described in Protective Groups in Organic Synthesis, published by John Wiley and Sons, 1980, etc. For example, employed are the methods using acids, bases, ultraviolet ray, hydrazine, phenylhydrazine, sodium N-methyldithiocarbamate, tetrabutylammonium fluoride, palladium acetate, trialkylsilylhalide (e.g., trimethylsilyliodide, trimethylsilylbromide, etc.), etc.; and reduction, etc.

Compound (I) can be isolated and purified by any known procedures, for example, through solvent extraction, pH adjustment, redistribution, crystallization, recrystallization, chromatography, etc. The starting compounds for compound (I) and their salts can be also isolated and purified according to the same known procedures as above, but without any isolation procedure, they may be used in the next step while they are in reaction mixtures.

Compound (I') or (I'') of the present invention can be produced according to the same method as that of the above-mentioned Compound (I).

The compound of the present invention may also be in the form of hydrates or non-hydrates thereof.

Where compound (I) includes optical isomers, stereo isomers, regio isomers and rotational isomers, those are within the scope of compound (I), and can be isolated as their single compound through per se known synthesis or separation. For example, where optical isomers of compound (I) exist, those resolved from their mixtures through optical resolution are within the scope of compound (I).

The optical isomers can be produced by per *se* known methods. Concretely, optically active synthetic intermediates or mixtures of racemate of the final product are subjected to ordinary optical resolution to give the corresponding optical isomers.

For the optical resolution, employable are *per se* known methods, such as a fractional recrystallization method, a chiral column method, a diastereomer method, etc.

1) Fractional Recrystallization Method

The method which comprises allowing a racemate to react with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine, etc.) to give a salt, which is then isolated through fractional recrystallization method, followed by, when desired, subjecting the isolated compound to neutralization to obtain free optical isomers.

2) Chiral Column Method

The method of separating a racemate or a salt thereof, which comprises utilizing a column for fractionating optical isomers (chiral column). In the case of liquid column chromatography, for example, a mixture of optical isomers is applied to a chiral column, such as ENANTIO-OVM (manufactured by Tosoh Corp.), CHIRAL SERIES (manufactured by Daicel Co.), etc., which is then eluted with water, various buffers (e.g., phosphate buffer) and organic solvents (e. g., ethanol, methanol, isopropanol, acetonitrile, trifluoroacetic acid, diethylamine, etc.), singly or as a suitable mixture of them, to isolate the individual optical isomers. In the case of gas chromatography, for example, a chiral column such as CP-Chirasil-DeX CB (manufactured by GL Science Co.), etc. is used for isolation.

3) Diastereomer Method

A racemic mixture is chemically reacted with an optically-active reagent to give a mixture of diastereomer, which is subjected to ordinary separation means (e.g., fractional recrystallization, chromatography, etc.) to give single compounds. The thus-isolated single compounds are then chemically processed, for example, through hydrolysis to thereby remove the optically-active reagent site from the compounds to obtain optical isomers. For example, where compound (I) has a hydroxy group or a primary or secondary amino group in the molecule, it is condensed with an optically-active organic acid (e.g., MTPA [α-methoxy-α-(trifluoromethyl)phenyl-acetic acid], (-)-menthoxyacetic acid, etc.) or the like to give the corresponding ester-type or amide-type diastereomer. On the other hand, where compound (I) has a carboxylic acid group, it is condensed with an optically-active amine or alcohol reagent to give the corresponding amide-type or ester-type diastereomer. The thus-isolated diastereomer is then subjected to acidic or basic hydrolysis, through which it is converted into the optical isomer of the original compound.

Compound (I) has an optical active center at 2-position in 3-(indol-3-yl)propanoyl group or(and) 3-position in 1,2,3,4-tetrahydroquinolin-1-yl. And in said optical active center, there exist (R)-isomer and (S)-isomer. Among those, preferred is Compound (I') or Compound (I'') of (R)-isomer.

The compound of the present invention has an excellent somatostatin receptor binding inhibition activity.

In the present invention, somatostatin receptor binding inhibition activity means the activity which inhibits binding between a somatostatin and a somatostatin receptor. It includes a somatostatin receptor agonist activity (agonist activity) and a somatostatin receptor antagonist activity (antagonist activity) , etc.

The compound of the present invention, especially the compound of the present invention has a selective somatostatin subtype 2 receptor (SSTR2) binding inhibition activity. Among those, it has a somatostatin subtype 2 receptor agonist activity.

The compound of the present invention through various intracellular signal transduction systems with which somatostatin is associated. The "intracellular signal transduction systems" include, for example, that which involves adenylate cyclase, $K^+$ channels, $Ca^{2+}$ channels, dephosphorylation of protein, phospholipase C/inositol trisphosphate production systems, MAP kinase, $Na^+/H^+$ exchanger, phospholipase A2, a transcription factor such as NF-κB. The compound of the present invention modulates a direct or indirect cell proliferation inhibitory action or apoptosis both of which are associated with somatostatin.

Further, the compound of the present invention is low in its toxicity, and enhances or inhibits production and/or secretion of a variety of hormones, growth factors and physiologically active substances, etc. by effecting on somatostatin receptors in mammals (e.g., human, cattle, horse, dog, cat, monkey, mouse and rat, especially, human).

The "hormones" include, for example, growth hormone (GH), growth hormone-releasing hormones (GHRH), thyroid stimulating hormone (TSH), prolactin, insulin, glucagon, etc. The "growth factors" include, for example, insulin-like growth factor-1 (IGF-1) and vascular endothelial cell growth factor (VEGF). Said "physiologically active substances" include, for example, vasoactive intestinal polypeptide (VIP), gastrin, glucagon-like peptide-1, amylin, substance-P, CCK (cholecystokinin), amylase, interleukins such as interleukin-6 (IL-6), interleukin-1 (IL-1), etc., cytokines such as TNF-α, etc., cardiotropin, etc.

Therefore, the compound of the present invention is safe, and useful for diseases associated with disorders of the above intracellular signal transduction systems (e.g., diseases accompanied by excess sthenia or suppression, etc.); disorders of regulating cell proliferation; diseases accompanied by disorders of production and/or secretion of hormones, growth factors, physiologically active substances, etc.; or facilitating growth, immune, gastroenteric or metabolic functions, etc; and the like.

For example, the compounds of the present invention are useful (1) for drugs for treatment of tumors such as acromegaly, TSH-producing tumors, nonsecretory (afunctional) hypophysial tumors, ectopic ACTH (adrenocorticotrophic hormone)-producing tumors, medullar thyroid carcinoma, VIP-producing tumors, glucagon-producing tumors, gastrin-producing tumors, insulinoma and carotinoid, (2) for drugs for treatment of diabetes such as insulin-dependent (type I) and non-insulin dependent (type II) diabetes mellitus or a variety of diseases associated with them, namely diabetic complications such as diabetic retinopathy, diabetic nephropathy, diabetic neuropathy, Doan syndrome and

orthostatic hypotension, (3) for drugs for treatment of obesity or overeating, etc caused by improvement of hyperinsulinemia or inhibition of appetite, etc. (4) for drugs for treatment of acute pancreatitis, chronic pancreatitis, pancreal/intestinal fistula, hemorrhagic ulcer, peptic ulcer, gastritis, hyperchylia, regurgitant esophagitis, etc. (5) for drugs for improvement of various symptoms accompanied by the *Helicobacter pylori* infection, for example, inhibitors of gastrin hypersecretion, etc (6) for drugs for inhibition of amylase secretion accompanied by endoscopic cholangiopancreatography, and drugs for prognostic treatment of surgical operation of pancreas, (7) for drugs for treatment of diarrhea due to intestinal malabsorption, promotion of secretion or dyskinesia of the digestive tracts (for example, short bowel syndrome, etc.), diarrhea due to the drugs for cancer chemotherapy, diarrhea due to congenital small intestine atrophy, diarrhea due to neuroendocrine tumors such as VIP-producing tumors, diarrhea due to AIDS, diarrhea due to graft versus host reaction accompanied by bone marrow transplantation, diarrhea due to diabetes mellitus, diarrhea due to celiac plexus blocking, diarrhea due to systemic sclerosis and diarrhea due to eosinophilia, etc. (8) for drugs for treatment of dumping syndrome, irritable colitis, Crohn disease and inflammatory bowel disease, etc. (9) for drugs for treatment of tumors or cancers (e.g., thyroid cancer, large bowel cancer, breast cancer, prostatic cancer, small cell lung cancer, non-small cell lung cancer, pancreatic cancer, stomach cancer, cholangiocarcinoma, hepatic cancer, vesical cancer, ovarian cancer, melanoma, osteosarcoma, chondrosarcoma, malignant pheochromocytoma, neuroblastoma, brain tumors, thymoma, renal cancers, etc.), leukemia (e.g., leukemia of basophilic leukemia, chronic lymphocytic leukemia, chronic myeloid leukemia, Hodgkin disease, and non-Hodgkin lymphoma, etc.); the drugs can be used for monotherapy or concomitant therapy with other anticancer drugs such as Tamoxifen, LHRH agonists, LHRH antagonists, interferon-$\alpha$, $\beta$ and $\gamma$, interleukin-2, etc.), (10) for drugs for prevention or treatment of hypertrophic cardiomyopathy, arteriosclerosis, valvular disease, myocardiac infarction (especially, myocardiac infarction post percutaneous transluminal coronary arterioplasty) and reangioplasty, (11) for drugs for treatment of hemorrhage of esophageal varicosis, cirrhosis and peripheral blood vessel disorders, (12) for drugs for treatment of diseases accompanied by general or local inflammation, for example, polyarteritis, rheumatoid arthritis, psoriasis, sunburn, eczema and allergy (e.g., asthma, atopic dermatitis and allergic rhinitis, etc.), because they modulate the secretion of physiologically active substances acting on the immune system (e.g., Substance P, tachykinin and cytokines), (13) for drugs for treatment of dementia (e.g., Alzheimer's disease, Alzheimer-type senile dementia, vascular/multi-infarct dementia, etc.), schizophrenia, epilepsy, depression, generalized anxiety disorder, sleep disorder, and multiple sclerosis, because they give influence on the production or secretion of nerve regulators, (14) for drugs for treatment of oculopathy (e.g., glaucoma, etc.), (15) for drugs for prevention or treatment of acute bacterial meningitis, acute virus encephalitis, adult respiratory distress syndrome, bacterial pneumonia, severe systemic mycotic infection, tuberculosis, spinal damage, bone fracture, hepatic failure, pneumonia, alcoholic hepatitis, virus A hepatitis, virus B hepatitis, virus C hepatitis, AIDS infection, human papilloma virus infection, influenza infection, metastasis of cancer, multiple myeloma, osteomalacia, osteoporosis, bone Paget disease, nephritis, renal failure, sepsis, septic shock, hypercalcemia, hypercholesterolemia, hypertriglyceridemia, hyperlipemia, systemic lupus erythematosus, transient ischemic attack and alcoholic hepatitis, etc., (16) for cure of organ transplantation, burns, trauma, and alopecia, etc. (17) as analgesics to suppress or relieve chronic or acute pain (e.g., postoperative pain, inflammatory pain, dental pain, bone disease (e.g., arthritis, rheumatism, osteoporosis, etc.) derived pain), (18) for imaging of tumors having somatostatin receptors after introducing radioactive substance (e.g., $^{123}$I, $^{125}$I, $^{111}$In, etc.) to compound (I) directly or via a suitable spacer, and (19) for targeting tumors having somatostatin receptors after introducing anti-cancer drugs to compound (I) directly or via a suitable spacer.

Somatostatin is associated with secretion of growth hormone (especially in the case of SSTR2), therefore, the compound of the present invention, when it is used directly or for the purpose of promoting secretion of growth hormone, can provide the same effect and use as growth hormone itself. Thus, the compounds of the present invention can be used for prevention or treatment of diseases or symptoms caused by insufficiency of growth hormone or IGF-1.

The "prevention or treatment of diseases or symptoms caused by insufficiency of growth hormone or IGF-1" includes, for example, treatment of insulin-dependent (type I) and non-insulin dependent (type II) diabetes mellitus or a variety of diseases associated with them, namely diabetic complications such as diabetic retinopathy, diabetic nephropathy, diabetic neuropathy, Doan syndrome and orthostatic hypotension, etc.; prevention of adverse effects caused by disassimilation of glucocorticoid; prevention or treatment of osteoporosis; stimulation of immune system (e.g., promotion of increase in hemocytes such as lymphocyte; strengthening of an antimicrobial activity or an antiviral activity); promotion of cure of burns and trauma; acceleration in the treatment of bone fracture; treatment of acute or chronic renal diseases; treatment or improvement of diseases or symptoms (short stature, delayed growth) associated with insufficiency of growth hormone in adults or infants; treatment of obesity; promotion of recovery after surgical operations; improvement of delayed growth associated with Prader-Willi syndrome and Turner's syndrome; treatment of delayed intrauterine growth and skeletogenous disorders; treatment of peripheral neuropathy; treatment of Noonan's syndrome, schizophrenia and depression; treatment or prevention of neurodegenerative diseases such as Alzheimer's disease and Parkinson's disease; treatment of pulmonary insufficiency and ventilation dependence; treatment of malabsorption syndrome; improvement of cachexia or protein loss caused by cancer or AIDS; promotion of weight increase or proteopexis in patients in the case of TPN (total parenteral nutrition); treatment of hyperinsulinemia; promotion of

induction of ovulation; improvement of menopausal disorders; improvement of senile constitution.

Further, the compound of the present invention is useful in mammals such as domestic animals for promotion of growth; increase in milk production; strengthening of an antimicrobial or antiviral activity by stimulation of immune system; stimulation in growth of wool in sheeps.

The compound of the present invention can be used with various concomitant drugs.

Examples of the concomitant drugs include a "agents for treating diabetes", "agents for treating diabetic complications", "agents for treating obesity", "agents for treating hypertension", "agents for treating hyperlipidemia", "agents for treating arthritis", "antianxiety agents", "antidepressant", "agents for treating osteoporosis", etc. Two or more kinds of these concomitant drugs can be combined in an appropriate ratio for use.

Examples of the above "agents for treating diabetes" include insulin sensitizers, insulin secretagogues, biguanides, insulins, α-glucosidase inhibitors, β3 adrenaline receptor agonists.

Examples of the insulin sensitizers include pioglitazone or its salt (preferably hydrochloride), troglitazone, rosiglitazone or its salt (preferably maleate), JTT-501, GI-262570, MCC-555, YM-440, DRF-2593, BM-13-1258, KRP-297, R-119702, CS-011, etc.

Examples of the insulin secretagogues include sulfonylureas. Concrete examples of the sulfonylureas include tolbutamide, chlorpropamide, tolazamide, acetohexamide, glyclopyramide and its ammonium salt, glibenclamide, gliclazide, glimepiride.

Other than the above, examples of insulin secretagogues include repaglinide, nateglinide, mitiglinide (KAD-1229), JTT-608.

Examples of biguanides include metformin, buformin, phenformin.

Examples of insulins include animal insulins extracted from bovine or porcine pancreas; semisynthetic human insulin which is enzymatically synthesized from insulin extracted from porcine pancreas; human insulin synthesized by genetic engineering, using *Escherichi Coil* or yeast. As insulin, also employed are insulin-zinc containing 0.45 to 0.9 (w/w)% of zinc; protamine-insulin-zinc produced from zinc chloride, protamine sulfate and insulin; etc. In addition, insulin can be an insulin fragment or derivative (e.g., INS-1, etc.).

Insulin can also include various types such as ultra immediate action type, immediate action type, two-phase type, intermediate type, prolonged action type, etc., and these can be selected depending on the pathological conditions of patients.

Examples of α-glucosidase inhibitors include acarbose, voglibose, miglitol, emiglitate, etc.

Examples of β3 adrenaline receptor agonists include AJ-9677, BMS-196085, SB-226552, AZ40140, etc.

Other than the above, examples of the "agents for treating diabetes" include ergoset, pramlintide, leptin, BAY-27-9955.

Examples of the above "agents for treating diabetic complications" include aldose reductase inhibitors, glycation inhibitors, protein kinase C inhibitors, etc.

Examples of aldose reductase inhibitors include torulestat; eparlestat; imirestat; zenarestat; SNK-860; zopolrestat; ARI-509; AS-3201, etc.

Examples of glycation inhibitors include pimagedine, etc.

Examples of protein kinase C inhibitors include NGF, LY-333531, etc.

Other than the above, examples of "agents for treating diabetic complications" include alprostadil, thiapride hydrochloride, cilostazol, mexiletine hydrochloride, ethyl eicosapentate, memantine, pimagedline (ALT-711), etc.

Examples of the above "agents for treating obesity" include lipase inhibitors and anorectics, etc.

Examples of lipase inhibitors include orlistat, etc.

Examples of anorectics include mazindol, dexfenfluramine, fluoxetine, sibutramine, baiamine, etc.

Other than the above, examples of "agents for treating obesity" include lipstatin, etc.

Examples of the above "agents for treating hypertension" include angiotensin converting enzyme inhibitors, calcium antagonists, potassium channel openers, angiotensin II antagonists, etc.

Examples of angiotensin converting enzyme inhibitors include captopril, enarapril, alacepril, delapril (hydrochloride), lisinopril, imidapril, benazepril, cilazapril, temocapril, trandolapril, manidipine (hydrochloride), etc.

Examples of calcium antagonists include nifedipine, amlodipine, efonidipine, nicardipine, etc.

Examples of potassium channel openers include levcromakalim, L-27152, AL0671, NIP-121, etc.

Examples of angiotensin II antagonists include losartan, candesartan cilexetil, valsartan, irbesartan, CS-866, E4177, etc.

Examples of the above "agents for treating hyperlipidemia" include HMG-CoA reductase inhibitors, fibrate compounds, etc.

Examples of HMG-CoA reductase inhibitors include pravastatin, simvastatin, lovastatin, atorvastatin, fluvastatin, lipantil, cerivastatin, itavastatin, ZD-4522, or their salts (e.g., sodium salts, etc.), etc.

Examples of fibrate compounds include bezafibrate, clinofibrate, clofibrate, simfibrate, etc.

Examples of the above "agents for treating arthritis" include ibuprofen, etc.

Examples of the above "antianxiety agents" include chlordiazepoxide, diazepam, oxazolam, medazepam, cloxazolam, bromazepam, lorazepam, alprazolam, fludiazepam, etc.

Examples of the above "antidepressants" include fluoxetine, fluvoxamine, imipramine, paroxetine, sertraline, etc.

Examples of "agents for treating osteoporosis" include, for example, bisphosphonates, vitamin D preparations, calcitonin preparations, PTH preparations, Osten, etc.

Other than the above, the concomitant drugs include "hormones promoting other growth hormone secretion (e.g., GHRH), GH or IGF-1", "cytokines or cytokine activity enhancing agents", etc.

A pharmaceutical composition of the invention can be produced according to a per se known method. Said pharmaceutical composition can be produced by mixing the compound of the present invention and a pharmacologically acceptable carrier according to any *per se* known method.

The dosage forms of the pharmaceutical composition include, for example, tablets (including sugar-coated tablets, film-coated tablets), powders, granules, capsules (including soft capsules), liquids, injections, suppositories, sustained release preparations (e.g., sustained-release microcapsules, etc.), etc. The compound of the present invention and the pharmaceutical composition of the present invention can be safely administered orally or parenterally (e.g., by local, rectal and intravenous administration, etc.).

The content of the compound of the present invention in a pharmaceutical composition of the present invention is, for instance, 0.1 to 100 weight percent of the whole composition. The dose of the pharmaceutical composition can be appropriately selected depending on the subject of administration, route of administration, disease, etc For instance, the dose per administration when a pharmaceutical composition of the invention is orally administered as an agent for treating glaucoma to an adult patient (body weight: about 60 kg), is about 0.1 to about 500 mg, preferably about 1 to about 100 mg, more preferably about 5 to about 100 mg in terms of an effective ingredient (compound of the present invention). These amounts can be divided into one to several doses per day for administration.

Here, examples of the pharmacologically acceptable carriers used for production of a pharmaceutical composition of the present invention include various organic or inorganic carrier substances which are commonly used as materials for pharmaceutical preparations, such as excipients, lubricants, binders, and disintegrators in solid preparations; solvents, solubilizing agents, suspending agents, isotonizing agents, buffering agents, soothing agents, in liquid preparations. In addition, additives such as antiseptics, antioxidants, coloring agents, sweeteners, absorbents, moistening agents, can be used, if necessary.

Examples of the excipients include lactose, sucrose, D-mannitol, starch, cornstarch, crystalline cellulose, light anhydrous silicic acid, etc.

Examples of the lubricants include magnesium stearate, calcium stearate, talc, colloidal silica, etc.

Examples of the binders include crystalline cellulose, sucrose, D-mannitol, dextrin, hydroxypropylcellulose, hydroxypropylmethylcellulose, polyvinylpyrrolidone, starch, saccharose, gelatin, methylcellulose, carboxymethylcellulose sodium, etc.

Examples of the disintegrators include starch, carboxymethylcellulose, carboxymethylcellulose calcium, crosscarmellose sodium, carboxymethylstarch sodium, L-hydroxypropylcellulose, etc.

Examples of the solvents include water for injection, alcohol, propylene glycol, macrogol, sesame oil, corn oil, etc.

Examples of the solubilizing agents include polyethylene glycol, propylene glycol, D-mannitol, benzyl benzoate, ethanol, trisaminomethane, cholesterol, triethanolamine, sodium carbonate, sodium citrate, etc.

Examples of the suspending agents include surfactants such as stearyltriethanolamine, sodium lauryl sulfate, lauryl amino propionic acid, lecithin, benzalkonium chloride, benzethonium chloride, glyceryl monostearate, etc.; or hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose, etc.

Examples of the isotonizing agents include glucose, D-sorbitol, sodium chloride, glycerin, D-mannitol, etc.

Examples of the buffering agents include buffer solutions of phosphate, acetate, carbonate and citrate, etc.

Examples of the soothing agents include benzyl alcohol, etc.

Examples of the antiseptics include paraoxybenzoates, chlorobutanol, benzyl alcohol, phenethylalcohol, dehydroacetic acid, and sorbic acid, etc.

Examples of the antioxidants include sulfite, ascorbic acid, etc.

The present invention will be explained in more detail by the following Reference Examples, Examples, Formulation Example and Experimental Examples. These are not intended to restrict the present invention, and may be modified within the range of not deviating the scope of this invention.

"Room temperature" in the following Reference Examples and Examples means a temperature of 0°C to 30°C. For drying an organic layer, anhydrous magnesium sulfate or anhydrous sodium sulfate was employed. Unless otherwise specifically indicated, "%" means percent by weight.

The IR absorption spectra were measured in a diffused reflection method using a Fourier transform infrared

spectrophotometer.

The meanings of the abbreviations used in the present specification are as follows:

s: singlet
d: doublet
dd: double doublet
dt: double triplet
t: triplet
q: quartet
m: multiplet
br: broad
J: coupling constant
Hz: Hertz
$CDCl_3$: deuterated chloroform
DMSO-$d_6$: deuterated dimethylsulfoxide
THF: tetrahydrofuran
DMF: N,N-dimethylformamide
DMSO: dimethylsulfoxide
WSC: 1-ethyl-3-(3-dimethylaminopropyl)carbodiimide hydrochloride
$^1$H-NMR: proton nuclear magnetic resonance spectrum (generally measured as the free form of each sample in $CDCl_3$)
IR: infrared absorption spectrum
Me: methyl
Et: Ethyl
HOBt: 1-hydroxy-1H-benzotriazol
IPE: diisopropyl ether

**Examples**

**Reference Example 1**

2-[(2-chloro-6-nitrophenyl)methylidene]malonic diethyl

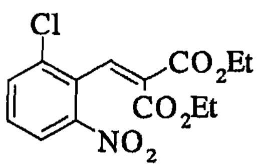

To a solution of 2-chloro-6-nitrobenzaldehyde(20.23 g) and malonic diethyl (17.63 g) in acetic anhydride (36 ml) was added potassium hydrogen carbonate (16.48 g). The mixture was stirred at 110°C for two hours. After the reaction solution was cooled down, the reaction solution was poured into water and extracted with ethyl acetate. The organic layer was washed with water and saturated brine. After drying, the solution was concentrated. The residue was purified by silica gel column chromatography (developing solvent: ethyl acetate/hexane = 4/1) and gave the title compound (34.89 g).

$^1$H-NMR δ: 1.00(3H,t), 1.37(3H,t), 4.03(2H,q), 4.36(2H,q), 7.48(1H,dt), 7.71(1H,dd), 8.00(1H,s), 8.04(1H,dd).

**Reference Example 2**

2- [(5-fluoro-2-nitrophenyl)methylidene]malonic diethyl

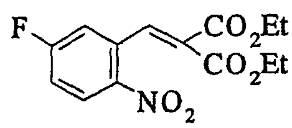

The title compound was obtained according to the same method as Reference Example 1.
$^1$H-NMR($CDCl_3$)δ: 1.10(3H,t), 1.36(3H,t), 4.15(2H,q), 4.35(2H,q), 7.10-7.30(2H,m), 8.13(1H,s), 8.28(1H,dd).

**Reference Example 3**

5-chloro-2-oxo-1,2,3,4-tetrahydro-3-quinoline carboxylic acid ethyl

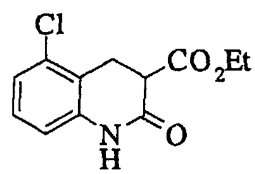

To a solution of 2-[(2-chloro-6-nitrophenyl)-methylidene]malonic diethyl (34.89 g) in ethanol (200 ml) was added sodium boron hydride (2.02 g) at 0°C. The mixture was stirred at 0°C for 30 minutes. Then, water was added to the mixture and extracted with ethyl acetate. The organic layer was washed with saturated brine, dried and concentrated. To the residue in the aqueous solution of acetic acid (200 ml) was added iron (25.7 g). The mixture was refluxed under heating for 90 minutes. Insoluble material was filtered off and the filtrate was concentrated. Water was added to the residue and extracted with ethyl acetate. The organic layer was washed with water and saturated brine, dried and concentrated. The obtained crude crystals were washed with IPE and the title compound (16.84 g) was obtained.
melting point: 174-176°C.

**Reference Example 4**

6-fluoro-2-oxo-1,2,3,4-tetrahydro-3-quinoline carboxylic acid ethyl

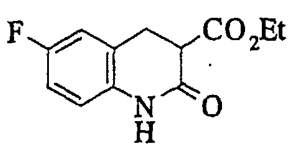

The title compound was obtained according to the same method as Reference Example 3.
melting point 166-168°C.

**Reference Example 5**

5-chloro-2-oxo-1,2,3,4-tetrahydro-3-quinoline carboxylic acid

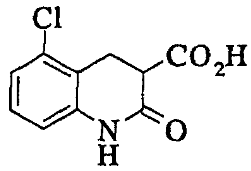

To a mixture of 5-chloro-2-oxo-1,2,3,4-tetrahydro-3-quinolinecarboxylic acid ethyl (15.23 g) in THF (180 ml) and methanol (120 ml) was dropwise added an aqueous solution of 1N sodium hydroxide (65 ml) at 0°C. The mixture was stirred at room temperature for 18 hours. To the reaction solution was dropwise added 1N hydrochloric acid (70 ml) at 0°C. Then, the mixture was extracted with ethyl acetate. The organic layer was washed with saturated brine, dried and concentrated. The obtained crude crystals were washed with IPE and the title compound (15.89 g) was obtained.

melting point: 179-186°C.

**Reference Example 6**

6-fluoro-2-oxo-1,2,3,4-tetrahydro-3-quinoline carboxylic acid

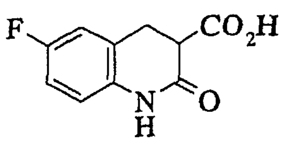

The title compound was obtained according to the same method as Reference Example 5.

melting point 144-147°C.

**Reference Example 7**

5-chloro-N,N-dimethyl-2-oxo-1,2,3,4-tetrahydro-3-quinolinecarboxamide

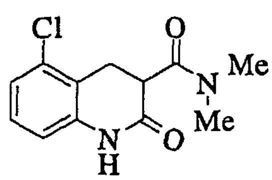

To a mixture of acetonitrile (100 ml) and THF (100 ml) was added 5-chloro-2-oxo-1,2,3,4-tetrahydro-3-quinolinecarboxylic acid (11.34 g), dimethylamine hydrochloride (4.95 g), HOBt (7.83 g), WSC (10.71 g) and triethylamine (17 ml). The reaction mixture was stirred at room temperature for 18 hours. To the reaction solution was added 10% of an aqueous solution of citric acid and extracted with ethyl acetate. The organic layer was washed with water, a saturated aqueous solution of sodium hydrogen carbonate and saturated brine, dried and concentrated. The residue

was washed with IPE and the title compound (6.601 g) was obtained.
melting point: 257-261°C.

**Reference Example 8**

6-fluoro-N,N-dimethyl-2-oxo-1,2,3,4-tetrahydro-3-quinolinecarboxamide

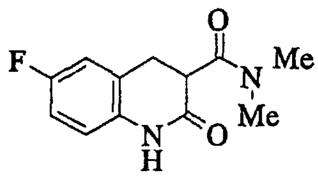

The title compound was obtained according to the same method as Reference Example 7.
melting point: 289-291°C. (decomposition)

**Reference Example 9**

5-chloro-3-(N,N-dimethylamino)methyl-1,2,3,4-tetrahydroquinoline

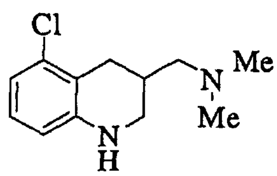

To a suspension of 5-chloro-N,N-dimethyl-2-oxo-1,2,3,4-tetrahydro-3-quinolinecarboxamide (5.059 g) in THF (180 ml) was added 1M borane-THF complex (80 ml). The reaction solution was refluxed under heating for 6 hours and left aside until it was cooled. The reaction solution was cooled down by ice, water (2 ml) and 6N hydrochloric acid (50 ml) was added thereto, stirred at room temperature for 15 hours and concentrated. The residue in methanol solution (50 ml) was refluxed under heating for 24 hours, to the residue was added an aqueous solution of 3N sodium hydroxide to make it as a base and extracted with ethyl acetate. The organic layer was washed with water and saturated brine, dried and concentrated. The residue was purified by alumina column chromatography (developing solvent: ethyl acetate/hexane = 1/10). The obtained crystals were washed with hexane and the title compound (3.52 g) was obtained.
melting point: 107~112°C.

**Reference Example 10**

3-(N,N-dimethylamino)methyl-6-fluoro-1,2,3,4-tetrahydroquinoline

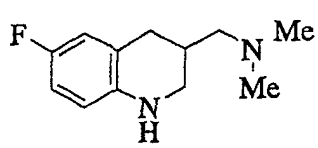

The title compound was obtained according to the same method as Reference Example 9.
melting point 104-105°C.

**Reference Example 11**

1-[2-(R)-amino-3-(indol-3-yl)propanoyl]-5-chloro-3-(R,S)-(N,N-dimethylamino)methyl-1,2,3,4-tetrahydroquinoline

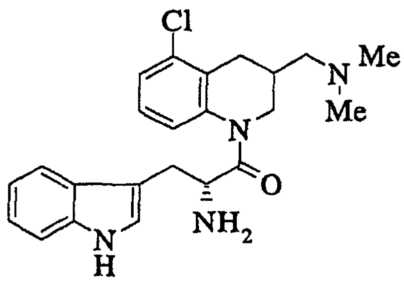

To a solution of N-(9-fluorenylmethoxycarbonyl)-D-triptophan (6.44 g) and DMF (0.14 ml) in THF (50 ml) was dropwise added oxalylchloride (1.6 ml) in THF (15 ml) solution at 0°C. The mixture was stirred at room temperature for 30 minutes. Then, to a mixture of a solution of 5-chloro-3-(N,N-dimethylamino)methyl-1,2,3,4-tetrahydroquinoline (1.13 g) in ethyl acetate (50 ml) and a saturated aqueous solution of sodium hydrogen carbonate (25 ml) was dropwise added the reaction solution at 0°C and stirred at room temperature for an hour, the organic layer was then separated. The organic layer was washed with saturated brine, dried and concentrated. The residue was purified by alumina column chromatography (developing solvent; ethyl acetate/hexane = 1:2 - 1:1) and concentrated. The residue was dissolved in methanol (60 ml), piperidine (2 ml) was added thereto and stirred for at room temperature for 12 hours. The reaction solution was concentrated and purified by alumina column chromatography (developing solvent; ethyl acetate/hexane = 1:2 - ethyl acetate/methanol = 20:1), the title compound was obtained as amorphous powders (0.828 g).
IR(KBr):3283, 2934, 2820, 2774, 1647, 1568, 1460, 1354, 1186, 741 $cm^{-1}$.

**Reference Example 12**

1-[2-(R)-amino-3-(indol-3-yl)propanoyl]-3-(R, S)-(N,N-dimethylamino)methyl-6-fluoro-1,2,3,4-tetrahydroquinoline

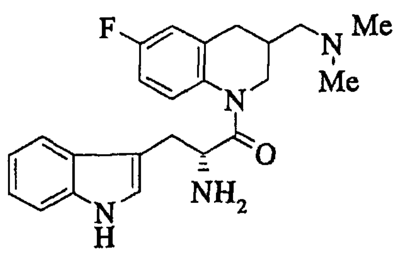

The title compound was obtained according to the same method as Reference Example 11.
IR(KBr):3289, 2928, 1644, 1497, 1456, 1244, 742 $cm^{-1}$.
MASS (APCIMASS), m/z 395[$(M+H)^+$].

**Reference Example 13**

2-(5-chloro-2-nitrobenzyl)malonic diethyl

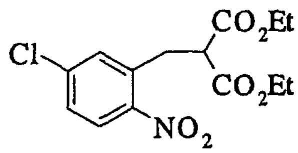

To a mixture of 5-chloro-2-nitrobenzaldehyde (25 g), malonic diethyl (21.6 g) in acetic anhydride (50 ml) was added potassium hydrogen carbonate (11.9 g) and the mixture was stirred at 110°C for 45 minutes. The reaction solution was poured into water and extracted with ethyl acetate. The organic layer was washed with water and saturated brine, dried and concentrated. The crude product of 2-(5-chloro-2-nitrobenzylidene)malonic diethyl was obtained.

To a solution of a crude product of 2-(5-chloro-2-nitrobenzylidene) malonic diethyl in ethanol (250 ml) was added sodium boron hydride (3.3 g) under ice cooling and the solution was stirred for 30 minutes. Then, to the reaction solution was added 10% of an aqueous solution of citric acid. The solution was acidulated and concentrated. To the residue was added water and the mixture was extracted with ethyl acetate. The organic layer was washed with water and saturated brine, dried and concentrated. The residue was purified with silica gel column chromatography (developing solvent; hexane - hexane:ethyl acetate = 4:1) and the title compound (43 g) was obtained.

oily substance:

$^1$H-NMR ($CDCl_3$) δ: 1.23(6H, t), 3.49(2H, d), 3.84(1H, t) , 4.19(4H, q), 7.10-7.46(2H, m), 7.99(1H, d).

**Reference Example 14**

2-(5-chloro-2-nitrobenzyl)-1,3-propanediol

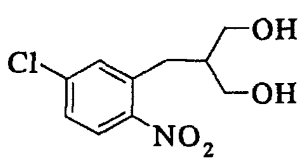

To a solution of a crude product of 2-(5-chloro-2-nitrobenzyl)malonic diethyl (30.0 g) in ethanol (300 ml) was added sodium boron hydride (10.3 g) under ice cooling. The mixture was stirred at room temperature for 12 hours. To the reaction solution was added 1N hydrochloric acid under ice cooling, stirred for 2 hours at room temperature and concentrated. The residue was extracted with ethyl acetate. The organic layer was washed with 1N hydrochloric acid, water and saturated brine, dried and concentrated. The residue was purified by silica gel column chromatography (developing solvent; hexane - ethyl acetate), the title compound (17 g) was obtained.

melting point: 56-58°C.

$^1$H-NMR ($CDCl_3$) δ: 1.90-2.20 (1H, m) , 2.80(2H, br s), 2.98(2H, d), 3.69(2H, dd), 3.85(2H, dd), 7.35(1H, dd), 7.43(1H, d), 7.92 (1H, d).

**Reference Example 15**

2-(R)-(5-chloro-2-nitrobenzyl)-3-hydroxypropyl hexanoate

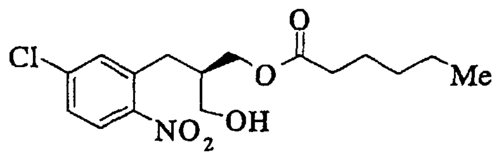

A mixture of 2-(5-chloro-2-nitrobenzyl)-1,3-propanediol (5.08 g), lipase PS-10527 (amano pharmaceuticals; 0.75 g), and vinyl hexanoate(10 ml) was shaked in IPE (500 ml) at 35°C for 21.5 hours. Enzyme was filtered off and the filtrate was concentrated. The residue was purified by silica gel chromatography (developing solvent: hexane/ethyl acetate = 3/1), the title compound 6.64 g (99% ee) was obtained.

$^1$H-NMR (400 MHz, DMSO-$d_6$) δ: 0.91 (3H, t), 1.25-1.35 (4H, m), 1.63 (2H, m), 2.19 (1H, m), 2.33 (2H, t), 2.93 (1H, dd), 3.03 (1H, dd), 3.51 (1H, dd), 3.62 (1H, dd), 4.13-4.23 (2H, m), 7.36-7.40 (2H, m), 7.94 (1H, dd).

IR (KBr): 3450, 2957, 1734, 1525, 1343, 1175, 832 $cm^{-1}$.

$[\alpha]_D^{27}$=+24.6° (c=1.02, ethyl acetate).

**Reference Example 16**

2-(R)-(5-chloro-2-nitrobenzyl)-3-hydroxypropyl propionate

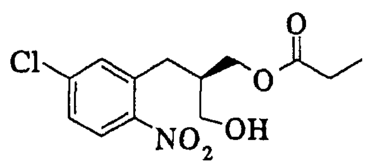

A mixture of 2-(5-chloro-2-nitrobenzyl)-1,3-propanediol (5.02 g), lipase PS-10527 (amano pharmaceuticals: 1.7 g), and vinyl propionate (20 ml) was shaked in IPE (500 ml) at 35°C for 11 hours. High-performance chromatography analysis was performed on this reaction solution. According to the analysis, the yield of monoacyl body was 91%, the enantiomer excess was 98% ee. Enzyme was filtered off and the filtrate was concentrated to dryness to give the oily substance. This product was employed silica gel chromatography (silica gel 200 g, hexane/ethyl acetate = 3/1) and the title compound was obtained as a yellowish oily substance (4.21 g, 98% ee).

$^1$H-NMR (400 MHz, DMSO-$d_6$) δ: 1.16 (3H, t, J = 7.6 Hz), 1.68 (1H, br s,), 2.2 (2H, m), 2.37 (2H, q, J = 7.6 Hz), 2.94 (1H, dd, J = 7.1 and 13.4 Hz), 3.02 (1H, dd, J = 7.6 and 13.4 Hz), 3.51 (1H, dd, J = 5.7 and 11.5 Hz), 3.62 (1H, dd, J = 4.1 and 11.5 Hz), 4.16 (1H, dd, J = 6.1 and 11.5 Hz), 4.21 (1H, dd, J = 4.9 and 11.5 Hz), 7.39 (2H, m, Ph), 7.94 (1H, d, J = 8.5 Hz).

IR (KBr) 3452, 1735, 1525, 1344, 1196, 833 $cm^{-1}$.

$[\alpha]_D^{28}$=+7.35° (c=1.02, ethanol)

HPLC condition: column; CHIRALPAK AD (Daicel chemical industries)

mobile phase; n-hexane/2-propanol (925/75)
velocity; 0.8 ml/min
temperature; room temperature
detection; UV (225 nm)
retention time; 20, 24 min.

**Reference Example 17**

6-chloro-3-(R)-hexanoyloxymethyl-1,2,3,4-tetrahydroquinoline

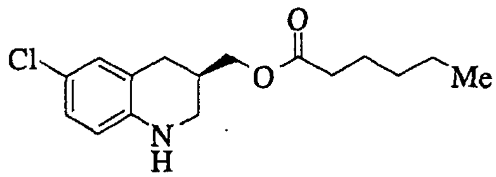

To a solution of 2-(R)-(5-chloro-2-nitrobenzyl)-3-hydroxypropyl hexanoate (400 mg) in acetonitrile (8 ml) was added triethylamine (0.3 ml) under ice cooling. Then, methanesulfonyl chloride (0.11 ml) was added thereto and stirred for 15 minutes. To the reaction solution was added a saturated aqueous solution of sodium hydrogen carbonate under ice cooling and the mixture was extracted with ethyl acetate. The organic layer was washed with water, saturated brine, dried and concentrated.

The crude product of 2-(S)-(5-chloro-2-nitrobenzyl)-3-[(methylsulfonyl)oxy]propyl hexanoate was obtained. The crude product of 2-(S)-(5-chloro-2-nitrobenzyl)-3-[(methylsulfonyl)oxy]propyl hexanoate was dissolved in THF (3 ml), acetic acid (1.5 ml)was added thereto under ice cooling. Then, zinc powders (760 mg) was added thereto under ice cooling and stirred under ice cooling for 30 minutes. The mixture was stirred at room temperature for two hours, zinc powders (760 mg) was added thereto and stirred for one hour. The reaction solution was filtered, the filtrate was concentrated under reduced pressure and the crude product of 2-(S)-(2-amino-5-chlorobenzyl)-3-[(methylsulfonyl)oxy] propyl hexanoate was obtained.

The crude product of 2-(S)-(2-amino-5-chlorobenzyl)-3-[(methylsulfonyl)oxy]propyl hexanoate was dissolved in THF (8 ml) and diisopropylethylamine (1 ml) was added thereto, stirred at 60°C for 12 hours under argon stream. To the reaction solution was added a saturated aqueous solution of sodium hydrogen carbonate and the mixture was extracted with ethyl acetate. The organic layer was washed with water and saturated brine, dried, concentrated. The residue was purified with alumina column chromatography (developing solvent; hexane - hexane:ethyl acetate = 10: 1) and the title compound (234 mg) was obtained.

oily substance:

$[\alpha]_D^{20}$ = -25.1° (c = 0.505, methanol).

**Reference Example 18**

1-benzyloxycarbonyl-6-chloro-3-(R)-(hydroxymethyl)-1,2,3,4-tetrahydroquinoline

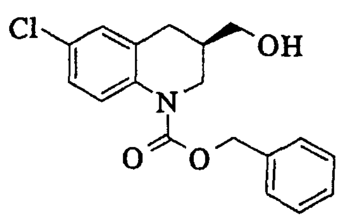

To a solution of 6-chloro-3-(R)-hexanoyloxymethyl-1,2,3,4-tetrahydroquinoline (3.86 g) in pyridine (20 ml) was dropwise added benzyl chloroformate (3.0 ml). After stirring at room temperature for 30 minutes, water was added to the reaction solution and the mixture was extracted with ethyl acetate. The organic layer was washed with 1N hydrochloric acid, water, a saturated aqueous solution of sodium hydrogen carbonate, water, saturated brine, dried and concentrated. The crude product of 1-benzyloxycarbonyl-6-chloro-3-(R)-hexanoyloxymethyl-1,2,3,4-tetrahydroquinoline was obtained.

The crude product of 1-benzyloxycarbonyl-6-chloro-3-(R)-hexanoyloxymethyl-1,2,3,4-tetrahydroquinoline was dissolved in a mixture of methanol (40 ml) and THF (40 ml) and an aqueous solution of 1N sodium hydroxide (20

ml) was added under ice cooling. The mixture was stirred at room temperature for 30 minutes, water was added to the reaction solution and concentrated. The residue was extracted with ethyl acetate. The organic layer was washed with water and saturated brine, dried and then concentrated. The residue was purified by silica gel column chromatography (developing solvent; hexane - hexane:ethyl acetate = 2:1), the title compound (4.30 g) was obtained.

oily substance:

$^1$H-NMR ($CDCl_3$) δ: 1.95(1H, br s), 2.12-2.34(1H, m), 2.50(1H, dd), 2.89(1H, dd), 3.40-3.70(2H, m), 3.71(1H, dd), 3.85(1H, dd), 5.20(1H, d), 5.27(1H, d), 7.04-7.20(2H, m), 7.24-7.52(5H, m), 7.61(1H, d).

$[\alpha]_D^{20}$ = -19.3° (c = 0.502, methanol).

**Reference Example 19**

1-benzyloxycarbonyl-6-chloro-3-(S)-[(N,N-dimethylamino)methyl]-1,2,3,4-tetrahydroquinoline

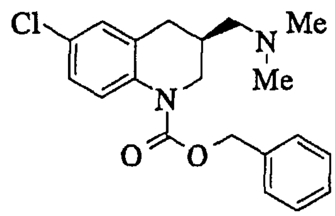

To a solution of 1-benzyloxycarbonyl-6-chloro-3-(R)-(hydroxymethyl)-1,2,3,4-tetrahydroquinoline (4.20 g) in acetonitrile (84 ml) was added triethylamine (3 ml) under ice cooling. Methanesulfonyl chloride (1.2 ml) was added thereto and stirred for 15 minutes. To the reaction solution was added a saturated aqueous solution of sodium hydrogen carbonate and water under ice cooling and the solution was extracted with ethyl acetate. The organic layer was washed water and saturated brine, dried and concentrated. The crude product of 1-benzyloxycarbonyl-6-chloro-3-(R)-[[(methylsulfonyl)oxy]methyl]-1,2,3,4-tetrahydroquinoline was obtained.

The crude product of 1-benzyloxycarbonyl-6-chloro-3-(R)-[[(methylsulfonyl)oxy]methyl]-1,2,3,4-tetrahydroquinoline was dissolved in DMSO (50 ml), an aqueous solution of 50% dimethylamine (25 ml) was added thereto and the solution was stirred at room temperature for 36 hours. To the reaction solution was added an aqueous solution of 5% potassium carbonate and the solution was extracted with ethyl acetate. The organic layer was washed with water and saturated brine, dried and concentrated. The residue was purified by alumina column chromatography (developing solvent; hexane - hexane:ethyl acetate = 10:1) the title compound (4.09 g) was obtained.

oily substance:

$^1$H-NMR ($CDCl_3$) δ: 2.0-2.4(3H, m), 2.20(6H, s), 2.46(1H, dd), 2.87(1H, dd), 3.25(1H, dd), 4.04-4.20(1H, m), 5.20 (1H, d), 5.27(1H, d), 7.04-7.20(2H, m), 7.24-7.52(5H, m), 7.68(1H, d).

$[\alpha]_D^{20}$ = -26.0° (c = 0.503, methanol).

**Reference Example 20**

6-chloro-3-(R)-[(N,N-dimethylamino)methyl]-1,2,3,4-tetrahydroquinoline

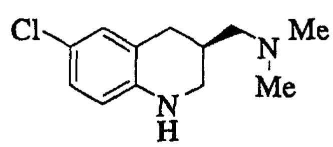

1-benzyloxycarbonyl-6-chloro-3-(S)-[(N,N-dimethylamino)methyl]-1,2,3,4-tetrahydroquinoline (3.89 g)was dissolved in 48% hydrobromic acid (20 ml) and the solution was stirred at room temperature for 18 hours. The reaction solution was diluted with water and the solution was extracted with hexane. To the aqueous layer was added an aqueous solution of 8N sodium hydroxide to adjust pH to 5. Then, potassium carbonate was added thereto to make the solution

to become basic and the solution was extracted with ethyl acetate. The organic layer was washed with water and saturated brine, dried and concentrated. The residue was purified by recrystallizing (hexane-ethyl acetate) and the title compound (1.47 g) was obtained.

melting point: 112-114°C

$[\alpha]_D^{20}$ = -46.7° (c = 0.503, methanol).

**Reference Example 21**

3-[[tert-butyl(diphenyl)silyl]oxy]-2-(S)-(5-chloro-2-nitrobenzyl)-1-propanol

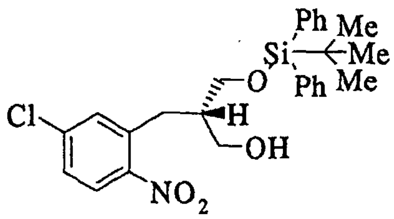

To a solution of 2-(R)-(5-chloro-2-nitrobenzyl)-3-hydroxypropyl propionate (4.21 g) in DMF (20 ml) was added imidazole (2.0 g) and tert-butylchlorodiphenylsilane(4.0 ml) successively under ice cooling. The mixture was stirred at room temperature for 30 minutes. Then, water was added thereto and the reaction solution was extracted with ethyl acetate. The organic layer was washed with water and saturated brine, dried and concentrated. The crude product of 3-[[tert-butyl(diphenyl)silyl]oxy]-2-(S)-(5-chloro-2-nitrobenzyl)propyl propionate was obtained.

To the solution of the crude product of 3-[[tert-butyl(diphenyl)silyl]oxy]-2-(S)-(5-chloro-2-nitrobenzyl)propyl propionate in methanol (40 ml) was added potassium carbonate (2.0 g) and stirred for 4 hours. Water was added to the reaction solution and the solution was extracted with ethyl acetate. The organic layer was washed with water and saturated brine, dried, and concentrated. The residue was purified by silica gel column chromatography (developing solvent; hexane - hexane:ethyl acetate = 5:1) and the title compound (6.15 g) was obtained.

oily substance:

$^1$H-NMR ($CDCl_3$) δ: 1.09(9H, s), 1.94-2.16(1H, m), 2.09(1H, t), 2.90(1H, dd), 3.03(1H, dd), 3.60-3.88 (4H, m) , 7.26-7.56(8H, m), 7.62-7.74(4H, m), 7.89(1H, d).

$[\alpha]_D^{20}$ = +1.2° (c = 0.376, methanol).

**Reference Example 22**

3-(S)-[[[tert-butyl(diphenyl)silyl]oxy]methyl]-6-chloro-1,2,3,4-tetrahydroquinoline

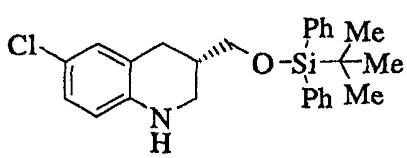

To a solution of 3-[[tert-butyl(diphenyl)silyl]oxy]-2-(S)-(5-chloro-2-nitrobenzyl)-1-propanol (6.00 g) in acetonitrile (120 ml) was added triethylamine (3.0 ml) and methanesulfonyl chloride (1.2 ml) successively under ice cooling. The mixture was stirred for 15 minutes, a saturated aqueous solution of sodium hydrogen carbonate was added thereto under ice cooling and water was also added thereto. The solution was extracted with ethyl acetate. The organic layer was washed with water and saturated brine, dried and concentrated. The crude product of 3-[[tert-butyl(diphenyl)silyl]oxy]-2-(R)-(5-chloro-2-nitrobenzyl)propyl methanesulfonate was obtained.

To the crude product of 3-[[tert-butyl(diphenyl)-silyl]oxy]-2-(R)-(5-chloro-2-nitrobenzyl)propyl methanesulfonate in THF (60 ml) was added acetic acid (60 ml) and zinc powders(12.2 g) successively under ice cooling. The mixture was stirred for 15 minutes under ice cooling. Moreover, the solution was stirred at room temperature for 30

minutes. Zinc powders (8.1 g) was added to the reaction solution and stirred for 30 minutes. The reaction solution was filtered, the filtrate was concentrated under reduced pressure and the crude product of 2-(R)-(2-amino-5-chlorobenzyl)-3-[[tert-butyl(diphenyl)silyl]oxy]propyl methanesulfonate was obtained.

To the crude product of 2-(R)-(2-amino-5-chlorobenzyl)-3-[[tert-butyl(diphenyl)silyl]oxy]propyl methanesulfonate was added THF (80 ml) and diisopropylethylamine (10 ml). The mixture was stirred at 60°C for 10 hours under argon stream. An aqueous solution of saturated sodium hydrogen carbonate was added to the reaction solution and extracted with ethyl acetate. The organic layer was washed with water and saturated brine, dried and concentrated. The residue was purified silica gel column chromatography (developing solvent; hexane - hexane:ethyl acetate = 10:1) and the title compound (5.14 g) was obtained.

oily substance:

$^1$H-NMR ($CDCl_3$) δ: 1.06(9H, s), 1.34-1.80(1H, m), 2.08-2.32 (1H, m), 2.50(1H, dd), 2.71(1H, dd), 3.07(1H, dd), 3.34-3.50(1H, m), 3.52-3.76(2H, m), 6.30-6.42(1H, m), 7.84-7.96(2H, m), 7.20-7.80(10H, m).

$[\alpha]_D^{20}$ = +13.3° (c = 0.433, methanol).

## Reference Example 23

1-benzyloxycarbonyl-6-chloro-3-(S)-(hydroxymethyl)-1,2,3,4-tetrahydroquinoline

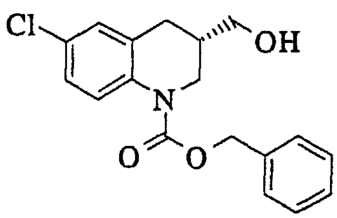

To a solution of 3-(S)-[[[tert-butyl(diphenyl)-silyl]oxy]methyl]-6-chloro-1,2,3,4-tetrahydroquinoline (4.63 g) in ethyl acetate (50 ml) was added an aqueous solution (50 ml) of potassium carbonate (7.3 g). To the reaction solution was dropwise added benzyl chloroformate (3.0 ml) for 15 minutes under ice cooling. Then, the reaction solution was stirred at room temperature for 30 minutes. Water was added to the reaction solution and the solution was extracted with ethyl acetate. The organic layer was washed with water and saturated brine, dried and concentrated. The crude product of 1-benzyloxycarbonyl-3-(S)-[[[tert-butyl(diphenyl)silyl]oxy]methyl]-6-chloro-1,2,3,4-tetrahydroquinoline was obtained.

To the solution of the crude product of 1-benzyloxycarbonyl-3-(S)-[[[tert-butyl(diphenyl)silyl]-oxy]methyl]-6-chloro-1,2,3,4-tetrahydroquinoline in THF (60 ml) was added a solution of tetra-n-butylammonium fluoride in THF (1 M, 30 ml) under ice cooling. The mixture was stirred at room temperature for 6 hours, water was added thereto and extracted with ethyl acetate. The organic layer was washed with water and saturated brine, dried and concentrated. The residue was purified by silica gel column chromatography (developing solvent; hexane - ethyl acetate) and the title compound (3.40 g) was obtained.

oily substance:

$^1$H-NMR ($CDCl_3$) δ: 1.95(1H, br s), 2.12-2.34(1H, m), 2.50(1H, dd), 2.89(1H, dd), 3.40-3.70(2H, m), 3.71(1H, dd), 3.85(1H, dd), 5.20(1H, d), 5.27(1H, d), 7.04-7.20(2H, m), 7.24-7.52(5H, m) , 7.61(1H, d).

$[\alpha]_D^{20}$ = +20.3° (c = 0.381, methanol).

**Reference Example 24**

1-benzyloxycarbonyl-6-chloro-3-(R)-[(N,N-dimethylamino)-methyl]-1,2,3,4-tetrahydroquinoline

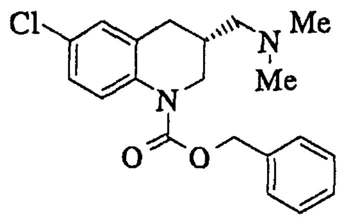

To the solution of 1-benzyloxycarbonyl-6-chloro-3-(S)-(hydroxymethyl)-1,2,3,4-tetrahydroquinoline (3.3 g) in acetonitrile (66 ml) was added triethylamine (3 ml) and methanesulfonyl chloride (1.0 ml) successively under ice cooling. After stirring for 15 minutes, to the reaction solution was added a saturated aqueous solution of sodium hydrogen carbonate under ice cooling. Furthermore, water was added thereto, the reaction solution was extracted with ethyl acetate. The organic layer was washed with water and saturated brine, dried and concentrated. The crude product of 1-benzyloxycarbonyl-6-chloro-3-(S)-[[(methylsulfonyl)oxy]-methyl]-1,2,3,4-tetrahydroquinoline was obtained.

To the mixture of the crude product of 1-benzyloxycarbonyl-6-chloro-3-(S)-[[(methylsulfonyl)-oxy]methyl]-1,2,3,4-tetrahydroquinoline in DMSO (40 ml) was added an aqueous solution of 50% dimethylamine (20 ml). The mixture was stirred at room temperature for 48 hours. An aqueous solution of sodium hydrogen carbonate and water was added to the reaction solution and the solution was extracted with ethyl acetate. The organic layer was washed with water and saturated brine, dried and concentrated. The residue was purified by alumina column chromatography (developing solvent; hexane - hexane:ethyl acetate = 10:1) and the title compound (3.3 g) was obtained.

oily substance:

$^1$H-NMR ($CDCl_3$) δ: 2.0-2.4(3H, m), 2.20(6H, s), 2.46(1H, dd), 2.87(1H, dd), 3.25(1H, dd), 4.04-4.20 (1H, m), 5.20(1H, d), 5.27(1H, d), 7.04-7.20(2H, m), 7.24-7.52 (5H, m), 7.68(1H, d).

$[\alpha]_D^{20}$ = +30.5° (c = 0.158, methanol).

**Reference Example 25**

6-chloro-3-(S)-[(N,N-dimethylamino)methyl]-1,2,3,4-tetrahydroquinoline

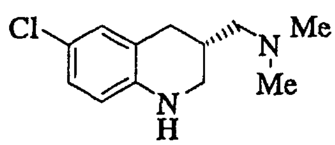

1-benzyloxycarbonyl-6-chloro-3-(R)-[(N,N-dimethylamino)methyl]-1,2,3,4-tetrahydroquinoline (3.2 g) was dissolved in 48% hydrobromic acid (16 ml) and the mixture was stirred at room temperature for 24 hours. The reaction solution was diluted with water and extracted with hexane. To the aqueous layer was added an aqueous solution of 8N sodium hydroxide to adjust its pH to approximately pH 5. Then, potassium carbonate was added thereto to make the solution to become basic. The solution was extracted with ethyl acetate. The organic layer was washed with water and saturated brine, dried and concentrated. The precipitate crystals were washed with hexane and the title compound (1.85 g) was obtained.

melting point: 110-114°C

$[\alpha]_D^{20}$ = +46.7° (c = 0.502, methanol).

**Reference Example 26**

2-[(2-nitrophenyl)methylidene]malonic diethyl

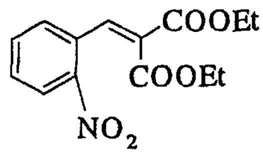

By azeotropic distillation, water was removed from a mixture of 2-nitrobenzaldehyde (10.0 g), malonic diethyl (15 ml), piperidine (1.3 ml) in benzene (90 ml) and while the distillation, the mixture was refluxed under heating for 25 hours. The reaction solution was concentrated and the residue was purified by silica gel chromatography (developing solvent: hexane/ethyl acetate = 4/1) and the title compound (7.16 g) was obtained.

$^1$H-NMR (DMSO-$d_6$) δ: 1.03 (3H, t), 1.36 (3H, t), 4.08 (2H, q), 4.34 (2H, q), 7.43 (1H, d), 7.64 (1H, dt), 7.57 (1H, dt), 8.19(1H, s), 8.21 (1H, dd).

IR (KBr): 1731, 1721, 1526, 1344, 1260, 1214, 1203 $cm^{-1}$.

**Reference Example 27**

2-(2-nitrobenzyl)-1,3-propanediol

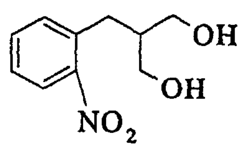

2-[(2-Nitrophenyl)methylidene]malonic diethyl (7.16 g) was dissolved in methanol (25 ml), sodium boron hydride (0.929 g) was added thereto and stirred at room temperature for 1.5 hours. To the reaction solution was added 1 N hydrochloric acid and the mixture was concentrated. The residue was dissolved in ethyl acetate and the solution was washed with water and saturated brine, dried and concentrated. The residue was dissolved in 0.1M phosphate buffer (pH 7.0) 30 ml and THF 30 ml. To the reaction solution was added sodium boron hydride (4.59 g) and the solution was stirred at room temperature for 4.5 hours. Then, to the reaction solution was added saturated ammonium chloride, acetic acid and 1N hydrochloric acid successively and the mixture was extracted with ethyl acetate. The organic layer was washed with saturated brine, dried and concentrated. The residue was purified by silica gel chromatography (developing solvent: hexane/ethyl acetate = 1/5) and the title compound (1.45 g) was obtained.

melting point: 103-104°C (re-crystallized solvent: hexane/ethyl acetate).

**Reference Example 28**

3-hydroxy-2-(2-nitrobenzyl)propyl acetate

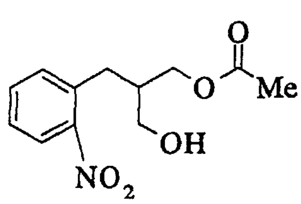

The mixture of 2-(2-nitrobenzyl)-1,3-propanediol (1.02 g), lipase P (amano pharmaceuticals; 1.0 g), vinyl acetate (1.0 ml) was shaked in IPE (300 ml) at 35°C for 5.5 hours. High-performance chromatography analysis was performed on the reaction solution. According to the analysis, the yield of monoacyl body is 92%, the enantiomer excess is 90% ee.

HPLC condition: column; CHIRALPAK AD (Daicel chemical industries)

mobile phase; hexane/2-propanol (925/75)
velocity; 0.8 ml/min
temperature; room temperature
detection; UV (224 nm)
retention time; 22, 27 min.

**Reference Example 29**

2-(bromomethyl)-1-chloro-3-nitrobenzene

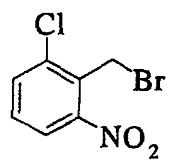

To a solution of 2-chloro-6-nitrobenzaldehyde (25 g) in methanol (600 ml) was added sodium boron hydride (5.1 g) at 0°C and the solution was stirred at the same temperature for 30 minutes. Then, diluted hydrochloric acid was gradually poured into the reaction solution. The mixture was stirred at room temperature and concentrated. To the residue was added ethyl acetate and water to perform separating extraction. The organic layer was washed with saturated brine, dried, concentrated. The residue was purified by silica gel chromatography (developing solvent: hexane/ethyl acetate = 3/1). The obtained crystals were washed with ice-cooled hexane and (2-chloro-6-nitrophenyl)methanol (23.0 g) was obtained.

Under nitrogen stream, to 2-chloro-6-nitrophenylmethanol (22 g) was added 48% hydrobromic acid (250 ml) and the mixture was stirred at 90°C for 30 minutes. The reaction solution was extracted with IPE. The organic layer was washed with saturated brine, dried and concentrated. The residue was purified by silica gel chromatography (developing solvent: hexane/ethyl acetate = 6/1). The obtained solid was washed with hexane and the title compound was obtained (27.2 g).

$^1$H-NMR ($CDCl_3$) δ: 4.88 (2H, s), 7.44 (1H, t), 7.70 (1H, d), 7.87 (1H, d).
IR (KBr): 1523, 1351 $cm^{-1}$.

**Reference Example 30**

2-(2-chloro-6-nitrobenzyl)malonic diethyl

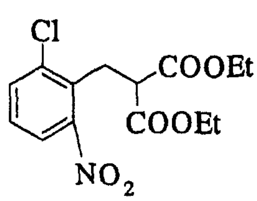

To a solution of malonic diethyl (19.3 ml) in dimethoxyethane (400 ml) was added sodium hydride (oiliness 60%, 5.1 g) at 0°C and added a mixture of 2-(bromomethyl)-1-chloro-3-nitrobenzene (26.5 g) in dimethoxyethane (100

ml) at 0°C successively. The solution was stirred at the same temperature for 30 minutes. Then, cold water was added to the reaction solution, stirred and concentrated. To the residue was added water and ethyl acetate to perform separating extraction. The organic layer was washed with saturated brine, dried and concentrated. The residue was purified by silica gel chromatography (developing solvent: hexane/ethyl acetate = 5/1) and the title compound (35.7 g) was obtained.

$^1$H-NMR ($CDCl_3$) δ: 1.23 (6H, t), 3.67 (2H, d), 3.81 (1H, t), 4.17 (4H, q), 7.36 (1H, t), 7.63 (1H, d), 7.76 (1H, d). IR (neat): 1733, 1534 $cm^{-1}$.

**Reference Example 31**

2-(2-chloro-6-nitrobenzyl)-1,3-propanediol

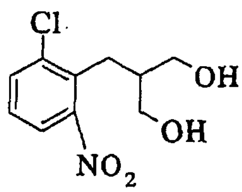

To a solution of 2-(2-chloro-6-nitrobenzyl)malonic diethyl (35 g) in diethylether (275 ml)was added methanol (11.5 ml). To the reaction solution was added lithium boron hydride (6.0 g) at room temperature. The reaction solution was gradually poured into cold diluted hydrochloric acid and the solution was extracted with ethyl acetate. The organic layer was washed with saturated brine, dried and concentrated. The obtained crude product was purified by silica gel chromatography (developing solvent: hexane/ethyl acetate = 2/2.5 - 1/1.5) and the title compound (15.9 g) was obtained.

amorphous powders:

$^1$H-NMR ($CDCl_3$) δ: 2.05-2.20 (1H, m), 2.25 (2H, br s), 3.10 (2H, d), 3.09-3.80 (4H, m), 7.33 (1H, t), 7.63 (1H, d), 7.69 (1H, d).

IR (KBr): 3508, 3431, 1526, 1359 $cm^{-1}$.

**Reference Example 32**

(+)-2-(2-chloro-6-nitrobenzyl)-3-hydroxypropyl-1-propionate

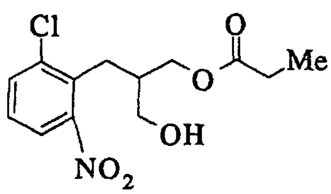

A mixture of 2-(2-chloro-6-nitrobenzyl)-1,3-propanediol (11.02 g), MEITO Lipase AL (Meito Industries; 0.3 g), vinyl propionate (50 ml) was shaked in IPE (1000 ml) at 35°C for 24 hours. High-performance chromatography analysis was performed on the reaction solution. According to the analysis, the yield of monoacyl body was 90% and the enantiomer excess was 98% ee. Enzyme was filtered off and the filtrate was concentrated. The residue was purified by silica gel chromatography (developing solvent: hexane/ethyl acetate = 3/1) and 12.59 g (93%, 96% ee) of the title compound was obtained.

$^1$H-NMR (DMSO-$d_6$) δ: 1.14 (3H, t), 1.8 (1H, br s), 2.28 (1H, m), 2.34 (2H, q), 3.14 (2H, d), , 3.55 (1H, dd), 3.61 (1H, dd), 4.11 (1H, m), 4.21 (1H, dd), 7.34 (1H, t), 7.63 (1H, t), 7.69 (1H, t).

IR (KBr): 3456, 1736, 1531, 1358, 1193, 801 $cm^{-1}$.

$[\alpha]_D^{28}$=+13.3° (c=1.11,ethanol)

HPLC condition: column; CHIRALPAK AD (Daicel chemical industries)

mobile phase; hexane/2-propanol (950/50)
velocity; 0.5 ml/min
temperature; room temperature
detection; UV (225 nm)
retention time; 51, 56 min.

**Reference Example 33**

2-(5-chloro-2-nitrobenzyl)-1,3-propanediol bispropionate

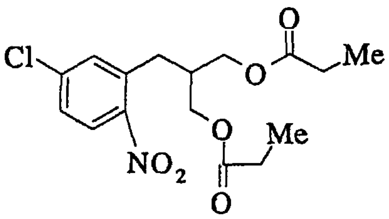

To a solution of 2-(5-chloro-2-nitrobenzyl)-1,3-propanediol (150 mg) in THF (5.0 ml) was added propionyl chloride (0.212 ml) and triethylamine (0.344 ml) successively. The mixture was stirred at room temperature for 5 hours. Then, the reaction solution was concentrated and IPE was added thereto. The organic layer was washed with water, saturated brine, dried and concentrated. The residue was purified by silica gel chromatography (developing solvent: hexane/ethyl acetate = 4/1) and the title compound (219 mg) was obtained.

IR (KBr): 1740, 1526, 1346, 1180, 835 $cm^{-1}$.

**Reference Example 34**

2-(5-chloro-2-nitrobenzyl)-3-hydroxypropyl-1-propionate

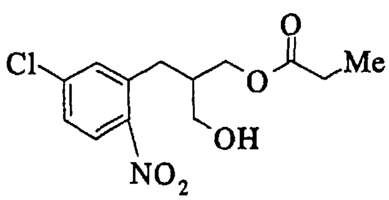

A mixture of 2- (5-chloro-2-nitrobenzyl)-1,3-propanediol-1-propionate (20 mg), Lipase PS (amano pharmaceuticals; 20 mg) and water(0.1 ml) was shaked in IPE (2.0 ml) at 35°C for 24 hours. High-performance chromatography analysis was performed on the reaction solution. According to the analysis, the yield of monoacyl body was 67% and the enantiomer excess was 91% ee.

HPLC condition: column; CHIRALPAK AD (Daicel chemical industries)

mobile phase; hexane/2-propanol (925/75)
velocity; 0.8 ml/min
temperature; room temperature
detection; UV (225 nm)
retention time; 21, 24 min.

**Reference Example 35**

1-[2-(R)-amino-3-(indol-3-yl)propanoyl]-6-chloro-3-(S)-(N,N-dimethylamino)methyl-1,2,3,4-tetrahydroquinoline

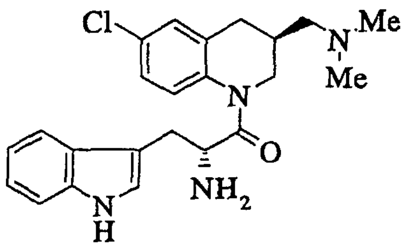

The title compound was obtained according to the same method as Reference Example 11.
IR(KBr): 3280, 2928, 1653, 1487, 1356, 1235, 1098, 743 $cm^{-1}$.
$[\alpha]_D^{20}$ = -240° (c = 0.501, methanol).
MASS (APCIMASS): m/z 411 [$(M+H)^+$].

**Reference Example 36**

3-(R,S)-(N,N-dimethylamino)methyl-1-[3-(indol-3-yl)-2-[(R)-4-(3-methyl-2-oxo-2,3-dihydro-1H-benzimidazol-1-yl)piperidylcarbonylamino]propanoyl]-1,2,3,4-tetrahydroquinoline

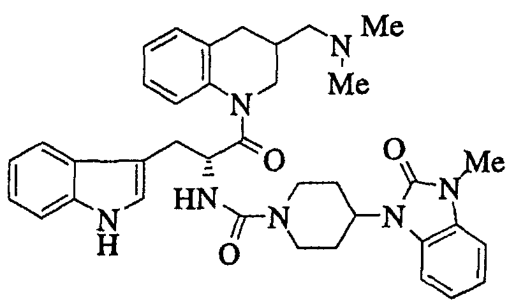

The title compound was obtained according to the same method as the later described Example 21.
IR(KBr): 3252, 2936, 1694, 1634, 1495, 1435, 1246, 750 $cm^{-1}$.
MASS (APCIMASS), m/z 634[$(M+H)^+$].

**Reference Example 37**

1-benzoyl-N-[(1R)-2-[3-(dimethylamino)methyl]-1,2,3,4-tetrahydro-1-quinolinyl]-1-(indol-3-ylmethyl)-2-oxoethyl]-4-piperidine carboxamide

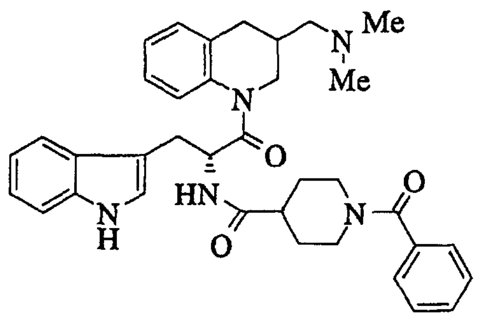

To a mixture of 1-[2-(R)-amino-3-(indol-3-yl)propanoyl]-3-(R,S)-(N,N-dimethylamino)methyl-1,2,3,4-tetrahydroquinoline (151 mg), 1-benzoyl-4-piperidinecarboxylic acid (104 mg) and HOBt (68 mg) in acetonitrile (5 ml) was added WSC (84 mg) and triethylamine (0.07 ml) at room temperature. The mixture was stirred at room temperature for 16 hours. Then, to the reaction solution was added an aqueous solution of 10% potassium carbonate. The solution was extracted with ethyl acetate. The organic layer was washed with saturated brine, dried and concentrated. The residue was purified by alumina column chromatography (developing solvent: ethyl acetate/methanol = 10/1) and the title compound was obtained as amorphous powders (205 mg).

IR(KBr): 3285, 2942, 1634, 1493, 1447, 743, 708 $cm^{-1}$.

**Reference Example 38**

3-(4-piperidinyl)propionic acid hydrochloride

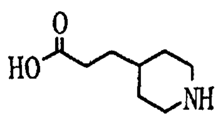

3-[1-(acetyl)-4-piperidinyl]propionic acid (10.34 g)was added to concentrated hydrochloric acid (30 ml) and the mixture was refluxed under heating for 6 hours. The mixture was concentrated and washed with ethanol and IPE. The title compound (9.195 g) was obtained.

melting point: 247-250°C

**Reference Example 39**

3-[1-(trifluoroacetyl)-4-piperidinyl]propionic acid

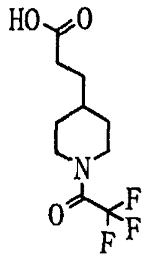

To a solution of 3-(4-piperidinyl)propionic acid hydrochloride (7.77 g) in methanol (40 ml) was added ethyl trifluoroacetate(6 ml) and triethylamine (11.2 ml) and, the solution was stirred at room temperature for 24 hours. A mixture of 4N hydrochloric acid/ethyl acetate (12 ml) was added thereto and concentrated. Ethyl acetate was added to the residue. Insoluble material was filtered off. The filtrate was concentrated. The residue was purified by silica gel column chromatography (developing solvent: ethyl acetate) and the title compound (9.132 g) was obtained.
melting point: 55-60°C.

**Reference Example 40**

(4-hydroxy-1-piperidinyl)(phenyl)methanone

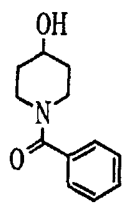

Under ice cooling, to a solution of 4-hydroxy-1-piperidine (10.12 g) and triethylamine (13.9 ml) in THF (100 ml) was added benzoyl chloride (14.06 g). The solution was stirred at room temperature for 12 hours, water was added thereto and extracted with ethyl acetate. The organic layer was washed with saturated brine, dried and concentrated. The residue was purified by silica gel column chromatography (developing solvent: ethyl acetate/hexane = 1/1) and the obtained crystals were washed with IPE. The title compound (14.24 g) was obtained.
melting point: 86-89°C.

**Reference Example 41**

tert-butyl 2-[(1-benzoyl-4-piperidinyl)oxy]acetic acid

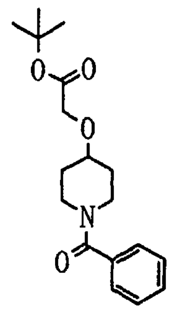

To a solution of (4-hydroxy-1-piperidinyl)-(phenyl)methanone (8.2 g) and tert-butyl bromoacetic acid (11.7 g) in toluene(80 ml) was added an aqueous solution (40 ml) of tetra-butylammonium hydrogensulfate (11.7 g) and NaOH (40 g). The mixture was stirred at room temperature for 24 hours. Then, water was added thereto and the solution was extracted with ethyl acetate. The organic layer was washed with saturated brine, dried and concentrated. The residue was purified by silica gel column chromatography (developing solvent: ethyl acetate/hexane = 1/1) and the obtained crystals were washed with IPE. The title compound was obtained (13.4 g).

$^1$H-NMR ($CDCl_3$) δ: 1.48 (9H, s), 1.54-2.08 (4H, m), 3.10-3.80 (4H, m), 3.84-4.20 (1H, m),4.02 (2H, s), 7.40 (5H, s).

**Reference Example 42**

2-[(1-benzoyl-4-piperidinyl)oxy]acetic acid

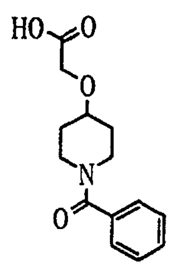

Tert-butyl 2-[(1-benzoyl-4-piperidinyl)oxy]acetic acid (11.46 g) was added to trifluoroacetic acid (35 ml), stirred at room temperature for 3 hours and concentrated. Ethyl acetate was added to the residue. The mixture was washed with water and saturated brine, dried and concentrated. The obtained crystals were washed with diethylether and the title compound was obtained (6.03 g).

melting point: 100-103°C

**Reference Example 43**

cis-4-(benzoylamino)cyclohexanecarboxylic acid

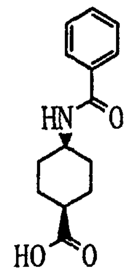

Under ice cooling, to a mixture of ethyl acetate (50 ml), an aqueous solution of 10% sodium carbonate (50 ml) and water (50 ml) was added a mixture of cis-4-aminocyclohexanecarboxylic acid (5.19 g) and benzoyl chloride (14.06 g) in ethyl acetate (20 ml). The mixture was stirred at room temperature for 12 hours and 1N hydrochloric acid (60 ml) was added thereto. The solution was extracted with a mixture of ethyl acetate/THF. The organic layer was washed with saturated brine, dried and concentrated. The obtained crystals were washed with IPE. The title compound (6.86 g) was obtained was obtained.

melting point: 194-196°C.

The compounds mentioned in the following Reference Examples were synthesized according to the same method as Reference Example 13.

**Reference Example 44**

2-(2,6-dinitrobenzyl)malonic diethyl

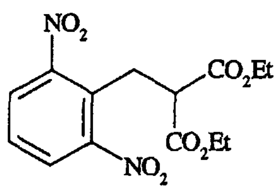

$^1$H-NMR ($CDCl_3$) δ: 1.23 (6H, t, J = 7.1 Hz), 3.71 - 3.75 (3H, m), 4.18 (4H, q, J = 7.1 Hz), 7.60 (1H, d, J = 8.2 Hz), 8.06 (2H, d, J = 8.1 Hz).

**Reference Example 45**

5-amino-2-oxo-1,2,3,4-tetrahydro-3-quinolinecarboxylic acid ethyl

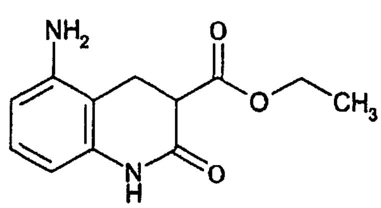

2-(2,6-dinitrobenzyl)malonic diethyl (6.47 g) was dissolved in ethanol (60 ml) and THF (60 ml). 10% Palladium-carbon (0.65 g) was added to the solution and the mixture was stirred at room temperature for 64 hours under 1 torr hydrogen atmosphere. Catalyst was filtered off and the solvent was concentrated. The obtained residue was washed with ethyl acetate/IPE, the title compound was obtained as yellowish powders (4.97 g).

$^1$H-NMR ($CDCl_3$) δ: 1.26 (3H, t, J = 7.1 Hz), 2.93 (1H, dd, J = 6.7, 15.8 Hz), 3.17 (1H, dd, J = 8.9, 15.8 Hz), 3.62 (1H, dd, J = 6.7, 9.0 Hz), 3.72 (2H, s), 4.23 (2H, q, J = 7.2 Hz), 6.24 (1H, d, J = 8.0 Hz), 6.42 (1H, d, J = 8.0 Hz), 6.99 (1H, t, J = 8.0 Hz), 8.10 (1H, s).

**Reference Example 46**

benzyl 3-[(dimethylamino)carbonyl]-2-oxo-1,2,3,4-tetrahydro-5-quinolylcarbamate

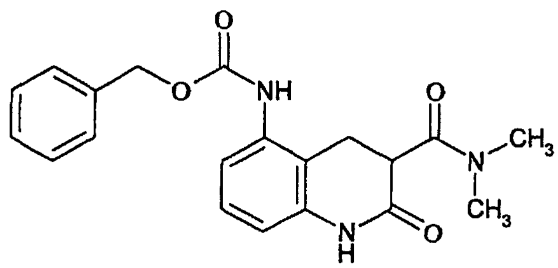

To a suspension of 5-amino-2-oxo-1,2,3,4-tetrahydro-3-quinolinecarboxylic acid ethyl (4.97 g) in THF (70 ml) was added an aqueous solution (70 ml) of sodium carbonate (7.15 g). The solution was cooled by ice and benzyl chloroformate (2.90 ml) was dropwise added thereto. The mixture was stirred for an hour. Then, water was added to the reaction solution and the solution was extracted with ethyl acetate.

To the suspension of the obtained residue in ethanol (50 ml) was added an aqueous solution (13 ml) of 2N sodium hydroxide. The mixture was stirred at 50°C for 1.5 hours and the solution was cooled down. 1N Hydrochloric acid (26 ml) was added thereto and stirred for an hour. The precipitates from the reaction were filtered and collected, washed with water and hexane and dried.

The obtained residue was added to a solution of dimethylamine hydrochloride (0.98 g), HOBt (2.23 g), WSC (2.88 g) and triethylamine (1.67 ml) in acetonitrile (70 ml). The reaction mixture was stirred at 50°C for 12 hours. After the solution was cooled down, an aqueous solution of 10% sodium hydrogen carbonate was added thereto. The precipitates from the reaction were filtered and collected, washed with water and IPE successively and dried. The title compound was obtained as light brown powders (3.19 g).

$^1$H-NMR (DMSO-$d_6$) δ: 2.87 - 3.30 (8H, m), 3.92 (1H, dd, J = 7.1, 12.0 Hz), 5.12 (2H, s), 6.70 (1H, d, J = 7.6 Hz), 7.00 (1H, d, J = 7.3 Hz), 7.11 (1H, t, J = 7.9 Hz), 7.35 - 7.44 (5H, m), 9.17 (1H, s), 10.32 (1H, s).

**Reference Example 47**

5-amino-N,N-dimethy-2-oxo-1,2,3,4-tetrahydro-3-quinolinecarboxamide

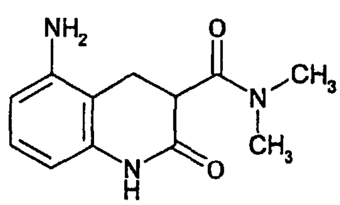

Benzyl 3-[(dimethylamino)carbonyl]-2-oxo-1,2,3,4-tetrahydro-5-quinolinyl carbamate(3.17 g) was dissolved in the mixture solvent of THF (120 ml)/methanol(120 ml) at 70°C. 10% Palladium-carbon (0.65 g) was added to this solution and stirred at 70°C for 12 hours under 1 torr hydrogen atmosphere. The solution was cooled down. Catalyst

was filtered off and the solvent was concentrated. The obtained residue was washed with THF/diethylether and the title compound was obtained as light yellowish powders (2.01 g).

$^1$H-NMR (DMSO-$d_6$) δ: 2.78 - 2.88 (5H, m), 3.02 (3H, s), 3.94 (1H, t, J = 10.3 Hz), 5.00 (2H, s), 6.11 (1H, d, J = 7.3 Hz), 6.27 (1H, d, J = 8.0 Hz), 6.80 (1H, t, J = 7.9 Hz), 10.04 (1H, s).

**Reference Example 48**

3-[(dimethylamino)methyl]-1,2,3,4-tetrahydro-5-quinolineamine

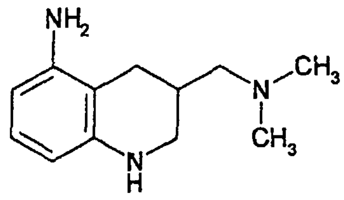

To a solution of 5-amino-N,N-dimethyl-2-oxo-1,2,3,4-tetrahydro-3-quinolinecarboxamide (2.01 g) in THF (20 ml) was added a solution of borane-THF complex in THF (1M, 100 ml). The reaction solution was refluxed under heating for 3.5 hours, water was added to the reaction solution under ice cooling and concentrated. The residue was dissolved in methanol (15 ml), 6N hydrochloric acid (60 ml) was added thereto and refluxed under heating for 12 hours. The reaction solution was cooled down then, an aqueous solution of sodium hydroxide was added thereto to make the solution to become basic. The solution was extracted with THF/ethyl acetate = 1/1. The organic layer was dried and concentrated. The residue was purified by alumina column chromatography (developing solvent; hexane/ethyl acetate = 2/1 - 1/1 - 1/2) and the title compound was obtained as a yellowish oily substance (1.02 g).

$^1$H-NMR ($CDCl_3$) δ: 2.09 - 2.25 (10H, m), 2.58 - 2.68 (1H, m), 2.87 - 2.97 (1H, m), 3.30 - 3.38 (1H, m), 3.55 (2H, s), 6.01 (1H, dd, J = 1.1, 7.9 Hz), 6.08 (1H, dd, J = 1.1, 8.9 Hz), 6.81 (1H, t, J = 7.9 Hz).

**Reference Example 49**

N-{3-[(dimethylamino)methyl]-1,2,3,4-tetrahydro-5-quinolinyl}acetamide

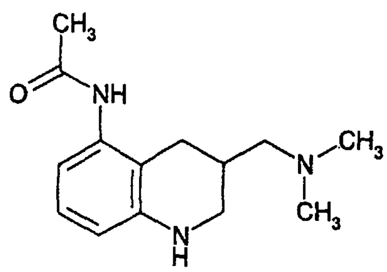

To a solution of 3-[(dimethylamino)methyl]-1,2,3,4-tetrahydro-5-quinolineamine (1.02 g) in pyridine (10 ml) was dropwise added a solution of acetic anhydride (0.472 ml) in THF (3 ml)under ice cooling. The solution was stirred for two hours. The reaction solution was concentrated and the residue was purified by alumina column chromatography (developing solvent: hexane/ethyl acetate = 1/1 - ethyl acetate - ethyl acetate/ethanol=20/1). The title compound was obtained as amorphous powders (1.04 g).

$^1$H-NMR ($CDCl_3$) δ: 2.17 - 2.33 (13H, m) , 2.72 (1H, dd, J = 2.6, 14.4 Hz), 2.89 - 2.99 (1H, m), 3.30 - 3.38 (1H, m), 3.95 (1H, s), 6.36 (1H, d, J = 7.3 Hz), 6.92 - 7.08 (3H, m).

The compounds mentioned in the following Reference Example 50-52 were synthesized according to the same method as Reference Example 11.

**Reference Example 50**

N-{1-[(2R)-2-amino-3-(1-indol-3-yl)propanoyl]-3-[(dimethylamino)methyl]-1,2,3,4-tetrahydro-5-quinolinyl}acetamide

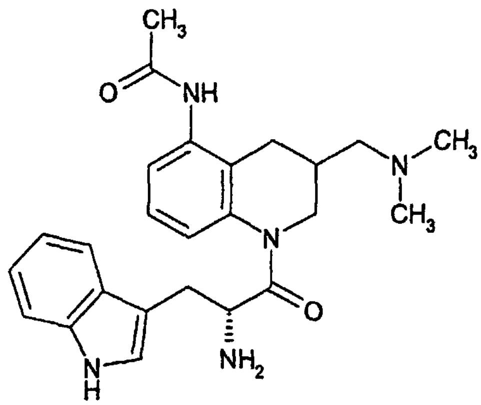

IR(KBr): 3287, 2940, 1651, 1456, 1289, 745 $cm^{-1}$.
MASS (APCIMASS), m/z 434 $[(M+H)^+]$.

**Reference Example 51**

1-[2-(R)-amino-3-(indol-3-yl)propanoyl]-6-chloro-3-(R)-(N,N-dimethylamino)methyl-1,2,3,4-tetrahydroquinoline

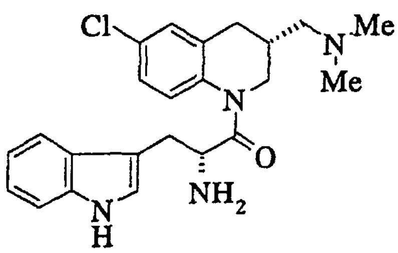

To a solution of N-(9-fluorenylmethoxycarbonyl)-D-triptophan (6.5 g) and DMF (0.57 ml) in THF (200 ml) was dropwise added a solution of oxalylchloride (8.20 ml) in THF (50 ml) at 0°C. The mixture was stirred at 0°C for 90 minutes and concentrated. The residue was dissolved in THF (300 ml). This reaction solution was dropwise added to a solution of 6-chloro-3-(N,N-dimethylamino)methyl-1,2,3,4-tetrahydroquinoline (6.50 g),tetrabutylammonium hydrogensulfate (0.50 g) and sodium hydroxide (powder, 4.00 g) in THF (80 ml) at 0°C. The mixture was stirred at room temperature for 30 minutes. The reaction solution was poured into ice-cold water (400 ml) and extracted with ethyl acetate (500 ml). The organic layer was washed with water (400 ml) and saturated brine (300 ml), dried and concentrated. The residue was purified by alumina column chromatography (developing solvent; ethyl acetate) and silica gel column chromatography (developing solvent; hexane - ethyl acetate 1:1, ethyl acetate, ethyl acetate-methanol 10:1). The obtained light yellow amorphous was dissolved in methanol (200 ml), piperidine (2 ml) was added thereto and stirred at room temperature for 24 hours. The reaction solution was concentrated and purified by alumina column chromatography (developing solvent; ethyl acetate/hexane = 1:2 - ethyl acetate/methanol = 10:1). The title compound was obtained as amorphous powders (8.91 g).

IR(KBr): 2942, 1645, 1487, 1456, 743 $cm^{-1}$.
$[\alpha]_D^{20}$=-198.4° (c=0.301, MeOH)

**Reference Example 52**

1-[2-(S)-amino-3-(indol-3-yl)propanoyl]-5-chloro-3-(R,S)-(N,N-dimethylamino)methyl-1,2,3,4-tetrahydroquinoline

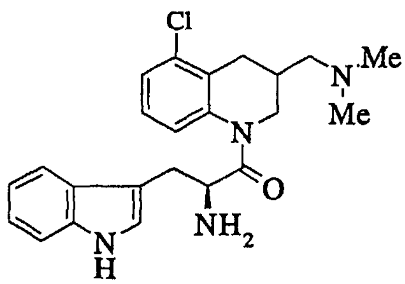

To a solution of N-(9-fluorenylmethoxycarbonyl)-L-tryptophan(3.30 g) and DMF(0.10 ml) in THF (30 ml) was dropwise added a solution of oxalylchloride(0.75 ml) in THF (20 ml) at 0°C. The mixture was stirred at room temperature for two hours, and then concentrated. The residue was dissolved in THF (30 ml). To this solution was dropwise added to 5-chloro-3-(N,N-dimethylamino)methyl-1,2,3,4-tetrahydroquinoline (0.50 g) and a solution of triethylamine (0.75 ml) in THF (15 ml) at 0°C. The mixture was stirred at 0°C for 30 minutes. Then, to the reaction solution was added to ice-cold water (50 ml) and extracted with ethyl acetate (50 ml). The organic layer was washed with saturated brine, dried and concentrated. The residue was purified by alumina column chromatography (developing solvent; ethyl acetate/hexane = 1:2 - 1:1). The obtained light yellow amorphous was dissolved in methanol (10 ml) and piperidine (0.5 ml) was added thereto. The solution was stirred at room temperature for 12 hours. The reaction solution was concentrated and purified with alumina column chromatography (developing solvent; ethyl acetate/hexane = 1:2 - ethyl acetate/methanol = 10:1). The title compound was obtained as amorphous powders (0.51 g).

$^1$H-NMR ($CDCl_3$) δ: 1.99-2.17(4H,m), 2.15(6H, s), 2.5(1H, br m), 2.8-3.2(2H, br m), 3.5(1H, br m), 4.5(2H, m), 6.9-7.4(8H, m), 7.9(1H, br m).

**Reference Example 53**

1-[2-(R)-amino-3-(1-indol-3-yl)-3-propanoyl] 6-chloro-3-(R)-(1-pyrrolidinylmethyl)-1,2,3,4-tetrahydroquinoline

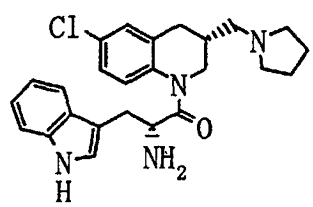

The title compound was obtained according to the same method as Reference Example 51.

IR(KBr): 2965, 2793, 1647, 1487, 741 $cm^{-1}$.

MASS (FAB), m/z 625.2[$(M+H)^+$]

**Reference Example 54**

6-chloro-3-(S)-(1-pyrrolidinylmethyl)-1,2,3,4-tetrahydroquinoline

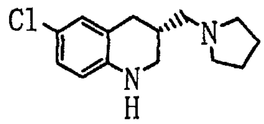

The title compound was obtained according to the same method as Reference Example 25.
IR(KBr): 3250, 2913, 2793, 1607, 1495, 1304, 1283, 1121, 801 $cm^{-1}$.
MASS (FAB), m/z 625.2[$(M+H)^+$]

**Reference Example 55**

1-benzyloxycarbonyl-6-chloro-3-(R)-(1-pyrrolidinylmethyl)-1,2,3,4-tetrahydroquinoline

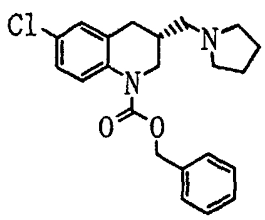

The title compound was obtained according to the same method as Reference Example 24.
IR(KBr): 2957, 2786, 1713, 1485, 1024, 816, 762, 737 $cm^{-1}$.
MASS (FAB), m/z 625.2[$(M+H)^+$]

**Reference Example 56**

1-[(1-methyl-1-indol-2-yl)carbonyl]-4-piperidinecarboxylic acid

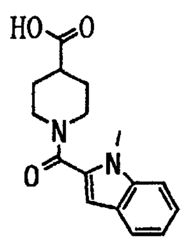

To the mixture of isonipecotic acid ethyl (5.50 g) in acetonitrile (70 ml)-THF(35 ml) was added (1-methyl-1-indol-2-yl)carboxylic acid (6.13 g), WSC (8.03 g), HOBt (5.39 g). The mixture was stirred at room temperature for over night. To the reaction solution was added a saturated aqueous solution of sodium hydrogen carbonate and the mixture was extracted with ethyl acetate. The ethyl acetate layer was washed with 1N hydrochloric acid, dried and concentrated. The obtained residue was dissolved in a mixture solution of methanol (50 ml)-THF (100 ml), an aqueous

solution of 1N sodium hydroxide (50 ml) was added thereto and stirred at room temperature for over night. To the reaction solution was added 1N hydrochloric acid (70 ml) and the mixture was extracted with ethyl acetate. The ethyl acetate layer was washed with saturated brine, dried and concentrated. The obtained crude crystals were washed with IPE and the title compound (9.505 g) was obtained.

melting point: 190-192°C

**Reference Example 57**

1-[(5-fluoro-1-methyl-1-indol-2-yl)carbonyl]-4-piperidinecarboxylic acid

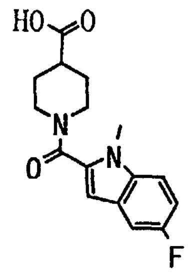

The title compound was obtained according to the same method as Reference Example 56.

IR(KBr): 2928, 1720, 1613, 1468, 1190, 787 $cm^{-1}$

**Reference Example 58**

4-[(benzoylamino)methyl]cyclohexane carboxylic acid

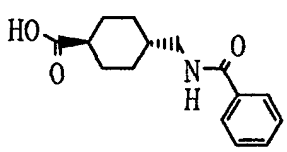

Under ice cooling, to a mixture of tranexamic acid (3.14 g), potassium carbonate (8.29 g) and water (120 ml) was added a solution of benzoyl chloride (2.81 g) in ethyl acetate (12 ml). The solution was stirred at room temperature for 12 hours. Then, 1N hydrochloric acid was added thereto and the pH of the solution was adjusted to approximately pH 4. The obtained precipitates were filtered and collected, washed with water and dried. The obtained crystals were washed with hexane and the title compound (4.76 g) was obtained.

$^1$H-NMR (DMSO-$d_6$) δ: 0.96 - 1.16 (2H, m), 1.34 - 1.70 (3H, m), 1.87 - 1.95 (2H, m), 2.02 - 2.10 (2H, m), 2.21 - 2.35 (1H, m), 3.33 (2H, t, J = 6.5 Hz), 6.26 (1H, t, J = 5.4 Hz), 7.38 - 7.54 (3H, m), 7.74 - 7.79 (2H, m).

**Reference Example 59**

tert-butyl N-(4-benzylidenepiperidin-1-yl)carbamate

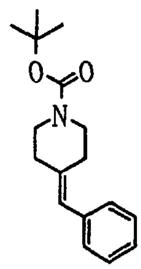

The mixture of benzylbromide (2.58 g) and phosphorous acid triethyl (2.49 g) was stirred at 100°C for 8 hours under heating. The reaction mixture was cooled down and dried under reduced pressure. Benzylphosphoric acid diethyl was obtained as a colorless oily substance.

To the solution of N-Boc-piperidone (2.99 g) and the above-mentioned product in THF (40 ml) was added sodium hydride and 60% oily substance (1.76 g) at room temperature. The solution was stirred at 80°C for 1.5 hours and cooled down. Then, the reaction mixture was poured into water and extracted with ethyl acetate. The organic layer was dried and the solvent was removed by evaporation. The residue was purified by silica gel column chromatography (developing solvent; hexane/ethyl acetate = 20:1 - 10:1) and the title compound (1.58 g) was obtained.

$^1$H-NMR ($CDCl_3$) δ: 1.47 (9H, s), 2.33 (2H, dt, J = 0.8, 5.9 Hz), 2.46 (2H, dt, J = 1.0, 5.9 Hz), 3.40 (2H, t, J = 6.2 Hz), 3.51 (2H, t, J = 5.9 Hz), 6.36 (1H, s), 7.17 - 7.33 (5H, m).

**Reference Example 60**

tert-butyl N-[4-[(2-fluorophenyl)methylidene]piperidin-1-yl]carbamate

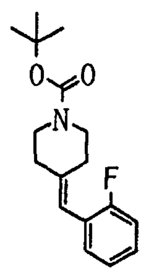

The title compound was obtained according to the same method as Reference Example 59.

$^1$H-NMR ($CDCl_3$) δ: 1.48 (9H, s), 2.31-2.39 (4H, m), 3.41 (2H, t, J = 6.0 Hz), 3.52 (2H, t, J = 5.7 Hz), 6.27 (1H, s), 6.96 - 7.18 (4H, m).

**Reference Example 61**

tert-butyl N-[4-(2-fluorobenzyl)piperidin-1-yl]carbamate

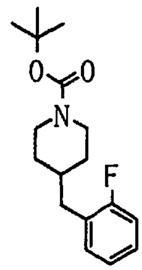

To a solution of tert-butyl N-[4-[(2-fluorophenyl)methylidene]piperidin-1-yl]carbamate (2.67 g) in methanol (50 ml) was added 10% palladium-carbon (0.27 g) and the solution was stirred at room temperature for 12 hours under hydrogen atmosphere. Insoluble material was filtered off and the mother liquid was concentrated. The residue was purified by silica gel column chromatography (developing solvent; hexane/ethyl acetate = 10:1) and the title compound (2.65 g) was obtained.

$^1$H-NMR ($CDCl_3$) δ: 1.08-1.27 (2H, m), 1.45 (9H, s), 1.69-1.79 (3H, m), 2.63-2.77 (4H, m), 3.30 (2H, d, J = 6.1 Hz), 4.05 -4.14 (2H, m), 4.50 (2H, s), 7.26-7.35(5H, s) .

**Reference Example 62**

tert-butyl N-[4-(4-chlorophenoxy)piperidin-1-yl]carbamate

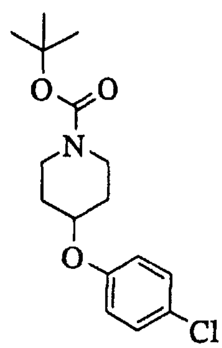

To a suspension of sodium hydride, 60% oily substance (1.0 g) in N,N-dimethylformamide (100 ml) was added 4-chlorophenol (3.21 g) at room temperature and the solution was stirred for 15 minutes. To the reaction mixture was added tert-butyl 4-[(methylsulfonyl)oxy]-1-piperidine carboxylate (6.98 g) at room temperature. The reaction mixture was stirred at 80°C for 8 hours and cooled down. Then, the reaction mixture was poured into water and extracted with ethyl acetate. The organic layer was washed with water, dried and the solvent was removed by evaporation. The residue was purified by silica gel column chromatography (developing solvent; hexane/ethyl acetate = 10:1 - 5:1) and the title compound (3.52 g) was obtained.

$^1$H-NMR ($CDCl_3$) δ: 1.47 (9H, s), 1.65-1.96 (4H, m), 3.29-3.39 (2H, m), 3.62-3.76 (2H, m), 4.36-4.46 (1H, m), 6.84 (2H, dd, J = 2.2, 6.8 Hz), 7.23 (2H, dd, J = 2.4, 6.8 Hz).

The compounds of the following Reference Example 63-64 were synthesized by the same method as Reference Example 62.

**Reference Example 63**

tert-butyl N-[4-(4-methoxyphenoxy)piperidin-1-yl]carbamate

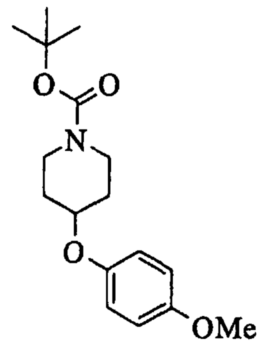

$^1$H-NMR ($CDCl_3$) δ: 1.47 (9H, s), 1.63-1.96 (4H, m), 3.23-3.35 (2H, m), 3.65-3.77 (5H, m), 4.26-4.38 (1H, m), 6.78 (2H, s), 6.84-6.85 (2H, m).

**Reference Example 64**

tert-butyl N-[4-(4-fluorophenoxy)piperidin-1-yl]carbamate

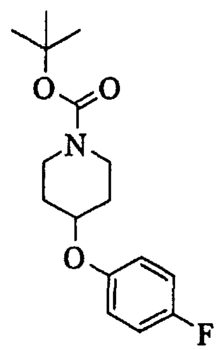

$^1$H-NMR ($CDCl_3$) δ: 1.47 (9H, s), 1.67-1.96 (4H, m), 3.25-3.37 (2H, m), 3.64-3.74 (2H, m), 4.32-4.42 (1H, m), 6.78-7.01 (4H, s).

**Reference Example 65**

tert-butyl N-[4-(4-cyanophenoxy)piperidin-1-yl]carbamate

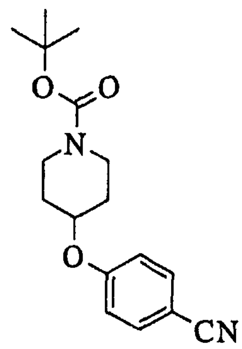

To a suspension of sodium hydride, 60% oily substance (0.44 g) in N,N-dimethylformamide (20 ml) was added tert-butyl 4-hydroxymethyl-1-piperidinecarboxylate (2.01 g) at room temperature and the solution was stirred for 15 minutes. To the reaction mixture was added 4-fluorobenzonitrile (1.45 g) at room temperature, and the mixture was stirred for 4 hours. The reaction mixture was poured into water and extracted with ethyl acetate. The organic layer was washed with water, dried and the solvent was removed by evaporation. The residue was purified by silica gel column chromatography (developing solvent; hexane/ethyl acetate = 10:1 - 5:1) and the title compound (2.12 g) was obtained.
$^1$H-NMR ($CDCl_3$) δ: 1.47 (9H, s), 1.67-2.02 (4H, m), 3.30-3.43 (2H, m), 3.63-3.75 (2H, m), 4.50-4.60 (1H, m), 6.95 (2H, dd, J = 2.0, 6.9 Hz), 7.58 (2H, dd, J = 2.1, 7.0 Hz).

**Reference Example 66**

tert-butyl N-[4-(benzyloxymethyl)piperidin-1-yl]carbamate

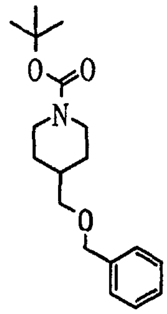

To a suspension of sodium hydride, 60% oily substance (2.2 g) in THF (120 ml) was added N-Boc-4-(hydroxymethyl)piperidine (5.38 g) at room temperature. The solution was stirred for 15 minutes. To the reaction mixture was added benzyl bromide (6.54 ml) at room temperature. The mixture was stirred at 80°C for two hours under heating. After cooling down, the reaction mixture was poured into water and extracted with ethyl acetate. The organic layer was dried and the solvent was removed by evaporation. The residue was purified with silica gel column chromatography (developing solvent; hexane/ethyl acetate = 20:1 - 10:1 - 5:1) and the title compound (5.26 g) was obtained.

$^1$H-NMR ($CDCl_3$) δ: 1.47 (9H, s), 2.33 (2H, dt, J = 0.8, 5.9 Hz), 2.46 (2H, dt, J = 1.0, 5.9 Hz), 3.40 (2H, t, J = 6.2 Hz), 3.51 (2H, t, J = 5.9 Hz), 6.36 (1H, s), 7.17 - 7.33 (5H, m).

**Reference Example 67**

tert-butyl N-[4-(phenoxy)piperidin-1-yl]carbamate

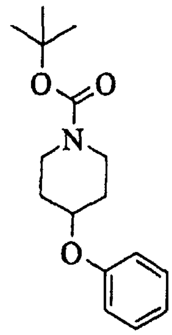

To a solution of tert-butyl N-[4-(4-chlorophenoxy)piperidin-1-yl]carbamate (2.73 g) in methanol (40 ml) was added 10% palladium-carbon (0.54 g) and the mixture was stirred at room temperature for 12 hours under hydrogen atmosphere. Insoluble material was filtered off. Then, the mother liquid was concentrated. The residue was purified with silica gel column chromatography (developing solvent; hexane/ethyl acetate = 10:1) and the title compound (1.94 g) was obtained.

$^1$H-NMR ($CDCl_3$) δ: 1.47 (9H, s), 1.64-1.96 (4H, m), 3.26-3.39 (2H, m), 3.63-3.76 (2H, m), 4.40-4.50 (1H, m), 6.83-6.98 (3H, m), 7.16-7.31 (2H, m).

**Reference Example 68**

tert-butyl N -[4-[(4-chlorophenyl)thio]piperidin-1-yl]carbamate

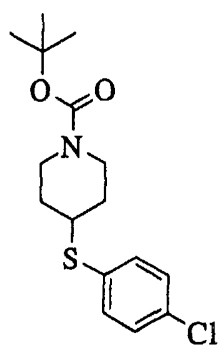

To a suspension of 4-chlorothiophenol (2.82 g) and potassium carbonate (3.11 g) in N,N-dimethylformamide (70 ml) was added tert-butyl 4-[(methylsulfonyl)oxy]-1-piperidinecarboxylate (4.19 g) at room temperature and the mixture was stirred at 70°C for 12 hours under heating. After cooling down, the reaction mixture was poured into water and extracted with ethyl acetate. The organic layer was washed with an aqueous solution of 2N sodium hydroxide and saturated brine successively. The layer was dried and the solvent was removed by evaporation. The residue was purified by silica gel column chromatography (developing solvent; hexane/ethyl acetate = 10:1 - 5:1) and the title compound (4.23 g) was obtained.

$^1$H-NMR ($CDCl_3$) δ: 1.45 (9H, s), 1.50-1.60 (2H, m), 1.84-2.05 (2H, m), 2.84-2.98 (2H, m), 3.11-3.22 (1H, m), 3.93-4.00 (2H, m), 7.25-7.38 (4H, m).

**Reference Example 69**

tert-butyl N-[4-[(4-fluorophenyl)thio]piperidin-1-yl]carbamate

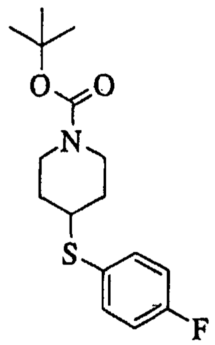

The title compound was obtained by the same method as Reference Example 68.

$^1$H-NMR ($CDCl_3$) δ: 1.44 (9H, s), 1.50-1.60 (2H, m), 1.82-1.94 (2H, m), 2.80-2.95 (2H, m), 3.02-3.16 (1H, m), 4.92-4.05 (2H, m), 6.97-7.05 (2H, m), 7.39-7.46 (2H, m).

**Reference Example 70**

tert-butyl N-[4-[(4-chlorophenyl)sulfonyl]piperidin-1-yl]carbamate

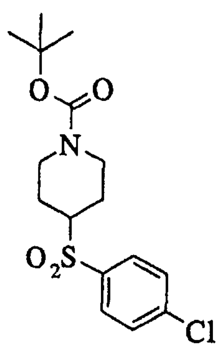

To a solution of tert-butyl N-[4-[(4-chlorophenyl)sulfonyl]piperidin-1-yl]carbamate (2.26 g) in acetone (50 ml) was added 70% m-chloroperbenzoic acid (3.45 g) at 0°C. The mixture was stirred at room temperature for 3 hours. The reaction mixture was poured into saturated aqueous solution of sodium hydrogen carbonate and extracted with ethyl acetate. The organic layer was washed with an aqueous solution of 2N sodium hydroxide and saturated brine successively, dried and the solvent was removed by evaporation. The residue was purified by silica gel column chromatography (developing solvent; hexane/ethyl acetate = 10:1 - 5:1 - 2:1) and the title compound (1.30 g) was obtained.

$^1$H-NMR ($CDCl_3$) δ: 1.43 (9H, s), 1.50-1.70 (2H, m), 1.94-2.00 (2H, m), 2.59-2.72 (2H, m), 2.94-3.10 (1H, m), 4.20-4.27 (2H, m), 7.56 (2H, dd, J = 2.1, 6.8 Hz), 7.81 (2H, dd, J = 2.0, 6.7 Hz).

**Reference Example 71**

tert-butyl N-[4-[(4-fluorophenyl)sulfonyl]piperidin-1-yl]carbamate

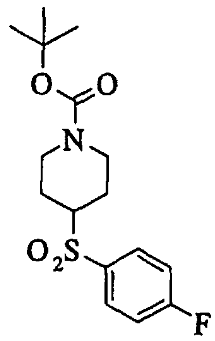

The title compound was obtained by the same method as Reference Example 70.

$^1$H-NMR ($CDCl_3$) δ: 1.43 (9H, s), 1.50-1.70 (2H, m), 1.94-2.05 (2H, m), 2.58-2.74 (2H, m), 2.94-3.10 (1H, m), 4.19-4.27 (2H, m), 7.22-7.31 (2H, m), 7.85-7.92 (2H, m).

**Reference Example 72**

4-benzylidenepiperidine• hydrochloride

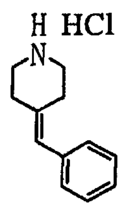

To a solution of tert-butyl N-(4-benzylidenepiperidin-1-yl)carbamate (1.58 g) in methanol (25 ml) was added concentrated hydrochloric acid (1.5 ml) at room temperature. The mixture was stirred at 60°C for 1.5 hours. After cooling down, the solvent was removed by evaporation. The obtained residue was washed with methanol/diethylether and dried. The title compound was obtained (1.08 g).

$^1$H-NMR (DMSO-$d_6$) δ: 2.51-2.68 (4H, m), 3.05-3.37 (4H, m), 6.46 (1H, s), 7.22 - 7.41 (5H, m), 9.39 (2H, s).

The following compounds mentioned in Reference Examples 73-84 were synthesized by the same method as Reference Example 72.

**Reference Example 73**

4-[(2-fluorophenyl)methylidene]piperidine • hydrochloride

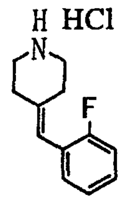

$^1$H-NMR (DMSO-$d_6$) δ: 2.50-2.65 (4H, m), 3.07-3.38 (4H, m), 6.38 (1H, s), 7.16 - 7.35 (4H, m), 9.39 (2H, s).

**Reference Example 74**

4-(2-fluorobenzyl)piperidine• hydrochloride

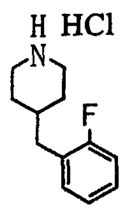

$^1$H-NMR (DMSO-$d_6$) δ: 1.33-1.52 (2H, m), 1.66-1.90 (3H, m), 2.57 (2H, d, J = 7.0 Hz), 2.68-2.86 (2H, m), 3.17-3.28 (2H, m), 7.10 - 7.32 (4H, m), 8.91 (1H, s), 9.15 (1H, s).

**Reference Example 75**

4-(benzyloxymethyl)piperidine• hydrochloride

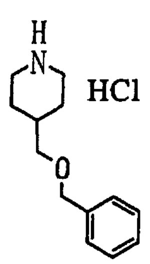

$^1$H-NMR (DMSO-$d_6$) δ: 1.71-1.90 (2H, m), 2.16-2.38 (3H, m), 3.11-3.29 (2H, m), 3.57-3.76 (4H, m), 4.85 (2H, s), 7.63 - 7.78 (5H, m), 9.24 (1H, s), 9.53 (1H, s).

**Reference Example 76**

4-(4-chlorophenoxy)piperidine• hydrochloride

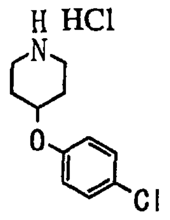

$^1$H-NMR (DMSO-$d_6$) δ: 1.78-1.93 (2H, m), 2.08-2.17 (2H, m), 2.98-3.27 (4H, m), 4.62-4.69 (1H, m), 7.02-7.08 (2H, m), 7.30 - 7.39 (2H, m), 9.26 (2H, s).

**Reference Example 77**

4-(4-methoxyphenoxy)piperidine• hydrochloride

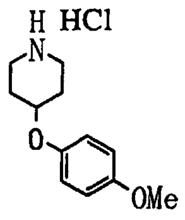

$^1$H-NMR (DMSO-$d_6$) δ: 1.73-1.90 (2H, m), 2.02-2.12 (2H, m), 2.96-3.27 (4H, m), 3.70 (3H, s), 4.45-4.58 (1H, m) , 6.83-6.97 (4H, m), 9.11 (2H, s).

**Reference Example 78**

4-(4-cyanophenoxy)piperidine• hydrochloride

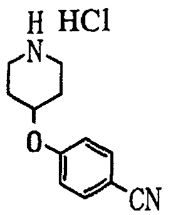

$^1$H-NMR (DMSO-$d_6$) δ: 1.82-1.97 (2H, m), 2.10-2.20 (2H, m), 3.00-3.27 (4H, m), 4.79-4.87 (1H, m), 7.19 (2H, d, J = 8.9 Hz), 7.79 (2H, d, J = 8.9 Hz), 9.30 (2H, s).

**Reference Example 79**

4-(4-fluorophenoxy)piperidine• hydrochloride

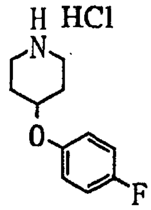

$^1$H-NMR (DMSO-$d_6$) δ: 1.82-1.97 (2H, m), 2.10-2.20 (2H, m), 3.00-3.27 (4H, m), 4.79-4.87 (1H, m), 7.19 (2H, d, J = 8.9 Hz), 7.79 (2H, d, J = 8.9 Hz), 9.30 (2H, s).

**Reference Example 80**

4-phenoxypiperidine hydrochloride

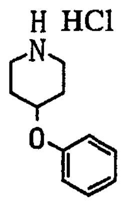

$^1$H-NMR (DMSO-$d_6$) δ: 1.80-1.93 (2H, m) , 2.05-2.17 (2H, m), 3.00-3.27 (4H, m), 4.62-4.72 (1H, m), 6.91-7.02 (3H, m), 7.26-7.34 (2H, m), 9.21 (2H, s).

**Reference Example 81**

4-[(4-chlorophenyl)thio]piperidine• hydrochloride

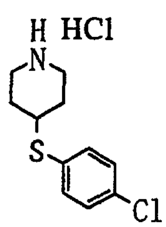

$^1$H-NMR (DMSO-$d_6$) δ: 1.64-1.82 (2H, m), 2.00-2.09 (2H, m), 2.88-3.00 (2H, m), 3.20-3.27 (2H, m), 3.47-3.60 (1H, m), 7.39-7.49 (4H, m), 9.24 (2H, s).

**Reference Example 82**

4-[(4-fluorophenyl)thio]piperidine hydrochloride

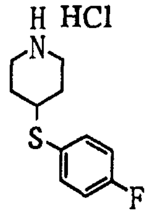

IR(KBr): 2730, 1491, 1219, 845, 544 $cm^{-1}$

**Reference Example 83**

4-[(4-chlorophenyl)sulfonyl]piperidine• hydrochloride

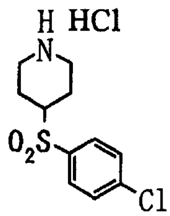

$^1$H-NMR (DMSO-$d_6$) δ: 1.65-1.86 (2H, m), 1.97-2.03 (2H, m) , 2.77-2.88 (2H, m), 3.28-3.37 (2H, m), 3.58-3.73 (1H, m), 7.77-7.90 (4H, m), 8.91 (1H, s), 9.42 (1H, s).

**Reference Example 84**

4-[(4-fluorophenyl)sulfonyl]piperidine• hydrochloride

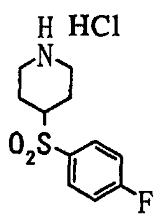

IR(KBr):2912, 2786, 1588, 1279, 1236, 1142, 590 $cm^{-1}$

**Example 1**

1-benzoyl-N-[(1R)-2-[3-(R,S)-[(dimethylamino)methyl]-6-fluoro-1,2,3,4-tetrahydro-1-quinolinyl]-1-(1-indol-3-ylmethyl)-2-oxoethyl]-4-piperidinecarboxamide

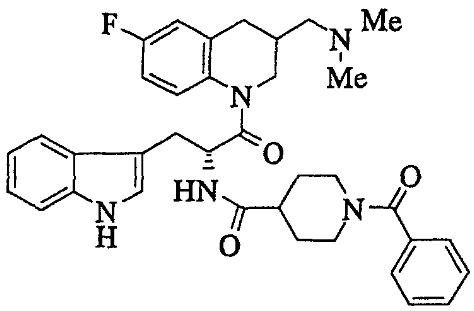

To a solution of 1-[2-(R)-amino-3-(indol-3-yl)propanoyl]-3-(R,S)-(N,N-dimethylamino)methyl-6-fluoro-1,2,3,4-tetrahydroquinoline (150 mg) in acetonitrile (3 ml) was added N-benzoylisonipecotic acid (115 mg), WSC (110 mg) and HOBt (61 mg). The mixture was stirred at room temperature for 3 hours. To the reaction solution was added a saturated aqueous solution of sodium hydrogen carbonate. The mixture was extracted with ethyl acetate. The ethyl acetate layer was washed with saturated brine, dried and concentrated. The residue was purified by alumina column chromatography (developing solvent; ethyl acetate - ethyl acetate/methanol = 10:1) and the title compound was obtained as amorphous powders (195 mg).

IR(KBr): 3289, 2942, 1632, 1497, 1447, 743, 708 $cm^{-1}$.

MASS (APCIMASS), m/z 610[$(M+H)^+$].

The following compounds mentioned in Examples 2-7 were synthesized by the same method as Example 1.

**Example 2**

3-(R,S)-(1-benzoyl-4-piperidinyl)-N-[(1R)-2-[5-chloro-3-[(dimethylamino)methyl]-1,2,3,4-tetrahydro-1-quinolinyl]-1-(1-indol-3-ylmethyl)-2-oxoethyl]propanamide

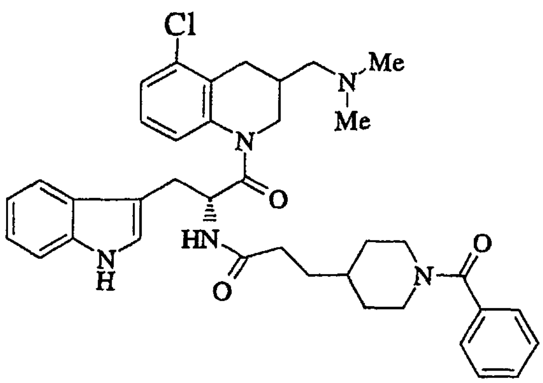

IR(KBr): 3260, 2932, 1636, 1456, 1281, 741, 710 $cm^{-1}$.

**Example 3**

1-benzoyl-N-[(1R)-2-[6-chloro-3-(S)-[(dimethylamino)methyl]-1,2,3,4-tetrahydro-1-quinolinyl]-1-(1-indol-3-ylmethyl)-2-oxoethyl]-4-piperidinecarboxamide

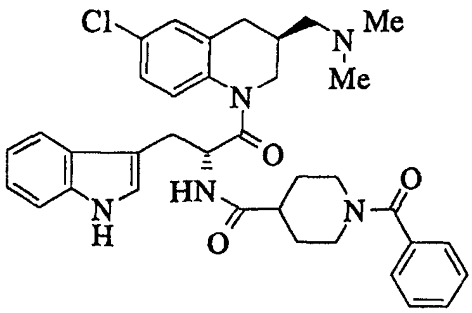

IR(KBr): 3283, 2942, 1624, 1487, 1447, 1281, 1231, 1096, 743 $cm^{-1}$.
$[\alpha]_D^{20}$ = -153° (c = 0.496, methanol).
MASS (APCIMASS): m/z 626 $[(M+H)^+]$.

**Example 4**

3-(1-benzoyl-4-piperidinyl)-N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]propanamide

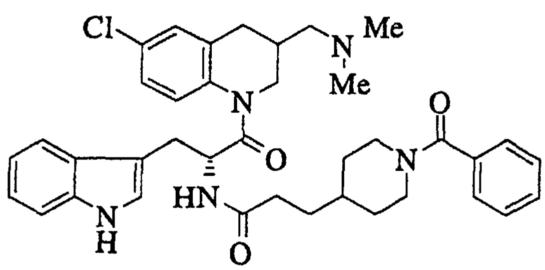

IR(KBr): 3287, 2934, 2863, 1634, 1487, 1445, 1279, 743 $cm^{-1}$.
MASS (APCIMASS), m/z 654$[(M+H)^+]$.

**Example 5**

benzyl 4-[[[(1R)-2-[6-chloro-3-(R,S)-[(dimethylamino)methyl]-1,2,3,4-tetrahydro-1-quinolinyl]-1-(1-indol-3-ylmethyl)-2-oxoethyl]amino]carbonyl]-1-piperidinecarboxylate

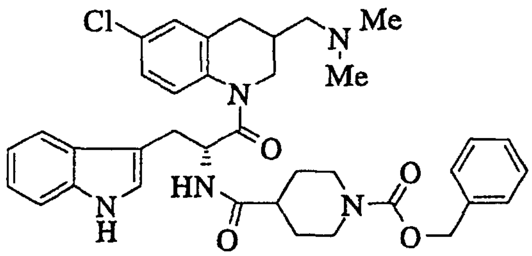

IR(KBr): 3308, 2948, 1686, 1634, 1487, 1433, 1215, 743 $cm^{-1}$.
MASS (APCIMASS), m/z 656[$(M+H)^+$].

**Example 6**

2-(1-benzoyl-4-piperidinyl)-N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]acetamide

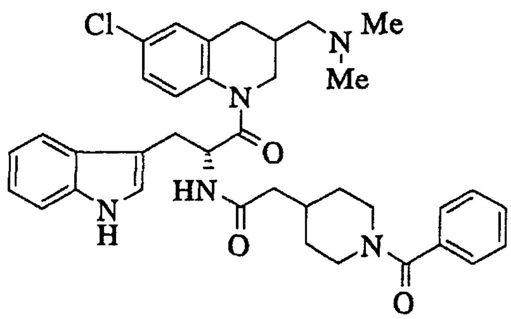

IR(KBr):3283, 2936, 1634, 1487, 1445, 1279, 743 $cm^{-1}$.
MASS (APCIMASS), m/z 640[$(M+H)^+$].

**Example 7**

1-benzoyl-N-[(1R)-2-[6-chloro-3-(R)-[(dimethylamino)methyl]-1,2,3,4-tetrahydro-1-quinolinyl]-1-(1-indol-3-ylmethyl)-2-oxoethyl]-4-piperidinecarboxamide

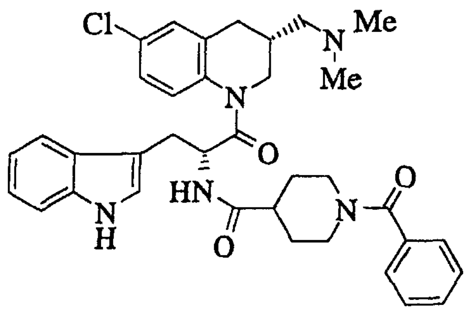

IR(KBr): 3289, 2944, 1634, 1487, 1435, 1280, 1094, 743 $cm^{-1}$.
MASS (APCIMASS), m/z 626[$(M+H)^+$].
$[\alpha]_D^{20}$ = -147°(c=0.498% methanol)

**Example 8**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(4-methoxybenzoyl)-4-piperidinecarboxamide

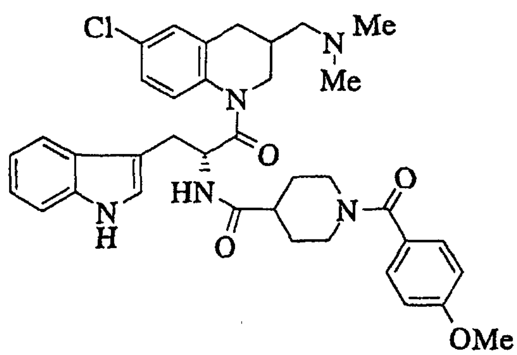

To a solution of 3-(R,S)-(N,N-dimethylamino)methyl-6-chloro-1-[3-(indol-3-yl)-2-[(R)-4-piperidylcarbonylamino]propanoyl]-1,2,3,4-tetrahydroquinoline (150 mg) in acetonitrile (3 ml) was added p-methoxy benzoic acid (57 mg), WSC (83 mg) and HOBt (46 mg). The mixture was stirred at room temperature for 2 hours. To the reaction solution was added a saturated aqueous solution of sodium hydrogen carbonate. The mixture was extracted with ethyl acetate. The ethyl acetate layer was washed with saturated brine, dried and concentrated. The residue was purified by alumina column chromatography (developing solvent; ethyl acetate/hexane = 1:4 - ethyl acetate/methanol = 20:1) and the title compound was obtained as amorphous powders (150 mg).
IR(KBr): 3289, 2942, 1634, 1613, 1487, 1439, 1250, 743 $cm^{-1}$.
MASS (APCIMASS), m/z 656[$(M+H)^+$].

The following compounds mentioned in Examples 9-20 were synthesized by the same method as Example 8.

**Example 9**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(2-methylbenzoyl)-4-piperidinecarboxamide

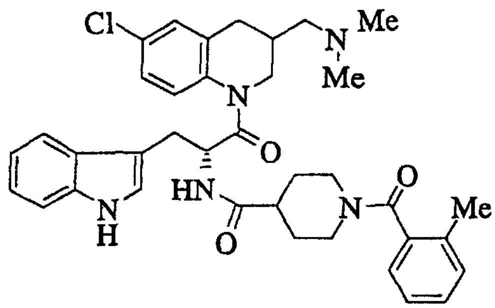

IR(KBr):3285, 2946, 1634, 1487, 1456, 1230, 743 $cm^{-1}$.
MASS (APCIMASS), m/z 640[$(M+H)^+$].

**Example 10**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(2-methoxybenzoyl)-4-piperidinecarboxamide

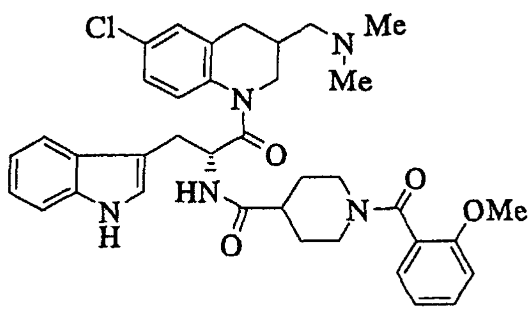

IR(KBr):3297, 2944, 1626, 1489, 1435, 1246, 743 $cm^{-1}$.
MASS (APCIMASS), m/z 656[$(M+H)^+$].

**Example 11**

1-(2-chlorobenzoyl)-N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-4-piperidinecarboxamide

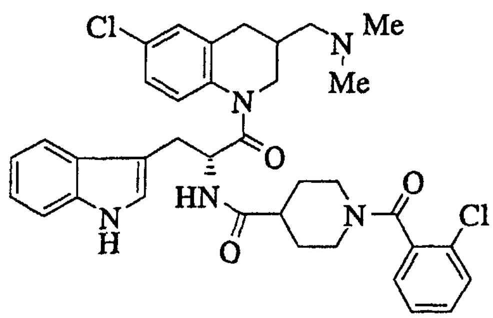

IR(KBr):3304, 2944, 2861, 1634, 1487, 1445, 743 $cm^{-1}$.
MASS (APCIMASS), m/z 660[$(M+H)^+$].

**Example 12**

1-(1-benzothiophen-2-ylcarbonyl)-N-[(1R)-2-[6-chloro-3-(R,S)-[(dimethylamino)methyl]-1,2,3,4-tetrahydro-1-quinolinyl)-1-(1-indol-3-ylmethyl)-2-oxoethyl]-4-piperidinecarboxamide

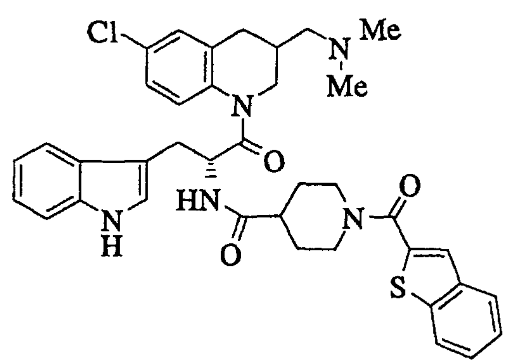

IR(KBr):3289, 2944, 1632, 1487, 1456, 1273, 743 $cm^{-1}$.
MASS (APCIMASS), m/z 682[$(M+H)^+$].

**Example 13**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(2-phenylacetyl)-4-piperidinecarboxamide

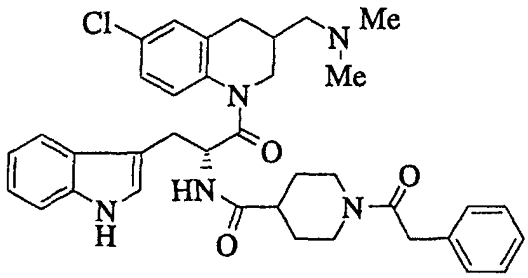

IR(KBr): 3297, 2944, 1632, 1487, 1456, 1100, 741, 729 $cm^{-1}$.
MASS (APCIMASS), m/z 640[$(M+H)^+$].

**Example 14**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(1-indol-2-ylcarbonyl)-4-piperidinecarboxamide

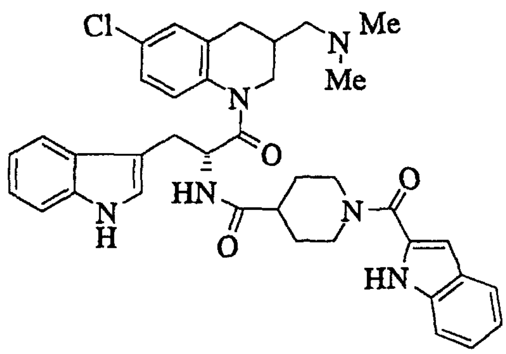

IR(KBr): 3283, 2938, 1638, 1601, 1528, 1487, 1439, 745 $cm^{-1}$.
MASS (APCIMASS), m/z 665[$(M+H)^+$].

**Example 15**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(1-indol-3-ylcarbonyl)-4-piperidinecarboxamide

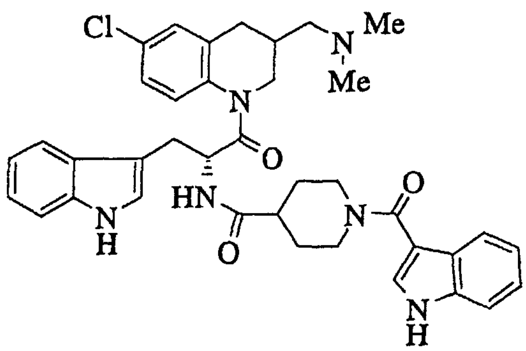

IR(KBr): 3283, 2942, 1636, 1595, 1532, 1487, 1439, 743 $cm^{-1}$.
MASS (APCIMASS), m/z 665[$(M+H)^+$].

**Example 16**

1-(1-benzofuran-2-ylcarbonyl)-N-(1R)-2-[6-chloro-3-(R,S)-[(dimethylamino)methyl]-1,2,3,4-tetrahydro-1-quinolinyl]-1-(1-indol-3-ylmethyl)-2-oxoethyl]-4-piperidinecarboxamide

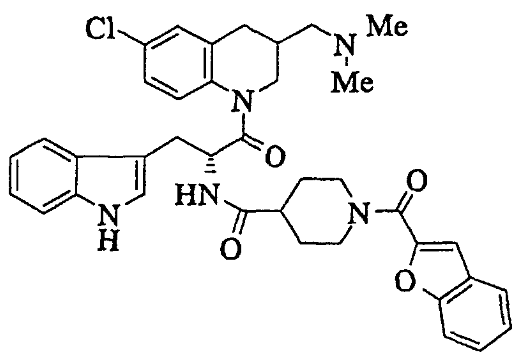

IR(KBr):3285, 2938, 1632, 1487, 1437, 1177, 745 $cm^{-1}$.
MASS (APCIMASS), m/z 666[$(M+H)^+$].

**Example 17**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(2-thienylcarbonyl)-4-piperidinecarboxamide

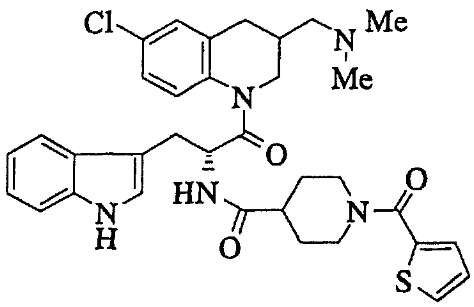

IR(KBr): 3291, 2938, 1636, 1522, 1487, 1439, 1273, 741 $cm^{-1}$.
MASS (APCIMASS), m/z 632[$(M+H)^+$].

**Example 18**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(3-thienylcarbonyl)-4-piperidinecarboxamide

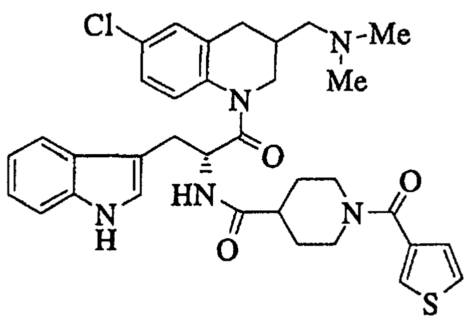

IR(KBr): 3293, 2942, 1634, 1526, 1487, 1445, 1275, 741 $cm^{-1}$.
MASS (APCIMASS), m/z 632[$(M+H)^+$].

**Example 19**

N-[(1R)-2-[6-chloro-3-(R,S)-[(dimethylamino)methyl]-1,2,3,4-tetrahydro-1-quinolinyl]-1-(1-indol-3-ylmethyl)-2-oxoethyl]-1-(3-pyridinylcarbonyl)-4-piperidinecarboxamide

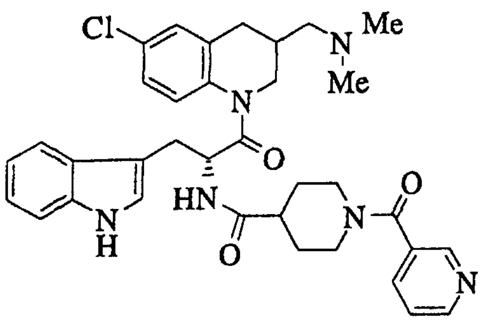

IR(KBr):3293, 2944, 1634, 1487, 1441, 1283, 741 $cm^{-1}$.
MASS (APCIMASS), m/z 627[$(M+H)^+$].

**Example 20**

N-[(1R)-2-[6-chloro-3-(R,S)-[(dimethylamino)methyl]-1,2,3,4-tetrahydro-1-quinolinyl)-1-(1-indol-3-ylmethyl)-2-oxoethyl]-1-[2-(4-chlorophenyl)acetyl]-4-piperidinecarboxamide

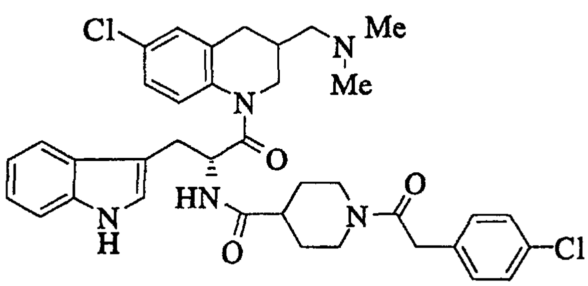

IR(KBr):3297, 2969, 1634, 1487, 1456, 1092, 743 $cm^{-1}$.
MASS (APCIMASS), m/z 674[$(M+H)^+$].

**Example 21**

N-[(1R)-2-[5-chloro-3-(R,S)-[(dimethylamino)methyl]-1,2,3,4-tetrahydro-1-quinolinyl]-1-(1-indol-3-ylmethyl)-2-oxoethyl]-4-phenyl-1-piperazinecarboxamide

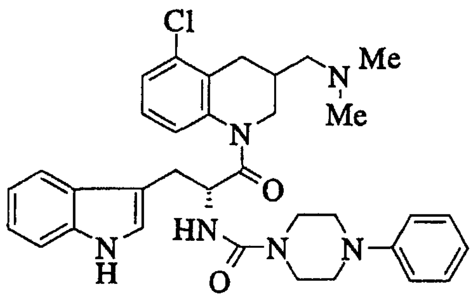

To a solution of 1-[2-(R)-amino-3-(indol-3-yl)propanoyl]-5-chloro-3-(R,S)-(N,N-dimethylamino)methyl-1,2,3,4-tetrahydroquinoline (154 mg) and N-ethyldiisopropylamine (0.07 ml) in acetonitrile (5 ml) was added N,N'-disuccinimidyl carbonate (96 mg). The mixture was stirred at room temperature for 30 minutes. To the reaction solution was added 1-phenylpiperazine (62 mg) and a solution of N-ethyldiisopropylamine (0.07 ml) in acetonitrile (5 ml). Furthermore, the reaction solution was stirred at room temperature for 3 hours. Then, to the reaction solution was added an aqueous solution of 10% potassium carbonate. The reaction solution was extracted with ethyl acetate. The organic layer was washed with saturated brine, dried and concentrated. The residue was purified by alumina column chromatography (developing solvent; ethyl acetate/methanol = 10/1) and the title compound was obtained as amorphous powders (136 mg).

IR(KBr):3266, 2971, 2820, 1636, 1458, 1233, 743 $cm^{-1}$

The following compounds mentioned in Examples 22-25 were synthesized according to the same method as Example 21.

**Example 22**

N-[(1R)-2-[3-(R,S)-[(dimethylamino)methyl]-6-fluoro-1,2,3,4-tetrahydro-1-quinolinyl]-1-(1-indol-3-ylmethyl)-2-oxoethyl]-4-phenyl-1-piperazinecarboxamide

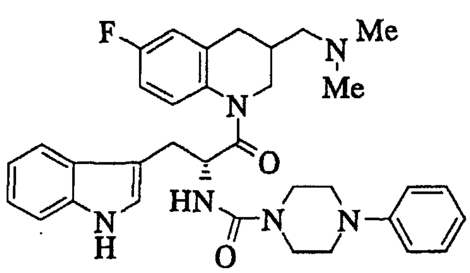

IR(KBr):3268, 2857, 2822, 1632, 1497, 1233, 760 $cm^{-1}$.
MASS (APCIMASS), m/z 583[$(M+H)^+$].

**Example 23**

N-[(1R)-2-[3-(R,S)-[(dimethylamino)methyl]-6-fluoro-1,2,3,4-tetrahydro-1-quinolinyl]-1-(1-indol-3-ylmethyl)-2-oxoethyl]-4-(2-oxo-2,3-dihydro-1-benzimidazol-1-yl)-1-piperidinecarboxamide

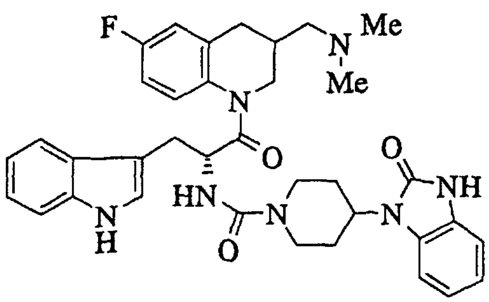

IR(KBr):3252, 2940, 1694, 1632, 1495, 1244, 756 $cm^{-1}$.
MASS (APCIMASS), m/z 638[$(M+H)^+$].

**Example 24**

N-[(1R)-2-[3-(R,S)-[(dimethylamino)methyl]-6-fluoro-1,2,3,4-tetrahydro-1-quinolinyl]-1-(1-indol-3-ylmethyl)-2-oxoethyl]-4-(3-methyl-2-oxo-2,3-dihydro-1-benzimidazol-1-yl)-1-piperidinecarboxamide

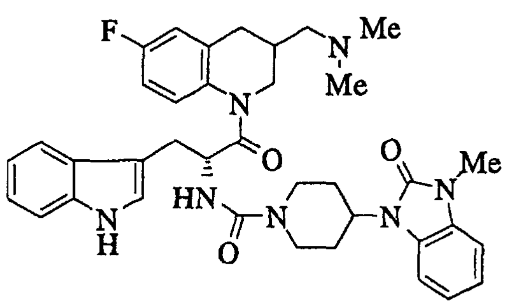

IR(KBr): 3254, 2938, 1694, 1634, 1497, 1435, 1242, 752 $cm^{-1}$.
MASS (APCIMASS), m/z 652[$(M+H)^+$].

**Example 25**

N-[(1R)-2-[5-chloro-3-(R,S)-[(dimethylamino)methyl]-1,2,3,4-tetrahydro-1-quinolinyl]-1-(1-indol-3-ylmethyl)-2-oxoethyl]-4-(2-oxo-2,3-dihydro-1-benzimidazol-1-yl)-1-piperidinecarboxamide

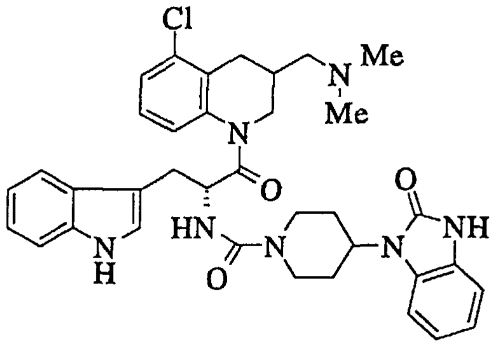

IR(KBr): 3272, 2971, 1694, 1634, 1483, 1464, 1246, 741 $cm^{-1}$.

**Example 26**

1-benzoyl-N-[(1R)-2-[5-chloro-3-(R,S)-[(dimethylamino)methyl]-1,2,3,4-tetrahydroquinolin-1-yl]-1-(1-indol-3-ylmethyl)-2-oxoethyl]-4-piperidinecarboxamide

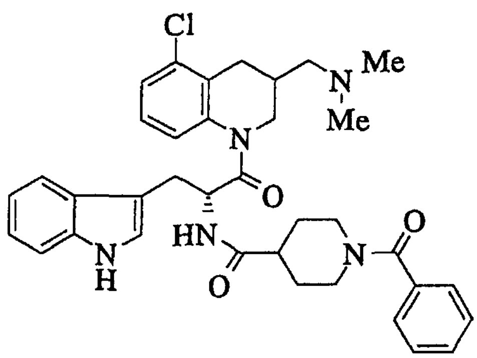

To a mixture of 1-[2-(R)-amino-3-(indol-3-yl)propanoyl]-5-chloro-3-(R,S)-(N,N-dimethylamino)methyl-1,2,3,4-tetrahydroquinoline (151 mg), 1-benzoyl-4-piperidinecarboxylic acid (94 mg) and HOBt (60 mg) in acetonitrile (5 ml) was added WSC (101 mg) at room temperature. The mixture was stirred at room temperature for 16 hours. Then, to the reaction solution was added an aqueous solution of 10% potassium carbonate. The mixture was extracted with ethyl acetate. The organic layer was washed with saturated brine, dried and concentrated. The residue was purified by alumina column chromatography (developing solvent: ethyl acetate/methanol = 10/1) and the title compound was obtained as amorphous powders (218 mg).

IR(KBr): 3283, 2934, 1634, 1281, 739, 708 $cm^{-1}$.

**Example 27**

N-[(1R)-2-[(3R,S)-6-chloro-3-[(dimethylamino)methyl]-1,2,3,4-tetrahydro-1-quinolinyl]-1-(1-indol-3-ylmethyl)-2-oxoethyl]-1-trifluoroacetyl-4-piperidinecarboxamide

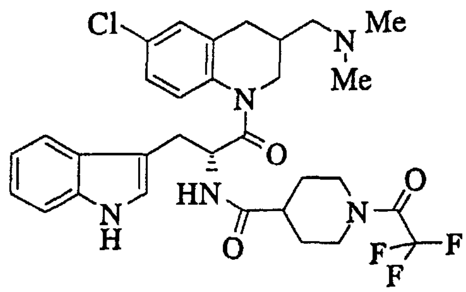

The title compound was obtained according to the same method as Example 1.

IR(KBr): 3308, 2946, 1694, 1634, 1487, 1175, 1144, 745 $cm^{-1}$.

MASS (APCIMASS), m/z 618 [$(M+H)^+$].

**Example 28**

N-[(1R)-2-[(3R,S)-6-chloro-3-[(dimethylamino)methyl]-1,2,3,4-tetrahydro-1-quinolinyl]-1-(1-indol-3-ylmethyl)-2-oxoethyl]-4-piperidinecarboxamide

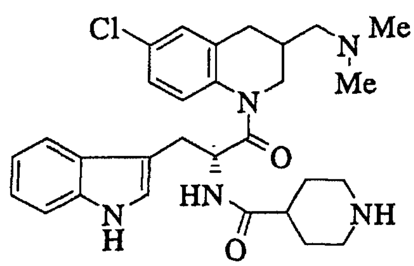

To a solution of N-[(1R)-2-[(3R,S)-6-chloro-3-[(dimethylamino)methyl]-1,2,3,4-tetrahydro-1-quinolinyl]-1-(1-indol-3-ylmethyl)-2-oxoethyl]-1-trifluoroacetyl-4-piperidinecarboxamide (200 mg) in methanol (4 ml) was added an aqueous solution of 10% potassium carbonate (2 ml). The mixture was stirred at room temperature for 2 hours. The reaction solution was concentrated under reduced pressure. To the residue was added water. The mixture was extracted with ethyl acetate. The ethyl acetate layer was washed with saturated brine, dried and concentrated under reduced pressure. The title compound was obtained as amorphous powders (155 mg).

IR(KBr):3279, 2944, 2822, 1636, 1487, 1233, 743 $cm^{-1}$.

MASS (APCIMASS), m/z 522[$(M+H)^+$].

**Example 29**

1-acetyl-N-[(1R)-2-[(3R,S)-6-chloro-3-[(dimethylamino)methyl]-1,2,3,4-tetrahydro-1-quinolinyl]-1-(1-indol-3-ylmethyl)-2-oxoethyl]-4-piperidinecarboxamide

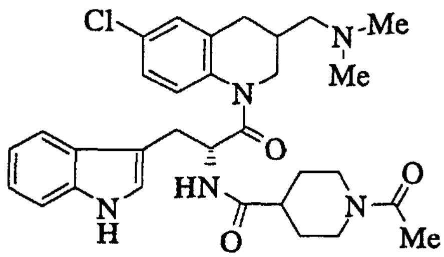

To a solution of N-[(1R)-2-[(3R,S)-6-chloro-3-[(dimethylamino)methyl]-1,2,3,4-tetrahydro-1-quinolinyl]-1-(1-indol-3-ylmethyl)-2-oxoethyl]-4-piperidinecarboxamide (150 mg) in ethyl acetate (1.5 ml) was added a saturated aqueous solution of sodium hydrogencarbonate. Under ice cooling, acetyl chloride (0.031 ml) was dropwise added thereto. The mixture was stirred for 30 minutes. Then, the ethyl acetate layer was separated. The ethyl acetate layer was washed with saturated brine, dried and concentrated under reduced pressure. The title compound was obtained as amorphous powders (80 mg).

The following compounds mentioned in Examples 30-36 were synthesized according to the same method as Example 1.

**Example 30**

3-(1-benzoyl-4-piperidinyl)-N-[(1R)-2-[(3S)-6-chloro-3-[(dimethylamino)methyl]-1,2,3,4-tetrahydro-1-quinolinyl]-1-(1-indol-3-ylmethyl)-2-oxoethyl]propanamide

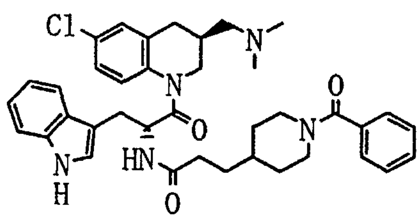

IR(KBr) : 3289, 2932, 1632, 1487, 1447, 741, 710 $cm^{-1}$.
MASS (APCIMASS), m/z 654[$(M+H)^+$].

**Example 31**

3-(1-benzoyl-4-piperidinyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

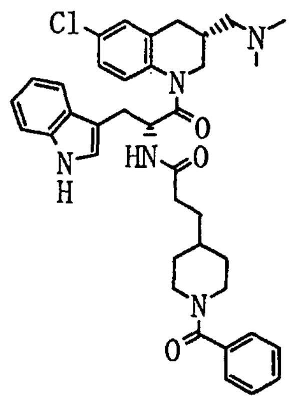

IR(KBr): 3289, 2936, 1634, 1487, 1445, 743, 710 $cm^{-1}$.
$[\alpha]_D^{20}$=-135.2°(c=0.302, MeOH)

**Example 32**

1-benzyl-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-piperidinecarboxamide

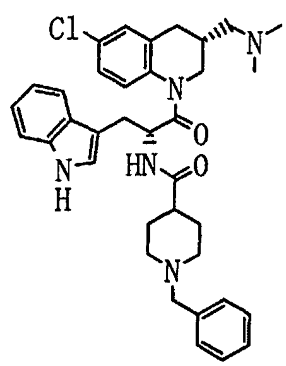

IR(KBr):3306, 2940, 2768, 1634, 1487, 1456, 741 $cm^{-1}$

**Example 33**

2-[(1-benzoyl-4-piperidinyl)oxy]-N-[(1R)-2-[(3R)-6-chloro-3- [dimethylamino]methyl]-1,2,3,4-tetrahydro-1-quinolinyl]-1-(1-indol-3-ylmethyl)-2-oxoethyl]acetamide

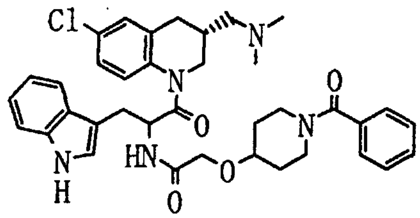

IR(KBr):3274, 2936, 1651, 1487, 1441, 1100, 743 $cm^{-1}$

**Example 34**

N-[cis-4-({[(1R)-2-[6-chloro-3-(R)-[(dimethylamino)methyl]-1,2,3,4-tetrahydro-1-quinolinyl]-1-(1-indol-3-ylmethyl)-2-oxoethyl] amino}carbonyl)cyclohexyl]benzamide

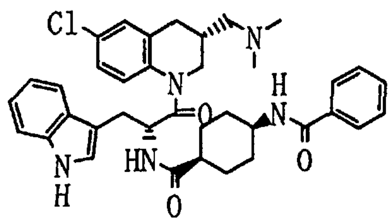

IR(KBr) : 3297, 2936, 1636, 1528, 1487, 743 $cm^{-1}$

**Example 35**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-1-trifluoroacetyl-4-piperidinecarboxamide

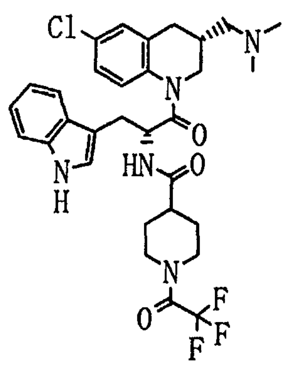

IR(KBr):3318, 2944, 1694, 1632, 1175, 1144, 743 $cm^{-1}$.

$[\alpha]_D^{20}$=-139.7°(c=0.308, MeOH)

**Example 36**

N-[(1R)-2-[(3R)-6-chloro-3- [(dimethylamino)methyl]-1,2,3,4-tetrahydro-1-quinolinyl]-1-(1-indol-3-ylmethyl)-2-oxoethyl]-3-[1-(trifluoroacetyl)-4-piperidinyl]propanamide

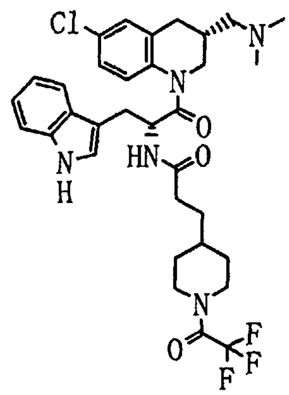

IR(KBr): 3301, 2938, 1688, 1344, 1487, 1204, 1146, 745, 667 $cm^{-1}$

**Example 37**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-piperidinecarboxamide

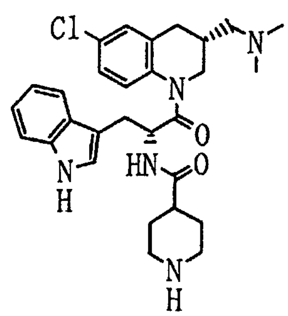

The title compound was obtained according to the same method as Example 28.
IR(KBr): 3285, 2942, 1634, 1487, 743 $cm^{-1}$.
$[\alpha]_D^{20}$ = -167.0°(c = 0.308, methanol).

The following compounds mentioned in Examples 38-50 were synthesized according to the same method as Example 8.

**Example 38**

N-[(1R)-2-[(3S)-6-chloro-3-[(dimethylamino)methyl]-1,2,3,4-tetrahydro-1-quinolinyl]-1-(1-indol-3-ylmethyl)-2-oxoethyl]-1-(4-methoxybenzoyl)-4-piperidinecarboxamide

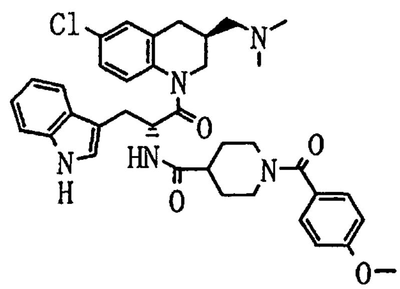

IR(KBr): 3289, 2946, 1609, 1487, 1435, 1250, 841, 743 $cm^{-1}$.
MASS (APCIMASS), m/z 656 $[(M+H)^+]$.

**Example 39**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-1-(4-methylbenzoyl)-4-piperidinecarboxamide

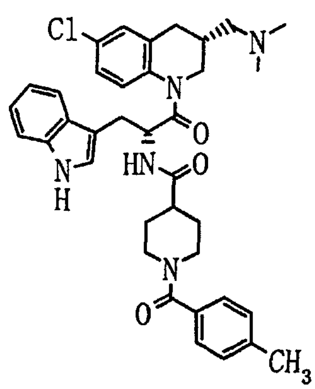

IR(KBr): 3303, 2942, 1630, 1439, 743 $cm^{-1}$.
MASS (FAB), m/z 640.3$[(M+H)^+]$

**Example 40**

N-[(1R)-2-[(3R)-6-chloro-3-[(dimethylamino)methyl]-1,2,3,4-tetrahydro-1-quinolinyl)-1-(1-indol-3-ylmethyl)-2-oxoethyl]-1-(4-fluorobenzoyl)-4-piperidinecarboxamide

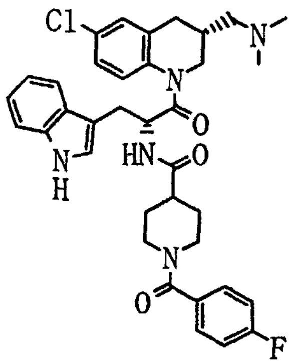

IR(KBr): 3297, 2944, 1636, 1437, 1227, 745 $cm^{-1}$.
MASS (FAB), m/z 644.2[$(M+H)^+$]

**Example 41**

1-(4-chlorobenzoyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-piperidinecarboxamide

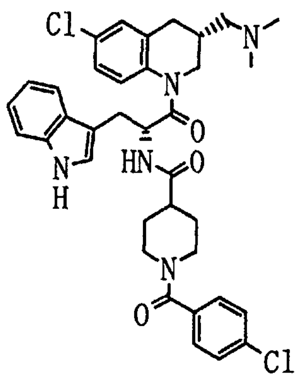

IR(KBr) : 3303, 2942, 1630, 1439, 743 $cm^{-1}$.
MASS (FAB), m/z 660.2[$(M+H)^+$]

**Example 42**

N-[(1R)-2-[(3R)-6-chloro-3- [(dimethylamino)methyl]-1,2,3,4-tetrahydro-1-quinolinyl]-1-(1-indol-3-ylmethyl)-2-oxoethyl]-1-(tetrahydro-2H-pyran-4-ylacetyl)-4-piperidinecarboxamide

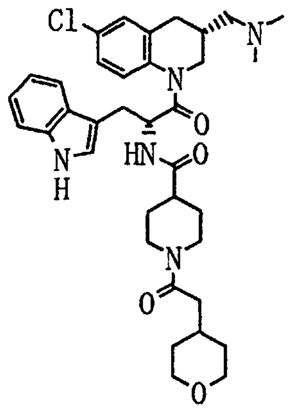

IR(KBr):3294, 2932, 1634, 1487, 1186, 1094, 741 $cm^{-1}$

**Example 43**

N-[(1R)-2-[(3R)-6-chloro-3- [(dimethylamino)methyl]-1,2,3,4-tetrahydro-1-quinolinyl]-1-(1-indol-3-ylmethyl)-2-oxoethyl]-1-(4-methoxybenzoyl)-4-piperidinecarboxamide

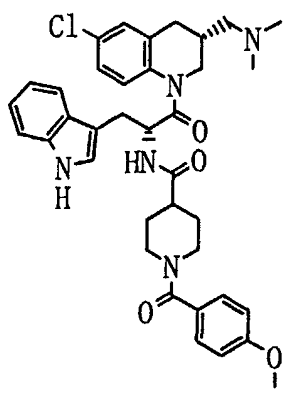

IR(KBr): 3277, 2938, 1634, 1613, 1487, 1435, 1250, 841, 743 $cm^{-1}$.
$[\alpha]_D^{20}$=-143.7°(c=0.309, MeOH)

**Example 44**

N-[(1R)-2-[(3R)-6-chloro-3- [(dimethylamino)methyl]-1,2,3,4-tetrahydro-1-quinolinyl]-1-(1-indol-3-ylmethyl)-2-oxoethyl]-1-(2-methoxybenzoyl)-4-piperidinecarboxamide

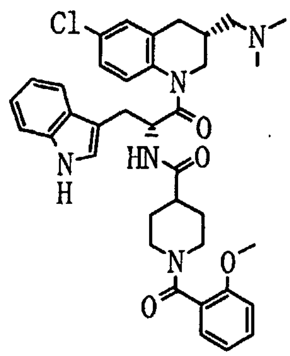

IR(KBr): 3283, 2940, 1632, 1487, 1250, 743 $cm^{-1}$.
$[\alpha]_D^{20}$=-140.2°(c=0.308, MeOH)

**Example 45**

N-[(1R)-2-[(3R)-6-chloro-3- [(dimethylamino)methyl]-1,2,3,4-tetrahydro-1-quinolinyl]-1-(1-indol-3-ylmethyl)-2-oxoethyl]-1-(2-methylbenzoyl)-4-piperidinecarboxamide

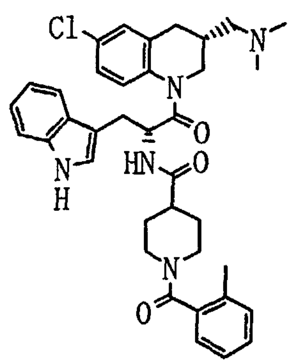

IR(KBr): 3300, 2946, 1634, 1487, 1441, 743 $cm^{-1}$.
$[\alpha]_D^{20}$=-143.3°(c=0.307, MeOH)

**Example 46**

1-(2-chlorobenzoyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-piperidinecarboxamide

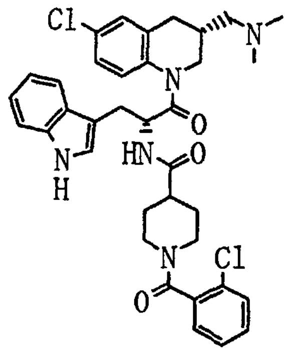

IR(KBr): 3287, 2944, 1634, 1487, 1445, 741 $cm^{-1}$.
$[\alpha]_D^{20}$=-139.9°(c=0.303, MeOH)

**Example 47**

N-[(1R)-2-[(3R)-6-chloro-3- [(dimethylamino)methyl]-1,2,3,4-tetrahydro-1-quinolinyl]-1-(1-indol-3-ylmethyl)-2-oxoethyl]-1-(indol-2-ylcarbonyl)-4-piperidinecarboxamide

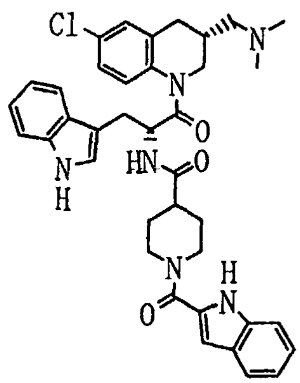

IR(KBr):3291, 2942, 1636, 1605, 1487, 1437, 747 $cm^{-1}$.
$[\alpha]_D^{20}$=-140.4°(c=0.307, MeOH)

**Example 48**

1-[4-(aminosulfonyl)benzoyl]-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-piperidinecarboxamide

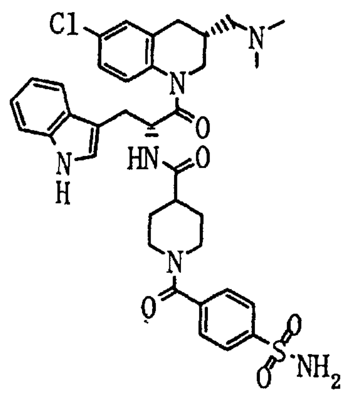

IR(KBr): 3268, 2942, 1634, 1487, 1339, 1165, 1096, 745, 608 $cm^{-1}$

**Example 49**

1- [4-(acetylamino)benzoyl]-N-[(1R)-2-[(3R) -6-chloro-3-[(dimethylamino)methyl]-1,2,3,4-tetrahydro-1-quinolinyl]-1-(1-indol-3-ylmethyl)-2-oxoethyl]-4-piperidinecarboxamide

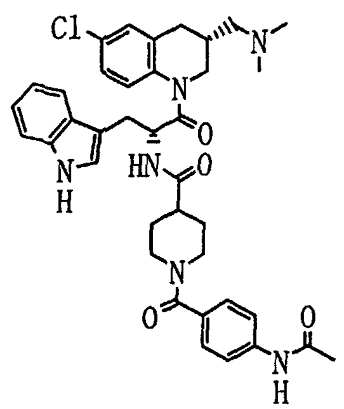

IR(KBr):3301, 2938, 1634, 1532, 1487, 1441, 743 $cm^{-1}$

**Example 50**

1-[(5-acetyl-2-thienyl)carbonyl]-N-[(1R)-2-[(3R)-6-chloro-3- [(dimethylamino)methyl]-1,2,3,4-tetrahydro-1-quinolinyl]-1-(1-indol-3-ylmethyl)-2-oxoethyl]-4-piperidinecarboxamide

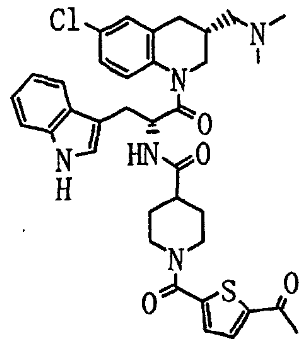

IR(KBr):3308, 2942, 1634, 1487, 1424, 1271, 743 $cm^{-1}$

**Example 51-1**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-1-(1-methylindol-2-ylcarbonyl)-4-piperidinecarboxamide

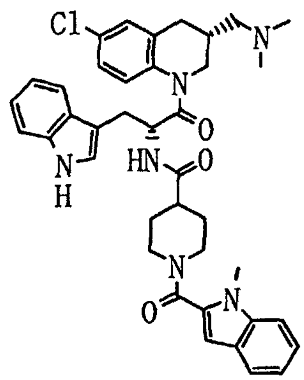

To a mixture of N-methylindole-2-carboxylic acid (60 mg) in acetonitrile (3 ml) and THF (3 ml) was added HOBt (54 mg), WSC (70 mg) and N-[(1R)-2-[(3R)-6-chloro-3-[(dimethylamino)methyl]-1,2,3,4-tetrahydro-1-quinolinyl)-1-(1-indol-3-ylmethyl)-2-oxoethyl]-4-piperidinecarboxamide (160 mg). The mixture was stirred at room temperature for 12 hours. To the reaction solution was added an aqueous solution of 10% potassium carbonate. The mixture was extracted with ethyl acetate. The ethyl acetate layer was washed with saturated brine, dried and concentrated. The residue was purified by alumina column chromatography (developing solvent; ethyl acetate/methanol = 50:1) and the title compound was obtained as amorphous powders (161 mg).

IR(KBr): 3303, 2968, 2942, 1632, 1487, 741 $cm^{-1}$

**Example 51-2**

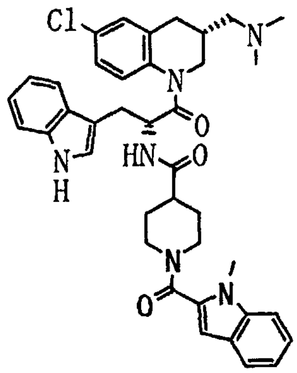

To a mixture of 1-[2-(R)-amino-3-(indol-3-yl)propanoyl]-3-(R)-6-chloro-(N,N-dimethylamino)methyl-1,2,3,4-tetrahydroquinoline (7.29 g) in acetonitrile (50 ml) - THF (50 ml) was added 1-[(1-methyl-1-indol-2-yl)carbonyl]-4-piperidinecarboxylic acid (5.323 g), WSC (4.28 g) and HOBt (2.872 g). The mixture was stirred at room temperature for 3 hours. To the reaction solution was added a saturated aqueous solution of sodium hydrogencarbonate. The mixture was extracted with ethyl acetate. The ethyl acetate layer was washed with saturated brine, dried and concentrated. The residue was purified by alumina column chromatography (developing solvent; ethyl acetate - ethyl acetate/methanol = 50:1) and then, silica gel column chromatography (developing solvent; ethyl acetate/methanol = 50:1 - 10:1). The title compound was obtained as amorphous powders (8.98 g).

**Example 52**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-(4-piperidinyl)propanamide

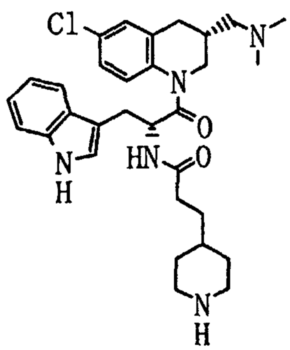

To a solution of N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(trifluoroacetyl)-4-piperidinyl]propanamide (194 mg) in methanol (4 ml) was added an aqueous solution of 10% potassium carbonate. The mixture was stirred at room temperature for 12 hours. To the reaction solution was added water. The mixture was extracted with ethyl acetate. The organic layer was washed with saturated brine, dried and concentrated. The obtained amorphous powders were washed with IPE and the title compound (146 mg) was obtained.

IR(KBr): 3287, 2924, 1636, 1487, 741 $cm^{-1}$

The following compounds mentioned in Examples 53-78 were synthesized by the same method as Example 8.

**Example 53**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-thienylcarbonyl)-4-piperidinyl]propanamide

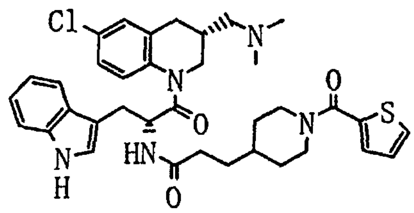

IR(KBr): 3299, 2926, 1634, 1487, 1443, 739 $cm^{-1}$.
MASS (APCIMASS), m/z 660[$(M+H)^+$].

**Example 54**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-pyridylcarbonyl)-4-piperidinyl]propanamide

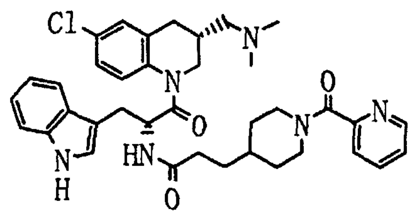

IR(KBr): 3287, 2928, 1634, 1487, 745 $cm^{-1}$.
MASS (APCIMASS), m/z 655[$(M+H)^+$].

**Example 55**

3-[1-(2-benzothiophenecarbonyl)-4-piperidinyl]-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

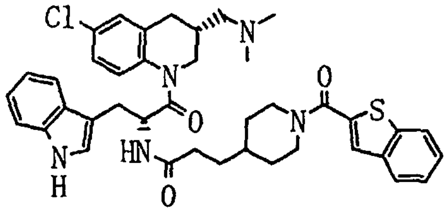

IR(KBr): 3297, 2934, 1635, 1487, 743 $cm^{-1}$.
MASS (APCIMASS), m/z 710[$(M+H)^+$].

**Example 56**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-methoxybenzoyl)-4-piperidinyl]propanamide

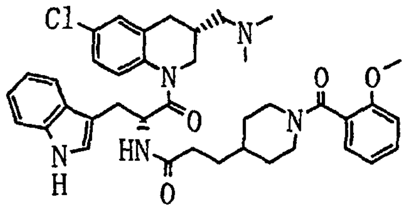

IR(KBr): 3272, 2934, 1634, 1487, 1248, 743 $cm^{-1}$.
MASS (APCIMASS), m/z 684[$(M+H)^+$].

**Example 57**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-[2-(methylsulfonyl)benzoyl]-4-piperidinyl]propanamide

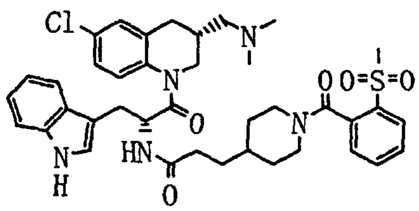

IR(KBr) : 3299, 2930, 1634, 1487, 1314, 1154, 781, 743 $cm^{-1}$.
MASS (APCIMASS), m/z 732[$(M+H)^+$].

**Example 58**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(cyclopentylcarbonyl)-4-piperidinyl]propanamide

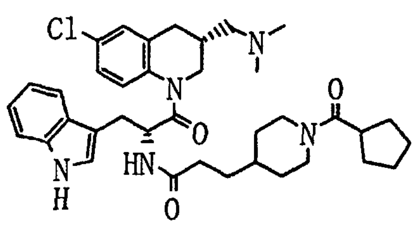

IR(KBr): 3285, 2944, 1638, 1487, 1439, 743 $cm^{-1}$.
MASS (APCIMASS), m/z 646[$(M+H)^+$].

**Example 59**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-[(4-chlorophenyl)acetyl]-4-piperidinyl]propanamide

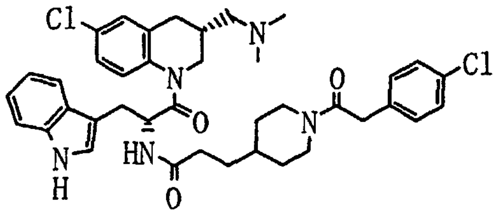

IR(KBr): 3287, 2934, 1640, 1489, 1092, 808, 743 $cm^{-1}$.
MASS (APCIMASS), m/z 702[$(M+H)^+$].

**Example 60**

N-[(1R]-2-[(3R)-6-chloro-3-[(dimethylamino)methyl]-1,2,3,4-tetrahydro-1-quinolinyl]-1-(1-indol-3-ylmethyl)-2-oxoethyl]-3-[1-[(3-chlorophenyl)acetyl]-4-piperidinyl]propanamide

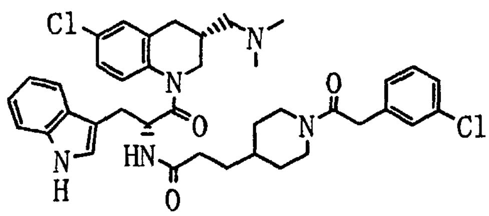

IR(KBr): 3299, 2930, 1636, 1487, 743, 685 $cm^{-1}$.
MASS (APCIMASS), m/z 702[$(M+H)^+$].

**Example 61**

N-(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-[(2-chlorophenyl)acetyl]-4-piperidinyl]propanamide

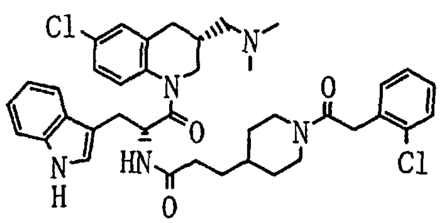

IR(KBr): 3291, 2934, 1636, 1487, 1040, 747 $cm^{-1}$.
MASS (APCIMASS), m/z 702[$(M+H)^+$].

**Example 62**

3-{1-[4-(aminosulfonyl)benzoyl]-4-piperidinyl}-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

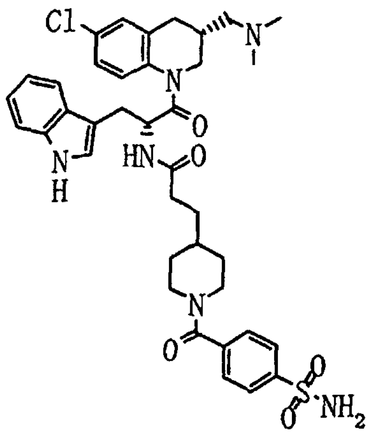

IR(KBr): 2936, 1632, 1489, 1456, 1339, 1165, 743, 667 $cm^{-1}$

**Example 63**

3-[1-(2-chlorobenzoyl)-4-piperidinyl]-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

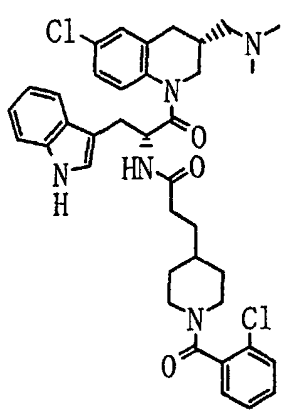

IR(KBr):3290, 2932, 2861, 1634, 1487, 1445, 743 $cm^{-1}$

**Example 64**

3-[1-(3-chlorobenzoyl)-4-piperidinyl]-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

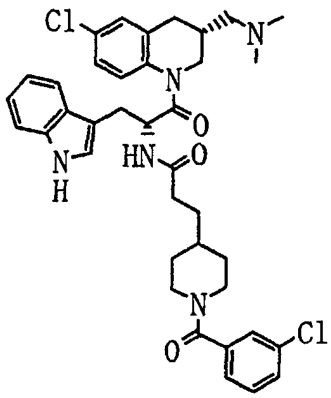

IR(KBr):3289, 2932, 1634, 1487, 1443, 1281, 741 $cm^{-1}$

**Example 65**

3-[1-(4-chlorobenzoyl)-4-piperidinyl]-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

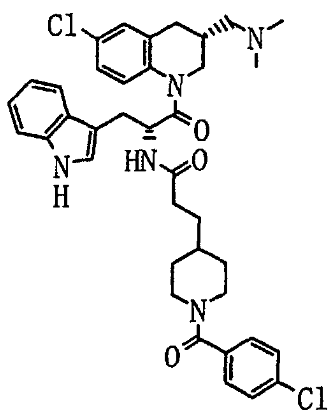

IR(KBr):3287, 2934, 1634, 1487, 1445, 1279, 1090, 837, 743 $cm^{-1}$

**Example 66**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-{1-[2-(trifluoromethyl)benzoyl]-4-piperidinyl}propanamide

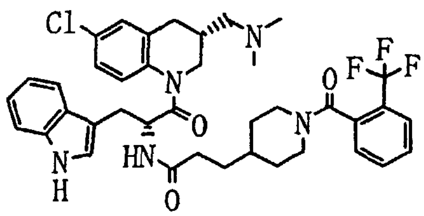

IR(KBr): 3289, 2936, 1634, 1489, 1441, 1318, 1175, 1130, 743 $cm^{-1}$

**Example 67**

3-{1-[3-(aminosulfonyl)-4-chlorobenzoyl]-4-piperidinyl}-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

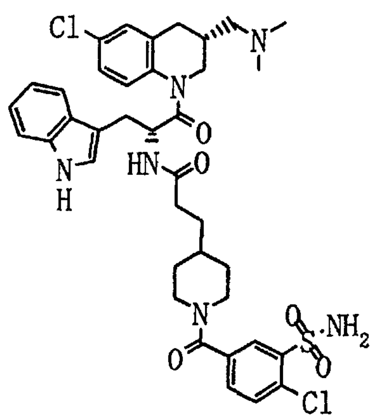

IR(KBr): 3275, 2971, 2934, 1634, 1487, 1445, 1339, 1167, 1042, 745, 594 $cm^{-1}$

**Example 68**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(4-methylbenzoyl)-4-piperidinyl]propanamide

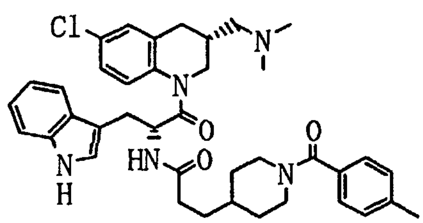

IR(KBr): 3266, 2934, 1638, 1439, 743 $cm^{-1}$.

MASS (FAB), m/z 668.3[(M+H)+]

**Example 69**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3- [1- (4-methoxybenzoyl)-4-piperidinyl]propanamide

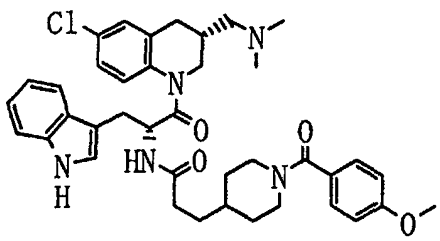

IR(KBr): 3285, 2936, 1638, 1439, 1250, 743 $cm^{-1}$.
MASS (FAB), m/z 684.2[(M+H)+]

**Example 70**

N-[(1R)-2-[(3R)-6-chloro-3-[(dimethylamino)methyl]-1,2,3,4-tetrahydro-1-quinolinyl)-1-(1-indol-3-ylmethyl)-2-oxoethyl]-3- [1-(4-fluorobenzoyl)-4-piperidinyl]propanamide

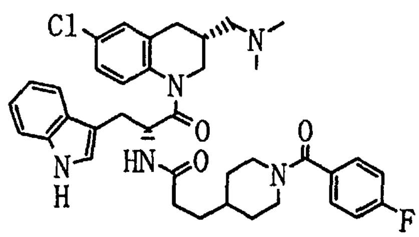

IR(KBr): 3287, 2932, 1636, 1443, 1233, 743 $cm^{-1}$.
MASS (FAB), m/z 672.2 [(M+H)+]

**Example 71**

3-{1-[4-(acetylamino)benzoyl]-4-piperidinyl}-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

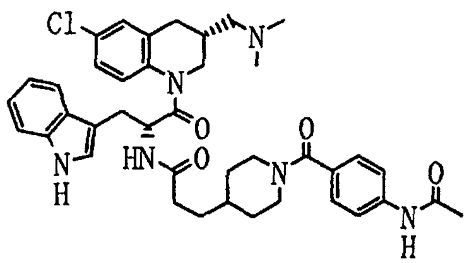

IR(KBr): 3303, 2934, 1638, 1530, 849, 723 $cm^{-1}$.
MASS (FAB), m/z 711.2[$(M+H)^+$]

**Example 72**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-methylbenzoyl)-4-piperidinyl]propanamide

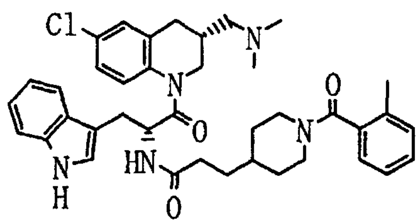

IR(KBr): 3291, 2930, 1636, 1441, 743 $cm^{-1}$.
MASS (FAB), m/z 668.3[$(M+H)^+$]

**Example 73**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-fluorobenzoyl)-4-piperidinyl]propanamide

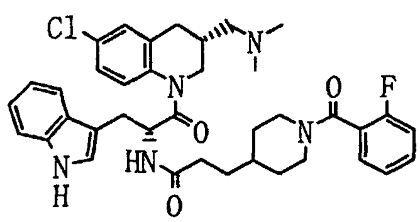

IR(KBr): 3304, 2934, 1632, 1456, 743 $cm^{-1}$.
MASS (FAB), m/z 672.2[$(M+H)^+$]

**Example 74**

3-[1-(2-acetylbenzoyl)-4-piperidinyl]-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide

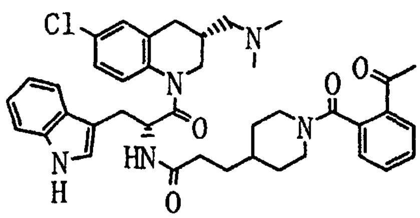

IR(KBr): 3303, 2932, 1636, 1437, 1260, 743 $cm^{-1}$.
MASS (FAB), m/z 696.2[$(M+H)^+$]

**Example 75**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(3-hydroxy-3-methylbutanoyl)-4-piperidinyl]propanamide

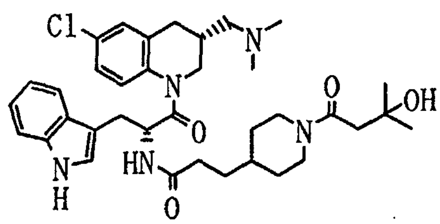

IR(KBr): 3277, 2932, 1640, 1487, 743 $cm^{-1}$.
MASS (FAB), m/z 650.3[$(M+H)^+$]

**Example 76**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-[(2-fluorophenyl)acetyl]-4-piperidinyl]propanamide

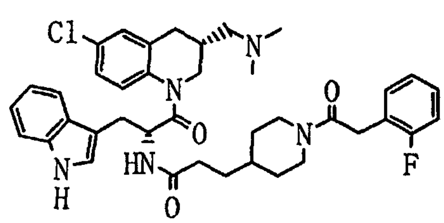

IR(KBr): 3301, 2932, 1638, 1491, 1233, 745 $cm^{-1}$.
MASS (FAB), m/z 686.2[$(M+H)^+$]

**Example 77**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-[(2-methylphenyl)acetyl]-4-piperidinyl]propanamide

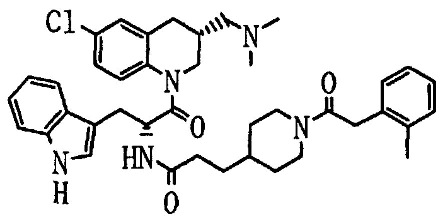

IR(KBr): 3281, 2930, 1636, 1456, 743 $cm^{-1}$.
MASS (FAB), m/z 682.2[$(M+H)^+$]

**Example 78**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-[(2-methoxyphenyl)acetyl]-4-piperidinyl]propanamide

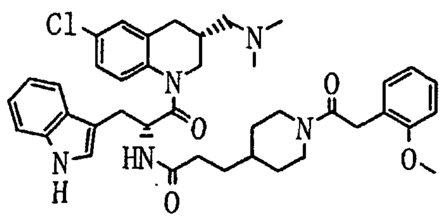

IR(KBr): 3281, 2938, 1638, 1491, 1246, 743 $cm^{-1}$.
MASS (FAB), m/z 698.3[$(M+H)^+$]

**Example 79**

N-(1R)-2-[(3R)-6-chloro-3-(dimethylamino)methyl]-1,2,3,4-tetrahydro-1-quinolinyl]-1-(1-indol-3-ylmethyl)-2-oxoethyl]-3-[1-(cyclohexylcarbonyl)-4-piperidinyl]propanamide

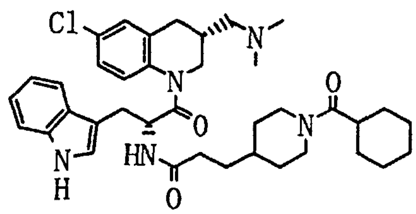

To a solution of 3-(4-piperidinyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide (180 mg) and triethyl amine (40 mg) in THF (6 ml) was added cyclohexanecarbonylchloride (58 mg) at 0°C. The mixture was stirred at 0°C for an hour. An aqueous solution of saturated sodium hydrogencarbonate was added thereto. The mixture was extracted with THF/ethyl acetate = 1/1.

The organic layer was washed with saturated brine, dried and concentrated. The residue was purified by alumina column chromatography (developing solvent: hexane/ethyl acetate = 1/1 - ethyl acetate - ethyl acetate/ethanol = 20/1) and the title compound was obtained as amorphous powders (144 mg).

IR(KBr): 3285, 2932, 1636, 1487, 1447, 741 $cm^{-1}$.

MASS (APCIMASS), m/z 660[$(M+H)^+$].

The following compounds mentioned in Examples 80-86 were synthesized according to the same method as Example 79.

**Example 80**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2,2-dimethylpropanoyl)-4-piperidinyl]propanamide

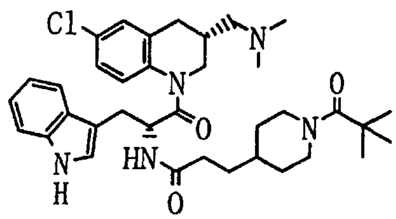

IR(KBr): 3287, 2932, 1634, 1487, 743 $cm^{-1}$.

MASS (FAB), m/z 634.2[$(M+H)^+$]

**Example 81**

2-[4-(3-[(1R)-2-[(3R)-6-chloro-3-[(dimethylamino)methyl]-1,2,3,4-tetrahydro-1-quinolinyl]-1-(1-indol-3-ylmethyl)-2-oxoethyl]amino-3-oxopropyl)-1-piperidinyl]-1,1-dimethyl-2-oxoethyl acetate

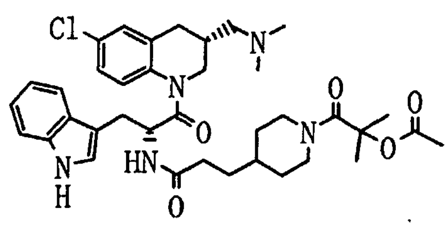

IR(KBr): 3275, 2934, 1576, 1437, 743 $cm^{-1}$.

MASS (FAB), m/z 678.3[$(M+H)^+$]

**Example 82**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(phenylsulfonyl)-4-piperidinyl]propanamide

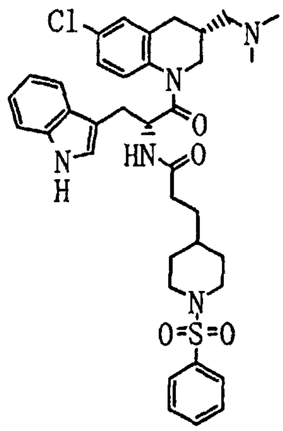

IR(KBr): 2934, 1645, 1489, 1339, 1167, 741, 579 $cm^{-1}$

**Example 83**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(phenylacetyl)-4-piperidinyl]propanamide

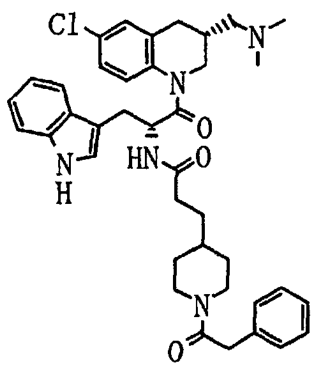

IR(KBr): 3272, 2934, 1634, 1487, 1456, 1271, 1233, 741 $cm^{-1}$

**Example 84**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-(1-isobutyryl-4-piperidinyl)propanamide

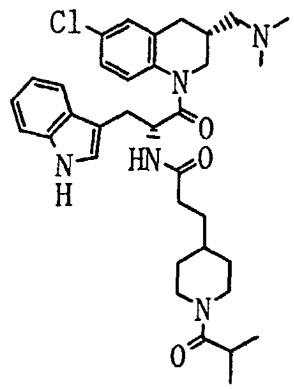

IR(KBr): 3282, 2932, 1640, 1487, 743 $cm^{-1}$

**Example 85**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-(1-propionyl-4-piperidinyl)propanamide

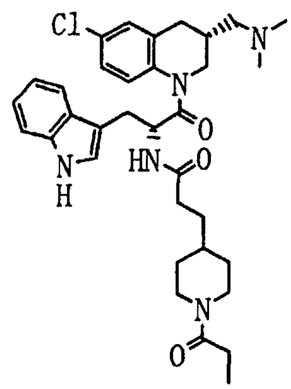

IR(KBr): 3279,2936, 1634, 1487, 1233, 743 $cm^{-1}$

**Example 86**

3- (1-acetyl-4-piperidinyl)-N-[(lR)-2-[(3R)-6-chloro-3-[(dimethylamino)methyl]-1,2,3,4-tetrahydro-1-quinolinyl]-1-(1-indol-3-ylmethyl)-2-oxoethyl]propanamide

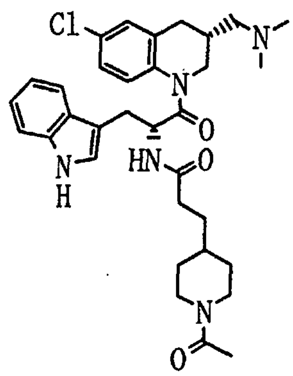

IR(KBr): 3268, 2934, 1634, 1487, 1456, 1271, 1235, 743 $cm^{-1}$

**Example 87**

3-(1-benzyl-4-piperidinyl)-N-[(lR)-2-[(3R)-6-chloro-3-[(dimethylamino)methyl]-1,2,3,4-tetrahydro-1-quinolinyl]-1-(1-indol-3-ylmethyl)-2-oxoethyl]propanamide

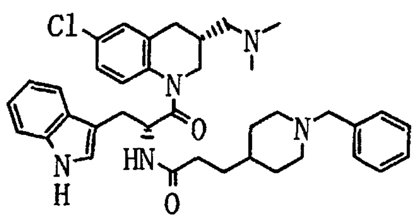

To a solution of 3-(4-piperidinyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide (193 mg) in ethanol (6 ml) was added benzaldehyde (39 μl) at room temperature. The reaction mixture was stirred for 15 minutes. To the reaction mixture was added sodium triacetoxyborohydride (82 mg) at room temperature. The reaction mixture was stirred at room temperature for 12 hours. Then, the solvent was removed by evaporation. To the residue was added a saturated aqueous solution of sodium hydrogencarbonate. The mixture was extracted with THF/ethyl acetate = 1/1. The organic layer was washed with saturated brine, dried and concentrated. The residue was purified by alumina column chromatography (developing solvent: hexane/ethyl acetate = 1/1 - ethyl acetate) and the title compound was obtained as amorphous powders (108 mg).

IR(KBr): 3299, 2934, 1487, 741 $cm^{-1}$.

MASS (APCIMASS), m/z 640[$(M+H)^+$].

The following compounds mentioned in Examples 88-95 were synthesized by the same method as Example87.

**Example 88**

3- [1-(2-chlorobenzyl)-4-piperidinyl]-N-[(1R)-2-[(3R)-6-chloro-3-[(dimethylamino)methyl]-1,2,3,4-tetrahydro-1-quinolinyl]-1-(1-indol-3-ylmethyl) -2-oxoethyl]propanamide

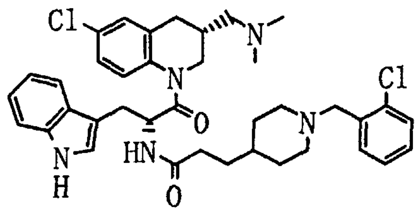

IR(KBr): 3289, 2932, 1636, 1487, 743 $cm^{-1}$.
MASS (APCIMASS), m/z 674[$(M+H)^+$].

**Example 89**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-fluorobenzyl)-4-piperidinyl]propanamide

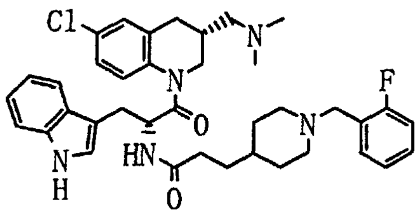

IR(KBr): 3302, 2924, 1636, 1487, 758, 743 $cm^{-1}$.
MASS (APCIMASS), m/z 658[$(M+H)^+$].

**Example 90**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-[2-(trifluoromethyl)benzyl]-4-piperidinyl]propanamide

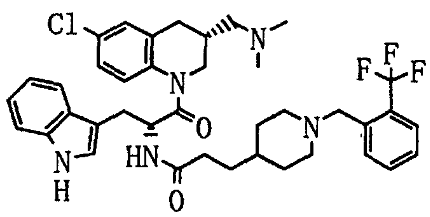

IR(KBr): 3301, 2926, 1634, 1487, 1314, 1121, 772, 743 $cm^{-1}$.
MASS (APCIMASS), m/z 708[$(M+H)^+$].

**Example 91**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-methoxybenzyl)-4-piperidinyl] propanamide

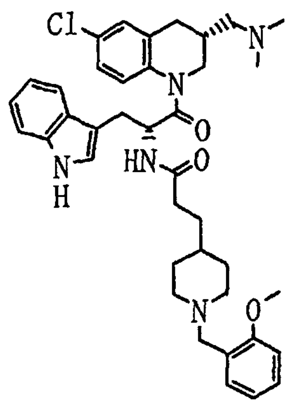

IR(KBr):3293, 2934, 1632, 1489, 1240, 1100, 741 $cm^{-1}$

**Example 92**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(3-methoxybenzyl)-4-piperidinyl] propanamide

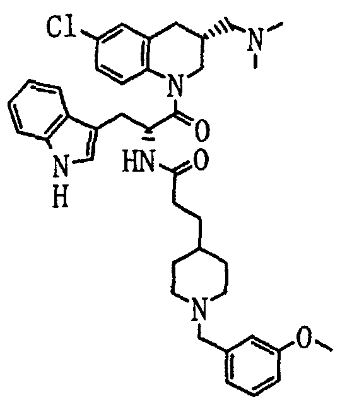

IR(KBr): 3301, 2922, 2768, 1632, 1487, 1456, 1265, 743 $cm^{-1}$

**Example 93**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(4-methoxybenzyl)-4-piperidinyl] propanamide

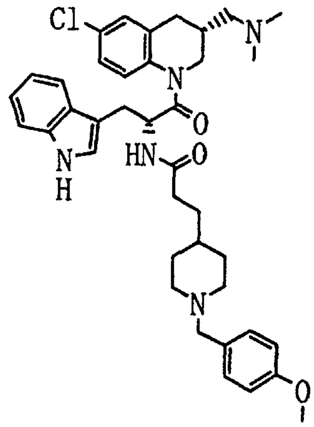

IR(KBr): 3289, 2926, 2768, 1634, 1512, 1487, 1456, 1246, 1179, 1101, 1038, 822, 741 $cm^{-1}$

**Example 94**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-methylbenzyl)-4-piperidinyl]propanamide

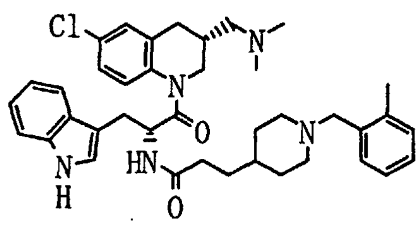

IR(KBr): 3291, 2922, 1632, 1487, 743 $cm^{-1}$.
MASS (FAB), m/z 654.3[$(M+H)^+$]

**Example 95**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1- (3-pyridinylmethyl) -4-piperidinyl]propanamide

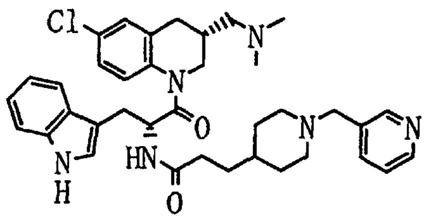

IR(KBr): 3270, 2932, 1638, 1487, 743 $cm^{-1}$.
MASS (FAB), m/z 641.2[$(M+H)^+$]

The following compounds mentioned in Examples 96-102 were synthesized according to the same method as Example 21.

**Example 96**

N-[(1S)-2-[(3R,S)-5-chloro-3-[(dimethylamino)methyl]-1,2,3,4-tetrahydro-1-quinolinyl]-1-(1-indol-3-ylmethyl)-2-oxoethyl]-4-(2-oxo-2,3-dihydro-1H-benzimidazol-1-yl)-1-piperidinecarboxamide

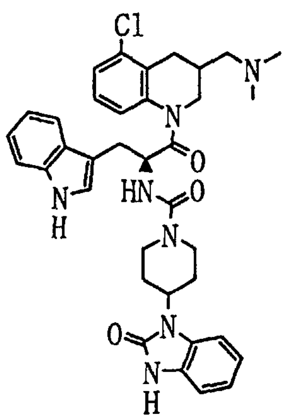

IR(KBr): 3248, 2938, 1696, 1460, 1373, 741 $cm^{-1}$.
MASS (FAB), m/z 654.2[$(M+H)^+$]

**Example 97**

4-benzyl-N-[(1R)-2-[(3R)-6-chloro-3-[(dimethylamino)methyl]-1,2,3,4-tetrahydro-1-quinolinyl]]]]-1-(1-indol-3-ylmethyl)-2-oxoethyl]-1-piperazinecarboxamide

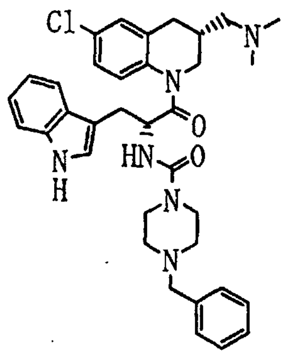

IR(KBr): 3268, 2938, 1632, 1487, 1233, 741 $cm^{-1}$.
MASS (FAB), m/z 613.3[$(M+H)^+$]

**Example 98**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-phenyl-1-piperidinecarboxamide

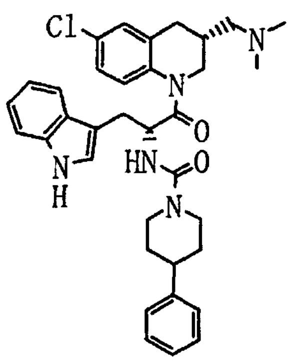

IR(KBr): 2934, 1630, 1487, 1229, 743 $cm^{-1}$.
MASS (FAB), m/z 598.2[$(M+H)^+$]

**Example 99**

4-benzoyl-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-1-piperidinecarboxamide

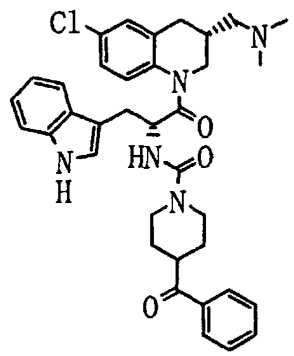

IR(KBr): 3254, 2946, 1636, 1487, 1209, 743 $cm^{-1}$.
MASS (FAB), m/z 626.3[$(M+H)^+$]

**Example 100**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-(4-chlorophenyl)-4-hydroxy-1-piperidinecarboxamide

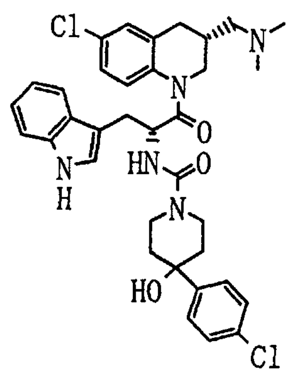

IR(KBr): 3303, 2942, 1624, 1487, 743 $cm^{-1}$.
MASS (FAB), m/z 648.2[$(M+H)^+$]

**Example 101**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-phenyl-1-piperazinecarboxamide

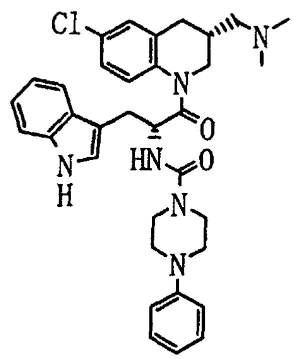

IR(KBr): 3279, 2820, 1634, 1489, 1231, 743 $cm^{-1}$

**Example 102**

4-benzoyl-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-1-piperazinecarboxamide

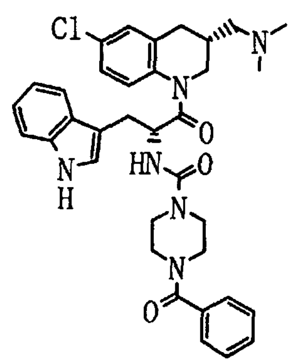

IR(KBr): 3274, 2971, 2934, 1634, 1487, 1435, 1256, 1009, 743, 710 $cm^{-1}$

The following compounds mentioned in Examples 103-104 were synthesized according to the same method as Example 1.

**Example 103**

1-benzoyl-N-[(1S)-2-[(3R,S)-5-chloro-3-[(dimethylamino)methyl]-1,2,3,4-tetrahydro-1-quinolinyl]-1-(1-indol-3-ylmethyl)-2-oxoethyl]-4-piperidinecarboxamide

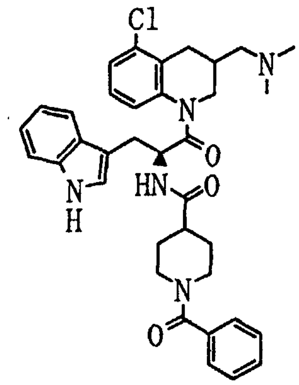

IR(KBr): 3299, 2944, 1636, 1458, 743 $cm^{-1}$.
MASS (FAB), m/z 626.2[$(M+H)^+$]

**Example 104**

3-(1-benzoyl-4-piperidinyl)-N-[(1S)-2-[(3R,S)-5-chloro-3-[(dimethylamino)methyl]-1,2,3,4-tetrahydro-1-quinolinyl]-1-(1-indol-3-ylmethyl)-2-oxoethyl]propanamide

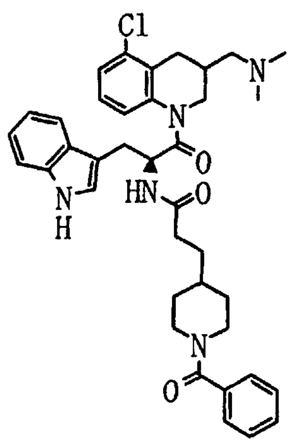

IR(KBr): 3272, 2928, 1636, 1458, 1281, 741 $cm^{-1}$.
MASS (FAB), m/z 654.3[$(M+H)^+$]

**Example 105**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-[1-(2-hydroxybenzoyl)-4-piperidinyl] propanamide

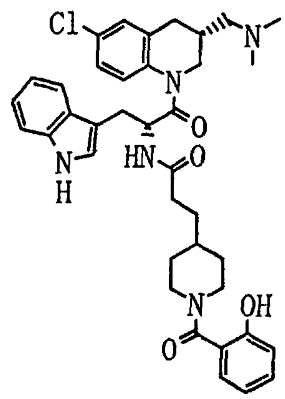

To a solution of acetyl salycylate (137 mg) in acetonitrile (15 ml) was added HOBt (142 mg), N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-3-(4-piperidinyl)propanamide (400 mg) and WSC (177 mg). The mixture was stirred at room temperature for 16 hours. Water was added thereto and the mixture was extracted with ethyl acetate. The organic layer was washed with a saturated aqueous solution of sodium hydrogencarbonate and saturated brine, dried and concentrated. To the mixture of the residue in methanol (2 ml) and ethyl acetate (2 ml) was added an aqueous solution of 10% potassium carbonate (2 ml) and the mixture was stirred for 48 hours. To the reaction solution was added water. The mixture was extracted with ethyl acetate. The organic layer was washed with saturated brine, dried and concentrated. The residue was purified by alumina column chromatography (developing solvent: ethyl acetate/methanol = 4/1). The purified amorphous powders were washed with IPE and the title compound (134 mg) was obtained.

IR(KBr): 3263, 2934, 1632, 1487, 1454, 743 $cm^{-1}$

The following compounds mentioned in Examples 106-107 were synthesized by the same method as Example 1.

**Example 106**

N-[(1R)-2-(5- (acetylamino) -3-[(dimethylamino) methyl]-1,2,3,4-tetrahydro-1-quinolinyl)-1-(1-indol-3-ylmethyl)-2-oxoethyl]-1-(trifluoroacetyl)-4-piperazinecarboxamide

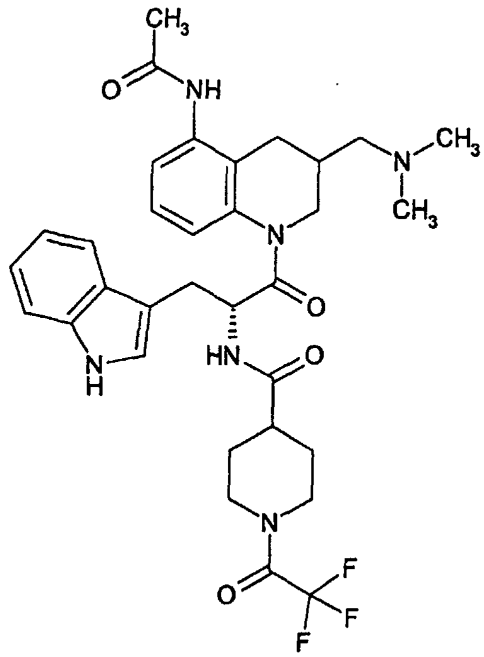

IR(KBr): 3299, 2934, 1686, 1458, 1204, 1175, 1144, 745 $cm^{-1}$.
MASS (APCIMASS), m/z 641 $[(M+H)^+]$.

**Example 107**

N-[(1R)-2-(5-(acetylamino)-3-[(dimethylamino)methyl]-1,2,3,4-tetrahydro-1-quinolinyl)-1-(1-indol-3-ylmethyl)-2-oxoethyl]-3-(1-benzoyl-4-piperidinyl)propanamide

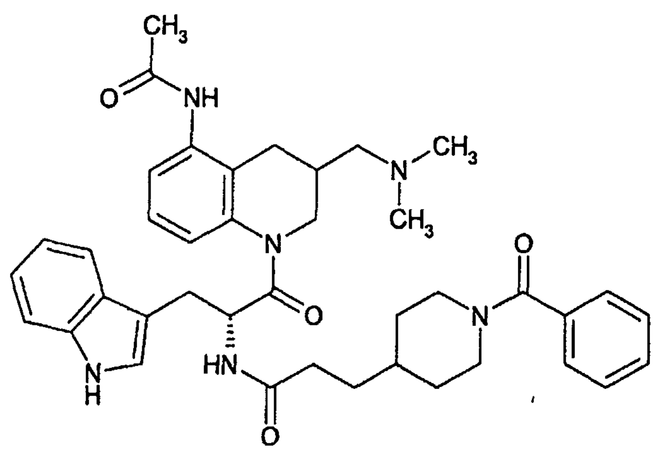

IR(KBr): 3272, 2930, 1634, 1456, 1283, 741, 710 $cm^{-1}$.
MASS (APCIMASS), m/z 677 $[(M+H)^+]$.

**Example 108**

N-[(1R)-2-(5-(acetylamino)-3-[(dimethylamino)methyl]-1,2,3,4-tetrahydro-1-quinolinyl)-1-(1-indol-3-ylmethyl)-2-oxoethyl]-4-piperazinecarboxamide

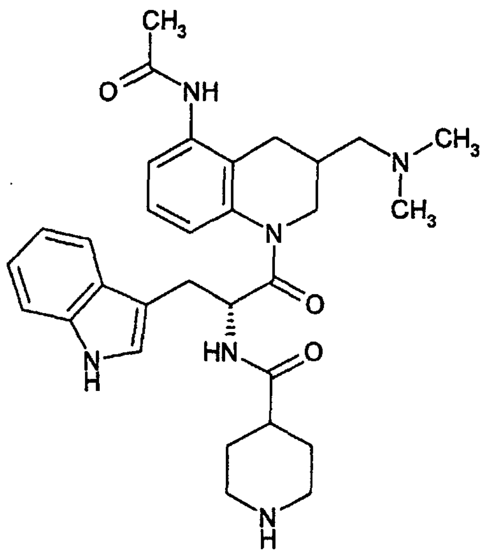

The title compound was obtained according to the same method as Example 28.

IR(KBr): 3291, 2934, 1647, 1458, 1202, 1177, 745, 613 $cm^{-1}$.

MASS (APCIMASS), m/z 545 $[(M+H)^+]$.

**Example 109**

N-[(1R)-2-(5-(acetylamino)-3-[(dimethylamino)methyl]-1,2,3,4-tetrahydro-1-quinolinyl)-1-(1-indol-3-ylmethyl)-2-oxoethyl]-1-benzoyl-1-piperidinecarboxamide

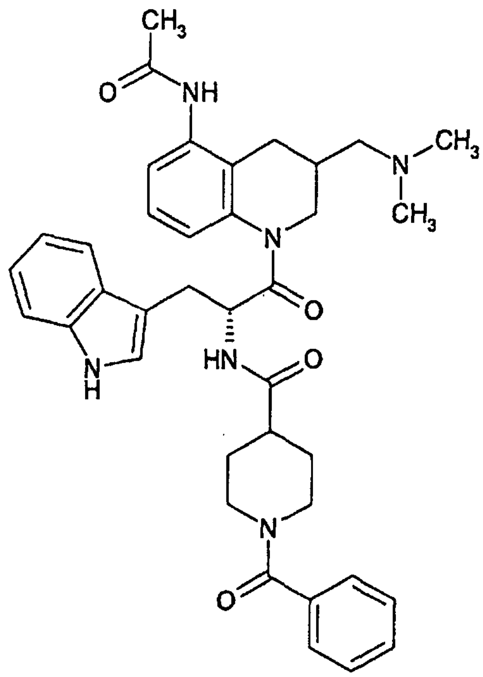

The title compound was obtained according to the same method as Example 79.
IR(KBr): 3289, 2944, 1636, 1456, 1283, 789, 743, 710 $cm^{-1}$.
MASS (APCIMASS), m/z 649 $[(M+H)^+]$.

The following compounds mentioned in Examples 110-111 were synthesized according to the same method as Example 21.

**Example 110**

N-[(1R)-2-(5-(acetylamino)-3-[(dimethylamino)methyl]-1,2,3,4-tetrahydro-1-quinolinyl)-1-(1-indol-3-ylmethyl)-2-oxoethyl]-4-(2-oxo-2,3-dihydro-lH-benzimidazol-1-yl)-1-piper idinecarboxamide

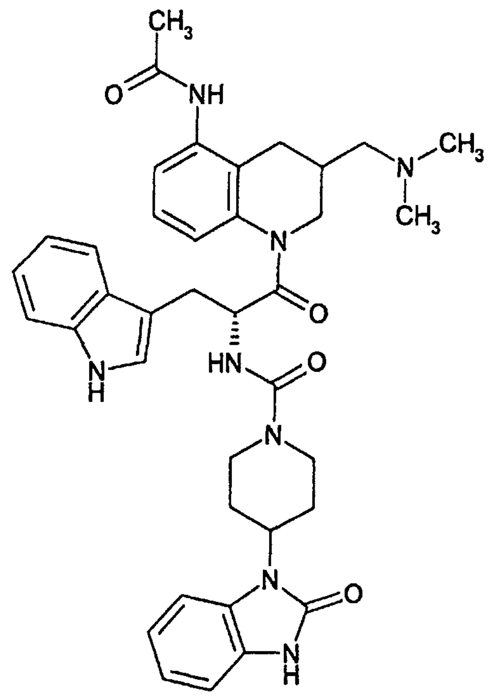

IR(KBr): 3300, 2944, 14634, 1456, 1233, 995, 743, 694 $cm^{-1}$.
MASS (APCIMASS), m/z 622 $[(M+H)^+]$.

**Example 111**

N-[(1R)-2-(5-(acetylamino)-3-[(dimethylamino)methyl]-1,2,3,4-tetrahydro-1-quinolinyl)-1-(1-indol-3-ylmethyl)-2-oxoethyl]-4-phenyl-1-piperazinecarboxamide

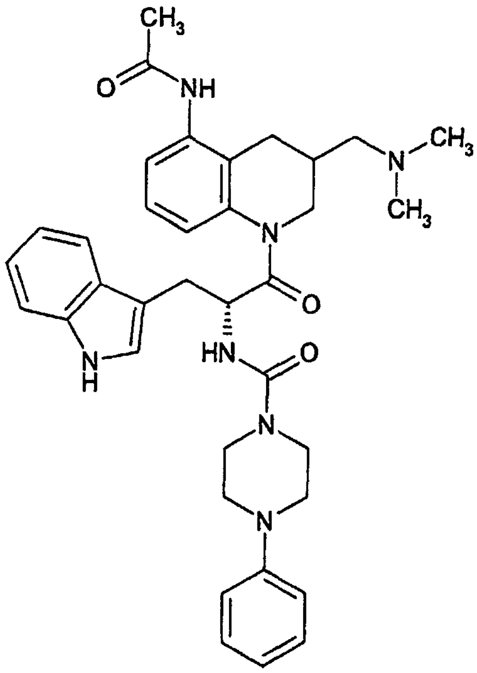

IR(KBr): 3260, 2940, 1694, 1372, 1246, 741 $cm^{-1}$.
MASS (APCIMASS), m/z 677 [$(M+H)^+$].

The following compounds mentioned in Examples 112-150 were synthesized by the same method as Example 1.

**Example 112**

N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-2-[[1-(trifluoroacetyl)-4-piperidinyl]oxy]acetamide

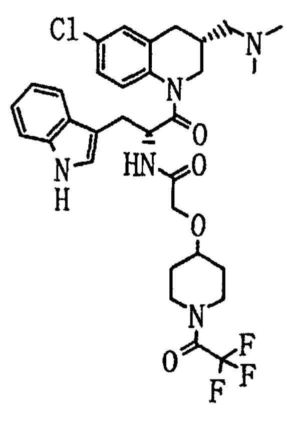

IR(KBr) :3310, 2941, 1680, 1180, 1140, 1103, 745 $cm^{-1}$.
MASS (FAB), m/z 654[$(M+H)^+$].

**Example 113**

N-[[4-[[[(1R)-2-[(3R)-6-chloro-3-[(dimethylamino)methyl]-1,2,3,4-tetrahydro-1-quinolinyl]-1-(1-indol-3-ylmethyl)-2-oxoethyl] amino]carbonyl]cyclohexyl]methyl]benzamide

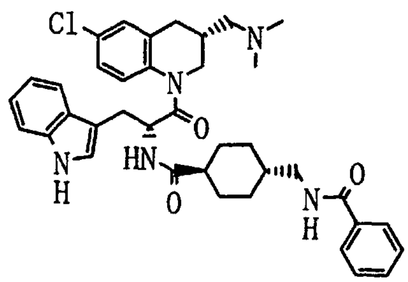

IR(KBr): 3304, 2928, 1640, 1487, 743 $cm^{-1}$.
MASS (FAB), m/z 654[$(M+H)^+$].

**Example 114**

1-benzoyl-N-[(1R)-2-[(3R)-6-chloro-3-(1-pyrrolidinylmethyl)-1,2,3,4-tetrahydro-1-quinolinyl]-1-(1H-indol-3-ylmethyl)-2-oxoethyl]-4-piperidinecarboxamide

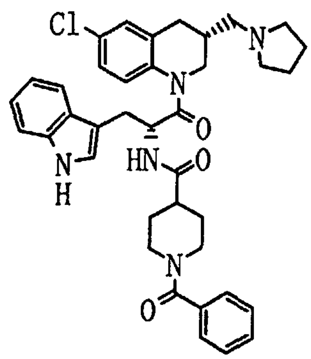

IR(KBr): 3291, 2926, 1628, 1437, 741 $cm^{-1}$.
MASS (FAB), m/z 652.2[$(M+H)^+$]

**Example 115**

3-(1-benzoyl-4-piperidinyl)-N-[(1R)-2-((3R)-6-chloro-3-(1-pyrrolidinylmethyl)-1,2,3,4-tetrahydro-1-quinolinyl)-1-(1H-indol-3-ylmethyl)-2-oxoethyl]propanamide

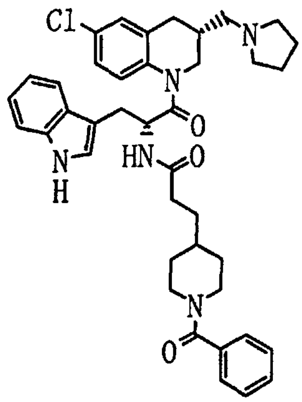

IR(KBr): 3293, 2926, 1632, 1445, 741 $cm^{-1}$.
MASS (FAB), m/z 692.9[$(M+H)^+$]

**Example 116**

N-[(1R)-2-((3R)-6-chloro-3-[(dimethylamino)methyl]-3,4-dihydro-1(2H)-quinolinyl)-1-(1H-indol-3-ylmethyl)-2-oxoethyl]-1-[(5-fluoro-1-methyl-1H-indol-2-yl)carbonyl]-4-piperidinecarboxamide

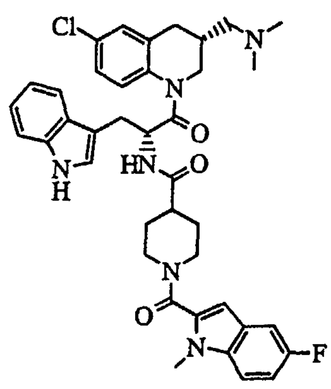

IR(KBr): 3304, 2942, 1628, 1192, 743 $cm^{-1}$.
MASS (FAB), m/z [$(M+H)^+$]

The following compounds mentioned in Examples 117-123 were synthesized according to the same method as Example 8.

**Example 117**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-1-(2,3-dihydro-1-inden-2-ylcarbonyl)-4-piperidinecarboxamide

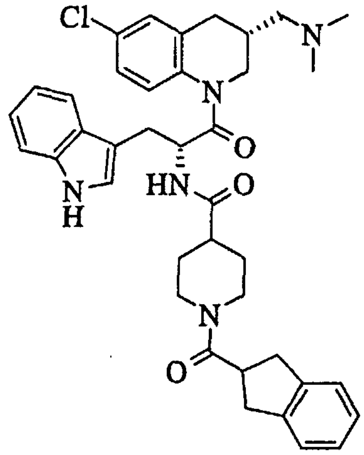

IR(KBr): 2923, 1629, 1487, 1204, 742.$cm^{-1}$.

**Example 118**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-1-(3-isoquinolylcarbonyl)-4-piperidinecarboxamide

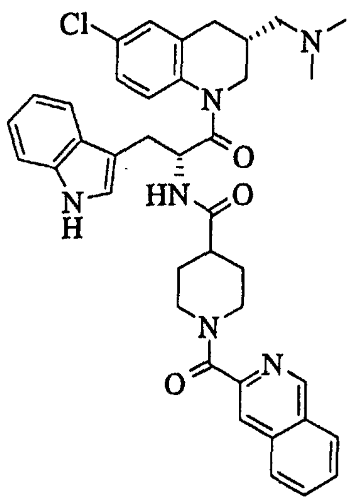

IR(KBr): 2923, 1624, 1488, 1092, 952, 743 $cm^{-1}$.

**Example 119**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-1-(1-indol-3-ylcarbonyl)-4-piperidinecarboxamide

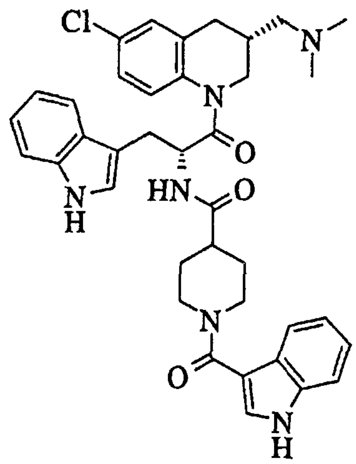

IR(KBr):3279, 1631, 1597, 1434, 1193, 1098, 745 $cm^{-1}$.

**Example 120**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-1-(phenylacetyl)-4-piperidinecarboxamide

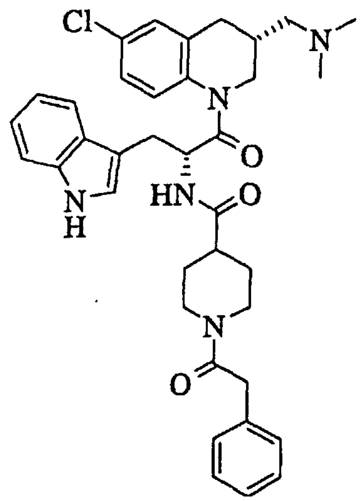

IR(KBr): 3274, 2932, 1634, 1488, 1099, 741 $cm^{-1}$.

**Example 121**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-1-[(1-methyl-1-indol-3-yl)carbonyl]-4-piperidinecarboxamide

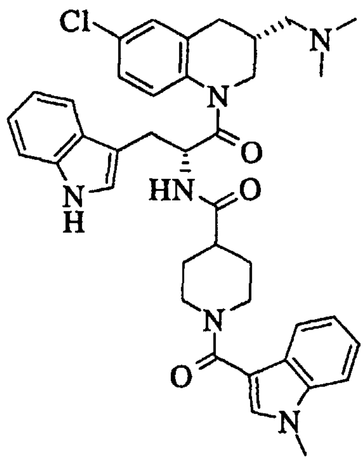

IR(KBr) :3294, 2933, 1636, 1488, 1231, 1097, 744.$cm^{-1}$.

**Example 122**

N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-4-[(1-indol-2-yl)carbonyl]-1-piperazinecarboxamide

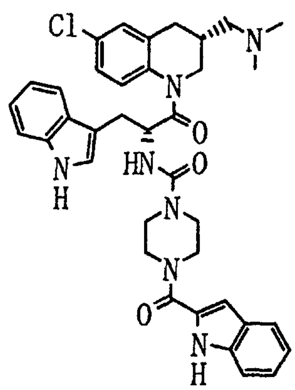

IR(KBr): 3283, 2938, 1628, 1250, 747 $cm^{-1}$.
MASS (FAB), m/z 666.4[$(M+H)^+$]

**Example 123**

N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-4-[(1-methyl-1-indol-2-yl)carbonyl]-1-piperazinecarboxamide

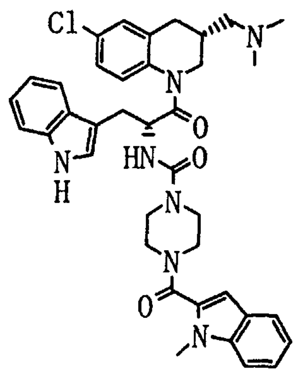

IR(KBr): 3293, 2938, 1628, 1227, 1007, 741 $cm^{-1}$.
MASS (FAB), m/z 680.4[$(M+H)^+$]

**Example 124**

2-{[1-(2-chlorobenzoyl)-4-piperidinyl]oxy}-N-[(1R)-2-((3R)-6-chloro-3-[(dimethylamino)methyl]-1,2,3,4-tetrahydro-1-quinolinyl)-1-(1-indol-3-ylmethyl)-2-oxoethyl]acetamide

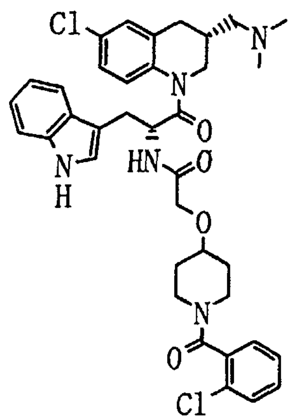

To a solution of N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-2-(4-piperidinyloxy)acetamide (300 mg) in acetonitrile (10 ml) was added p-chlorobenzoic acid (100 mg), WSC (120 mg) and HOBt (100 mg). The solution was stirred at room temperature for 16 hours. To the reaction solution was added a saturated aqueous solution of sodium hydrogencarbonate. The mixture was extracted with ethyl acetate. The ethyl acetate layer was washed with saturated brine, dried and concentrated. The residue was purified by alumina column chromatography (developing solvent; ethyl acetate/hexane = 1/1 - ethyl acetate/methanol = 20/1) and the title compound was obtained as amorphous powders (210 mg).

IR(KBr): 3291, 2940, 1653, 1001, 743 $cm^{-1}$.
MASS (FAB), m/z 690.2[$(M+H)^+$]

The following compounds mentioned in Examples 125-128 were synthesized according to the same method as Example 124.

**Example 125**

N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-2-[[1-(2-methylbenzoyl)-4-piperidinyl]oxy]acetamide

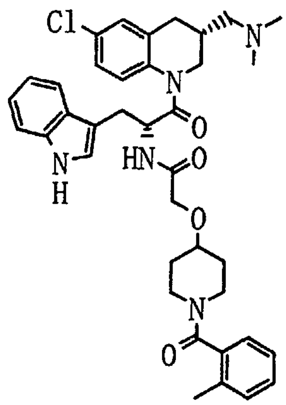

IR(KBr): 3275, 1638, 1096, 743 $cm^{-1}$.
MASS (FAB), m/z 670.3[$(M+H)^+$]

**Example 126**

N-[(1R)-2-((3R)-6-chloro-3-[(dimethylamino)methyl]1,2,3,4-tetrahydro-1-quinolinyl)-1-(1-indol-3-ylmethyl)-2-oxoethyl]-2-[[1-(4-fluorobenzoyl)-4-piperidinyl]oxy]acetamide

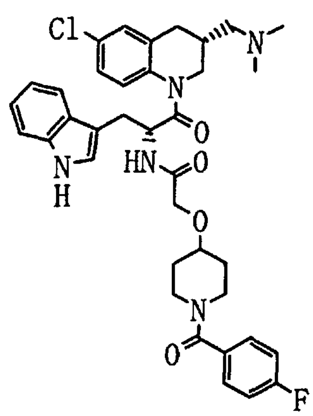

IR(KBr): 3291, 2942, 1640, 1100, 743 $cm^{-1}$.
MASS (FAB), m/z 674.2[$(M+H)^+$]

**Example 127**

N-[(1R)-2-((3R)-6-chloro-3-[(dimethylamino)methyl]1,2,3,4-tetrahydro-1-quinolinyl)-1-(1-indol-3-ylmethyl)-2-oxoethyl]-2-[[1-(4-methylbenzoyl)-4-piperidinyl]oxy]acetamide

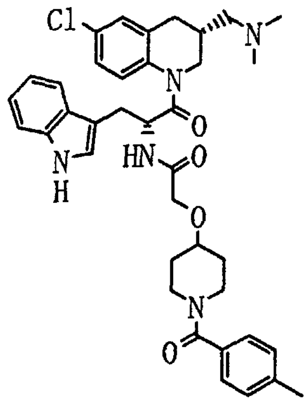

IR(KBr): 3289, 2928, 1638, 1437, 1100, 743 $cm^{-1}$.
MASS (FAB), m/z 670.2[$(M+H)^+$]

**Example 128**

N-[(1R)-2-((3R)-6-chloro-3-[(dimethylamino)methyl]1,2,3,4-tetrahydro-1-quinolinyl)-1-(1-indol-3-ylmethyl)-2-oxoethyl]-2-[[1-(phenylacetyl)-4-piperidinyl]oxy]acetamide

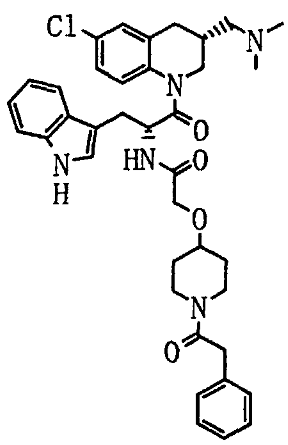

IR(KBr): 3291, 2942, 1638, 1437, 1101, 743 $cm^{-1}$.
MASS (FAB), m/z 670.2[$(M+H)^+$]

The following compounds mentioned in Examples 129-150 were synthesized according to the same method as Example 21.

**Example 129**

N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-(4-fluorobenzoyl)-1-piperidinecarboxamide

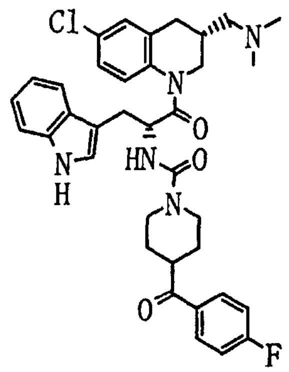

IR(KBr): 3275, 2944, 1636, 1487, 1231, 968, 743 $cm^{-1}$.
MASS (FAB), m/z 644[$(M+H)^+$].

**Example 130**

4-(4-chlorobenzoyl)-N-[(1R)-2-[(3 R)-6-chloro-3-[(dimethylamino)methyl]-1,2,3,4-tetrahydro-1-quinolinyl]-1-(1-indol-3-ylmethyl)-2-oxoethyl]-1-piperidinecarboxamide

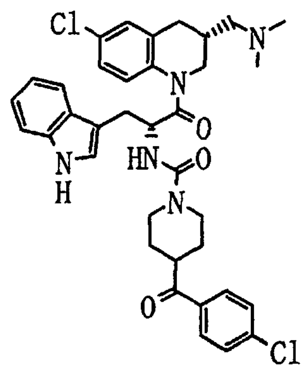

IR(KBr): 3270, 2944, 1636, 1287, 1092, 968, 741 $cm^{-1}$.
MASS (FAB), m/z 660[$(M+H)^+$].

**Example 131**

N-[(1R)-2-[(3 R)-6-chloro-3-[(dimethylamino)methyl]-1,2,3,4-tetrahydro-1-quinolinyl]-1-(1-indol-3-ylmethyl)-2-oxoethyl]-4-(2-fluorobenzylidene)-1-piperidinecarboxamide

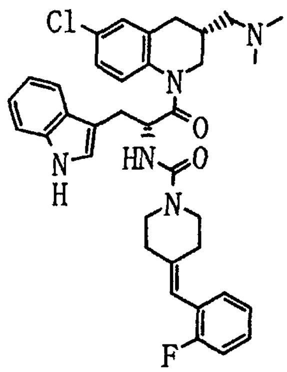

IR(KBr): 3274, 2940, 1485, 1229, 756, 743 $cm^{-1}$.
MASS (FAB), m/z 628 [$(M+H)^+$].

**Example 132**

4-[(benzyloxy)methyl]-N-[(1R)-2-[(3 R)-6-chloro-3-[(dimethylamino)methyl]-1,2,3,4-tetrahydro-1-quinolinyl]-1-(1-indol-3-ylmethyl)-2-oxoethyl]-1-piperidinecarboxamide

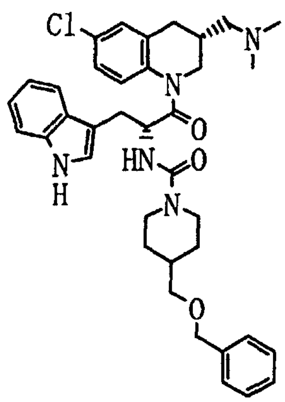

IR(KBr): 3274, 2938, 1632, 1487, 1100, 741 $cm^{-1}$.
MASS (FAB), m/z 642[$(M+H)^+$].

**Example 133**

4-benzylidene-N-[(1R)-2-[(3 R) -6-chloro-3-[(dimethylamino)methyl]-1,2,3,4-tetrahydro-1-quinolinyl]-1-(1-indol-3-ylmethyl)-2-oxoethyl]-1-piperidinecarboxamide

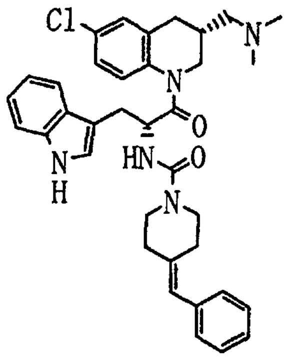

IR(KBr): 3274, 2940, 1632, 1487, 1229, 741 $cm^{-1}$.
MASS (FAB), m/z 610[$(M+H)^+$].

**Example 134**

4-benzyl-N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-1-piperidinecarboxamide

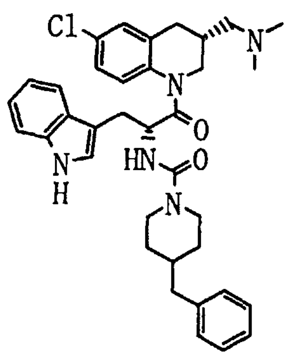

IR(KBr): 3268, 2928, 1628, 1485, 1233, 741 $cm^{-1}$.
MASS (FAB), m/z 612.3[$(M+H)^+$]

**Example 135**

N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-4-(4-methoxybenzyl)-1-piperidinecarboxamide

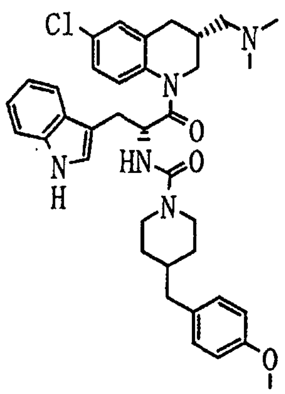

IR(KBr): 3256, 2913, 1624, 1508, 1244, 743 $cm^{-1}$.
MASS (FAB), m/z 642.2[$(M+H)^+$]

**Example 136**

N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-4- (2-fluorobenzyl)-1-piperidinecarboxamide

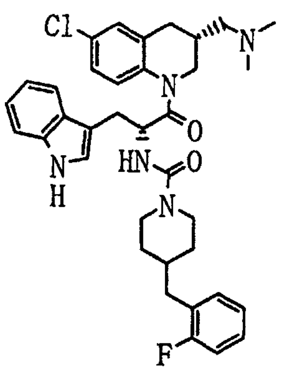

IR(KBr): 1638, 1491, 1229, 741 $cm^{-1}$.
MASS (FAB), m/z 630.3[$(M+H)^+$]

**Example 137**

N-[(1R)-2-((3R)-6-chloro-3-(1-pyrrolidinylmethyl)-1,2,3,4-tetrahydro-1-quinolinyl)-1-(1-indol-3-ylmethyl)-2-oxoethyl]-4-phenyll-1-piperazinecayboxamide

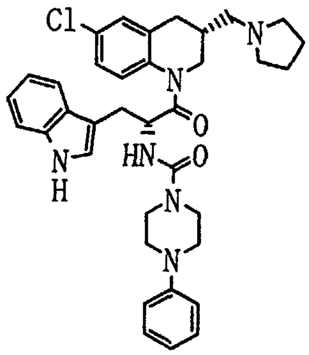

IR(KBr): 3268, 2911, 1630, 1485, 1231, 743 $cm^{-1}$.
MASS (FAB), m/z 625.2[$(M+H)^+$]

**Example 138**

4-benzyl-N-[(1R)-2-[(3R)-6-chloro-3-(1-pyrrolidinylmethyl)-1,2,3,4-tetrahydro-1-quinolinyl]-1-(1-indol-3-ylmethyl)-2-oxoethyl]-1-piperazinecarboxamide

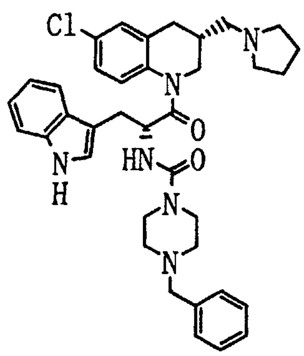

IR(KBr): 3285, 2928, 2801, 1630, 1485, 999, 741 $cm^{-1}$.
MASS (FAB), m/z 639.2[$(M+H)^+$]

**Example 139**

N-[(1R)-2-((3R)-6-chloro-3-(1-pyrrolidinylmethyl)-1,2,3,4-tetrahydro-1-quinolinyl)-1-(1-indol-3-ylmethyl)-2-oxoethyl]-4-(2-oxo-2,3-dihydro-lH-benzimidazol-1-yl)-1-piperidinecarboxamide

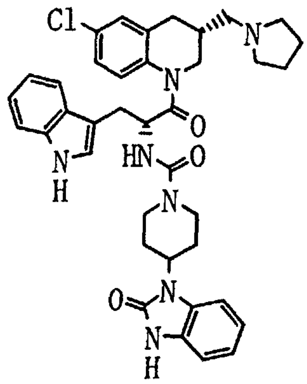

IR(KBr): 3247, 2930, 1692, 1483, 739 $cm^{-1}$.
MASS (FAB), m/z 680.2[$(M+H)^+$]

**Example 140**

N-[(1R)-2-[(3R)-6-chloro-3-[(dimethylamino)methyl]-1,2,3,4-tetrahydro-1-quinolinyl]-1-(1H-indol-3-ylmethyl) -2-oxoethyl] -4- (4-methoxybenzoyl)-1-piperidinecarboxamide

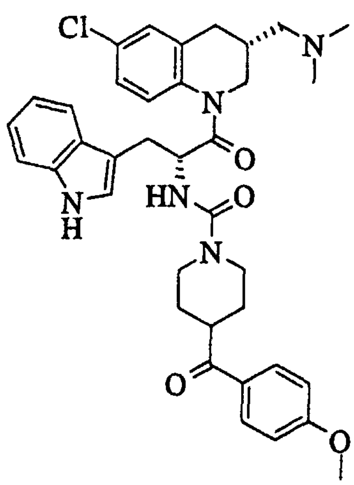

IR(KBr): 3316, 2942, 1634, 1258, 968, 743 $cm^{-1}$.
MASS (FAB),m/z656.3 [$(M+H)^+$]

**Example 141**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-(3,4-dimethoxybenzoyl)-1-piperidinecarboxamide

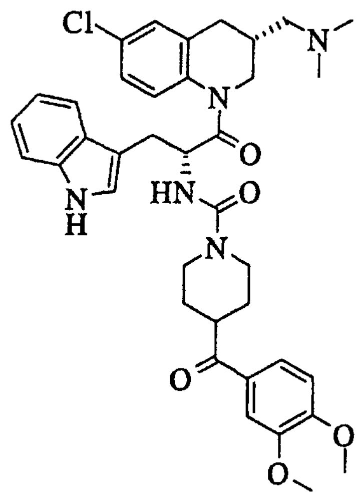

IR(KBr): 3310, 2938, 1632, 1514, 1265, 1161, 1022, 743 $cm^{-1}$.
MASS (FAB),m/z686.3 $[(M+H)^+]$

**Example 142**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-[(1-methyl-1-indol-2-yl)carbonyl]-1-piperidinecarboxamide

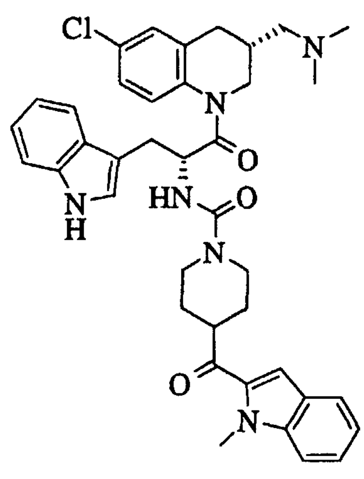

IR(KBr): 3293, 2944, 1632, 1485, 1184, 968, 741 $cm^{-1}$.
MASS (FAB), m/z679.3 $[(M+H)^+]$

**Example 143**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-(4-chlorophenoxy)-1-piperidinecarboxamide

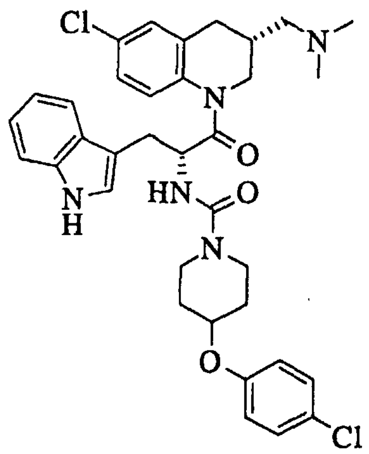

IR(KBr): 3274, 2942, 1632, 1487, 1235, 826, 743 $cm^{-1}$.

**Example 144**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-(4-methoxyphenoxy)-1-piperidinecarboxamide

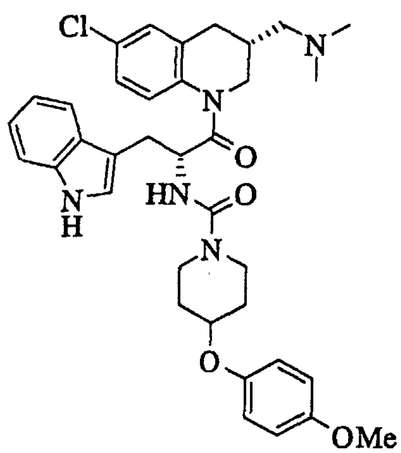

IR(KBr): 3274, 2944, 1632, 1505, 1227, 1038, 824, 745 $cm^{-1}$.

**Example 145-1**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-(4-cyanophenoxy)-1-piperidinecarboxamide

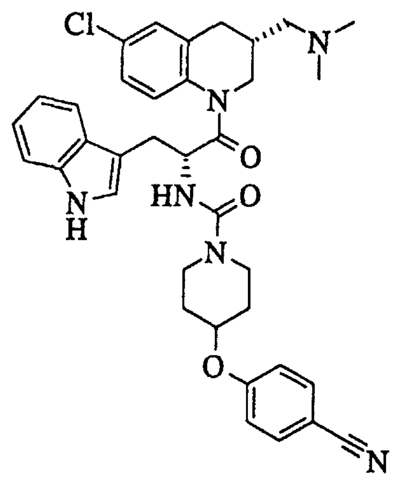

IR(KBr): 3306, 2944, 2222, 1634, 1505, 1254, 1034, 835, 743 $cm^{-1}$.

**Example 145-2**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-phenoxyl-1-piperidinecarboxamide

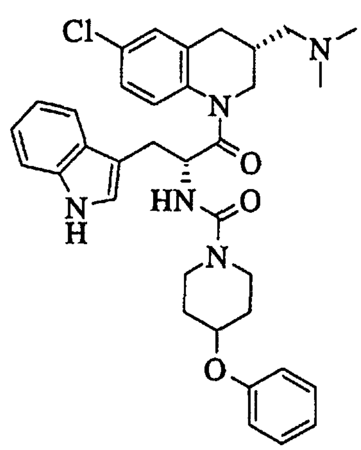

IR(KBr): 3275, 2942, 1632, 1487, 1229, 1042, 743, 693 $cm^{-1}$.

**Example 146**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-(4-fluorophenoxy)-1-piperidinecarboxamide

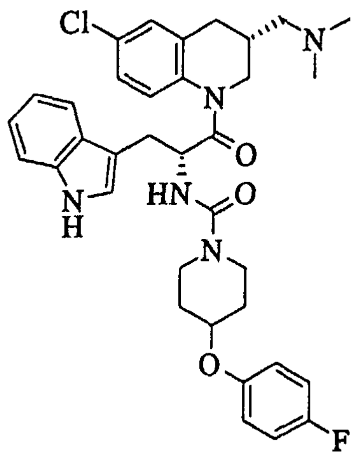

IR(KBr): 3295, 2942, 1632, 1503, 1206, 828, 762 $cm^{-1}$.

**Example 147**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-[(4-fluorophenyl)thio]-1-piperidinecarboxamide

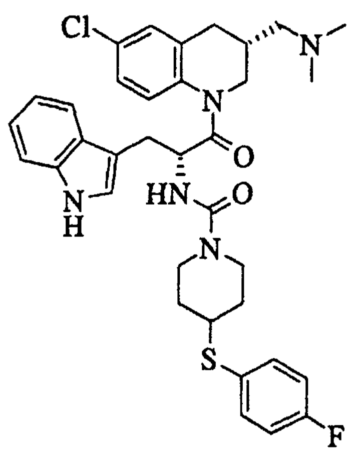

IR(KBr): 3277, 2971, 1632, 1487, 1233, 833, 743 $cm^{-1}$.
MASS (FAB),m/z648.2 $[(M+H)^{+}]$

**Example 148**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-[(4-fluorophenyl)sulfonyl]-1-piperidinecarboxamide

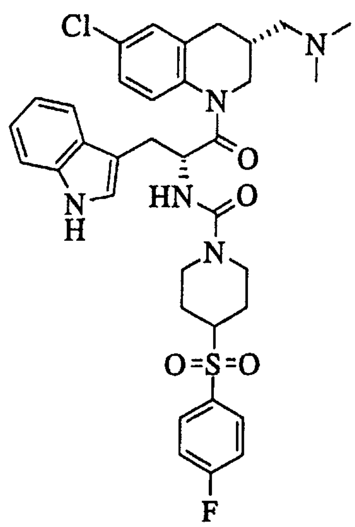

IR(KBr): 3293, 2936, 1630, 1489, 1230, 1144, 839, 743 $cm^{-1}$.
MASS (FAB),m/z680.2 $[(M+H)^+]$

**Example 149**

N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-[(4-chlorophenyl)thio]-1-piperidinecarboxamide

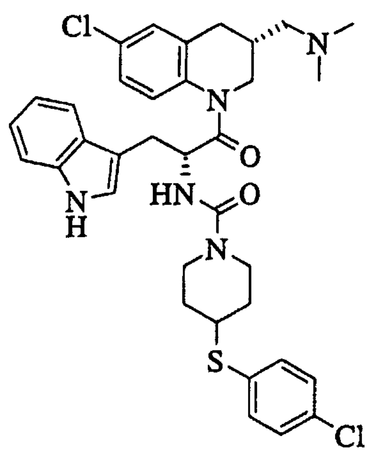

IR(KBr): 3291, 2942, 1632, 1487, 1096, 1011, 822, 743 $cm^{-1}$.

**Example 150**

N-[(1R)-2-[(3R)-6-chloro-3-[(dimethylamino)methyl]-1,2,3,4-tetrahydro-1-quinolinyl]-1-(1H-indol-3-ylmethyl)-

2-oxoethyl]-4-[(4-chlorophenyl)sulfonyl]-1-piperidinecarboxamide

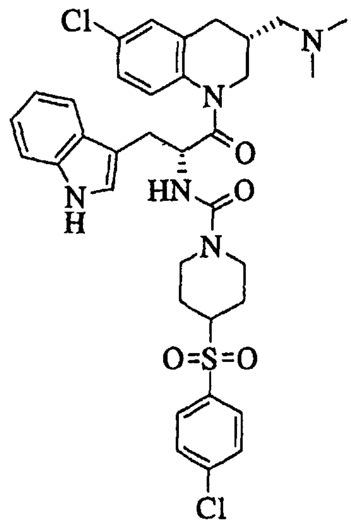

IR(KBr):3306, 2938, 1636, 1487, 1148, 1090, 752 $cm^{-1}$.

**Example 151**

N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-2-(4-piperidinyloxy)acetamide

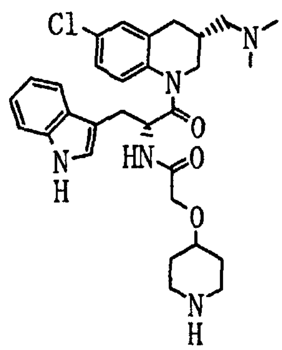

The title compound was obtained according to the same method as Example 28.
IR(KBr): 3399, 2940, 1642, 1487, 1101, 743 $cm^{-1}$.
MASS (FAB), m/z 552.3[$(M+H)^+$]

**Example 152**

N-[(1R)-2-((3R)-6-chloro-3-[(dimethylamino)methyl]-1,2,3,4-tetrahydro-1-quinolinyl)-1-(1-indol-3-ylmethyl)-2-oxoethyl]-3-[1-(2-hydroxy-2-methylpropanoyl-4-piperidinyl)propanamide

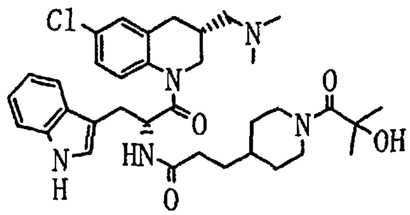

To a solution of 2-[4-(3-[(1R)-2-[(3R)-6-chloro-3-[(dimethylamino)methyl]-1,2,3,4-tetrahydro-1-quinolinyl]-1-(1-indol-3-ylmethyl)-2-oxoethyl]amino-3-oxopropyl)-1-piperidinyl]-1,1-dimethyl-2-oxoethyl acetate (80 mg) in ethanol (6 ml) was added 5N sodium hydroxide (0.1 ml). The mixture was stirred at room temperature for 16 hours. To the reaction solution was added water. The mixture was extracted with ethyl acetate. The organic layer was washed with saturated brine, dried and concentrated. The residue was purified by alumina chromatography (developing solvent: hexane/ethyl acetate = 1/1, ethyl acetate/methanol 10/1 - 5/1, methanol) and the title compound was obtained as amorphous powders (50 mg).

IR(KBr): 3275, 2934, 1576, 1437, 743 $cm^{-1}$.

MASS (FAB), m/z 636.3[$(M+H)^+$]

**Example 153**

N-[(1R)-2-(3R)-6-chloro-3-[(dimethylamino)methyl]-1,2,3,4-tetrahydro-1-quinolinyl]-1-(1-indol-3-ylmethyl)-2-oxoethyl]-1-piperazinecarboxamide

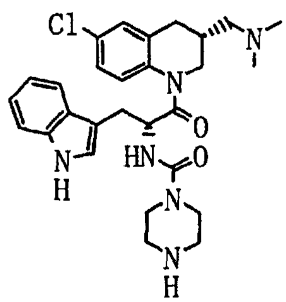

To a solution of 1-[2-(R)-amino-3-(indol-3-yl)propanoyl]-6-chloro-3-(R)-(N,N-dimethylamino)methyl-1,2,3,4-tetrahydroquinoline (300 mg) and N-ethyldiisopropylamine (0.14 ml) in acetonitrile (10 ml) was added N,N'-disuccinimidyl carbonate (200 mg). The mixture was stirred at room temperature for 30 minutes. Then, to the reaction solution was added a solution of 1-trifluoroacetylpiperazine (150 mg) and N-ethyldiisopropylamine (0.14 ml) in acetonitrile (5 ml). Furthermore, the mixture was stirred at room temperature for 48 hours. Then, to the reaction solution was added water. The mixture was extracted with ethyl acetate. The organic layer was washed with saturated brine, dried and concentrated. The residue was purified by alumina column chromatography (developing solvent; hexane/ethyl acetate = 4/1 - 1/1) and the title compound was obtained as amorphous powders (250 mg).

$^1$H-NMR ($CDCl_3$) δ: 1.93-2.17(4H, m), 2.13(6H, s), 2.8(4H, br m) , 3.0(1H, br m), 3.3(1H, br m), 3.4(4H, br m) , 3.5(1H, br m), 5.2(1H, br m), 5.6(2H, br m), 6.8-7.5(8H, br m), 7.9(1H, br s) .

MASS (FAB), m/z 523.2[$(M+H)^+$]

| Formulation Example 1 | |
|---|---|
| (1) Compound obtained in Example 1 | 50.0 mg |
| (2) Lactose | 34.0 mg |
| (3) Corn Starch | 10.6 mg |
| (4) Corn Starch (pasty) | 5.0 mg |
| (5) Magnesium Stearate | 0.4 mg |
| (6) Carboxymethyl Cellulose Calcium | 20.0 mg |
| Total | 120.0 mg |

The above (1) to (6) were admixed in an ordinary manner, and tabletted using a tabletting machine, to obtain tablets.

**Experimental Example 1**

The followings are some examples of the pharmacological actions of the compounds of the present invention, which should not be construed as being limiting to them. The gene manipulation using *E. coli* was conducted in accordance with the method described in the 1989 Edition of Molecular Cloning.

(1) Cloning of human somatostatin receptor protein subtype 4 (hSSTR4) DNA

DNA oligomers S4-1 and S4-2 were synthesized based on the known human SSTR4 DNA sequence (Rohrer etal., Proc. Natl, Acad. Sci., USA 90, 4196-4200, 1993). The sequence of S4-1 is 5'-GGCTCGAGTCACCATGAGCGCCCCCTCG-3' (Sequence No. 1) and that of S4-2 is 5'-GGGCTCGAGCTCCTCAGAAGGTGGTGG-3' (Sequence No. 2).

Human chromosomal DNA (Clonetech Inc. Catalog No. CL6550-1) was used as the template. To 0.5 ng of said DNA was added 25 pmol of each of the above-mentioned DNA oligomers and the polymerase chain reaction was carried out using 2.5 units of PfuDNA polymerase (Strata gene). The composition of the reaction mixture was in accordance with the directions attached to said Pfu DNA polymerase.

The conditions of the reaction were as follows: One cycle consisting of the reactions at 94°C for 1 minute, at 66°C for 1 minute and at 75°C for 2 minutes, and 35 cycles were repeated. The reaction mixture was subjected to electrophoresis on 1% agarose gel to find that the DNA fragments of the intended size (about 1.2 kb) were specifically amplified. Said DNA fragments were recovered from the agarose gel in the usual manner and ligated to pUC118 cleaved at the Hinc II site to transform into the competent cells, *Escherichia coli* JM109. The transformant having plasmid containing said DNA fragments was selected out and the sequence of the inserted DNA fragments was confirmed by the automatic sequence analyzer employing fluorochroming, ALF DNA Sequencer (Pharmacia). As the results, the amino acid sequence expected from the nucleotide sequence was completely in agreement with the sequence described in the above-mentioned reports by Rohrer et al.

(2) Construction of the expression plasmid of human somatostatin receptor protein subtype 4 (hSSTR4) DNA

pAKKO-111 was used as the expression vector in CHO (Chinese Hamster Ovary) cells. pAKKO-111 was constructed as follows: The 1.4 kb DNA fragment containing SR$\alpha$ promoter and poly A appositional signal was obtained from pTB1417 described in JP-A-H5(1993)-076385 by the treatment with a restriction enzyme (Hind III) and a restriction enzyme (Cla I). On the other hand, the 4.5 kb DNA fragment containing dihydrofolic acid reductase gene (dhfr) was obtained from pTB348 [Naruo, K. et al., Biochem. Biophys. Res. Commun., 128, 256-264, 1985] by the treatment with a restriction enzyme (Cla I) and a restriction enzyme (Sal I). These DNA fragments were treated with T4 polymerase to make the terminal blunt-ended and ligated with T4 ligase to construct pAKKO-111 plasmid.

Then, 5 $\mu$g of the plasmid having human SSTR4 DNA fragment was digested with a restriction enzyme (XhoI) and subjected to electrophoresis on 1% agarose gel to recover the 1.2 kb DNA fragment encoding human SSTR4. Next, 1 $\mu$g of the above-mentioned expression vector pAKKO-111 (5.5 kb) was digested with Sal I to prepare the cloning site for insertion of human SSTR4 DNA fragment. Said expression vector fragment and the 1.2 kb DNA fragment were ligated with T4DNA ligase. The reaction mixture was introduced into *E. coli* JM 109 by the calcium chloride method to obtain the expression plasmid pA1-11-hSSTR4 in which human SSTR4 DNA fragment was inserted in regular direction against the promoter from the transformants. This transformant is expressed as *Escherichia coli* JM109/pA-1-11-hSSTR4.

(3) Transfection and expression of human somatostatin receptor protein subtype 4 (hSSTR4) DNA in CHO (dhfr$^-$) cells

1 x $10^6$ CHO (dhfr$^-$) cells were cultured for 24 hours in HAM F12 medium containing 10% bovine fetal serum on a laboratory dish of 8 cm in diameter. The cells were transfected by 10 μg of the human SSTR4 DNA expression plasmid, pA-1-11-hSSTR4 obtained above using the calcium phosphate method (Cell Phect Transfection Kit; Pharmacia). The medium was switched to Dulbecco's Modified Eagle Medium (DMEM) containing 10% dialyzed bovine fetal serum 24 hours after the transfection to select the colony-forming cells (i.e. dhfr$^+$ cells) in this medium. Further, the selected cells were cloned from a single cell by the limiting dilution method and the somatostatin receptor protein expression activity of these cells was measured as follows: Human SSTR4 receptor expression cell strain was diluted with a buffer solution for assay [50 mM of Tris hydrochloride, 1 mM of EDTA, 5 mM of magnesium chloride, 0.1% of BSA, 0.2 mg/ml of bacitracin, 10 μg/ml of leupeptin, 1 μg/ml of pepstatin and 200 units/ml of aprotinin (pH 7.5)] to adjust the cell number to 2 x $10^4$/200 μl. 200 μl of the dilution was placed in a tube and to this was added 2 μl of 5 nM [$^{125}$I]-somatostatin-14 (2000 Ci/mmol, Amersham). The mixture was incubated at 25°C for 60 minutes. For measurement of non-specific binding (NSB), the tube to which 2 μl of somatostatin-14 ($10^{-4}$ M) was added was also incubated. To the tube was added 1.5 ml of a buffer solution for washing [50 mM of Tris-hydrochloride, 1 mM of EDTA and 5 mM of magnesium chloride (pH 7.5)] and the mixture was filtered by GF/F glass fiber filter paper (Whatman) and washed further with 1.5 ml of the same buffer solution. The amount of [$^{125}$I] of the filter was measured by a γ-counter. Thus, a highly somatostatin-binding cell strain, hSSTR4-1-2, was selected.

(4) Cloning of rat somatostatin receptor protein subtype 4 (rSSTR4) DNA

DNA oligomers S4-3 and S4-4 were synthesized based on the known rat SSTR4 DNA sequence (Bito.H etal., J. Biol. Chem., 269, 12722-12730, 1994).

The sequence of S4-3 is 5'-AAGCATGAACACGCCTGCAACTC-3' (Sequence No. 3) and that of S4-4 is 5'-GGTTTTCAGAAAGTAGTGGTCTT-3' (Sequence No. 4).

As the template, a chromosomal DNA prepared from Sprague-Dawley rats by using Easy-DNA™ KIT (Invitrogen) was used. To 0.5 ng of said DNA was added 25 pmol of each of the above-mentioned DNA oligomers and the polymerase chain reaction was carried out using TaKaRa LAPCR KIT (TaKaRa).

The conditions of the reaction were as follows: One cycle consisting of the reactions at 95°C for 30 seconds, at 65°C for 2 minutes and 30 seconds, and 30 cycles were repeated. The reaction mixture was subjected to electrophoresis on 1% agarose gel to find that the DNA fragments of the intended size (about 1.2 kb) were specifically amplified. Said DNA fragments were recovered from the agarose gel in the usual manner and ligated to a vector (pCR™ 2.1 (Trade name)) of ORIGINALTA CLONINGKIT (Invitrogen) to transform into the competent cells, *Escherichia coli* JM109. The transformant having plasmid containing said DNA fragments was selected out and the sequence of the inserted DNA fragments was confirmed by the automatic sequence analyzer employing fluorochroming, ALF DNA Sequencer (Pharmacia). As the results, the amino acid sequence expected from the nucleotide sequence was completely in agreement with the sequence described in the above-mentioned reports by Bito. H et al.

(5) Construction of the expression plasmid of rat somatostatin receptor protein subtype 4 (rSSTR4) DNA

pAKKO-111 was used as the expression vector in CHO cells. 5 μg of the plasmid having rat SSTR4 DNA fragment obtained above was digested with a restriction enzyme (EcoRI), treated with T4 DNA polymerase, and subjected to electrophoresis on 1% agarose gel to recover the 1.2 kb DNA fragment encoding rat SSTR4. Next, 1 μg of the above-mentioned expression vector pAKKO-111 (5.5 kb) was digested with a restriction enzyme (ClaI), treated with T4 DNA polymerase and Alikaline Phosphatase, to prepare the cloning site for insertion of rat SSTR4 DNA fragment. Said expression vector fragment and the 1.2 kb DNA fragment were ligated with T4 DNA ligase. The reaction mixture was introduced into *E. coli* JM 109 by the calcium chloride method to obtain the expression plasmid pA1-11-rSSTR4 in which rat SSTR4 DNA fragment was inserted in regular direction against the promoter from the transformants. This transformant is expressed as *Escherichia coli* JM109/pA-1-11-rSSTR4.

(6) Transfection and expression of rat somatostatin receptor protein subtype 4 (rSSTR4) DNA in CHO (dhfr$^-$) cells

1 x $10^6$ CHO (dhfr$^-$) cells were cultured for 24 hours in α-MEM medium (containing ribonucleoside and deoxynucleoside) containing 10% bovine fetal serum on a laboratory dish of 8 cm in diameter. The cells were transfected by 10 μg of the rat SSTR4 DNA expression plasmid 1 pA-1-11-rSSTR4 obtained above using the calcium phosphate method (Cell Phect Transfection Kit; Pharmacia). The medium was switched to α-MEM medium (free of ribonucleoside and deoxynucleoside) containing 10% dialyzed bovine fetal serum 24 hours after the transfection to select the colony-forming cells (i.e. dhfr$^+$ cells) in this medium. Further, the selected cells were cloned from a single cell by the limiting

dilution method and the somatostatin receptor protein expression activity of these cells was measured by the binding method mentioned above. Thus, a highly somatostatin-binding cell strain, rSSTR4-20-25, was selected.

(7) Preparation of CHO cell membrane fractions containing somatostatin receptor 4

Human and rat somatostatin receptor 4 expressing CHO cell strain, hSSTR4-1-2 or rSSTR4-20-25 (1 x $10^9$) was floated on a phosphate buffered saline supplemented with 5 mM EDTA (PBS-EDTA) and centrifuged. To the cell pellets was added 10 ml of a homogenate buffer for cells (10 mM $NaHCO_3$, 5 mM EDTA, pH 7.5), which was homogenated using a Politron homogenizer. The supernatant obtained by centrifugation at 400 x g for 15 minutes was further centrifuged at 10,000 μg for 1 hour to give a precipitate of the membrane fraction. The precipitates were suspended in 2 ml of a buffer solution for assay [25 mM of Tris-HCl, 1 mM of EDTA (Ethylenediaminetetraacetic Acid), 0.1% of BSA (Bovine Serum Albumin), 0.25 mM of PMSF (Phenylmethylsulfonyl Fluoride), 1 μg/ml pepstatin, 20 μg/ml leupeptin, 10 μg/ml Phosphoramidone, pH7.5], which was centrifuged at 100,000 x g for 1 hour. The membrane fraction recovered as precipitates was suspended again in 20 ml of the buffer solution for assay, which was placed in tubes and stored at -80°C. The suspension was thawed and used at every use.

**Experimental Example 2**

(1) Cloning of human somatostatin receptor protein subtype 1 (SSTR1) DNA

DNA oligomers S1-1 and S1-2 were synthesized based on the known human SSTR1 cDNA sequence (Proc. Natl. Acad. Sci., USA vol.89, p.251-255, 1992). The sequence of S1-1 is 5'-GGTCGACCTCAGCT AGGATGTTCCCCAATG-3' (Sequence No. 5) and that of S1-2 is 5'-GGTCGACCCGGGCTCAGAGCGTCGTGAT-3' (Sequence No. 6). Human chromosomal DNA (Clonetech Inc. Catalog No. CL 6550-1) was used as the template. To 0.5 ng of said DNA was added 25 pmol of each of the above-mentioned DNA oligomers and the polymerase chain reaction was carried out using 2.5 units of Pfu DNA polymerase (Stratagene). The composition of the reaction mixture was in accordance with the directions attached to said Pfu DNA polymerase. The conditions of the reaction were as follows: One cycle consisting of the reactions at 94°C for 1 minute, at 63°C for 1 minute and at 75°C for 2 minutes, and 35 cycles were repeated. The reaction mixture was subjected to electrophoresis on 1% agarose gel to find that the DNA fragments of the intended size (about 1.2 kb) were specifically amplified. Said DNA fragments were recovered from the agarose gel in the usual manner and ligated to pUC118 cleaved at the Hinc II site to transform into the competent cells, *Escherichia coli* JM109. The transformant having plasmid containing said DNA fragments was selected out and the sequence of the inserted DNA fragments was confirmed by the automatic sequence analyzer employing fluorochroming, ALF DNA Sequencer (Pharmacia). As the results, the amino acid sequence expected from the nucleotide sequence was completely in agreement with the sequence described in the above-mentioned literature.

(2) Construction of the expression plasmid of human somatostatin receptor protein subtype 1 (SSTR1) DNA

pAKKO-111 was used as the expression vector in CHO (Chinese Hamster Ovary) cells. PAKKO-111 was constructed as follows: The 1.4 kb DNA fragment containing SRα promoter and poly A appositional signal was obtained from pTB1417 described in JP-A-H5 (1993)-076385 by treatment with Hind III and Cla I. On the other hand, the 4.5 kb DNA fragment containing dihydrofolic acid reductase (DHFR) gene was obtained from pTB348 [Biochem. Biophys. Res. Commun., 128, p.256-264, 1985] by treatment with Cla I and Sal I. These DNA fragments were treated with T4 polymerase to make the terminal blunt-ended and ligated with T4 ligase to construct pAKKO-111 plasmid. Then, 5 μg of the plasmid having human SSTR1 DNA fragment obtained under the above (1) was digested with the restriction enzyme Sal I and subjected to electrophoresis on 1% agarose gel to recover the 1.2 kb DNA fragment encoding human SSTR1. Next, 1 μg of the above-mentioned expression vector pAKKO-111 (5.5 kb) was digested with Sal I to prepare the cloning site for insertion of human SSTR1 DNA fragment. Said expression vector fragment and the 1.2 kb DNA fragment were ligated with T4 DNA ligase. The reaction mixture was introduced into *E. coli* JM 109 by the calcium chloride method to obtain the expression plasmid pA1-11-SSTR1 in which human SSTR1 DNA fragment was inserted in regular direction against the promoter from the transformants. This transformant is expressed as *Escherichia coli* JM109/pA-1-11-SSTR1.

(3) Transfection and expression of human somatostatin receptor protein subtype 1 (SSTR1) DNA in CHO ($dhfr^-$) cells

1 x $10^6$ CHO ($dhfr^-$) cells were cultured for 24 hours in HAM F12 medium containing 10% bovine fetal serum on a laboratory dish of 8 cm in diameter. The cells were transfected by 10 μg of the human SSTR1 cDNA expression plasmid 1 pA-1-11-SSTR1, obtained under the above (2) using the calcium phosphate method (Cell Phect Transfection

Kit: Pharmacia). The medium was switched to DMEM medium containing 10% dialyzed bovine fetal serum 24 hours after the transfection to select the colony-forming cells (i.e. DHFR+ cells) in this medium. Further, the selected cells were cloned from a single cell by the limiting dilution method and the somatostatin protein activity was measured as follows: Human SSTR cDNA expression cell strain was diluted with a buffer solution for assay [50 mM of Tris hydrochloride, 1 mM of EDTA, 5 mM of magnesium chloride, 0.1% of BSA, 0.2 mg/ml of bacitracin, 10 μg/ml of leupeptin, 1 μg/ml of pepstatin and 200 units/ml of aprotinin (pH 7.5)] to adjust the cell number to 2 x $10^4$/200 μl. 200 μl of the dilution was placed in a tube and to this was added 2 μl of 5 nM [$^{125}$I]-somatostatin-14 (2000 Ci/mmol, Amersham). The mixture was incubated at 25°C for 60 minutes. For measurement of non-specific binding (NSB), the tube to which 2 μl of somatostatin-14 ($10^{-4}$ M) was added was also incubated. To the tube was added 1.5 ml of a buffer solution for washing [50 mM of Tris hydrochloride, 1 mM of EDTA and 5 mM of magnesium chloride (pH 7.5)] and the mixture was filtered by GF/F glass fiber filter paper (Whatman) and washed further with 1.5 ml of the same buffer solution. The amount of [$^{125}$I] of the filter was measured by a γ-counter. Thus, a highly somatostatin-binding cell strain, SSTR1-8-3, was selected.

(4) Cloning of human somatostatin receptor protein subtype 2 (SSTR2) DNA

DNA oligomers PT-1 and PT-2 were synthesized based on the known human SSTR2c DNA sequence (Proc. Natl. Acad. Sci., USA vol.89, p.251-255, 1992). The sequence of PT-1 is 5'-GGTCGACACCATGGACATGGCGGATGAG-3' (Sequence No. 7) and that of PT-2 is 5'-GGTCGACAGTTCAGATACTGGTTTGG-3' (Sequence No. 8). Human pituitary gland cDNA (Clonetech Inc. Catalog No. 7173-1) was used as the template. To 1 ng of said cDNA was added 25 pmol of each of the above-mentioned DNA oligomers and the polymerase chain reaction was carried out using 2.5 units of Taq DNA polymerase (TaKaRa Shuzo). The composition of the reaction mixture was in accordance with the directions attached to said Taq DNA polymerase. The conditions of the reaction were as follows: One cycle consisting of the reactions at 94°C for 30 seconds, at 52°C for 20 seconds and at 72°C for 60 seconds, and 30 cycles were repeated. The reaction mixture was subjected to electrophoresis on 1% agarose gel to find that the DNA fragments of the intended size (about 1.1 kb) was specifically amplified. Said DNA fragments were recovered from the agarose gel in the usual manner and ligated to pUC118 cleaved at the Hinc II site to transform into the competent cells, *Escherichia coli* JM109. Two strains (No. 5 and No. 7) of the transformant having plasmid containing said DNA fragments were selected out and the sequence of the inserted DNA fragments was confirmed by the automatic sequence analyzer employing fluorochroming, 373A DNA Sequencer (Applied Biosystem). As the results, point mutation was confirmed at one site between Sal I and Bst PI in the sequence of the 770 base fragment of No. 5 strain, and point mutation was also confirmed at one site between Bst PI and Sal I in the sequence of the 360 base fragment of No. 7 strain. Therefore, the fragments remaining after removing the Bst PI-Sal I fragment of No. 5 strain and the Bst PI-Sal I fragment of No. 7 strain were purified by electrophoresis on agarose to construct a plasmid in which these fragments were ligated by the ligation reaction. Confirmation of the insertion sequence of the DNA fragment of this plasmid revealed that it was completely in agreement with the sequence described in the above literature.

(5) Construction of the expression plasmid of human somatostatin receptor protein subtype 2 (SSTR2) DNA

pAKKO-111 mentioned under the above (2) was used as the expression vector in CHO (Chinese Hamster Ovary) cells. 5 μg of the plasmid having human SSTR2 cDNA fragment obtained under the above (4) was digested with the restriction enzyme Sal I and subjected to electrophoresis on 1% agarose gel to recover the 1.1 kb DNA fragment encoding human SSTR2. Next, 1 μg of the above-mentioned expression vector pAKKO-111 (5.5 kb) was digested with Sal I to prepare the cloning site for insertion of human SSTR2 DNA fragment. Said expression vector fragment and the 1.1 kb DNA fragment were ligated with T4 DNA ligase. The reaction mixture was introduced into *E. coli* JM 109 by the calcium chloride method to obtain the expression plasmid pAC01 in which human SSTR2 DNA fragment was inserted in regular direction against the promoter from the transformants. This transformant is expressed as *Escherichia coli* JM109/pAC-01.

(6) Transfection and expression of human somatostatin receptor protein subtype 2 (SSTR2) DNA in CHO ($dhfr^-$) cells

1 x $10^6$ CHO ($dhfr^-$) cells were cultured for 24 hours in HAM F12 medium containing 10% bovine fetal serum on a laboratory dish of 8 cm in diameter. To the cells was transfected 10 μg of the human SSTR2 cDNA expression plasmid, pA-C01, obtained under the above (5) by the calcium phosphate method (Cell Phect Transfection Kit: Pharmacia). The medium was switched to DMEM medium containing 10% dialyzed bovine fetal serum 24 hours after the transfection to select the colony-forming cells (i.e. $DHFR^+$ cells) in this medium. Further, the selected cells were cloned from a single cell by the limiting dilution method and a cell strain which highly expresses human SSTR2, SSTR2-HS5-9, was selected.

(7) Cloning of human somatostatin receptor protein subtype 3 (SSTR3) DNA

DNA oligomers S3-1 and S3-2 were synthesized based on the known human SSTR3c DNA sequence (Mol. Endocrinol., vol.6, p.2136-2142, 1992). The sequence of S3-1 is 5'-GGTCGACCTCAACCATGGACATGCTTCATC-3' (Sequence No. 9) and that of S3-2 is 5'-GGTCGACTTTCCCCAGGCCCCTACAGGTA-3' (Sequence No. 10). Human chromosomal DNA (Clone Tech Inc. Catalog No. CL6550-1) was used as the template. To 0.5 ng of said DNA was added 25 pmol of each of the above-mentioned DNA oligomers and the polymerase chain reaction was carried out using 2.5 units of Pfu DNA polymerase (Strata gene). The composition of the reaction mixture was in accordance with the directions attached to said Pfu DNA polymerase. The conditions of the reaction were as follows: One cycle consisting of the reactions at 94°C for 1 minute, at 63°C for 1 minute and at 75°C for 2 minutes, and 35 cycles were repeated. The reaction mixture was subjected to electrophoresis on 1% agarose gel to find that the DNA fragments of the intended size (about 1.3 kb) was specifically amplified. As the results, the amino acid sequence expected from the nucleotide sequence was completely in agreement with the sequence described in the above-mentioned literature.

(8) Construction of the expression plasmid of human somatostatin receptor protein subtype 3 (SSTR3) DNA

pAKKO-111 mentioned under the above (2) was used as the expression vector in CHO cells. 5 μg of the plasmid having human SSTR3 DNA fragment obtained under the above (7) was digested with the restriction enzyme Sal I and subjected to electrophoresis on 1% agarose gel to recover the 1.3 kb DNA fragment encoding human SSTR3. Next, 1 μg of the above-mentioned expression vector pAKKO-111 (5.5 kb) was digested with Sal I to prepare the cloning site for insertion of human SSTR3 DNA fragment. Said expression vector and the 1.3 kb DNA fragment were ligated with T4DNA ligase. The reaction mixture was introduced into *E. coli* JM 109 by the calcium chloride method to obtain the expression plasmid pA1-11-SSTR3 in which human SSTR3 DNA fragment was inserted in regular direction against the promoter from the transformants. This transformant is expressed as *Escherichia coli* JM109/pA-1-11-SSTR3.

(9) Transfection and expression of human somatostatin receptor protein subtype 3 (SSTR3) DNA in CHO ($dhfr^-$) cells

1 x $10^6$ CHO ($dhfr^-$) cells were cultured for 24 hours in HAM F12 medium containing 10% bovine fetal serum on a laboratory dish of 8 cm in diameter. The cells were transfected by 10 μg of the human SSTR3 DNA expression plasmid, pA-1-11-SSTR3, obtained under the above (5) using the calcium phosphate method. The medium was switched to DMEM medium containing 10% dialyzed bovine fetal serum 24 hours after the transfection to select the colony-forming cells (i.e. $DHFR^+$ cells) in this medium. Further, the selected cells were cloned from a single cell by the limiting dilution method and the somatostatin receptor protein expression activity of these cells was measured by the binding assay mentioned under the above (3). Thus, a highly somatostatin-binding cell strain, SSTR3-15-19, was selected.

(10) Cloning of human somatostatin receptor protein subtype (SSTR5) DNA

DNA oligomers S5-1 and S5-2 were synthesized based on the known human SSTR5c DNA sequence (Biochem Biophys. Res. Commun., vol.195, p.844-852, 1993). The sequence of S5-1 is 5'-GGTCGACCACCATGGAGCCCCTGTTCCC-3' (Sequence No. 11) and that of S5-2 is 5'-CCGTCGACACTCTCACAGCTTGCTGG-3' (Sequence No. 12). Human chromosomal DNA (Clonetech Inc. Catalog No. CL6550-1) was used as the template. To 0.5 ng of said DNA was added 25 pmol of each of the above-mentioned DNA oligomers and the polymerase chain reaction was carried out using 2.5 units of Pfu DNA polymerase (Stratagene). The composition of the reaction mixture was in accordance with the directions attached to PfuDNA polymerase. The conditions of the reaction were as follows: One cycle consisting of the reactions at 94°C for 1 minute, at 66°C for 1 minute and at 75°C for 2 minutes, and 35 cycles were repeated. The reaction mixture was subjected to electrophoresis on 1% agarose gel to find that the DNA fragments of the intended size (about 1.1 kb) were specifically amplified. Confirmation of the insertion sequence of said DNA fragment by the method mentioned under the above (1) revealed that the amino acid sequence expected from the nucleotide sequence was completely in agreement with the sequence described in the above-mentioned literature.

(11) Construction of the expression plasmid of human somatostatin receptor protein subtype 5 (SSTR5) DNA.

pAKKO-111 mentioned under the above (2) was used as the expression vector in CHO cells. 5 μg of the plasmid having human SSTR5 DNA fragment obtained under the above (10) was digested with the restriction enzyme Sal I and subjected to electrophoresis on 1% agarose gel to recover the 1.1 kb DNA fragment encoding human SSTR5. Next, 1 μg of the above-mentioned expression vector pAKKO-111 (5.5 kb) was digested with Sal I to prepare the cloning site for insertion of human SSTR5 DNA fragment. Said expression vector fragment and the 1.1 kb DNA fragment were

ligated with T4 DNA ligase. The reaction mixture was introduced into *E. coli* JM 109 by the calcium chloride method to obtain the expression plasmid pA1-11-SSTR5 in which human SSTR5 DNA fragment was inserted in regular direction against the promoter from the transformants. This transformant is expressed as *Escherichia coli* JM109/pA-1-11-SSTR5.

(12) Transfection and expression of human somatostatin receptor protein subtype 5 (SSTR5) DNA in CHO ($dhfr^-$) cells

1 x $10^6$ CHO ($dhfr^-$) cells were cultured for 24 hours in HAM F12 medium containing 10% bovine fetal serum on a laboratory dish of 8 cm in diameter. To the cells was transfected 10 μg of the human SSTR5 cDNA expression plasmid, pA-1-11-SSTR5, obtained under the above (11) by the calcium phosphate method. The medium was switched to DMEM medium containing 10% dialyzed bovine fetal serum 24 hours after the transfection to select the colony-forming cells (i.e. $DHFR^+$ cells) in this medium. Further, the selected cells were cloned from a single cell by the limiting dilution method and the somatostatin receptor protein expression activity of these cells was measured by binding assay mentioned under the above (3). Thus, a highly somatostatin-biding cell strain, SSTR5-32-4, was selected.

## Experimental Example 3

Measurement of the binding inhibition rate of $^{125}$I-Somatostatin

The receptor binding inhibition rate (%) of the subject compound was calculated using each of the membrane fractions prepared in Experimental Examples 1 and 2.

The membrane fraction was diluted with a buffer solution for assay to adjust the concentration to 3 μg/ml. The diluate was placed in tubes each in quantity of 173 μl. To this were simultaneously added 2 μl of a solution of a subject compound in DMSO and 25 μl of a 200 pM radioisotope-labeled somatostatin-14 ($^{125}$I-somatostatin-14: Amersham). For measurement of the maximum binding, a reaction mixture added with 2 μl of DMSO and 25 μl of a 200 pM $^{125}$I-somatostatin was prepared. For measurement of non-specific binding, a reaction mixture added with 2 μl of a 100 μM somatostatin solution in DMSO and 25 μl of a 200 pM $^{125}$I-somatostatin solution was prepared at the same time. The mixtures were allowed to react at 25°C for 60 minutes. Then, the reaction mixture was filtered by aspiration using a Whatman glass filter (GF-B) treated with polyethylenimine. After filtration, the radioactivity of $^{125}$I-somatostatin-14 remaining on the filter paper was measured by a γ-counter. The binding inhibition rate (%) of each subject compound was calculated by the following formula:

$$(TB-SB)/(TB-NSB) \times 100$$

SB: radioactivity when a compound was added
TB: maximum binding radioactivity
NSB: non-specific binding radioactivity

The binding inhibition rates were measured by changing the concentrations of the subject compound, and the 50% inhibiting concentration of the subject compound ($IC_{50}$ value) was calculated by the Hill plots.

| [Results] | | | |
|---|---|---|---|
| | $IC_{50}$ (nM) | | |
| Example No | SSTR2 | SSTR3 | SSTR5 |
| 14 | 0.6 | 70 | 300 |
| 31 | 2 | 60 | 300 |
| 51 | 0.3 | 80 | 400 |
| 130 | 2 | 40 | 400 |
| 145-2 | 1 | 10 | 200 |

This shows that the compound (I) of the present invention, salts thereof or prodrugs thereof have a binding inhibition effect on the human and rat somatostatin receptor.

## Industrial Applicability

Compound (I), (I') and (I'') of the present invention, salts thereof or prodrugs thereof have an excellent somatostatin receptor binding inhibition action with low toxicity. Therefore, compounds (I), (I') and (I'') of the present invention, salts thereof or prodrugs thereof are useful for disorders of an intracellular signal transduction system (e.g., diseases accompanied by excess sthenia or suppression, etc.); diseases accompanied by disorders of regulating cell proliferation; diseases accompanied by disorders of production and/or secretion of hormones, growth factors, or physiologically active substances, etc.; in a mammal.

## Claims

1. A compound of the formula:

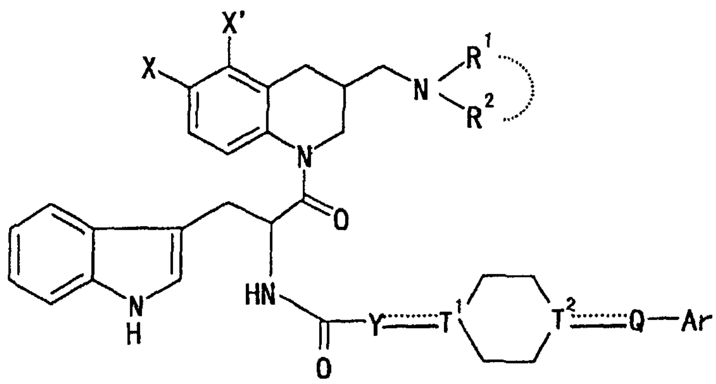

(I)

wherein X and X' are the same or different, and each represents a hydrogen atom, a fluorine atom, a chlorine atom or an amino optionally having substituents, and at least one of X and X' represents a fluorine atom, a chlorine atom or an amino optionally having substituents;

$R^1$ and $R^2$ represent a hydrogen atom or $C_{1-6}$ alkyl optionally having substituents, or $R^1$ and $R^2$, together with the adjacent nitrogen atom, form a nitrogen-containing heterocyclic ring optionally having substituents;
Y and Q are the same or different, and each represents a bond or a spacer having a main chain of 1 to 6 atoms;
... represents a single bond or a double bond;
$T^1$ and $T^2$ are the same or different, and each represents $C(R^9)$ ($R^9$ represents a hydrogen atom, a hydroxy or $C_{1-6}$ alkyl) or N, when each of the adjacent ... is a single bond, and C when the adjacent ... is a double bond; and
Ar represents an aromatic group optionally having substituents, a $C_{3-9}$ cycloalkyl group optionally having substituents, a 3 to 9-membered saturated heterocyclic group optionally having substituents, a hydrogen atom or a halogen atom; provided that 6-chloro-3-(R,S)-(N,N-dimethylamino)methyl-1-[3-(indol-3-yl)-2-[(R)-(4-phenylpiperazin-1-yl)carbonylamino]propanoyl]-1,2,3,4-tetrahydroquinoline; 6-chloro-3-(R,S)-(N,N-dimethylamino)methyl-1-[3-(indol-3-yl)-2-[(R)-4-(2-oxo-2,3-dihydro-lH-benzimidazol-1-yl)piperidinocarbonylamino]propanoyl]-1,2,3,4-tetrahydroquinoline and 1-benzoyl-N-[(R)-2-[6-chloro-3-[(N,N-dimethylamino)methyl]-1,2,3,4-tetrahydroquinolin-1-yl]-1-[3-(indol-3-yl)propanoyl]-4-piperidinecarboxamide are excluded; or a salt thereof.

2. A compound of the formula:

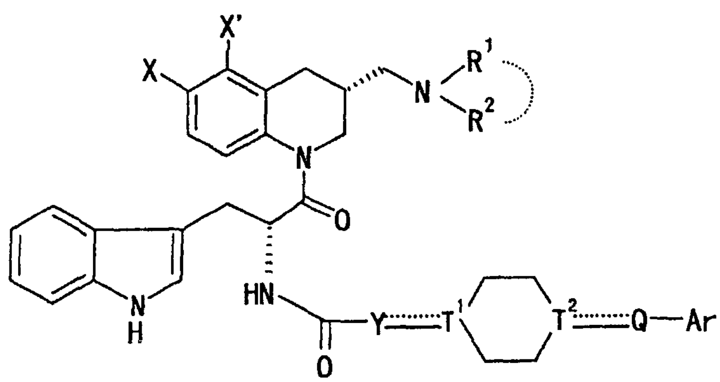

(I')

wherein X and X' are the same or different, and each represents a hydrogen atom, a fluorine atom, a chlorine atom or an amino optionally having substituents, and at least one of X and X' represents a fluorine atom, a chlorine atom or an amino optionally having substituents;

$R^1$ and $R^2$ represent a hydrogen atom or $C_{1-6}$ alkyl optionally having substituents, or $R^1$ and $R^2$, together with the adjacent nitrogen atom, form a nitrogen-containing heterocyclic ring optionally having substituents;
Y and Q are the same or different, and each represents a bond or a spacer having a main chain of 1 to 6 atoms;
... represents a single bond or a double bond;

$T^1$ and $T^2$ are the same or different, and each represents $C(R^9)$ ($R^9$ represents a hydrogen atom, a hydroxy or $C_{1-6}$ alkyl) or N, when each of the adjacent ... is a single bond, and C when the adjacent ... is a double bond; and

Ar represents an aromatic group optionally having substituents, a $C_{3-9}$ cycloalkyl group optionally having substituents, a 3 to 9-membered saturated heterocyclic group optionally having substituents, a hydrogen atom or a halogen atom; or a salt thereof.

**3.** The compound according to claim 1, wherein compound (I) is represented by the formula:

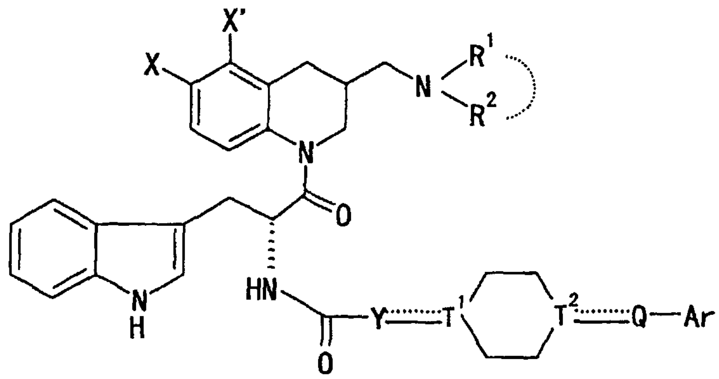

(I'')

wherein each symbol has the same meaning as in claim 1.

**4.** The compound according to any of claims 1 - 3, wherein X and X' are the same or different, and each represents a hydrogen atom, a fluorine atom or a chlorine atom, and at least one of X and X' represents a fluorine atom or a chlorine atom;

... represents a single bond;
$T^1$ and $T^2$ are the same or different, and each represents CH or N; and
Ar is an aromatic group optionally having substituents.

**5.** The compound according to any of claims 1 - 3, wherein X is a fluorine atom or a chlorine atom and X' is a hydrogen atom.

**6.** The compound according to any of claims 1 - 3, wherein X is a chlorine atom and X' is a hydrogen atom.

**7.** The compound according to any of claims 1 - 3, wherein $R^1$ and $R^2$ are each $C_{1-6}$ alkyl, or $R^1$ and $R^2$ form a 5- or 6-membered cyclic amino group together with the adjacent nitrogen atom.

**8.** The compound according to any of claims 1 - 3, wherein $R^1$ and $R^2$ are each $C_{1-6}$ alkyl.

**9.** The compound according to claim 1, wherein the spacer having a main chain of 1 to 6 atoms represented by Y and Q is a divalent group compring of 1 to 3 groups selected from -O-, -S-, -CO-, -SO-, $-SO_2-$, $-NR^8-$ ($R^8$ is a hydrogen atom, an optionally halogenated $C_{1-6}$ alkyl, an optionally halogenated $C_{1-6}$ alkyl-carbonyl, an optionally halogenated $C_{1-6}$ alkylsulfonyl) and an optionally halogenated divalent $C_{1-6}$ non-cyclic hydrocarbon group.

**10.** The compound according to any of claims 1 - 3, wherein Y is a bond, $C_{1-2}$ alkylene or $-CH_2O-$.

**11.** The compound according to any of claims 1 - 3, wherein Y is a bond or $C_{1-2}$ alkylene.

**12.** The compound according to any of claims 1 - 3, wherein Q is =CH-, $-CH_2-$, -O-, -S-, -CO-, $-SO_2-$, $-CO-CH_2-$, $-CH_2-NH-CO-$ or $-CH_2-O-CH_2-$.

**13.** The compound according to any of claims 1 - 3, wherein Q is -CO-.

**14.** The compound according to any of claims 1 - 3, wherein ... represents a single bond, $T^1$ is CH and $T^2$ is N.

**15.** The compound according to any of claims 1 - 3, wherein ... represents a single bond, $T^1$ is N and $T^2$ is CH.

**16.** The compound according to any of claims 1 - 3, wherein ... represents a single bond, $T^1$ is N and $T^2$ is N.

**17.** The compound according to any of claims 1 - 3, wherein Ar is a monocyclic aromatic group optionally having substituents.

**18.** The compound according to any of claims 1 - 3, wherein Ar is a fused aromatic group optionally having substituents.

**19.** The compound according to claim 17, wherein Ar is phenyl which may have 1 or 2 substituents selected from a halogen atom, a cyano, an optionally halogenated $C_{1-6}$ alkyl and an optionally halogenated $C_{1-6}$ alkoxy.

**20.** The compound according to claim 18, wherein Ar is indol-2-yl which may have 1 or 2 substituents selected from a halogen atom, an optionally halogenated $C_{1-6}$ alkyl and an optionally halogenated $C_{1-6}$ alkoxy.

**21.** The compound according to claim 18, wherein Ar is inden-2-yl, isoquinolyl or 2-oxo-2,3-dihydro-1H-benzimidazol-1-yl.

**22.** The compound according to claim 2, which is of the formula:

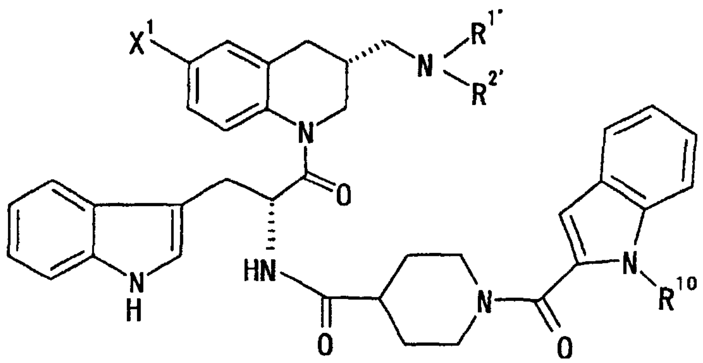

or

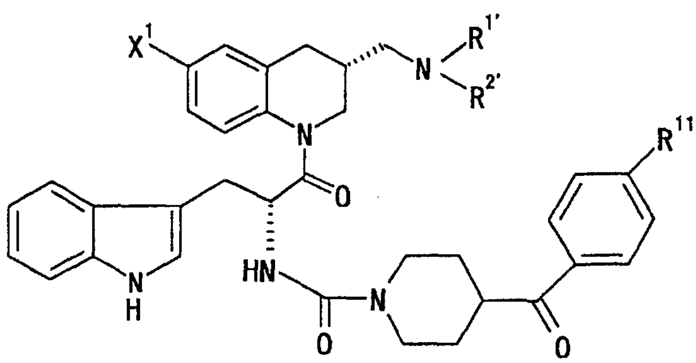

wherein $X^1$ represents a hydrogen atom, a fluorine atom, a chlorine atom or an amino optionally having substituents;

$R^{1'}$ and $R^{2'}$ each represent a hydrogen atom or a $C_{1-6}$ alkyl;
$R^{10}$ represents a $C_{1-6}$ alkyl; and
$R^{11}$ represents a halogen atom.

**23.** The compound according to claim 22, wherein $X^1$ represents a chlorine atom, $R^{1'}$ and $R^{2'}$ each represent a $C_{1-3}$

alkyl, $R^{10}$ represents a $C_{1-3}$ alkyl, and $R^{11}$ represents a halogen atom.

**24.** N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-1-(1-methylindol-2-ylcarbonyl)-4-piperidinecarboxamide, N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-1-(3-isoquinolylcarbonyl)-4-piperidinecarboxamide, N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-4-(4-fluorobenzoyl)-1-piperidinecarboxamide, 4- (4-chlorobenzoyl) -N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]-1-piperidinecarboxamide, N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-(4-chlorophenoxy)-1-piperidinecarboxamide, N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-phenoxy-1-piperidinecarboxamide, N-[(1R) -2-[(3R)-6-chloro-3-[(dimethylamino)methyl]-1,2,3,4-tetrahydro-1-quinolinyl]-1-(1H-indol-3-ylmethyl)-2-oxoethyl]-4-[(4-fluorophenyl)sulfonyl]-1-piperidinecarboxamide, N-[(1R)-2-[(3R)-6-chloro-3-[(dimethylamino)methyl]-1,2,3,4-tetrahydro-1-quinolinyl]-1-(1H-indol-3-ylmethyl)-2-oxoethyl]-4-[(4-chlorophenyl)sulfonyl]-1-piperidinecarboxamide, 3-(1-benzoyl-4-piperidinyl)-N-[(1R)-2-[(3R)-6-chloro-3-[(dimethylamino)methyl]-1,2,3,4-tetrahydro-1-quinolinyl]-1-(1-indol-3-ylmethyl)-2-oxoethyl]propanamide, 2-[(1-benzoyl-4-piperidinyl)oxy]-N-[(1R)-2-[(3R)-6-chloro-3-[dimethylamino]methyl]-1,2,3,4-tetrahydro-1-quinolinyl]-1-(1-indol-3-ylmethyl)-2-oxoethyl]acetamide, or a salt thereof.

**25.** A prodrug of the compound according to any of claims 1 - 3.

**26.** A method for producing a compound of claim 1 or a salt thereof, which comprises reacting a compound of the formula:

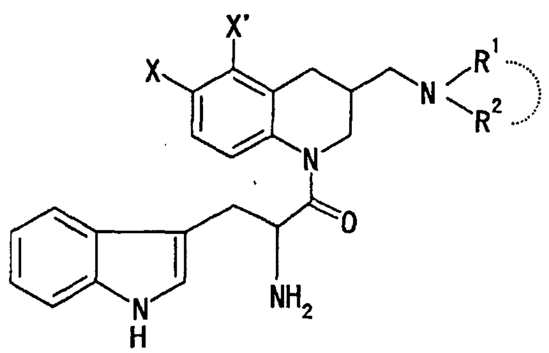

(IV)

wherein each symbol has the same meaning as in claim 1, or a salt thereof, and a compound of the formula:

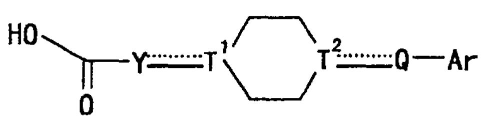

wherein each symbol has the same meaning as in claim 1, or a salt thereof.

**27.** A method for producing a compound of the formula:

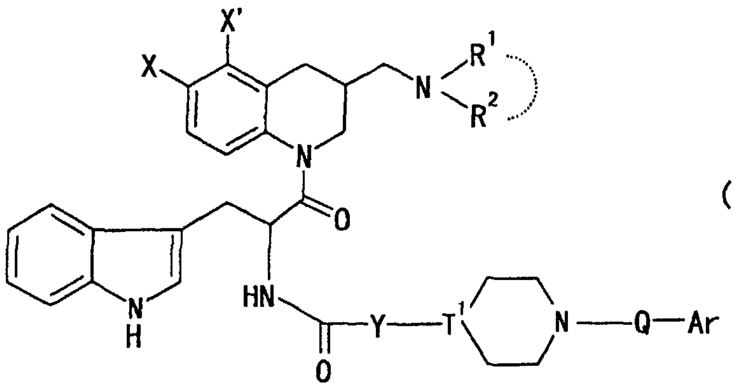

(Ia)

wherein each symbol has the same meaning as in claim 1, or a salt thereof, which comprises reacting a compound of the formula:

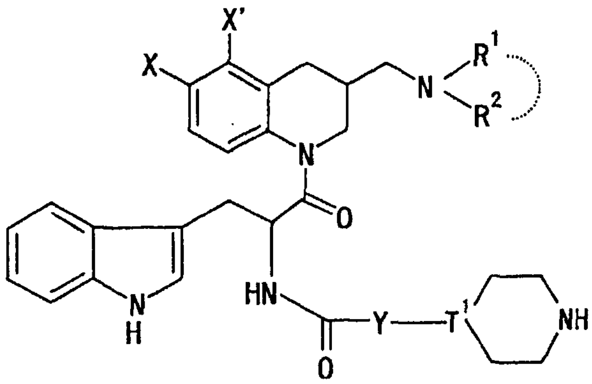

(VII)

wherein each symbol has the same meaning as above, or a salt thereof, and a compound of the formula:

$L^1$-Q-Ar

wherein $L^1$ is a leaving group, and other symbols have the same meanings as in claim 1, or a salt thereof.

**28.** A pharmaceutical composition comprising the compound according to any of claims 1 - 3, a salt thereof or a prodrug thereof.

**29.** The composition according to claim 28, which is a somatostatin receptor binding inhibitor.

**30.** The composition according to claim 29, which is a somatostatin subtype 2 receptor binding inhibitor.

**31.** The composition according to claim 28, which is a somatostatin receptor agonist.

**32.** The composition according to claim 31, which is a somatostatin subtype 2 receptor agonist.

**33.** The composition according to claim 28, which is a prophylactic or therapeutic agent for diabetes or diabetic nephropathy.

**34.** The composition according to claim 28, which is a prophylactic or therapeutic agent for tumors such as acromegaly, TSH-producing tumors, nonsecretory (afunctional) hypophysial tumors, ectopic ACTH (adrenocorticotrophic hormone)-producing tumors, medullar thyroid carcinoma, VIP-producing tumors, glucagon-producing tumors, gastrin-producing tumors, insulinoma and carotinoid.

**35.** The composition according to claim 28, which is a prophylactic or therapeutic agent for diarrhea due to neuroendocrine tumors, or diarrhea due to AIDS.

**36.** A method for inhibiting somatostatin receptor binding, which comprises administering to a mammal an effective amount of the compound according to any of claims 1 - 3, a salt thereof or a prodrug thereof.

**37.** Use of the compound according to any of claims 1 - 3, a salt thereof or a prodrug thereof for manufacturing a somatostatin receptor binding inhibitor.

**38.** A compound of the formula:

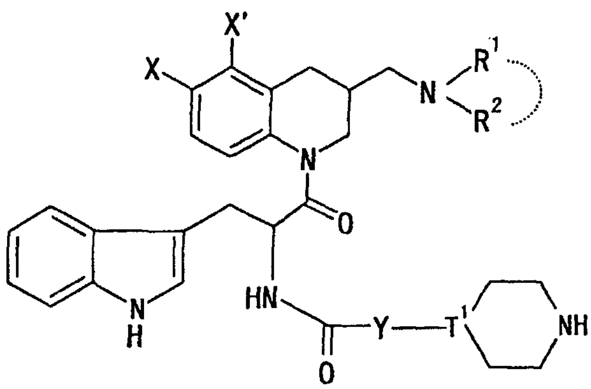

(VII)

wherein each symbol has the same meaning as in claim 1, or a salt thereof.

## INTERNATIONAL SEARCH REPORT

International application No.
PCT/JP00/06937

A. CLASSIFICATION OF SUBJECT MATTER
Int.Cl[7] C07D401/14, 405/14, 409/14, A61K31/4709, 496, 497, A61P43/00, 1/00, 3/10, 13/12, 35/00

According to International Patent Classification (IPC) or to both national classification and IPC

B. FIELDS SEARCHED

Minimum documentation searched (classification system followed by classification symbols)
Int.Cl[7] C07D401/14, 405/14, 409/14, A61K31/4709, 496, 497, A61P43/00, 1/00, 3/10, 13/12, 35/00

Documentation searched other than minimum documentation to the extent that such documents are included in the fields searched

Electronic data base consulted during the international search (name of data base and, where practicable, search terms used)
CAPLUS, REGISTRY (STN)

C. DOCUMENTS CONSIDERED TO BE RELEVANT

| Category* | Citation of document, with indication, where appropriate, of the relevant passages | Relevant to claim No. |
|---|---|---|
| PX | WO, 99/52875, A1 (TAKDA CHEMICAL INDUSTRIES, LTD.), 21 October, 1999 (21.10.99), & AU, 9952655, A & JP, 2000-226373, A | 1-35,37,38 |
| A | WO, 95/14666, A1 (MERCK & CO.INC.), 01 June, 1995 (01.06.95) & AU, 9512945, A & EP, 730578, A1 & JP, 9-505601, A | 1-35,37,38 |
| A | WO, 96/38471, A1 (PFIZER INC.), 05 December, 1996 (05.12.96) & NO, 9602162, A & AU, 9654554, A & FI, 9704368, A & EP, 828754, A1 & JP, 10-510511, A & US, 5936089, A | 1-35,37,38 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 November, 2000 (28.11.00) | 12 December, 2000 (12.12.00) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP00/06937

**Box I Observations where certain claims were found unsearchable (Continuation of item 1 of first sheet)**

This international search report has not been established in respect of certain claims under Article 17(2)(a) for the following reasons:

1. ☒ Claims Nos.: 36
because they relate to subject matter not required to be searched by this Authority, namely:

The invention as set forth in claim 36 pertains to methods for treatment of the human body.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

SEQUENCE LISTING

<110> Takeda Chemical Industries, Ltd.

<120> Amine Derivatives

<130> Case2654

<150> JP 11-286939

<151> 1999-10-07

<150> JP 2000-215837

<151> 2000-07-11

<160> 12

<210> 1

<211> 28

<212> DNA

<213> Artificial Sequence

<220>

<223>

<400> 1

GGCTCGAGTC ACCATGAGCG CCCCCTCG 28

<210> 2

<211> 27

<212> DNA

<213> Artificial Sequence

<220>

<223>

<400> 2

GGGCTCGAGC TCCTCAGAAG GTGGTGG 27

<210> 3

<211> 23

<212> DNA

<213> Artificial Sequence

<220>

<223>

<400> 3

AAGCATGAAC ACGCCTGCAA CTC 23

<210> 4

<211> 23

<212> DNA

<213> Artificial Sequence

<220>

<223>

<400> 4

GGTTTTCAGA AAGTAGTGGT CTT 23

<210> 5

<211> 30

<212> DNA

<213> Artificial Sequence

<220>

<223>

<400> 5

GGTCGACCTC AGCTAGGATG TTCCCCAATG 30

<210> 6

<211> 28

<212> DNA

<213> Artificial Sequence

<220>

<223>

<400> 6

GGTCGACCCG GGCTCAGAGC GTCGTGAT 28

<210> 7

<211> 28

<212> DNA

<213> Artificial Sequence

<220>

<223>

<400> 7

GGTCGACACC ATGGACATGG CGGATGAG 28

<210> 8

<211> 26

<212> DNA

<213> Artificial Sequence

<220>

<223>

<400> 8

GGTCGACAGT TCAGATACTG GTTTGG 26

<210> 9

<211> 30

<212> DNA

<213> Artificial Sequence

<220>

<223>

<400> 9

GGTCGACCTC AACCATGGAC ATGCTTCATC 30

<210> 10

<211> 29

<212> DNA

<213> Artificial Sequence

<220>

<223>

<400> 10

GGTCGACTTT CCCCAGGCCC CTACAGGTA 29

<210> 11

<211> 28

<212> DNA

<213> Artificial Sequence

<220>

<223>

<400> 11

GGTCGACCAC CATGGAGCCC CTGTTCCC 28

<210> 12

<211> 26

<212> DNA

<213> Artificial Sequence

<220>

<223>

<400> 12

CCGTCGACAC TCTCACAGCT TGCTGG 26